# EUROPEAN PATENT APPLICATION

(11) **EP 2 813 571 A1**
(43) Date of publication of application: **17.12.2014**
(21) Application number: 13382224.7
(22) Date of filing: 13.06.2013
(51) Int. Cl.: C12N 15/113, A61K 31/195, C12Q 1/68

(54) **Method for the treatment of lung cancer**

(71) Applicant: Institut d'Investigació Biomèdica de Bellvitge (IDIBELL), 08908 Hospitalet de Llobregat (ES)
(72) Inventor: Esteller Badosa, Manel, E-08908 Hospitalet de Llobregat - Barcelona (ES)
(74) Representative: ABG Patentes, S.L.

(57) **Abstract**

The invention relates to the use of the amplification of SETDB1 gene in lung cancer patients as predictive marker for the determination of the response to a SETDB1 antagonist, particularly to a SETDB1-specific RNAi or to mithramycin. The invention relates to methods of treatment, methods for predicting the clinical response to a SETDB1 antagonist and methods for selecting a lung cancer patient for being treated with a SETDB1 antagonist based on the detection of SETDB1 gene amplification.

SEQUENCE LISTING

<110>  Institut d'Investigació Biomèdica de Bellvitge (IDIBELL)

<120>  METHOD FOR THE TREATMENT OF LUNG CANCER

<130>  P9741EP00

<160>  1

<170>  PatentIn version 3.5

<210>  1
<211>  156757
<212>  DNA
<213>  Artificial Sequence

<220>
<223>  RP11-42A12 BAC


<220>
<221>  misc_feature
<222>  (451)..(451)
<223>  n is a, c, g, or t

<400>  1
tagacaagga gtggtgaatt ttcatccaaa aatctttggc ggattccctt gctctcccgc      60

tccccagtct aacagtagtt taaaacagaa gatcagcaac atttggagag tttttttttt     120

ctctgcattc tgatgataaa cttgatttat tcaattccaa aaatttcttt attctgaggt     180

tgtctttcta tattttgagt ataaaaccat ggccatacaa tgttggaccc accaaggttc     240

ttctcttcac ttgtttattt gtacagtctc tttgtaatct cagaatttct tgagtaaact     300

gggaactgtt atacataatt ctgtgttttt gagcattctt tgtaaacagg aatcaggatt     360

gggggtgggg aaggagggag gagtgggagt gctgatttgt gtaacggaaa aatcatagat     420

tacttatgtg atggggcaaa ggttttaaaa nactcttagt gtcctcagat ttccccttat     480

actatttgct gaagaaaatc atgtccttga ctcccttcct taccctctcc tggcaaggga     540

aggggtcccc tcctgcctca gggtctctgt cctcctgggg ttaggagaag cgcatccaac     600

tccctctcca agtgctggag aggaggctaa gtctttccca tgggctgggg gctttgctgt     660

cccaaggacg gctggggagg aagaggaggc agaggcggaa gaggaggcag aggcggaaga     720

ggaagccggc tgagatttgt gaagcagctc ttgctaccgc gggtaggaga gggcgcgcgg     780

gacccaagag cctccgtgcg gagccaggcc tcaggagcag ccgccttggt cctggaaggg     840

atgcctccct ccatcctgtc tccaaagaat gtagggagac cttctcaccc ggtgggaaag     900

cgacgtctcc catgctttcc caccacggac gccttatgtc ccttttcctg ctagtgtctg     960

accgccagcc tgcgggggct gcggctgcaa cggttccaag ctgggtggag gctgcctgcc    1020

cgcgagccaa gagccacgct aggaggcagg agtcgggaag tcgtgtgcag gcccggagga    1080

gactcaagct acgggtcagg cttttttgcc aattgccaaa ttcagcgtgt aaaacagaga 1140

gctcacatct gtctgtttgt aagcttcggt gagaacaaca aggaaggccc tcaagatccc 1200

taggagggcg gcgaagggtc tctggcgaag ggtcaccgcg tcggcggtga gccaaggtat 1260

gtcaggagag agtgcgaacc cagcccccgg gtagcacgaa caccaccgca gacaagggcc 1320

ccgcttgtgg ggaaatcgca gggccagtgc agccgcagtg cggtggacta gcctcatcca 1380

gggagaaccc ccttcctgac cacggtctgt cccccgccag gtaagaggag aggagggaca 1440

gtacagcctc ctgtcccgta gccgcaggct cagggtcacc accttcccct ctgcttggcc 1500

ggcgccattc ttccaaacca ctctccgcca aagattccac ggacagtcac ctacaagaca 1560

acccagccgt cctttcagca gcggatcccg ccccgcagcc accgcgccct ctcaccccag 1620

cgcttctacc caccgcctct gcccagtctg tgcacttcac ctccctcgcc ccggctcccc 1680

tctgagctta cagtggacag ggggttcgtc cccaaccctt cttgggaata ctgaatagaa 1740

aagggaggag cgtgcgcaat tccttggtag tgtgtagacg gacattgtgg gatttgactg 1800

tggtaccatc gctttgacgt cctagtgatt tttacacctg ccttctgctt agaacaccgg 1860

ccacagtttt ccatttgtgt ctactccacc tgctgtcttt gttggataag tgaacatcgt 1920

ccccctctac cactcaatca acaaaagggc ggtcctggag gacctcaccc gccgctcacc 1980

tgctgctcac ccccctccca accttgcgga catcgcctcc ggtcgcctct tcgtaaggcc 2040

taagaagcac gttagctgcg aacggaggtg aggaggctca gctgaccgcc tgtgtcagct 2100

gacaacgtgt gacacgcaca aacgccagga cttggcttgg cctctctctt agttatttgc 2160

agctctgccc aatcggcgcc tccgggacgg cggagacggt gggcttcttg ggtgcagctc 2220

cacgaaggct ggcatccctg cacagcggtg aacacctgag ggagacgctc agtctctctc 2280

taaagccact tctgcggatg acacggagat aaataagagc agtgtgtcat gagaggccgt 2340

ccaccaggac ttgccctcct ttgccagggt ttgcacctag cagagagact gttctgcctc 2400

tggcccttgg agcaggctgg ctgacagcgg agtaaagaaa aattactgcg ggtgtgcagt 2460

cagtgcaaaa caattctctg accgataatt gaacccggga tgcggtggtg aaagagctga 2520

atcatagcca ctagaccagc acgggggcac gggagggtct ttctcaacct tcttgccata 2580

taagtgactg tttccctgtg cgctgggagg acttgggcct tgtgggggtc gttggtcgct 2640

cctggagtcg tctcacaagg cctttccctc cctcctttct ccaaaagagg agcccggagg 2700

cgacgcaccc agtactctgc gaggcccacc aaggccacga gtcccgagtc ctggagcgag 2760

ttgcagcgtc caggctgcac ctgaccactg acctagaaat gtgcctttgc gaagcggcag 2820

cgcatggaga aacagtcgct ggtggccagg gccagagtcg cgcaccgggt agccagaaca 2880

ggcaggagcg aggaggctcc cggggtgaga acgggcaccc ccgcacatca taaaggactc 2940

agaccttggc aaccccgaca tcataaagga ctcacacgga tgtggaaacc gagacgagct 3000

ggatggaaaa ctttccaggg gggctccggg gccctcaact gctctctgac cttcccctgc 3060

aacctgcgac acctgccatt ttcccatctt aggcgacggc aacgctaccc ttccttatgc 3120

tccaggcaaa actcctagag ttccctctga cgctggagtt ttttcctcaa atccaaaatc 3180

caattggtca ccaattcgtg atttcccgtc ggccaagtgc gtgggcattg atctacacgc 3240

gaatttctcc acctcggcgg aatggccact taggggtggg ggagggcccc tccggtggat 3300

tgtaaggtgt ttagcagcat ccctcgcctc cgctgactag atagatgcca ggggttaaca 3360

ttctccctcc gggcttcccc tagtctggcc tagtgtccca gcggggatgg gggagtagtc 3420

tgagggtgaa gttgccgcct attgagaacc actggtgctc atagtcctgc cctctgagct 3480

tgtgcagagg actctccaga tgaaggctca ggggtcgatc cagctcgaga ccccctcgct 3540

ccccccgcac agtcggacct taaaattgga gggttttaac atctcgacat catgagattc 3600

tacctttagg tcttttcttc cgttctgtcc gccaaatcgg cctcttccga gtttgttgac 3660

cagggccagc caggcagaag gctggtttcg ctcaaagagg atcccctcgc cctcctggag 3720

cttcaggcct gtttccgttg ggtctgcaga gaagccttag gggccacttc cttcggcatg 3780

gggaggggga acaggtgctc gctgcgcggg gaagaagagg gtttgactgc tgttctcgac 3840

ccccagcgcc aaacctccac cccggtgcgc gcccctccag gctcctgctg gtcccactcg 3900

ccggtaatta ggtctccggt cagcctcagc tcccgagaca cccaggccca gaaggacacg 3960

taagggaaag cagctattca cttcggtcct gtccgcaagg gatccctttc tgacgggaaa 4020

gaaaggtggc gagtcccgtt ctgttgagta agcggaagag agatcaaagg gaagagaaga 4080

aaaatcctgt gaggtttcag gatctaaagt taccatgaag tcgacctaac ctcgtctgga 4140

ggtcctcccg tggctggcga aggtgagtcg agtctccgtc ttcagtttgt caaagtggac 4200

aaagcctacg acaactgcca gtaccagcta ctaggcatcc gcagtctttc cagttggtgg 4260

tactggaggt ccctacccag gatttccgtg gattccaagg gtacaactaa acagcttttg 4320

ttgttccaac tgctcttatt tgaagaaagg gccgatctgt tgagaatcac ataggaaaac 4380

cacttccata agagtgtagc tactgttgtt gagactgtca cattctccct cctcctcctt 4440

cctcttccta gaaatgtgca atgaaaatta atccccacga tcggcctgtt gtactttcaa 4500

tgtacagttg gcagaaccta aggtaactct aggaggttcc gggatgaata tttgaagtat 4560

gacacagtca tttttatttg cacgtgcatt ccaaaaacac attggacaaa cacatccagt 4620

tcacctgtgc cacacttaca aagacgcgaa aagaggggct acatacaaac aagtgaaaga 4680

tgtaatcaaa cggcttttaa atgtctgctg tttccactga aggtcttctt ctctggatac 4740

acctaccagt gtcttctctt tctccaaaac cagacattca agtgccagac cctgaaatca 4800

gttttctggt tccaacttgt tcttccccat tctgtatctc tgcccctcca ctctcttctt 4860

tcatatgcag gaaagttcag ctctccctca gtttccaatt tgcaacatcc cattcgaaga 4920

atgcaatgac cagcatcaaa aattagacac cttccatggg actccgtctc tctcttagtt 4980

ggagcttcta taacaatgta ccataaattg ggtgactcat cctaaacaga catttctcac 5040

agttctggag ttttcaagtc taagatcaag gtgccagcat ggtagggtta tggtgaggaa 5100

cctcttcctg gtcgtagact gccgtcttcc cactgtgtct tcaggtggct gaaagagggg 5160

gaaagagctc tctagtgtcc cttttatggg ggcactaatc ccactcagga ggactccact 5220

ctctggatct aattacctcc caaaggcccc tcctaataat tttaacacat ttcctggggt 5280

gggcagggga aggttagggt tccaatcaag gtattttggg gggacacaaa cattaggtct 5340

cccgtgatct ttctagaaga agaaattata catgacccaa agttttgtac atttcacagt 5400

tagaaataaa gaaaactttt tctaagattt tgaaaggcct gaaacgtgag gccaaccaac 5460

cccatcacta aaatctggac tctgtgctga ttgttgtgga ctttttgcgt gttcatcctc 5520

cctgcagaca actgaagatt cattctctcc agagggtaga agagaagatg tattgggaga 5580

acccaccccc aatatttcaa agtaggttct ttctattttc cgtaagtgtc agctggctga 5640

aaaataaaga gacagtacaa agagaggaat tttgcagctg ggccaccagg ggtggcatca 5700

catatcagta ggaccatgat gcctgcctga gtctcagacc accaagtttt cattaagggt 5760

ttcaaaaggg gagggtgtgt aagaacagag agtaggtaca aagatcacat gcttcaaaga 5820

gcaaaaagca gaaccactga taagggtcta acaaagatca catgcttctg agggaacagg 5880

gcaaagggca aaagcagaac cactgataag ggtccaacaa agatcacggg gcaaagggca 5940

aaagcagaac cactgataag ggtctgtgtt cagtggtgca catattgtct tgataaacat 6000

cttaaacaac agaaaaaagg gttccagagc agagaaccgg tctgaccaca aatttaccag 6060

agctgagttt tcctaaccct agtaagcctg agggttctgc aggagaccag ggcttatctc 6120

agtccttatc tcactgcaca agacagacat tcccagagtg gctgttcata gacctccccc 6180

cagggatgca ttcttttccc agggtattaa tattaatatt ccttgctagg gaaagaattt 6240

agcaataagt ttcctacttg cacgtccaag taggccccct gcaagaagaa aattatggct 6300

ctttttgccc gaccccacag gcagtcagac cttctggttg tcttcccttg ttccataaaa 6360

attgttatta ttctgttctt tttcaaggtg cactgatttc atattgttca aacacacatg 6420

ttttacaatc aatttgtaca attaacacaa ttatcacagt ggtcctgatg tgacgtacat 6480

cctcagctta tgaagataac aggattaaga gattaaagac aggcataaga aattataaaa 6540

gtattatttg agaactgata aatgtgtata ttaagatgaa atcttcacaa tgtatgttcc 6600

tctgctgcag ctgcagcttg tccctctgtt tgggttccct tacttcctgt aacaaggatc 6660

cttggcccca agggggcaat cagcatagat gagcaaagac ttgtgtccca cactgaaatg 6720

agagggatga agtgacagca tatcttgatc tatgtgatgg ttacataagt tatgacctat 6780

ttcaaagctt attatgctgt gtgaaaaaga acaaaaccag aggacttgca caacctgaca 6840

ttcagactca ctctaagctg ttataaacaa ggcagtatgc aagcggcatt ggaagacaca 6900

aatacatcaa cagactgact tgggagatct gaaactgacc acagctgtaa ggtccatagg 6960

ttttttgttt gtttgttaat ttctttttgt tttctgagac agggtctcac tctgtcaccc 7020

aggctggagt gcagtggcct gatctcagct cactgcaaac tctgcttccc atgctaatgc 7080

aatcctcctg cctcagcctc ccaggtagtt catactacat gtgcatttta gtagacatga 7140

ggttttgcca tgttgtccag actggtctcc aactcctggg ctcaagtgat ccaccagcct 7200

ctgtctctca cagtgctggg attacaggca tgaaccacca tgaccagtca caggttttca 7260

ataaaagcag ttcaatgggg caaaggaaat agtttcaatt aattgacttt cacaaagaag 7320

aaaggggaga tgaacaaggt tatttctccc tcacactaca cttgaaagta acttgaggca 7380

cattatagac caaaacttaa aagctaaaga tataaagcat ttcaaggata ctttgtgact 7440

tgttggtaga cagagattta ttaaacaggg cacagaaaaa aaatgtataa aggaaagact 7500

tgataaatta gacttcatca atattagcca tatcttctca tcaaaagatg ccacaaagaa 7560

cttgaaaaaa tgaatcagcc tcagggagaa aatagtcaca aaacctatct gacccccaaa 7620

ccctgtaact ataatgtatg gtgagatctt cttacctggt aacgaaaaca acaacaacaa 7680

aacaaaaaca aaaaataatc aacccaagta tgctagaagg gtaaaagact tgaacagata 7740

cataagaata aaaggtacac acatggtcaa taaagcacat acaatatgtt tagttatcag 7800

gaaaatggaa attaagacca cactgagaca ccactataac tcccagtagc atggctaaat 7860

ttatagggag aggaaacacc caatcttgga gaagatgaag aacaaccaca actctcacac 7920

gtcattggtg gaaatgtaaa atgacacaac tactttggga cgttttgtca ttttttatga 7980

acttgcccat atcccactct ttggtaccag caattcggca cccaggtttt cccaagagac 8040

acgaaaacag atgccctcaa aatctcttgt gagaagaaag ttcaaagcag gcttattcag 8100

aatagccaat accagtttat tcagaatagc caatatccag actctcatta tgagggtggg 8160

ggtggggcgg gaatggcaaa caaactgtgc tgtttctttg caatggaata ctactcagca 8220

atgaaagagg aatgaacttc tgctacactc agcaatggat agagccagag acattatgca 8280

gagagaaaga agcatgtaga acagagtaga tgcaccgtga ttccatctgc gtgaagttct 8340

agaagcaaag gtaaagtgaa aacaatctgg agagtgattg ttcacaaata aggcagagga 8400

caacgacaat gatttttata attctagtga ttacgttgtt gattgagtag tcactaaatc 8460

caggcacatc tgacaacaac ggtgtaaggc agagtttgcg aagtggcaca accttccata 8520

gaaaaaatgt tgaggcacat aaaatgtaag atacttaacc aaggtcaggc aaggactgac 8580

acagatttcc aacacagaac ctgggttctt ggacatagat tagactggac ctgtccactt 8640

ctctgtttca ggtgtctctg ggattctcag ctgcaccaga ggagagagag aaggacagag 8700
aaggagacag agggagagag gagtagggaa gaaatggagg gaggcacagg gcctgtagct 8760
gttgaagagc agagaagtaa ctcttgccag tgtatttaca gtatagaaaa gttttctggc 8820
gatcaggaca ttagcagatg ctaagcttgc tgtggtcagg agatgaattt gatatgccat 8880
agatttagaa attaaaacga aaccctaaag ttattggaag agaatgtagc agaatacatt 8940
tctgccttaa gagataacag gaaaaaataa agcaaagtag aatttaacca aagttatcaa 9000
cttctgctca tcaagaatca ccatgaaaag ggagaaggtg agccacagac tgggagaaga 9060
taattgcccc gtgtttacct gaaaaaaaaa ataactcaaa tcttgaatat agagagtact 9120
ttttacaaat cagtaataaa aggcaaagcc aattgaatta gggaaaatgc ttcaatagcc 9180
tctcttctca aaggattgct aaatggtgaa aacgcattag taaatatgct caacagcatt 9240
actcattaaa ggaaacacaa attgaagcca tgatgtggta ccgttttaca tccaccagaa 9300
tgggtgtatt taaaagacag taaacaccac gtgctgtcaa ggatgttcag ccagtggaac 9360
tctccttgct ggtgagaaag cattctggaa gaaaccattt ggccgtttct aataaagtta 9420
acattacacc taccatatga ttgaacaatt ccagtacttg gtattcctca aaggaaatga 9480
atgcacgtgt ccacaaaaag accccacaca agaagaatct tcattcgcct tgtccagcta 9540
gtggaatctg tttcttacca gcaggagtag aagtctgaac atttctcttg gtatcatttc 9600
tcttggcctc ttggtatcag agtcgccaag gaaaatacaa cgcagtcaag cactggacgg 9660
aaaaacaaca cattattcac acagagagga gacacagccg ggtgtgggct ccgcttcagt 9720
agtgggcgtt ggtcccccat ggccatctgg tccctccatt tggcctgcac acacagcctc 9780
ttgagaagca gaagaaaggg cccctccccc tcccctcagc ggggagcaga tacagcagct 9840
ggccaggtgc cagttgatgc acatgtttaa gtggaacaaa ggggtacaca gtctgatcgg 9900
gggaaagata ctcccacaca aggaggtgag tccagcacag gctgtgagga ctccttatct 9960
cttgggaagg aagtgctcca ggcccaaggc ccattcctac ccggcagagt ggaggtggaa 10020
agacccctgg cacgagattg cctttcccca cactttcctg ttggcataca aacttggcga 10080
cgagctggga gacaagccat tgtaggccac aacaaagagt ttgaagttta aagtcattaa 10140
ataacagccc cagtgactgg aaaacaggtt ctgagggcac cattgccttc gtgtaagcca 10200
ccaacagggc tgactgggtt atttgctgtg tgaggctcgg aaatggacgg agcacgtcca 10260
gcgcttccct tggattcctc gccaggttag tgggctgtga ggtctctttt cctttccctg 10320
aggcgcctgg ttgcagggag cgaaagtgga tagctgacgg cctggaagaa cgacattgcc 10380
gtgactcgga ttcgaaccga ggttgctgcg gccacagcgc agagtactaa ccactatacg 10440
atcatggcga gccacaggcc aagcgatgat agctgagctt gctctactaa ttttgacatc 10500
aatagatttc gattattagg cggctttgtt cccagacagc tacaggggga tcgcgctatt 10560

ctttcatacc cgctctgctt ttccctccct tcctcccaat cctcatatgt gatgagaaaa 10620

attcttatct cctccactcc agcctgggcc tctctcgcga agtctctctg tcctcattcg 10680

cacctcctct acttctctcc ggcctctccg tatttctgct tttcaccctt cctctctccc 10740

gccggaccga ccgaagcaga ggtgagcgag ggtagcgcaa gcagcgtagc gagtcaggtg 10800

aaggcggagc caagaactgc gagagctgtc actagagccc accgggcgtt ctgcttgctt 10860

cccttcggac ccacgcgatt caggctcagg gagtcgcgga cccgggcgtt tccaggatgc 10920

tgggtctgat gcctgcgagg ggcaggacga ggcgaaggcc attccgctgc ccttccagct 10980

tcagaatcac ccaatgcacg ggtttcaacc gtagccggca agctgcccct ctccggtttt 11040

ccgaggcttt ggagcctgca caattgttct acaaaaggcg gtggctccgc ctaacgttag 11100

atggacgctt gctctgcgtg gtgataccag agcgagagtc gactgttcct gtgtcgcagt 11160

cgggtggtgt gcttgcttcc aagtctggag gcgggtggtt cgagctcgtt ctatggaacg 11220

tttcctgcat tttaaaaatg cttaaaccgc tggcaacttt cttctatcca gcacatgcac 11280

tcatggaaag gcagagcgag accaacttta ccctcctctc atccctctcc ctctccctcc 11340

ccctctgccc ctccccctcc ccctccccct ccccctccct ctccctctcc agacggagtc 11400

tcgctctgtt gccaggatgg agtgcagtgg cgcgatctcg gctcactgca acctccgact 11460

ctctggttca agcaattctc cttcctcagc ctccagagta gctaggctta taggcacaag 11520

ctaccacgcc cagctaattt ttgtattttt tagtagagac ggggtttcac catgttggcc 11580

agggtggtgt ggatctcctg acctgatgat ccgcccgctt agcctcccca gagtgctggg 11640

attacaggcg tgaaccaccg cgcctgacac tctctgtcat tcatttagcc ctttccatac 11700

gcttttgagg ttaaattggt tctgtgattt tactattaca tataatgctc tagtcattct 11760

tgtatgggca ctttcagcca ctggtgtaag tatttctata gggtagaggt gcaattacta 11820

tgtggaaagc aattactcta tcatagtgtt cacacggttt ttatatcatt ccattctctc 11880

tcctctgttt gcttattaga tataactctt tcttttgtta ttttagtaat tgccttaggg 11940

cttatagcaa accattttaa cttatcacag tcaaccttca agtgatatta tacctcgcct 12000

tctggccttg atgctgatgc tgtagctgac atgcactttg cttttacgca tgttacaaac 12060

cccacaacac attgttattg cttttgttta aatggtcaaa tttttacaaa agaaatttac 12120

agaataaaaa aaaaactagt atagttaccc atttctggtg ttcatttcct cgtgtagatc 12180

tggattttca tctggtattg gattccttct gcctggaaga ctcctttgac gtttcttgta 12240

gtgtgggttg gctggtagtg aattctttca gcttttgtac atctctaaat gtccttattt 12300

tacttccagt tttgataatt tattttttgt ggcatagtat tctaggttaa cttcccctgc 12360

cctcagtacc ttaaagatgt tgcttctatg tctgctcatg tgcattgctg ctcctggtag 12420

aggccggcaa gagcagaaag gaagctgatg caatcctcat ctttgttcct gtgtatgtac 12480

aatgtatttt tcttctggcc ggatttgagc aatattatta tgaggtatct aggagtggtt 12540

ttcttcatgt ttcttgtgct ttggtttcct cagctccttg ggtcactgga tttataattt 12600

ttgttatgat tggggacttt tcagctattg tttcttcaaa aattggccgg acacagtggt 12660

tcacaacttc aggaggccaa gtcgggaggt gttgagtgag gcctgaaatt taagaccagc 12720

ctgggcaatg tactgagacc ccattcctaa taaaagtaac aaagaaagaa ataaatgttt 12780

cctttccccc ctgctccttc tcagggactt ccattactca taatttaaac ctcttaagtc 12840

tgccctacag ttcactgata atcttttatt ttaaaaaaat ctcttttgtc tgtgagtttc 12900

attttgcata ttttcaatta ttcatggttc catgttccct gatcatttct tctgcaacgt 12960

ctaatctgct gttgatccca tccagtgaat ttatcatctc tgatattgtg gttttggcgt 13020

gatatcggct cactgcaacc tcctcctccc aggttcaagt gatcctccgg cttcagcctc 13080

ccaagtagct gggactacag gcatgtacca cgataccagg ctaacttttt gtattttgag 13140

tagagatgga ggtttcaccg tgttagccag gatcgtcttc atctcctgac tttgtgatcc 13200

acctgactca gactcccaaa gtgctgggat tacagtcgta agccaccgca cccagccccc 13260

tcccttcctt ccttccttcc ctccttcctt ccttccttga gacagggtct caccctgtag 13320

cccaagctgg acttcagtgg tgtaatcttg gctcacgcaa cttctgcctc ccaggctcaa 13380

acaatcctcc cactgcaacc tcctgagtag ccgggactac aggcacacac caccatgcct 13440

ggctaattat ttgaattttt ttggtagaga tgaggtttcc ttatgttgcc cagtctggtc 13500

tgaaactcct gagctcaagg gatcagccca cctcagcctc ccaaactgct gggattacag 13560

gtgcctgttt gtttgcagag aggagcagtg agcagatttc aggcaggaga gttaatcggg 13620

catccgttac tcaaactttg ccgggagcct aaggcatctc tttacatgtt cttagtttga 13680

cttcaacagg aggtgtgtca gtttatcttc caacatattt ttccaactca atgagagagc 13740

cttttccttc gtgctcttgc cagcacagca gtttccatat atcagtctga tgtaccagtg 13800

agggggtggt gccctgcctg atttacctaa gcaggaggtg cagcagaaag acatggtgga 13860

cagttcccag agatactctc tggcacgtgt tccacagcta aacctccaag atgtttagaa 13920

agtcttccag taattgtaat tgttttctct ggtgatctac ctaagcccag agaaaactgg 13980

ctggagagac cctgaactgc ttcctgtcaa gaggcttatc aaataggagg ggaaagaatg 14040

gctccttcta gaaatttgtt ccttttctct tttctttatg aattgcattc tgcttttgtt 14100

gaatgcaaac gatctacaga ctgttctccc tcctagacaa gggtgaattg gcatgctaat 14160

aactacctct gtgttattcc ttccttgatg gtgtttgttt tgttttgttt ttctgtaaaa 14220

ctcatctcac tggagacaaa tctctggatc ctgcacctca ccatgcttct tatgcagacc 14280

tggatccttg gagttattgg ctccctttgc cttgtttatt ttgttgactc tcttcagcaa 14340

aactgaactc caacttttac accctaaatc tggctcttgg acatataggt ccttttgctt 14400

tctgactcct cctcaaccca gccctgcctt tccaacctct ccatctgtat ctgtgtgcct 14460

gtgtctgtct gtctgtctct cataccccag gctgccccca gtattaccca ctttcttctc 14520

cccctcttca gtgttcaggg tacaccttct tctctgccta cagagtccca caggctgagg 14580

gtgcgtcccc ttacagagag gggagctcat tccagaaggt gccctctaga cttggaatta 14640

accctacaaa caacacccag gtttgaaaag ctttttctgt cttcccatta cctgcattat 14700

tcaaaggact aaaagtgggg agtccagtag aattcttgat gtctctcaga agcctgcatt 14760

ggaatgcctt cattcaccga gcatatgttg ggtgctcaca aggtgtgaag ccttgctcta 14820

ggtgagtagc tgcatggtga gcaagtcctg ccccaccgag cccacatctg gaggaatcaa 14880

aagggaccgg gcagtgggaa cacaaccagc tcgtgtcctc cccacaagtg tttccccatt 14940

cgggcacagg gaacacaagc ggtaagcatg acagataaag catagcgaag gatggggaag 15000

tgggtgactt cagaccaaga ggctactgtg gtcatgtgca gtggggccac ttccgacagg 15060

ggaagtgcag ggggacagtg tgggccaggg agaatgtcgt ggatctggca ttcctgcatc 15120

ttgccctcac tcacatggcc tggtgtcaac tcagaaatgt gcccaaccac acaacttctg 15180

gcctccgtgt cacctagaag acatctagaa cccatgcaat agcacaaaga agtgaggatg 15240

tgcagcctgg ggatgggggc atttaataat agacaggtgt ctgaggcaga tgtcctcaga 15300

tttccaggca ccacttgcct ctttgtggag caatcagggc aatcatcggc aaaaaactaa 15360

agactactcg tcctgttcca tgcagaggcg tagttaacct tgtaactatt cttttctgac 15420

cttcattctt ccacgtccag atatatgggg gtctttgttg cccatctgct tcaagttaga 15480

tgaagaaaat cctgcctgaa ggcttaaggg tttgttagaa aaataaatgc tgttcagaaa 15540

aggtgcattt taggcaaaag ctcagttctg gagctccaga acctacctat cccaagccca 15600

atacacacct cctttctctc cctccctgct catcgcacct gccttcgctg attcttcagg 15660

ctgcacatct ctgagaagat gacaatccct agcagaagaa caagagaacc ttggacactt 15720

ctgtccatga agccatagac gcgagcagat agcctcattc catggcggtg tgactctgaa 15780

gtcactcagg ttttctgtgc ctccatttcc acattgtaaa atgggcccaa taggaatgag 15840

tttctgaatg ggttgttcca agtttaaata agacacggca gtaaaacatt gaacacacac 15900

cctgatgcat agtaaacgct ccaagagcag tagccattat tactatcatt atcatcggtg 15960

gcttcatgac ggctattatc gttatctaca tccatctttg aaataattgc atatcctttt 16020

agggtatgga gggattgcaa catataacaa atcctaactg atgaacattt cttattacaa 16080

atattgctgc tgggaacata tttctgtatt ttaatcatca cctgtgtgtt tctctatcaa 16140

atcttctcac acaatttggg gatgttgcaa aagtcattcc ctgaccggga atcgaacgcg 16200

ggccgcggca gtgaaagcgc caaatcctag ccacttgacc accagacaca gaaatctcct 16260

tgtcagagga aacaagagaa ggtaaagatt aactttctaa gaggaggaga gccccactga 16320

aaatggctct ctgtgaccca agcgggctgc aggactgcag ggctccaagc agtcactgcc 16380

cagctgctca acacctctca cctctgctag cctggccact gctttgggct gtattgagga 16440

caggccacaa ggaaactgag cggtgttcca ttctagcctt gctgtagcat ggtgtgaggc 16500

agagccaggc acttgctctc tttccgagcc tcagtttcct tgccaccaaa gtgcggacgt 16560

ggctctcaga gggtgatgag ccgagtcctt ctagttcccg atctctgaac tggctcaagg 16620

ggctctaccg ataaagacat gtgtaggggt cccctttcgc ccccaactag ctcaattagt 16680

agagcatgaa actctgaatg gatgggtctt tggctcgagc tccaggtgca gatttaggtt 16740

cattttcttt tgttccgcca cacaattcga ccggttccgc aactgagctc gggtcgcgag 16800

gaagccaagt gggcttcaca ggaaagacct ccttggtttc agagacattg tagctgtcag 16860

cttgaaatct tcagaaagaa gatttcggcg atagatgaga agactggaaa tttctggatc 16920

taaagctaac atgaagtccg cctcatccgg cgccctcgtc tcccgatcca attcacggcg 16980

ggcaaaccag gcctggttcc taatgctcag cgaagggttt caggtagtaa tggaactcct 17040

cccgtgcttc cagaagctgc tgcagccggc ggggtttgag cgccggagtt ccgggcggca 17100

gtagtgtgga ccccgcccct ccaggtctat gggccccgcc tcgggacctt atccctggcg 17160

tccggccttc agggctcttg gctttgacag ggtaccgact ttcgtggtcc ttccaggagc 17220

tgcaggaccc tgcctgcccg cccttcactc aagtcaaggg gcctcatccc tgtgctcttt 17280

cctcgctttg ggctctccac tcagggtgtc aacagaggta ggtcaggtga gaaacctgga 17340

tttccacaca cacctggcta caaggagatg gcaccactcc ccttctcctg cagaagcaga 17400

ctcagaaaaa aggtaattaa aacagaaggt ttcaataaaa tcgagagtct gataacataa 17460

tacccaaaag tccaagtttc tattgaaaat cattcattac actaagaacc agaaaatctt 17520

attctgaatg aaaatagaca gcaaatacac accggcaata agatgagaga ggggtcggga 17580

ttgtctgaca aagattttaa agcaagcaag cctgatgaaa atgatgcagt aattatgagc 17640

acattcgaaa caaatggaca aatagaaagc cttagcaaat aaatacaaga cttacaggag 17700

aaacaaatgg aagtattaga agtggaaaat acaacaacca aagtaaaaag tccggcacat 17760

aggctcaaca gcagaatgga tggtgcagag caaagaaatc catccactgc agatgaaaaa 17820

atagaaatta cacaatctta acaagcaaaa aatagactgg gtgagaaagt gaatagaacc 17880

tcagggactt gtggaactgc ctcacaaggt ctcactttca tgttgttatc tatgtctgga 17940

aggggaagaa gaagaggaag aggctgaaaa cataaagaag tcaatggcta aaagcttgcc 18000

agagttggca agagacagaa acctacaggt tcaagaagct gtgaagaccc caaataggag 18060

aaaacccaaa gacgtctatg gcaagataca tgaaaaaata aaatgtagga aatgaaagac 18120

aaagaaaata tcttgaaagc aacagagaaa ggtgacactt tacctagaaa ggaaaaacaa 18180

ttctggtgat gatggatttc tcatcagaaa ccaaggaggc agaaagaagt tgtgcagtgc 18240

ttttcaagcc atgaaagaaa acaattgtca gcccagactc ctctgtccag cagaaacatc 18300

tttcagggat taagaggaag atcaaaactg tttcagatta agaagagcta agggaatctc 18360

tgaccaccag ttctacctta aaacaaaagg ctaaagggag ttctctagac agaaaggaag 18420

tgattaaaga aggaaccttg ggacatcagg aagaaaattt aaaaaaaaaa aaaaaaagta 18480

agcaaaatag gccttccttc ttctcttgag ttttctaaac tatgattgac ggcttcagaa 18540

aaaaatcata gcatcattca aagtggttct aactctccaa aaagaaaatg tttgaaacaa 18600

ttataaactg ggagggtgaa gagaccggta aggaaggtaa ctcatctatc attcactcat 18660

actggtacaa caatgacacc agtatactgt gtggtaagac atagatgcac agacatatga 18720

atatcaggga aaaccaagag ggctatccaa acagatatat gcaaaaacac tatggataag 18780

gcaaaacacc attctcaaag acgttcaagt caccacagga aggtaagaaa aagaaagcat 18840

atccagaaaa cgacaccaga gagaacaaac acaaaaagaa aaatataatg gcagacttaa 18900

gccctaccat atcaatcatc atattaaata taaatggcct aagtacacca attacaagac 18960

agagattggc agagtaggtt aatggacatg acccaactac atcctggctg caaagagact 19020

accttcaaat atgatgatac aggaaagttg aaatcaaaag gatggaaaaa gatacatcat 19080

gcaaacctta gacaaaggaa agcaagactg gctatattaa tgccagatat agtatatttc 19140

agaacaaaga aactaatcag agacagagag ggacattata taatgaggaa acaaccaatc 19200

cacgaactcc tctcctgaca aaagtcataa atgtctatgc aacaacaact gaaagtactg 19260

aacagagaac taggcaaatc cacaaatata gctatatgct tcgaagttcc cctttcttaa 19320

caatttatac aaaaaccaga cagaaaatca acaagaatat agaagaaccc aacagcatga 19380

tcaaccagcg gggcttaaat tgacaatttt agaacaccca accacgtcag aatacacatt 19440

gttaagggat gcattgtgcc cggccccccc agattcatat gttgaaatca gaacattagt 19500

ggcaactagt aatatactag ttaaaatcac aatgagaaat tgctaacata cctatcagaa 19560

tggccaaaag aaaagcatga ccacatcaag tgctgtcaag gattcagaga aagctggatc 19620

actcatacat tgctggtgga aatgtaaaaa gtgacagccc actctggaga atatctgtat 19680

agtttttact ttttaaacta tatgtgcaac taccatacca cctaggaatt gcattctctg 19740

catttatcac agagaaatag aatcttatgt tcacacaaaa tccatgtact taaagtttat 19800

agcagccttg tttttaatag ccaaaactgg gggaaaaatg tatagacgac tttcatgagg 19860

tgaatggtta aacaaactgt gatatatatc catagcactg aagagtcctc aataattttt 19920

taaaaaacaa caaactattg atacccacaa taacctggat gaatctccac agaattatgc 19980

tgagtggaca aagggtaatc ccaaaggtga catgtaggag tccatttatg caaattattc 20040

ttgaaataaa taaattataa aaatagggaa tagattagta gttgccaggg gttaaggagg 20100

gaaggaggag cagcactgaa gagacagggt catggctata aaaaggcaac atgatgaatc 20160

ctagtattga tggaaatgtt ctgtaccttg cttgtatcaa tgtcaatctc ctggtacagt 20220

ggcatttgca atacatttgc aatatttgca aatattacta agatgttacc attggaggaa 20280

actgagtaaa ggatacaagg gacctctctt tttctctctg agttcctaag attgtatgtg 20340

aattattaaa atgtaaaaat taacttaaga aaacctaagt atatttctcc tttactgatc 20400

atgcctttgc ttaaaaacat taaggcaaca atatttttcc caacaccctt tattttcttg 20460

aagacagttt taacaaaagc tgttcttagg gcttttccta aaagtttaaa acttccagta 20520

atccttactg ttcttctttg agaaccctcc agggttttgt tttccaaaag aataaagaca 20580

aaaactggca actggcatat aatactatat tagtcttagt tctccagaga aagaaaatga 20640

gcaggataca cacacataca cacacagaag cagagaagcc caagttctac tctgagtaag 20700

ctctagacct aggagagtca aatatagttc aagacaaagt ccaaggctta aggcagaaga 20760

ctgatgtccc agctcaaaga cagtgcagca gaaaaacttc tttcttaacc cttcatccta 20820

gtcaagccgt ccatgggttg gatgaggccc actcacactc aggagggaaa tctgcttcaa 20880

tcagtttact gattcaaatg tcaatctctt ccagaaacac cctcagagac acagtgagaa 20940

atcatgtcta accaaacatc tgggcactcc ctggcccagt caaattgaca caaaaattaa 21000

ccttcacaaa tgctctcaat agaagtgatt aataaatgta gcatcatgta ctgcctgttt 21060

atcccgtgac aattaatata ttccagcata acattggctt ctgttccctt actgtaggaa 21120

cacataggag tttcatttaa agtgaggata acttttacct tatggagtct ctccctaccc 21180

gtacctacca ttgccacttg tggattgtca ttttgccatg gctttcttat tcataatatc 21240

aataagctgt atactttgct aatgtaagtt tgaatttcat cctggctgca taagtcactt 21300

tccattttga caaagcctag aaatattttt gtttatctcc tgtatattgg taacacatac 21360

ctattgtggg gcccactctc aatctcatgg ttttctttat tttcattcag ctctcaatag 21420

atatgcctag gctgcttgaa catcactgaa aatggcctgt ggccttgtcc atctatcaga 21480

gtccagtaaa atcctggcca tggaatgggg ggatggaaag tgagaaccac caaactcagc 21540

acacaaagac tcagtttaaa cctctgcgct aagagatgac cagactctat catggctcct 21600

aatagcataa gaccatgtcc catggatgac cctaggctcc catgacaatg cctacctaag 21660

aaagctcaag gctaccaggg gaacttactt tagttctagc caatacctgc tgatgatggt 21720

aggttctttc ttatagcatt tactaagaag ggaatccctc ctccatccct ttgagatatg 21780

tatgtatctc ctagggactc cagtgtgtct ttccacagga tttgaaagcc attcctttta 21840

ggtgtagtcc tcaggaagga cagaatctct gtcttccggt ctctgtggga ggttaggatc 21900

ctaacatcta atattgccag ctggcagaaa cagctgtcct gatgaggcag aagcttacaa 21960

tgatcaatct ttcataatgt ttcacttgtg ccctgaccct gccctctccc ctgcctcact 22020

ctctcattag aatgccatca cctctgcaca aacgggatgg aattcagccc cctctgcttt 22080

ggtcagtagt tactgaataa aatctgtttt cacagctttg actaatgtct gactttatct 22140

ttgacaaaat aagtgcaagc aattgttttg agcaacacag cagccacaaa gagaggcaca 22200

gacatgtcgg gagagggagg aatcatctga cccttctcat ttcacaggca tctattcctg 22260

cctggtgttt atgagcctga gacttggctg ggctgccctg gtgtatcact ccatggctct 22320

ggggcaagac ttgagtaggc tggtggtggc acttggtgtg accacagcca ccttcaaaga 22380

tgccattaag ggttcacaag aaaatgaaag gaaacataga gaaactgatt ttgaaagttg 22440

ttgcctctcc tgcttcctca ttcagagtgt gtatgatatt gctgtgtgat gttagttgga 22500

ctgcaattta gcacaacagc tgttctaata attggcagtt tctcatcgag ttaagcctac 22560

ctgaaaggga agcacactta cctttattga tcacccccgt taaatgaaca atacatccaa 22620

caaggcttct accaaaaatg ttcttattaa cctcatttgt atactaagga aaaactggga 22680

acaacacaaa ccttcatcaa cagaagaatt taatacggac tgttatatag tcataaaacg 22740

atcccagaag ttcgctgcat aacatcaaac ccaggaaccc acactgatgc cactgatggc 22800

agaaaatctt gatacaaaaa taagagagac gagagacaga aaatgtgcta taaccatagt 22860

agtgggtaaa agccaaagag ctcagtttct attcatgata ttcgatagaa gaacagaagg 22920

aacatgagcc aatagctcta agggtccagg tctgcacaag gtgcctgttg ggtgcagggt 22980

tctgttggct ccagtgttgt atctcaaatg ggctaatttt tatggacata gccactgcag 23040

aaatctatca aagaataata aagggatagc tatttgcatg ttaatttacc attgtatagg 23100

agggagaacg ggccttagat tggtttggtc aaataaaagc aaaaccgaga tttatgaaga 23160

aaagaggaaa ggggagagga aaaaagtcat ctgggaactt tgtgattttg ctccccttgg 23220

ataagcgact tgacagaaag ctgtcaaaga ggtcattgtg acccatcttc tccataccca 23280

catgacttgg tcaccacaga aaacaattac agatgttgga ggactttaaa acatcctaga 23340

agtttaacag ctcatcctat gccacagaat atctgtgagt aatgtccacc atgtccttcg 23400

gttgtacacc ccctcagtta agccaggagg acgcctcttc cctcagtgga atatcatacg 23460

tcgatgtgta gaaagttggt tgtgccagca agtaaatgac aaaaggacaa ttgatagaat 23520

tgggaaacac attggagtat gatttgctac tgctcccacg gaagataaat ttgcagaatg 23580

tactcaaatt agaagcatta tataaatgct gtaatgtaaa aatggcacat cctgttctct 23640

acactataga aatatctgca tgagtgacca aagatatgtg tacaagaatg gtggagattg 23700

cagcatcatt tgtaatccta aagtcatgaa acccacctca tttcaaaaac atcttgaaaa 23760

gggttaaatc atgcacaaac agaggaagaa atgctgtttt cacaaacaat ggcgaggatc 23820

accatgagga tgagtgattc cactggtcac attattgata gagcaatcag taaacccagg 23880

cacatcctga gaataggata ctactgtaat agctctccta ttactaaaca tgaaaaactc 23940

gaggcacttt actcccacaa gccttccatg ggagcaaaga acatttttat cacactagaa 24000

gtgagttcct ctctaaggtc tctgggattc ctgagtttga cagatacaag gcaccatggg 24060

ccttgccaag cctcagccct agaagatggg gaggaggaca tgtgtgtctc ctgattaggc 24120

tggagttgat tgtatatcaa cagaaacaac aaaaatcaaa tcatgaacta tttttctatg 24180

gggaagtttt gttttgtttt gttttgcgac agggtctcac ttggtcgccc aggctggagt 24240

gtactggtgc aaacaaggct cactgcagcc tcgacctcct ggggccaagt gatattcttg 24300

cctcagcccc caagtagctg agacgacagg ctaatttttg tattttttgt agagacaaga 24360

tttcgacatg ttgctgaggc tcgtcgcgaa ggggaagtaa gttcttttta aaaagcattt 24420

attccgattg atttctccgc atcctgtggg gcgcgcaagg ggcaaggcct cccgtggcct 24480

ctcttgccac tctgaaccag ggagctgtgg gggctcagct gcgttgtttt tgggtgcgtg 24540

tctggggcag gaggccgggg ccgggcaagg ggtgaatcca acagggaact gcgaggagga 24600

gggcaaggaa gtcggtagag gcatgcacca gggaaacttc ccagtagctt tgtgcagcga 24660

taaggactgc acaaggagga gatgagaagt gttttcttat tgtatcagaa tggggaagaa 24720

aggggggaaa aacggagaga caccaagaaa gaacgaaatc tacgcggacc cttgggtctc 24780

caaaagggaa gcagtcctgc attaagttta aagaaggagc gcggcgcgct tcagctcctg 24840

tggcttgccg tgatcttatt gtggttagta ctttacgcgg tggccgcagc agcctgggct 24900

cgaatcccag tcgtggcaac gtccattcta gggtgtagcg ctcttctttc cctttagcct 24960

ctaacctcag cctaggctgc gctgttgacc tgcggctaga gaggtcacaa ctttcctgtg 25020

acttcggaac tctctgtggt gtcgagaata gaaggaaacc ctaccctgaa tgcctagggg 25080

tccggttatc ttgaaagact tttcctcctc tctgagcaag tacaacaccc aaaagatgag 25140

gtgccccctc tcagtcagcc ttattggaat ctttaatgtt aacatgatgt ggccgtaccc 25200

gtttttctgg ctcagatatt tcaaaaatcc gtggggcctt tttcaggcca aatcagaaat 25260

gaacaagaga agatgagagt tcgcactcca actgggtatt ttaagcaaaa cctaaagaat 25320

acaataaaat ctaaagtttg tgactttctc caaccccaat ttttaatttt gtttttattt 25380

atttatttat ttattttgct atcagtaaat gattgaagta taatccacag agaccaaagt 25440

gcaccaatct taagtgtgtg agccagtgca ttttgacaaa agaaaccatc acctatgtca 25500

agttatggta cattttcaac agaccaagaa gtcattagag cccctttcca gtcaatcctc 25560

accccatccc catttaggca atcaccattc tgacttctat ctccgtgatt tactcttgct 25620

tcttgtagaa atcacgtaaa ggactacatt gattttacct gttttctttt aatgcaacaa 25680

tgatatgtga tgcttccatt ttatgacatg tatctcagct ctttggtgtt attgatgagt 25740

gttattccaa catgtgaata tactacagtt gatggacaat tgggctattt ctagttttgg 25800

ctattgtgaa taaggtgttt acaaatattc tactatgggt ctttttatgg acctgtgcct 25860

tcatttctat tgagtataaa tctagtaatg gaatggctgg gtcataaata ggtgtatttt 25920

taactgtatt agaaactatc agacagtatt ttagaatggt ggcactattg tattttctct 25980

ctctctcttt tttttttttt tttttttttt gagacagagt ctcgctttgt cacccaggtt 26040

ggagtgcaat tgcaggatct ccactcacta caacctccac ctcacgggtt caagcaattc 26100

tcctgcctca gcctcctcct gagtacttgg gattacagga gtgcaccacc atgcctggct 26160

aatttttgta tttttagtag agacagggtt tcaccatgtt ggccaggctg gtctcgaaat 26220

cctgacctca tgatctgccc acctcagcct accaaagtgc tgggattaca ggcgtgagcc 26280

atcacacctg gcctatatgc aaacttctgt atcagaatag aaaagttgag aagatagtac 26340

acagaatgga gtaaagagga tggaatgggt ttttggaaac caattcacaa tttaaagttg 26400

ttttaacacc cctaatcccc aaagaaaaat gtatattctc caaagacatc cctttcttaa 26460

aatatgcaga gaaagtgtgg gataaaagag catgtagtaa acttctcaga acgtgtgtct 26520

ggaagtggac aggggaatgg gagtgggatg aacagagaaa agaaggaaag acagtaaggg 26580

ccttgctgag agcagccacc ataatgtgcc ctgagctaag gtacatgatt accttcttcc 26640

tgtgcacctg agatctaaca gaatgtattg acttatcagg gatatggaat ttcaaaaaca 26700

ctacaggaaa tcaaaaaatt taccaaaaca gaacagtcta gttaagaaag gcaggaagag 26760

attttataaa caaagcaaaa acacagaaat catacagtga ggtggtggga atgagactat 26820

gcgcatcgtc tccaatataa cactaattac tgctaattat aatggattta agtatctcat 26880

attggataga aaaggaaatg ccagctctaa tgctttaatg gtggaaaagg gttaaatatt 26940

caaagcagtg cacattatta ttgttagtag tagtagtagt attttgagat agagtctcac 27000

tcttgccaga ctggagtgca gtggctcgat ctcggctcac tgcaacctct acctcctgtg 27060

ttcaagcgat tcttctgcct caggctcctg agtagctggg actataggtc gcgcgccacc 27120

atgcccagct aatttttgta tttttagtag agattttagt agagatttca tatcggccag 27180

gctggtctca aactcctgac ctggtgaccc acccgcctcg acctcccaaa gtgctgagat 27240

tacagatgtg aaccaccgca ccaggtcaaa agcagtgcac attatcatta gcaagctagc 27300

ccagaaagag aaaaccaaac accgtgtgtt ctgacttata agttggagcc gaatatgaga 27360

atgcttggat acataggttt cgggggtgag gacaacacac actggagcct gttggaaggt 27420

gggggttggg aggagggaga gcatcaggaa gagtagctaa tggatgctgg gcttagtacc 27480

tgggtgatgg gaaggtcagt gcagcaaacc accatggcac acatttacct atgagacaaa 27540

cctgcacata ctgcacatgt acccctgaac ttaaagtaaa agttggaaat aaaaaatttt 27600

ttaaagtcaa gaaaacaaac aacaaaaaga gtagtgcaaa tctgtaccat gctcatttgt 27660

accactcttt tgtttgtacc accaaacaaa agaatgagag tgaaaacatg aatatctgac 27720

aaaagtaaac agcaaaagaa cactttatgt taggagtaat accctagcat ctacctagaa 27780

tgaaatgaaa ctgtttttcc ctgaactggc ctcccaagct gaactcttgg gactgtgcct 27840

gggatgctcc aggagaccag ggtgataatt gaacttccct cctcctacac ctctatcgag 27900

ttcctggaaa acgccagcga tcagatcttg cttttaactt cttatacttt acagagacct 27960

aaacaagagg gaagaaatgg acatttcatg ccaagatttc ttcccttcac ctcacactcg 28020

agttcctggg ttctgacggc cttttttgag gaaagtgctg atgtttgcgt caaggttggg 28080

aggaaagggt ttgggaggta ccaggcaatg gggaggcaaa gagaagacat caacaagcga 28140

ggcaagaact ttccacaggc tgacactgga tacaagacca gagacaaaaa gctgggtgaa 28200

tttctgatgt tttcctacag aggtttttta tccagttgca cacagcctac tcagaataag 28260

gctttttcaa gtcaaccctt ttaatgtgaa gccataattt tgtggtgttg cctgatgcaa 28320

atctccactg tgagcctttg ctctgggtgt cttttttgtt agtcacatct tccaaagaga 28380

acatggtgaa aatcttgact cctttcccca agtctgtgtc ttaactgttt ccaaggctgc 28440

agagctcggg ggtcaggagg cattggcggt acagtggcag aattctcgcc tcccacacgg 28500

gagacctgga ttccatttcc ggcaaatgca gcgtcctgtc tttgtgccct gtggttttca 28560

catttactta ctgtgccacg ctaatgaaga aaaaaaaagg agcggggggt cgggggaacc 28620

actgtgtact cctcaggttc tttccgtagc ggaatttatt tttcttttat ttatttattt 28680

ttgtttgttt gtttgtttga gacagagtct cggtctgtgg cccagattgg agtgcacttg 28740

cactatcctg gctcactgca acctccacct tccgggctca agtgattctc atgcctcagc 28800

ctcccaagta gctgggatta caggcacatg ccaccatttc ctgctaattt ttgtattttt 28860

agtagagacg gggtttcacc atgttggcta ggctgatctt gaactactga cctcacatga 28920

tccatcagcc tcggcctccc aaagtgctgg gattacaggt gtgaactccg ccccggagcg 28980

gaatttacct aagcagagac tctctttgaa gcagcctgtg ggtctggttg gtctccgcgt 29040

ttctacatat ttggccctaa aaccggcacg acttttatcg tctctgttgt tggatctctg 29100

cagtacaaag agtcacctgg tcactgattc tctgcctgga ccttggatag accattggga 29160

cacatcttgc gatggagtgc gactcaagca atgacattcc tgaaaggaga gaagctgtgg 29220

ggttgggcgg acacacgcaa tgttggggaa ctgggaccca gggagagggg agacgtgtga 29280

gaaggaaccc acgtgcagat gaggggagct gcagagaggg gcctcggacc ttcctgttga 29340

ggaaagccca gtctttagtg gtctcggcaa attaaaggat tggccccgag tggagagcag 29400

catatcccca aaactcattt ctatcagccc tgtttgtttt acactgcacc aggcaaatct 29460

tctgtccacg ggattctttt taatgcttct ctttaagcag cgtggtgggg aggccatggc 29520

gatcattagc tctggaggcc tggggaggac actctgaaga agaaagccct gcgattatcc 29580

ggtttcaccc tgttttcatc tactctttgc ctcagcgaag tttatattgg gtcctctatg 29640

ttacatcaca gctgctgcac tggcggatta agagccgctg acaagtctct ttcgggaagg 29700

ggtaagaagc gcagctggcc cagagccatg gagagagacg caagagcaga accgaaaata 29760

ctttcctcct tcttgttctc tctctgctat tggtgaacat caaccttcca cacaaaccgg 29820

ttcaatactg ccttggtctc tacaggaggt ttaccaattt ttacagttcc cgtaagccat 29880

gttttcttct cgtttctaca gttcttccag ggttgagttt caggagctgg tcagcagtcc 29940

agactagttc aacatcctgg ttcaaaacca tttgtctcgt ttcgctacta gggtaaagct 30000

tgcctcctag accagagtta agacgtgttc cctcggcttc tgttttctag agagaattgg 30060

catagtttct tccttaaatg tttggtagtg aaaccatcta gacctggtgc tttgttctgg 30120

aaggttgtta attgttgctt caattgcatt aatagataga gacctatcta ggttgtctat 30180

ttctccttgt atgagttttg gtcaacttta tctttcaagg acttggtcca attcattgaa 30240

gttatcaaat ttgtgagcag agaattgtgc ataatagtcc tctactatcc ttttaatgtc 30300

cacgagatct gtagtgaggg tcattgtttt ctttttcata ttagtaattt gtaccttttc 30360

tcattgtttc ttggctcacc agctaaaggt ttatcaattt tattggtcct ttcaaagaac 30420

actctttagg ttttgttgac atttttctaa tgatttctgg ttttcaattt ccttgatttc 30480

tgctttagtg tttattgttt cttgtcttct actcactttg gacttgattt gctcttgttt 30540

tctttgagtt tcctaaagtg caagtttgga ttattgattt tagattttta tttgtaatat 30600

ttaatctttt aaaatccatt attattttac tttttgtaca gtaattcttt tagtgtaata 30660

taattttgtt ctggtaaaaa tttcagtttt attttatgta ccaagtgtac atgtatagga 30720

ttgttacatg gatatattgg acccaggtag ttcacatagt acccaatagc tagtttttca 30780

acccactctc cctccctccc tccctacctc atggtagtcc acagtgtcta cggttcccag 30840

gtttatgtct gtgtgaactc aatatttagg ttccgctcct aagtgagaac attcggtatt 30900

tcattttctg attctgcatt agtttcctcc agagtatggc ctcctcctct ctccatgtta 30960

ttgcaaaaga catgatttca ttctgtttta tgggtgtata gtattccatg ttgtatatat 31020

accacatttt ctttatttaa tccaccactg atgggcatct agcttgattc catgtcttcg 31080

ctattgtgaa tagcctggtg ataatcatag gagtgcatgt gtctttttct tttaatgatt 31140

tacatttctt tgagtatata cccagtaacg agattactgg ttgaataaca gctctgttgt 31200

aggttctttg agaaatctcc aaactgcttt ccacagtgac tgaactaatt tacattcact 31260

ccaagcattt ataaactttc tctccagagc cttattagca tctgttggtt tttgactttt 31320

taataatggc cattctgagt gatgtgagat ggtatctcat tgtagctttg atttgcattt 31380

ctctgattat tagtgatgat gagcatttat tcctgtgttt gctggctgct tggatgtctt 31440

cttttatgaa gtgtctgttc atgtctcttt gcccactttt taatgggatt ttttttcttg 31500

ttgatttttt taaagttcct tatagatttt ggacattaga cctttgtcag attcacagtt 31560

tgtgaatatt ttctcccatt ctgtcggtgg tctgttgact ctgttgatgg tttcttttgc 31620

tgtgcagaag ctctttcatt gaattaggtc ttacttgtga atttttgtct ttgctacaat 31680

tgcttttgtg gacttggccg taaattcttt gccacggctg atgttgagaa tgatatttcc 31740

taggttttct tctacggttt ttctgctttg aggtcccagt atatattgtg tcattgcaaa 31800

tgacaaccaa ccagaaacta atcattacaa gccaagaaag ggaacttatt ggtgaccagg 31860

aaaaatgtcc aacaaatgaa aattagtaaa agagattcct gggtgtcttt ttctgtaaag 31920

cctggggcat tgttgtctgc agagttcaca ttttctgtaa agggccaggg agataggaag 31980

tattgtaggc ttctgagcca agaggcaaaa ttaaggatat tatgtaggtg cacatataac 32040

caggaagaaa acaaatttct aagccatttt tattgatgag attctaaact aacaatagca 32100

actgagtaga acttctcagg tggtaaatgt tcgctgcatt gagagcctag gctcagcttt 32160

cctagaatcc ctggtacagc cctccctttc caccccttag gctgtttcct ccctgttttc 32220

ctcagaagag ttccatacaa aagacctact actctgagaa acagtgaagt actcctgggg 32280

cccaccaaag cacacgagtc aagaagtgga tggccttgtc ccagctatgg ctcagatcac 32340

ctttgcagga tgacatcttg ttgggagatt gtcatcagag ccagggacat ggaagatgct 32400

gtcagaagga tgaaaggact ggtgcccagg gggagaagtg gccaagcaac tctgcattca 32460

gcctcacaaa aatctcaagg ggatgtagaa atgggaacag ggcgaatgga aaactctttc 32520

aagcagggtt ttggggtcct gagctgatct ctatttttcc tcctacaacc tgccgcacag 32580

gctattttct cacctctgtg gatggaaact gcatccttcc atatgctcag tccaaaaatc 32640

tttgagtttt ctttgactgc tgatctttcc ttcacaccct atagctgttt agtcaagaat 32700

tcacattatg ctgagcatca ggtgtgctcc ttaatccaca ccatcctttt cccccagttt 32760

ataaaacttg taaagtcatt ctcctgataa agacaatagg atttccatga aattgcacaa 32820

tttttatcct tcctggatac atgactcatg taagagctct gctattgttt gctaccattt 32880

ccccctattt ttatttcata cctgtttgac ctgcattctt tcccataagc caggcacttc 32940

agctcaggtg tgctttccta atcatagaat tagcaatagc agccttgcta acaacttctt 33000

cctggcccca ccaccaggca aatgtcacag gatttaatag gtaaagaatc gtaggatcgc 33060

tgagcatggt ggctcatgcc tgtaatccag gtctttggga ggctgaggtg ggtggatcac 33120

ttgaggtcag agtttgagac cagcctgacc aacatggaga aaccccgtct ctactaaaaa 33180

tacaaaatta gccaggcatg gtgacgcatg cctgtaatcc cagctacttg agaggctgag 33240

gcaggaatat tgcttgaatc tgcgaggcgg aggttgcagt gagcctagat tgcgccactg 33300

cactccagcc tgggcaacaa gaactaaact ccatttcaaa aaaaaaaaaa aaaaaaagaa 33360

tcataggatc gtgcctatta ctcggtttta taaaatgaat tgttttcccc tgaattcata 33420

tgttgaaagc ttaaccccca acttgactgt atttagagat agagtcttta aggaggtaat 33480

taaggttaat taggccatac agttggggtc ctaatctggt cagaatggtg tccatataag 33540

aagaggaagg gcatcaaaga atctgtctct ttctccacac aggcacagag gaaaggctat 33600

gggagcacaa agcaagaaag tggacttctg caagccagga agaaaggcct ccccagaaac 33660

caactctgtg agcacatcga tctttgactt ttggcctcca gaatattgag aaaataaaag 33720

tctgttgtta aaccacccaa cctatggtat ctttctatgg cagcctgagc tgactaatac 33780

ccttggtagg tgggtggcag tcaccttact gggctacatg atgtgaagtt aaagtaactt 33840

tagcaatagg caccatgtaa tttcattgta tagatattat gtcttttgca ctgatattgc 33900

ttttttagtt gtatgtatat tgatgcctgg gcctagcatt acaagaccag tcaaaatggg 33960

aagaaagaaa agaaggtggg aaagatacca agttccataa ttgcatcctc tacccacatc 34020

cagttcttag aatcttttct gctggaggct gcaccttgtg tacactcaaa ttcaatctga 34080

gcacacaatc atttaaacaa ccaaggcaac ttttccaatt ttcagcatca cgcgcttgag 34140

gaaacaagta tttcatcact aatttaatat tcattgagat aattgttctc aggttgatga 34200

aaaatgtggt gtatccattt actgttgtct tcttctgggt gaagaatgtt gcctttactc 34260

tctagctcac atttgaccag aaactggagt cccagcaaat cttagtagcc acttaggaag 34320

cctattttaa gatattccca atctcaaact tgttctaaga ctagtatgct tattgaggag 34380

aaagagcttc atgacaaaaa ttcaactaga gctgtgggtg acagttgtgt gtttcagtgc 34440

tggtctgggc ccaggtttcc catgagcacc taatgaatag gctcatttgc agagatcgtc 34500

aaggtgttca ctatgtctct ctcacagcct caaaagaagt gatcggctaa aagaatttgt 34560

gtagtatcct gaagggaaat tagtccagaa atagaggaca agataaggat tttataaaac 34620

agacatgtga ttcaacagtt gtttaagatg tagaaagctg caagagacag ttgttcccac 34680

tctaatgaca agaaaaagct gtatggtctg caaaatcata tgtgttttga tcccaccaga 34740

gagctgaagt tgcaaggccc ctgagtcagc tgaagtccaa agcatgagaa acccccctac 34800

agaaggacaa acagatgaag cagttcatct ttaacagata atggaaggaa aagatggaag 34860

ccataaatgt tggttggaga aaaatcactg aagttttcat catttcttta gttttttttt 34920

tttttttttt tgatacggag tctcactgtt gcccaggctg gagtgcagtg gcgcgatctt 34980

ggctcactgc aggctgcacc ccccggggtt cacgccattc tcctgcttca gcctccctag 35040

tagctgggac tacaggtacc cgccaccccg cccggctaat tttttgtatt tttagtagag 35100

acgcggtttc accgtgttag ccaggatggt ctcgatctcc tgacctcgtg aaccacccgc 35160

ctcagcctcc caaagtgctg ggattacagg cttcatcatt tcttaaaggc caagtgtgaa 35220

ctattgtgac agcttagaat ctctgagagt cccagacaca tagcctgttt tgcccattta 35280

ctgggtcttt cctcccacgt acttagtgct gataagacac atgtagcagg gaaagagaat 35340

ttctcgagct tgccacagtg gtgtaggcat ataagtcatg aatgcatgat agagcaccac 35400

cagagcacag gcacaaaacg tggttgcttc tctcacataa aacaaaaggc atctaccact 35460

gatgttggag ctagaaacct gtccaaccct tgcccccagg tataggccag ctgctgaggc 35520

agagttagac acaaaagctg tctatactgg acaaggggag gagacctgct cttgcccaag 35580

acttcccatc attgaaaggt agagttccac cactcccagg ggatgtcata ggaacactag 35640

gactcaggca cccatggcct gaggtttggt acacattcag tgctatcagt aatatgagtt 35700

ggtttcaatc gatcatttga gttggttgca gaccctttcc tttagtgagg aaaaactgtg 35760

atgtgggtac aaagtttcca cgtttaggaa agattggccc gtccttcagg atattatatt 35820

gattggattt ctctcaacct cctttagttg tgtttaccgt tattaaaatt cagtgacatt 35880

cacttggatg aggtggtaat aaaaacaagt tgtgagaatt tctagtgatt tttgatccca 35940

agccatgtat cactgttggg ccttcatgtg tgtacttgaa aacaaaacat gtacaattgt 36000

cgcactggtt tgaagatttt aatggtgaaa gcaacctaat cagtaatcag tactctatct 36060

agaaaccaat cttggaaata tgtgattatg ctcttttaaa atagctgaaa agaaggccgg 36120

gtgttctggc tcattcctac aatcccagca ctttgagggg ccgagttggg cagatcactg 36180

aaggccagga gttcaaggcc agaagcctgg ccaagatgat gaaaccctgt ctttactaaa 36240

aacacaaaaa tcagctgggg gtggtggtgc acacctgtaa ttccagttac tcaggaggct 36300

gaggcaggaa aatcccttga acatgggact cgaaggtgga ggttgcagtc agctgagatc 36360

ctgctactgt accccaacct aggtgacaga gggagattct gtctcaaaat aaataaataa 36420

ataaaatagc tgaaaagaaa tgactctgtg tttattctca ctttgttctt gttttgttgt 36480

atagtattta aataaaagca gattatttat cctcatactg aatttccaaa actgatattt 36540

gcatttaaca ttttgttaaa tgatggagag aaaatttaat tgtcttactt tgaaaagttt 36600

ggcataggtt ctgtcacatt tttgatgctt tcaatcgcag tctgtcatta gaatgctggc 36660

aactagttac atgcaaggag tatcctaacc actttaatac aatggtttga agtgctgcag 36720

gcagtaacta ctggacacca aatcacagtg tttttattgg tgacatgtta gagagaaata 36780

gaactttctg cagtacaaat gtcctattaa caaatccttg tgctattagc ttacaggact 36840

ttgactcctg ggtctgaaaa aggcacctac tcctgctaaa tcttgaatat tgacaccatt 36900

caatgcctca tcttcagacc ctgaggaaga cgacaatcca aatgaactgc tttcatgaga 36960

aacagaacaa gaaattgaaa caatttaatc tctctagacc caacaatccc acctgccccc 37020

ttttccacct catgccccct cttcctctgg acagaagaga tgggcacatg acaatggtaa 37080

ggccggagtc tgagggataa gtttagttca gagactttct tttctttctc ttttcttttc 37140

tttccttcct tccttccttt ttcctttctt tctttctttt tttttttttt ttttttgcat 37200

ttttggtaga cacaatgttt caccatattg cccaggctag actcaaactc ctaagttcaa 37260

gtgatccacc caccctggcc tctcaaattc agagattttc tataaattag acattacact 37320

caggagcaca ctgatgcaag gccagcaact ggccccgtgt atcagaataa cagggttttc 37380

tagagcgttc atctgatttt tcatagaaaa gtataaaagg tcatgaaagg tttttgaaac 37440

tcttacctta tggtcaaact aattgtaatt aaatttgtgt ataaggtttt attaaaatta 37500

tctttaacat tttaatacac cacacaaagg taaaatttag ttttctcttt tgatcaaaat 37560

gtttgtgtaa tattaataat agataaaata tttttgttta ccttttggct aaatgattga 37620

ctattataag gtgacctgtg accctatttt gtgatatcaa gtgtctcaaa actttgatat 37680

ctggcaaact ttctaagagt aaaatttcta aattcagccc tttggacctc aaattaactt 37740

tttggatatt aggttcttga agtccaagag agatatatta ggcttcttag gcttatttgt 37800

tatgtagaat tatgcatgaa gcattgtcaa atctgaggtg gtgtttcact tcctttgggt 37860

tctattgaca cagatgtgtt gttaatgtgt gttccaggat tgtatgagat tcctagagtt 37920

ctgatatgct gttggtaata attatgatta ttacgttaaa ttgttgtgtg ccacagaacc 37980

aaccaaattc ccttgtcaac tgtatcttta actatggctg tcttaaggct tttgtcatcc 38040

acaattgttg ttttgctttg attcttctca aaaagtgact tataatcagt cacagtccag 38100

gccttggttt tttggggaag tccatgaaaa ggactcttgg atgcaggttt ctgataactc 38160

tggagatcgt gccattaact aaagagaaaa tttccagggc actaacagaa aggctgatgt 38220

gttcatagag attgctagcc cagtatgaag caaagcagga gatgattgca tggattaaac 38280

taatggagga ctgaaataag tttttatggc ctttgtttgt ttgtttgttt gaaacatggt 38340

ttattcttca gagtctggag aacttttttc cttttgagct atttatagcc tgaaacaatt 38400

gagtagaata gtcttctaaa cagaatttga ggcatatttt tctctctctg ctgaatttct 38460

ccagaatttg taaactgaat attcttaatt catgacaatg tgtttatttg cataagttca 38520

ataagattct gttttctttt ataacctgac acaggtggag gaattggtaa ttttcccagg 38580

gctttgcctg aaatgtcatt gtgaaatgtt ccagcaaagc ccatttagga gagcctatgt 38640

gaacaatgat tcttgctgca ctttgtgtgg gtaatcaggc caagtatatg ggactgaagc 38700

ttcttttgca ggtaggttgg tcctgctgtg ttttatctta gtggaaatgg ggaactggag 38760

agaggaaggt tgtgtttcag gagaaaccat ggtattagat gaatgtttga ttcctgggtg 38820

gccatgcggt cacccatggt gtggagctgc ccaggatgcc ccttctccac atgacacagc 38880

cagaaagatg gatgaccaga ttccccatga ttgaggaact gacaaataga aagtggggac 38940

tgaaatcgat gcaatagtcc cacagacagt gtttttggat agatgtagaa atggaccctt 39000

ctagtcttaa agcttgaaac ttacatttgt tttatctgag ttcattcctc aggaaaggac 39060

cctcagcctc tcaaaaagca tcatagacct gaaactcagc agatcacagc atccagacaa 39120

tgagatgcca gacccctcac tcatcataag tgcttcctca cccctccagt tcctcttttc 39180

ctactcacag ttacgtttct tccctgctac ataaactcct cactttagtt gatcagggag 39240

attgatttga gactgatctc ccgtctcctc agctgcagca cacagttaaa gccttcttcc 39300

ttggcaatac tcaatgtctc aatgattggc tttctgtgag ctgagccgcg ggcctagacc 39360

aaacacctgg gctttcagta acaatacatg tacactttta aagcctgcaa ttaatgggtc 39420

atgagatatt gagaaattgg aatgacatca acttggccaa ttctgagaaa atacactgtg 39480

cttaaatgtc agggccccgc ctcctgacct tgacagtata tggttgattt tgagtcatag 39540

caggtgagta tggcctggtt tgagtgtttt atagaaatat ataatatggc tgattgcatt 39600

tattttaaat ccaaaaaatt tccatttctg gtgagcaaat tttgttaaaa ccaaccaaac 39660

aaacaaacat agaagtatat gaacaagaca atgaatgatt ccctgcctcc caatctcatt 39720

tgattgctgt cttctgttat atactacagg gaagaagggg gcaaggcacc caggatgtcc 39780

catgtcctgt tttgacaatc atctggcctc cttgggcaaa cagaaaagaa ggaaagcatc 39840

aagaaaataa cagaaatttt gcagaaattt catctccggt ctgggcgcag tggctcacac 39900

ctgtaatccc agcactttgg gaggccaagg tgggcaaatc acttgaggtc aagagttcaa 39960

taccagcctg gccaacatgg caaaacctca tctctactaa atatacaaaa tttaaccagg 40020

catggtggcc cacgcttgta atcccagcta ctggggatta caatcacttg aacccagcag 40080

atggaggtgg cagtgaggag agatcaccca ctgccactgc actgcactcc agcctgagca 40140

acagaaggaa actctgtctc aatggaaaga aagaaagaaa gaaagaaaga aagaaagaaa 40200

gaaagaaaga aagaaagaaa gaaaggaagg aaggaaggga aggaaggaag aaaagaaaga 40260

aagaaagaaa gaaagaaaga aagaaagaaa gaaagaaaga aagaaagaaa gaaggaagga 40320

aggaaagaaa gaaagggaga gagagagaga aaagaaaaga aaagaggaag aaacgaagaa 40380

aggaagaaag aaatttcacc taggcacagg aaactctatg acagaggctt caggggctct 40440

tctctggccc taaactcgca gtggattttt gcctcatttt ccgaggtgca aatgaaatga 40500

tagaagtcga tcaaaggaga gggcagagag aaagcaagaa ggatggctcc ttcctgcagg 40560

tccaccttcc tggagtgttg cttgtgggat aatagaaatg ccttgacatg ggtgggtggg 40620

aactattgct acttattagg aattcagacg cttctggcaa attcgcattg tgaggttcca 40680

tggtgtaatg gtaagcactc tggactctga atccagcgat ccgagttcga gtctcggtgg 40740

aacctttctg tttaattagg aaggcaatgc cgtgttttac tccctaaatg gaatggggat 40800

tctgctgacg ttcagaagct ctgcgctgcg ggcctccgtg tcccttctgc tcccgctgtc 40860

tgagcctcgg gatgcacgcc gctctcctca tcccctcttc ggtgttcggg gtcccagaaa 40920

ccgggttctc acagtggccc ggaccacagg gaaatcgccg aatcatccat tcggggtcaa 40980

agagcctgga ggatcccctt tcagggccag cctcctggca cccagcaatc ggaggcccgg 41040

actcgccccg ccagggaccc agcgacccag caagtcctca cccccctcca gggcgaccct 41100

aggcctcggg gagaggtttc cactaaaaag gctgccacct ctcgatccta gaccccgaga 41160

ttcctttcac ccgggaaatc tcttaatcgc atcctttgtc attttggtaa gtaaacggtg 41220

gcgtgagaaa ttttgatggg gttagctcag tcactggaga ttgtgtctca gtttaggatc 41280

ggtggttgga gacctaactt gggagtagca cttttttatt cgcatggccc atccaattcc 41340

gaatgttaat ttattctgtt cttcatattc agatttttat gccgaatttc tagctcctac 41400

gtctctattg cgggccctaa aagtcgctgt ttcactaagc tttggaacct gactccggga 41460

aatttcattc tttcctccca agatctcagc cggaaagaga gatccgcgcg ggctgggagc 41520

gcgggttctg ttcctcggcc gccccggctg aactcccgca ggaccacggg gaagaggagc 41580

ctgagcagct ctgaggggcc agtctctgac ggtggggctc cccctcgtcc cgtcaggacc 41640

ctgccccaag gagaagacgc cttagaacgc taccagagtt aaaaatgttt tctcctgcgc 41700

ttcagagcac ttgggatgag gttcctgatg ggtcttggga gtgaactaag atgtcactcc 41760

aggcgctcag gaccatcgct ctgattttca tattttgggg ctgagagcgg ttacgaagga 41820

ctgaaaaaca ccgcagtctt tgaggggctc agtttggagg acgagctggg ggaggggggc 41880

ggtgagaaga cgccttcttg gctccttgga gtggactggc aggtaaggaa aaataaactc 41940

cgtttcccct tgcaggctac actgcagggc ctgggatgga caattggaca gtcaaagagt 42000

aacaatattt atgtagaggt tggctgcatg cattaaaaat tgttatccct acagataagt 42060

gtaggaaggg accacggcac tgcggttcac caccagcttt gcagcatatg taactatgaa 42120

aacagtgtat agtgcataaa aaattcattc ctgttcacaa ataattgatc acaaccagtt 42180

acaggtttct ttgttccttc tccacttcca ctgcttcact tgactagcct tacaggaaaa 42240

aaaaaaatca tttgtaaata tgatattgtt tcagttgatc ctcagcccgc ccgttcccca 42300

ccccctgaaa gtagctggaa taacaggtgt gctaattttt gtatttttag tagagacagg 42360

gttacgccat tttggccagg cagctctcga acttaagaac tcaggtgatc cgcctgcctg 42420

ggcctccctg aacctctctg ctgggattac aggcatgagc caccccactt ggccatcatt 42480

tgtattttga atttaaatat tttgtagtca tcactcttca aagattctgg gttttgttgc 42540

atgtgcatga tgggattcag gacatgctac tccaaagtat aacatcttgg ggtattgaat 42600

attttatgct aaaggaatat gagtaaacca cagaagcagg aaggtcactg tcaccctccc 42660

cctgcccttc ttccctgaag tcgattgtaa gactcttatg tgagaggtgc cctctctgta 42720

cccagaggaa aggtgcatcc tggagtgtga agacacaggg acacagagaa gagcctgaac 42780

acagaggcct ggccacattt tccccagttt gttactgtta gatcatactt ttattgtctt 42840

atcatacttc tccatgacta tccattcttc aataaaacta ctgttaaaaa acaaataaac 42900

aaaaaactga ggtttagctg tttccatggg ccttcatttt ctttggaagg cgctcctgtc 42960

acataaaact tacattaaac aaatacatat gttcttctct tattagtctg tgttttgctg 43020

tgagggtctc agcagtgaac ctaggctggg tgaggaaaag tcatatttcc tccccgacat 43080

gtacaaatgt cttccactgt aaagctataa aaattttatt ggggcagatg tgaagcaaaa 43140

aattgaatca ttattcccct ttggatgagt tgaacagatg agctaataaa ctcccagttc 43200

cccccagata gaaatacttc tctagctcat tccccgaagc tccttgaggg tcaaacttgt 43260

tggagattga ggggccttcc tgggatctcc cagcccagga ggttgctgct ggtgagagga 43320

ttgctgcagt ctccctggga gggtgcatgg tcactggaaa gtatcactgc tataacttca 43380

atacaaccgg ttttcatcac atctgctctt tgtgcagtgt tacaggcaaa atacaagagc 43440

taacatgcac atgagtaaca catgcacatc atcaactgtg cctgcaaccc aaagtgctac 43500

atgtcacaca cagttgatga gtgtgcgtat gtttactcat ttagtcctta cctcaggcag 43560

aggtcacgcc atcaccttca tcttccatat gagaaaactg agatgcacaa agatgttaag 43620

tgacctgccc agggtcactc agccagcaaa ggaggatttg aaagtgggaa tttgaagtga 43680

aggcagcctg tctccagaag acattctcag aaccactgtg tttatactgc tcctaacaaa 43740

accagccttg caaaaattat gacagtgaga aaaacctgac ataggaaaac tatgactgtg 43800

aaagaaatct gacctaactg actgcatctt gcttctaacc tctgagctgc ccttgtccat 43860

tcctgggcat aggccaggct aactatgaga ggagtttagt tccaagtcta agtctgaaac 43920

agagatgatt gcccattcct gagacaaacc ccttcctttc ttggggatca gacctcctta 43980

gcaaagctaa cagatcaggt acgagattgg aaattatggc tcaggagcca tgcagccaga 44040

agccacatga ttcctaagct ccccaactgt gccttatata acagtattag tgtcaaacct 44100

aagatttgac agggtgttca aggtattttt cagaccctgc cttatgatgg actagctggc 44160

acacccagac cggtaaactg tctcatcttg tcctgtggcc ctcacccagg gactgactca 44220

gtgccataaa caagctctga cactgtgatt tcatccccaa cgcaaccaat cagctttgcc 44280

catttcctag ccccctgcct gccaaactat ccaaaaaaaa aaaaaaaaaa ccctagtctc 44340

cgaatttggg gggaggcaga tttgagtaat aataaacccc tgtcctggtc cctcagctgc 44400

ctttgcattt attaaatttt ttctctattg tagaaagcct gctgtgctca gctccattgg 44460

cttttctggg caaccagcaa gataaaccta gcaggcaatt acactaataa aagaaaaaca 44520

caaatggtaa gcatgcttga aaagaaatgt aagtctgtat atttcttttg aagaaacaca 44580

aaaggaggag gaatgataag ataggcaata cactgtccta aatattttca tgattctcct 44640

gcctcccgca tttccttctt atcccaataa ctgctcccca gaaaccttca acttaaacat 44700

ggtcctgcca gaactgggtt tgtccttccc tttacatttt cctctcacct gccccacacc 44760

ccttccatat ctaatctgtc ctttcacttc caagtcattg agtttttcca tttaaaaata 44820

tgaaagatac ggaaagaaga agataaccag ttatccatat tagcaaagta ctgttcacat 44880

atgtacatat ttttcttcca aacaatttta aagttactct catttaaaaa aaaattatac 44940

atgcttagca tagaccatac caccaataca gaaaaagtac agatacataa aatttctgat 45000

cacgttaagt tcaaaataat ttttgttaac attagggcaa cactcattga gtcatgttct 45060

tctggtatat ctacagagac agtgtatatt ttaggtagac tggcccccgc cccatgcacc 45120

cataaaagaa aaaatagaac aataaagaaa gctatatata tatatatata tatatatata 45180

tatatatatg agttatatat tttatatata tatatccaca tacatatatg cttttgtaaa 45240

attgagctta gactgtattt ttattttaca aatattccat ttaatagtgc atgaatttaa 45300

ctgaagacag agacagaaac acaaatgaag gaattcctga actctcaatc acatttagag 45360

agacattttc taaaggaatc ttctaaagct aaacgtacat taaattaagt agaaggcatt 45420

atgatttgca gtggttttcc ttttttttgt tttttgtttt gttttgtttt gttttgtttt 45480

gttttttgag accgagtctt ctctgtcgcc caggctggag tgcagtggca cgatctcggc 45540

tcactgcaac ctctgcctcc caggttcaag caattctcct gcctcagcct cccaagtagc 45600

tgggactaca ggtgtgcacc accacatcca gctaattttt gtatttttag tagagatggg 45660

gtttcactat gttggctagg atggtctcga tctcttgccc tcgcaatccg tctgcctcgg 45720

cctcccaaag agctgggatt acaggcgtag gccactgcac ccggccttct ttttcttttc 45780

ttttcttttt tttttttttt agcatggcat cactcactct gtcacccaga ctggagtgtg 45840

gtggcgccag ctcagcttac tgcaacttcc acctcccagg ttcaagagat tctcctgcct 45900

tagcctccca agtagctggg attacaggcg cccgccacca cgcccagcta attttttttt 45960

ttgtattttt agtagagatg aggtttcacc atgttggccc gctggtttca aactccttac 46020

ctcaggtgat ccgcctgcct cagcctccca gagtgctagg attacaggcg tgagccaccg 46080

cacccagcct gcagcctgta gtggttttct tattactttt tacaatcaaa ttgattaagg 46140

cattggttga atatggtaca atgcatccat ttcaagtttg atgacatgtc tattactcca 46200

aaatgtcctt ttgtaccctt tgtagtcagt cccctcgact ccctgaaccc agcaatcaat 46260

gatgtagttt ctgtcactat acaggttagt ttcacctgtt ctagaaattc agcttgatgg 46320

cgccacagag gatgcactct tttcatcact cagcatgagg tttttgagat tcaccaatgc 46380

tatctcatgt ttccataatt tgtcccatct tcccctggaa gaatatatca caataggtcc 46440

attcacctct tgatgcatat gaacggtttc cagtttcggg actattgtaa acaaagctgc 46500

taagaacaag ttctttttgt gaatatatgt tttgtaaatt ccatttgtca ttctttagta 46560

ttaacttatt ttgtaaaagg atgtcacctt cacaagtgca aaattcattc aaatgttttt 46620

tgaaaaaagt tgaggcctga agcagcagtc tacacaattt cctttcttat atttcataat 46680

taaggggaag aaactttatc tgggatatta tttcctatca gcaaatcctt aatgtaagcc 46740

acacacgatg ctagctgtac tattagacta tcttctgtaa tctgtgcaat aactccaaga 46800

agtacttcat attattattt tcctgtaaca ctttccaact tgaagcctag caaattgaag 46860

gacctggccc aaggtcaaac agaaggcaag gggcagcgcc aggactggaa gaaggccttc 46920

ccaaaggcag acagaagctc atgtctttgt cagaacctcg gtatttctga tgcgcctgga 46980

agccaatatc caagccgttc tcagccaccg gattggtggg tccttttgct cagaaactcc 47040

tcgatgagga atcccaagca tacgttagtg atgacggtcc cgaaacaagg gacatttctt 47100

aaacattcta aaaacccttt tgctcatcgg taacgcaatg aaagttgttt tcggtcttgc 47160

ttcatatatt ttatatttga gcaaggcaat gtccgctgtt ggggttagaa gtaaatggct 47220

cgtcttcatc aaactagatc agtccgtaag aagtacggct tccactcgca gtgtcctgaa 47280

cactcgctgg gcgtcctgtg cgttctagcc ggttttcttt ggagccgcgg gtttctgcac 47340

cgcgcccggg ttgggatggc agaggcttct cgggacgtgc ggatctatga gtgccgtccc 47400

cagggtagcc accaggggac gccataggcc ttccttccgt ggtcctcttt gcctttttcg 47460

tttgggccag gtagtttcta ttgaccagga cattagagat cagaagtggg tggggtcaga 47520

aaacacaccc tgggagaggc tggcgaaatg cccagaacct ccatcactag gcctggggtt 47580

ttcttctgta gtggaattct tgtgctcatg tagtgcaggg acaaaaccaa ttagatactt 47640

ctgggagtta aaaagagatg aatttacaat gccatttgag aaggggtatt aaggaaacgt 47700

ccgaatttgc cagggtactg atgcgtgtct ggtgcaaaac gcaggttgag agagagctaa 47760

gtacttcctg tccatgaagg tgataagcga gggcctgaag agggacgggg gaggacactg 47820

gcgccagaga tagaaagagg cggcttgggg gtgggaagca tgggtcgggg tgctatttag 47880

aaagttgcct ggcaatggat gtgggatgtg gaagcgagac atcaaacaag aagctgtcac 47940

ttaaatgaaa tctgaagaaa gactagatat gtaaacggca ggagatagtg gatccggctc 48000

ctcataaaat ttcccgcttt atatttcagg gaggatcgca gcgcattttc gccaagacag 48060

gtgagactgc ggttctgacc tgcgtgtctc gggatgaatt gcgttagggc acctgggcgc 48120

cggtagaccc cttcccctcc gcaaagtaag cgtgttatgt ctacaaccca acgggaacac 48180

taagagcccc aaaggccctg atttcttccc aaagaacagc gcccgtctgc gtagtttgta 48240

cctggctcta ccaagtgaga acacattccc cgctagcaca gaaatcctac aaactcctga 48300

gggggctgcg cttagaagca gaggctgtgt aaaaggtgat tgggggctag ggaaaaacaa 48360

gaagattttt acagagcgtg agaccccaag agaatggaga ctatggacca atctctgcaa 48420

aaagcagcct catgttgaaa ggaaggagct gtgcggcctt cgcgctactt agcttgtgtt 48480

aaacaggaac acaacgcctg acgcttattt ctccggcagg aacgacggcg acatgttact 48540

tcctgtttcc ctgccatccg gctataagaa taacacacca ccaccaagat tatacagcaa 48600

aaaaaagcga gaagtagacc tttgcatacc aactgctttt tgcactggaa aattcagggg 48660

aaagcgcgaa cgcagtcccc cactaccaca aattacgcag tcgagtttcc cacagttggg 48720

gaaatcgcag aggtcaacac atccggagtg caatggataa gcctcgctct ggaaaaacca 48780

ctttcatgat catactatct cccctgccag gtaagtgtga gttcacacat ctccgccctg 48840

cgacagctcc atacgcctca acctttacag acacggtcac ttgccccgcg cacccgcacc 48900

cccaccccca ccccgcccca tgccacccgg agccctccta gccctgacac acagctggga 48960

ctctcacgtc caaccagcgg tcctggactt gctcccaaag caggagaaat ccttcgtggg 49020

aagcagcagc ccctaagctg cctcatctac gtaggaatcg ccctatccgt gatgtcaccg 49080

acagcgcctt tcccggtccc catctgctct tccgcccgac cctccgccac tcagccgacc 49140

aacccgctgc gggagccggc ggaggaagtg aagtctacct ctatctcctt tccctccctt 49200

ccccacccct ggtctctccc aaagaagtag gttcttagcc tgtgatgcca aggacccctt 49260

tggcggccag ctggagcctg ggcgctcttc tttgagtaat ggcttttaat gcgcaaacta 49320

gaaagtttag gattacaaag aaaaccggtt cttttcacat acggttatcc ttgtgatgta 49380

ccattccgct tgacagtgga agtcgttcaa tatcagagag aaacagtatc tatgaaacta 49440

gagaggctgc tcagatgact gcaaaccagc catccttact tgttttatca ctaggaatat 49500

tataaagacg gctgtccagt ttcatcaatc ttgtagggtt tttttcaaat acagcaatgt 49560

acaaaaatgt gctgccccag cagagcacac tggacactca ggcatggtgc tggagtcgtt 49620

ttgccatctc ttcactgcct tctctagacc ttagcgctta cctcatgtta acactttata 49680

tgctgcaaag acagaaacaa agtcatccaa atgtggcaaa aatattttgg ggaaatttta 49740

tttcaggtat ttaactgatt acttttaata tgtaggcaac aacaccaaac tctgacaata 49800

ctccgcagac ttattaatgg tttccacccc cgtctctgca aatctatcag gcaagttttt 49860

cccgcccttt actttcatat aaactgagtc atacactgtg tagtgagttt tggggggagt 49920

gttactgccc aacaggttca ccttgcctgc tgcctagaca gagccaattt tttgacacag 49980

gggaattgca ataaagagtt atttaaacag agctggctgt gtgggagacc agacttttat 50040

ttttactcaa atcagcctcc tcaagcattc cgggaccagt ttttaaggat aatttggtgg 50100

atgggggcca gtgagttggg agttctgatt ggtcaggtca gagatgaaat cacagggagc 50160

tgaagctgtc gtcttgtcct gggtcagttc atgtgtgggg gacacaagac cagatgagcc 50220

aggttatcag tctagctggt gccagctgat ccactgagtg cagggtcggc aaaatacctc 50280

aagcactgat cttaggtttt ccaataatga tgtgatctcc aggaacaatt tggggaggtt 50340

tagaatcttg cagccagagg ctacatgact cctaaaccat aatttctaat ctcgtggcta 50400

atttgttagt cctgcaaatg cagtctagtc cccaggcagg aaaggggata ttatcatctt 50460

tgtttcacag ctaaaccata aactaagttc ctcccaaagt tattttggtc tgagcccagg 50520

aatgaacaag gacagcttgg atgttagaaa caagatggaa tcagatcaga tctctttcac 50580

cgtgataatt gtcccagttc taatttttca aaggcagttt cagaagcaga ggcataggtt 50640

tgacttcttt cactcagtct tatgtctatg aatttttcac cttattcata tggttatttg 50700

tttctgtgtg tcttactttg tatgaatata cattaactta tttattcatt tgactgttgt 50760

tgaacatgtt gacctaaaag aaagaggctg aagcacaaaa cataatatag agtttacttg 50820

agccaacatg aagacagctg cctggaagac tcacacccaa ggaaccttgg atctgagctc 50880

cctttggctt ttgtcacaag caggttttta aggtcaaaag cagggacaga cagtaggctg 50940

atagaaaggt gcctgtcagg aattctcatt ggtttacaga aataacattg attggtgatt 51000

ggctttatgt tgttaagtta taggctatgg ggtatagtgt ttggtgtggc attattagga 51060

taatttacag cgacttgagg cgatatcaag cagtttcaac agatgaatac atagctcaaa 51120

aggaaggaat ggaaaggggt ttctctctcg atttaacatc tctctgggcc tgatcatttg 51180

aaagggctca cattcctcag ataaaaattt ttttaagtcc tcctctccct ctccctcgtc 51240

tccctctccc tcctctccct ctccccacgg tctccctctc cctctctttc cacggtctcc 51300

ccctgatgcc gagccaaagc tggactgtac tgctgccatc tcggctcact gcaacctccc 51360

tgcctgattc tcctgcctca gcctgctgag tgcctgcgat tgcaggcgcg cgccgccacg 51420

cctgactggt tttcgtattt ttttggtgga gacggggttt cgctgtgttg gccgggctgg 51480

tctccagctc ctaaccacga gtgatccgcc agcctcggcc tcccgaggtg ccgggatggc 51540

agacggagtc gcgttcactc agtgctcaat ggtgcccagg ctggagtgca gtggcgtgat 51600

ctcggctcgc tacaacctcc acttcccagc tgcctgcctt ggccccgcaa agtgccgaga 51660

ttgcagcctc tgcccggccg ccaccccgtc tgggaagtga ggagcgtctc tgcctggccg 51720

cccatcgtct gggatgtgag gagcctctct gcctggctgc ccagtctgga aagtgaggag 51780

tgtctctgcc cggccgccat cccatctagg aagtgaggag cgtctctgcc aggccgccca 51840

tcttctgcga tgtggggagc gcctctgccc tgccgccccg tctgggatgt gaggagcgtc 51900

tctgcccggc cgccccgtct gagaagtgag gagaccctct gcctggcaac cgccccgtct 51960

gagaagtgag gagcccctcc gcccagcagc cacaccctct gagaagtgag gagcccctcc 52020

gcccggcagc cactccgtct gggaagtgag gagcgtctct gcctggcagc caccccgtct 52080

gggagggagg tgggggtcag ccccccgccc ggccagccgc cccgtccgga agggaggtgg 52140

gggggttagc ccccctcccg gccagccgcc ccatccggga gggagtttgg ggggtcatcc 52200

ctctgcccgg ccagctgccc gtccgggagg gaggtggggg ggtcagcccc ccacccggcc 52260

agccgccccg tccgggaggt gaggggcgcc tctgcccggc cgcccctact gggaagtgag 52320

gagcccctct gcccggccag ccgccccgtc caggaaggat gtgggggggt cagccccggg 52380

cccggccagc cgccccatcc gggaggtgag gggcgcctat gcctggccgc ccctactggg 52440

acgtgaggag cccctctgcc cggccagccg ccccgtccag gagggaggtg ggggggtcag 52500

accccgcccg gccagccgcc ccatccggga ggtgaggggc gcctctgccc ggccgcccct 52560

actgggaaga gaggagcccc tctgcccggc cagccgcccc atccgggagg gaggtgggag 52620

ggtcagcccc ccacccggcc agccgcccca tccgggaggg aggtggggag gtcagccccc 52680

cgcccggcca gccgccctgt ccgggaggga ggcgggggtg tcagcccccc gcccggccag 52740

ccgccccgtc cgggaggtga ggggcgcctc tgcccggccg cccctactgg gaattgagga 52800

gcccctctgc ccggccacca ccccgtctgg gaggtgtact caacagctca ttgagaacgg 52860

gccatgatga caatggccgt tttgtggaat agaaaggggg gtaaggtggg gaaaagattg 52920

agaaatcgga tggttgccat gtctgtgtag aaagaggtag acatgggaga cttttcattt 52980

tgttctgtac taagaaaaat tcttctgcct tgggatcctg ttgatctgtg accttacccc 53040

caaccctgtg ctctctgaaa catgtgctgt gtccactcag ggttgaatgg attaagggtg 53100

gtgcaagatg tgctttgtta aacagatgct tgaaggcagc atgctcgtta agagtcatca 53160

ccactcccta atctcaagta cccagggaca caaacactgc ggaaggccgc agggtcctct 53220

gcctaggaaa accagagacc tttgttcact tatctgctga ccttccctcc actattgtcc 53280

tgtgaccctg ccaaatcccc ctctgcgaga aacacccaag aatgatcaat aaaaaaaaaa 53340

aaaaaaaaaa attttaaaaa tctttcttct tgaaagcatt gatgatcaaa agctcagtca 53400

aggtgtccct ctttgccaga aaggctcatt cccagatagt cctatcccag gcaggggaca 53460

gggaaggaaa ggagccacct caccaaggaa tttttaagaa tgaccacaag tccaattgaa 53520

agggcattac agccagacac ggtgactcac acctgtaatc ccagaacttc aggaggccaa 53580

ggtgggtgga ttgcttgagt cgaggagttc gagaccagcc tgggcaatat ggcgaaaccc 53640

catctccaca aaaaatacac aaattagcca ggtgtagtgg cacacacctt agtcccagct 53700

actcaggagg ctgaggtggg aggattgctt gagcctggaa gttcaaggct gcactgagct 53760

aagatcacac cactgcactc cagcccaggt gacagagtga gactcagtct caataaaaaa 53820

aaaaaaaaaa aaaaaaacag aaagaaaaga aagaaaaagg gcatcataga atggcaaaaa 53880

aaaaaaaaaa agagagagag agagagagag aggcctcagt taagtctata gctgttgtca 53940

cctgttgaat catctctagt cttcagaata ccatgaaatt agttttctca gaagtaaaac 54000

gatgagaaat acataacaat aatgacattg cacttcatca ttccatgctc ttagacattt 54060
atagggccat ggatggtgat cttttcaaga gacaaaaata aatgctggtg cagaacaggt 54120
aagtgcgcta taatttgagt acatcagctt ttgagcattt aaatcatgca tcagaaattt 54180
agagcaagag ggcaggttgg atcctgatag caggagtgtt tttcttttaa ctttccagta 54240
gcatcatggt gtttaccact gtcatttcag agtgacgtga caattttttg tttgattttg 54300
tactttttat ttttattata attacttttt gagacaggga ctcactctgt ctcccaggct 54360
ggagttcagt gcaagatcac agctcagtac agccttgacc tcttgacctc caaggctcaa 54420
tggctcagcc tcctaagcag ctgggattac aggcacacac caccacaccc agcttattta 54480
tttatttttt gagacagggt ctcactctgt tgtccaggct ggagtgcagt ggcatcatca 54540
gggttcactg caggcttgaa ctcctgagct caagtcatcc tctcacctca tgctcctcag 54600
tagctggact acaggtgggc accaccacac ccagctaatt tgtatatatt ttacagagtc 54660
agtgtttcac cattttgccc aggctagtct caaacccctg ggctccagtg atcctcctgc 54720
cttggcctcc cagagtgctg ggattacagg ccgttatttt gacaatacga ttatgatggg 54780
ttaaactgag atgtgaggca ggaaggttag ggtgagtcac atagattcga taagcaggtt 54840
ttgcttgata tgaccctgag ttgcatcgtg agtgcaatca ggaggaatac aggtgaaatt 54900
tagcacagga tgtaccaacg ggccattatg gccaaaagtg gttggtggtt taaggtggca 54960
aacccagcac cttgttaaat ttcccacagt gcaatggttt gtgagattct gatcagagaa 55020
ttatccctcc ctgctataat caaattaatt agggacagtg aaacagaaat ctgatcatag 55080
gcattttgct atgtaggtat gtatatgtat atacacatac acgcaatagg actagtgtgt 55140
catatcttaa gagaatacac atataggatg attttaccaa aaccaccaag agaggacccc 55200
tacaacttaa gggagcccta gtggtgtatt attccagtca acagaaaaag agactcaggt 55260
gcagtgagca ctggaactgt attgttgata caggccagta tcttgggcaa aagcggacta 55320
caacagatgt cagctacttg ccatctcggt caggaagact gagttacctt taagtgaaaa 55380
tctccactaa cagatgtcca ctctggcagt gctgcctttt acaaaagaaa catgaatccg 55440
tgtgtcaacg cccttaagtt tactggcaca gattgttcag taatacctgt tgtgagttgg 55500
gtacattggc ccatgcctgt ggtcccaact atttggaaga ttgaggcaga aggatggctt 55560
aagcccaaga gttcaaatct acagtgagtc atgattgtgc cactgcattc tggcttgggg 55620
aacagagtga gatcccatct caaaaaaata acataaaata ataatacctg atgcggtccc 55680
ttctaacagt cttgggagga gtattttatt tgatgttgtt tccagtaaac aacaacatct 55740
ccaggttata ggccaagatt tttgatattt tcatctccca agaattcatt atgaaaggaa 55800
tcctgtgtta atgtagagtt tttagtaagt tttgtgagat cttagcagta atggagaatg 55860
tcacctttga gaagtgcata ttcataagtg tcttcaccta accactgcat gggtcttcct 55920

gttattattt caaagagaga gagtgtggac attttccaga actagtggga gatccttggc 55980

caagaaaggt taaaaacttc tgttagcttt gcctatggag tttttattgt atcatcagtg 56040

cattccatca gcccagagga ttaggagtga taggcacaat ggaattgttg ggacatagac 56100

aaaactgtac aaatggatcc aattacctga ccgatgaagt gtgttccttg gttgctatgg 56160

aattcagatg gcactctcca tgaagaatta ccttttctaa tcacaatcta ctaacctact 56220

acttgggctg tggctcatcg gcatggaaat gctcacccca gtgagcacat acaaattagc 56280

accagaaggt atctgtaacc ctcagatgga gacagctgga taaagtctaa gtgctatgtc 56340

tcaaagggtc ctaaaggtaa aatgttcctg tgacccatga agtggttttc cagggttatg 56400

tttgggacag acagtatagc aaaaataaac cttttctact atcataggca atgattttca 56460

ataccattgc tttccccatg ctaacatttt gtctgaattc caataagtca gcttatgaag 56520

tactgtaaaa agggtgactg aagcctaaag ggtggatcag tgtatcattg ggcccatacc 56580

atatgcctgt ttctggaaga aacttgtttc ctttatcctt ccagagatcc ctctcctgct 56640

tgggagctcc cacttatgct tcttcttctg tttttttttt tttttttttt ttttgagcca 56700

gagtctcggt ctgtcaccca ggctgtagtg cagtggtgca atctctgctc actgcaacct 56760

ctgcctccca ggttcaagtg attctcatgc ctctgcctcc caagtagcag ggattatagg 56820

catgtgtcac cacaccggct aatttttgta tttttagtag agacagggtt tcaccatgtt 56880

ggccaggctg gtctcaaact cctgacctca ggtgatctgc ccgcctcggc ctcccaaagt 56940

gctgggatta caggcatgag ccaccatgct cagcccactt gtacttctaa agaagtcttc 57000

aatgggtcat aagtcagcaa ggtcattttg agaagttctg tgtctgatgt caccttcaga 57060

gctgcattct tagctgcagc gtcagcaaaa tggttgccct tgctctccgg aatatctaat 57120

tttgaatgac caggaactcc tttgatgatg gccaaagatt caggttttca aattgcttct 57180

aagaggtcag agacttactg gtcatttttc atggcttgaa ctgatgaaat cagaaaccta 57240

tgttgcttct aaagcattcc aaaatcatca cctactccaa atgcacctct gctgccagtg 57300

taaatgttag gctgaggtga gaggattgac tgagcccagg agttcattta ggtccagcct 57360

ggaaaacaca gcgagacctc atctctagaa aaaaaacaaa gaattagctg ggcattgtgg 57420

cacgcacctc agctacttgg gaggctgagg tgggaggatt gctagagctg agagattgag 57480

gctgtggtgg gccatgatgg agccactgca ctctagcctg ggtgacagag tgagaatctg 57540

tcacaaacaa tcaataaata aatctcaacc gtttaaaata tcaatgacag atttgcttgg 57600

ccttttaagt gcattgctga attctccaat ctacattttt aggaacaacc tggggaatgg 57660

caacatggag gtatttggcc aaaatgcaga ccctttcatt gtctgcttca gtctgttttt 57720

aagaatcctc taaaggatta ttccaatttg cccttttcaa ccatttaatg gccctgcctt 57780

cttaccccag catgtgaatt aattgataaa gatcagaatg tccaggctca aaagtttggg 57840
caattaagtc aaattacctc ccaaacacta taggatcttg gaggctgctt tagaattaga 57900
aggggaaaat gtatttaaat ttcaatcagg gaaatctttt tttttttttt ttttttttga 57960
tatagagtcc tgctctgtca cccaggctgg agtgcaatgg tgcaatcttg gctcactgca 58020
acctccattt cccaagttca agtgattctc ctgcctcagc atcctgagta gctgggatta 58080
cagtcatgtg ccaccatgac cggctaattt ttgtattttt agtatagatg gggtttcacc 58140
atgttgtcca ggctggtctt gaactcctga actcatgatc tgcccaccta gtcctcccaa 58200
actgctggga taacaggcgt gagccactgc acccggccca gggaagtctt ttatgatatt 58260
cttaattcac atttggtgaa agcagtatac agaatggtag gctcccttct tcctgtggat 58320
tttaccttga aaggggctgt cagaacagaa gtttcaggga agggaccaga gccctgggga 58380
ctttccttag gtgatggtga tggaagaaga ggcaaggcag gaaaggaggg ctcaggtaag 58440
ggagactatg caggtacagg ggcaggagaa gaaggagcag ttgggggcga aagagacaaa 58500
gcctcctcaa tatttctcag ttcagaaaac atgtcagttt agctccttca gcttacttcc 58560
tcactggaga taattttctc agttctttta gaaatttcta ggtataattg gaagtaagtc 58620
tcccatttaa tttggttcta aaaccaaatt tttccaattg tgcacactaa taaattattt 58680
taagcatttc aaaatatccc cattttcttc attctgctta tgatttttcc tggttaggtg 58740
ggtccagttt cttggatatt tacaagagaa tgcctcataa gtgttataca taaacccagc 58800
tgatgtttct aaaggtggtt gtttcttcgc agtgtgctaa attttgaagc ttgatttccc 58860
ataattcagg agcttttcag atgaccaagg cctgcgatgt gttttgggtc acagtgtggt 58920
gcttatgaag tcactcctac agataacacc ggagttgtgt attgctgtct cagtggttcc 58980
cactgtttct aaccaccagg tggccacaga gtgcatgtgc tatgagggac ccatcccaca 59040
tatgccctcc cagaagagca ggaaaggggg ttcataaaga tgttcttctg atagggagtg 59100
ccctatgagg ccaccttcac atggtgagta atgaccccga cacctctgca acatccccga 59160
tcacttggag cacctgaatg gatcagcggg agcagatcac ttcttttctt cagggcaagg 59220
gaacacacct ccgtgcactt tccagttaag aacaggaact agtcacaaat gaaaagctaa 59280
gtccgaatgt tcaaaacaac atgctacata aaacgaaacc accagagcat acctgccaat 59340
accttggccc agatcttcct gctgaggaac agaggcagaa aaggcagaag ctctctgggg 59400
agctctttgc ctgactgtga cccaaacttt ctgtcctgac gcagaggtgg aaaaggcagt 59460
tatttccccc tgaaactcaa atctcttact cacagggtaa agacagaaaa cctccgttct 59520
agaaagggag agctggaaag gacactcaag caaactccag gccttcaacc aaagagtggg 59580
aaggtccaga tttcaggagg attcaccact tactactgcc caactctttc tctcagagtc 59640
agaactcaag ggcttcagat ctggccaggc acccagtcca ggaggaatgt ggtgtgatct 59700

gagagatgaa aatacatgcc cccttagaga ctaaggcaga ctcgaaagtt aaggaaacaa 59760

aagttagcta cttgtccagg gttcagggct catctggcat ggcaacttcc tgaattccta 59820

tggctacagg aaaaaccaca ctcttgctaa actccttaac aatagaacta gcaagcaaat 59880

tgtcaaaacc ttcccagccc ttctcaactg gatttacaac ccagatcact agacctctga 59940

tgggacagaa gagtggcctt ccattctctc ctgataagca actgcggata tcaagccagt 60000

ttcagccatg ttacagggac tgcacacaaa ccgacttggt gtccagcagt tcaccttttg 60060

acataaaaag ccaatttcca ccttatttta atgctaaaat cccttcccaa tgtgaagatg 60120

gggtgcatgt tacatatatg tttacccttt gcacatgagc tcaatacccc tcagaaatgg 60180

cgtttctcca aacctgctga atatgtataa ctccattgtg tgatacacac cctgtgaggc 60240

actaaaacca aactgccctc tctctctctc tgaagagaaa gcaccttttc aaagacacac 60300

accagagact gtctcttcct ggactgcaaa ctgatctcac caatgaaatt cttactacta 60360

tgtagccacc ctggaggcct ttgagaggat agtcagatgg tcagagatga gttgctctga 60420

atcctgctcc cagggccaaa aacgtcaacc tagaaggaag gggcttaaac acaaaatatg 60480

atttaaagag tttatttgag ccaatgtgag gatagttgcc tggaaggctg agacccaagc 60540

aatcttggat atgggttctg tttggccttt gttacaagca ggtttgtaaa gaccaaaaag 60600

ggagacaggg agttggctgg tagaaagttg cttatcagga actcccactg gtttacagaa 60660

ataacattga ttagggatta gctatacatt gttcagctac agggagtgag agatggtgtc 60720

cagtgcacgt gattaggtta ctttatagct aactgtggca atagcaagca gtttcagtgg 60780

atgaatacat agctcaaaag tagggaagtg gccagtggca ctggcccatg cctataattc 60840

caacactttg ggaggccgag gtgggcagat cacctgaggt taggagtttg agaccagcct 60900

ggccaacatg atgaaacact gtctctacta aatatacaga aaaaaaaaaa aaaaaaatag 60960

gcaggcattg tggttcatgc ctttagtcgc agcaactcag gaggctgtgg caggagaatc 61020

acttcaaccc gactggagga ggttgcagtg ggccaaaatc ctgtcactgc actccagcct 61080

gggcaacaga gtgagattcc atctcaatac aataaattat tttttaaaaa aatttaaaaa 61140

gcagggaagt aggatgtggt tggtgtttca ttttaatgcc tagctctagg catggtaact 61200

tgaaagcgcc cattttcctc acatgaaaaa gttcttttct ttcttttctt ttcttttttt 61260

tttttttttt ttggagacac actctcactt actgtgccac ccatgctaaa gtgaagtggc 61320

atgatctctg ttcactgcaa cctccacctc cagggatcaa gcgattctca tgcctcagct 61380

tcctgagtag ctgggattag aggtgccagc caccatgcct ggctaatttt tgtattgtag 61440

tagagatggg gtttcaccat gttggccagg ctggtctcga actcctgacc tcagatgatc 61500

cacccgcctc agcctcccaa agtgctggga ttacaggtgt gagccaccat gcccagccaa 61560

gaccattaat ttaatctaca gctaaatcta ttatattttc atgttacaaa ttcagcaaga 61620

aaagttttcc ctaatgaaac atcacattta aagcataagt agttaaaaaa aaaaaaaaaa 61680

gtaagagaca gggtgttgct gtcatccagg ggagagtgca ctggtgcaac cacagctgac 61740

aaacctggaa attctgggct caggctgtcc tcccagctca gcctgaattt taacttttta 61800

gatgtgacat cttgtcctgt tgcccgggct ggtctccaat tccttgccta aagcaatcct 61860

cccacctcag cctcctgatt tgctgggatt acaggtgtga gtcaaccagc ctggcattgc 61920

aaaaaaaaaa aaaaaaaaaa gtcaagaaat tattcttatt ctttatttta ttttattttg 61980

agttgaggtc ttactctgtc acccaggcta gagtgcagtg gtacaatgat agttcactgc 62040

agcctggatc tcctgggctc aagtgatcct cctgcctcag cctcccaagc atctgggact 62100

acagacgtga gccactgtgc tgactagttt ttcttttaat cagctcattt tttaaaattt 62160

gtagagacag gcgtctcacc atgttgccca ggctggtatc gaactcctgg tttcaatcct 62220

tcttccctcc tcagctgcct aaagtgctaa gactacaggt gtgagcacca cgccctgctt 62280

aattttcata ttttaatttt atatacgtaa ttattactgt ccttaagata actggggcag 62340

taatctcctt aaagttttag agacttaatt tatctgttca ctccactgga agagtatgcc 62400

aattggtttc ataagaagat ttatttataa atcagaaaaa ttctttccac caaactaggg 62460

gtcattcaaa agcaaataat tgccttaagt aacatcattt aaggtgaaaa cagatgcagt 62520

ggtatctatt aataatgctt ctcagtgtgg gaaacaaact gaaaaaatga acatcattag 62580

atccttggaa aaccctcact gctgaaaatc tgagtaattc tgtgataccc ttttgagtct 62640

tccaggacat tctctttccg gagcacataa aagtgagtaa aatcattttc atccattttc 62700

attaagagct gaacttcctt gatgttctgg agattattaa atttgatttg tggccaggcg 62760

cagtggctta tgcatgtaat cctagcactt tgggaggcca aggtgggcag atcacttgag 62820

ttcaggagtt tgagaccagc ctggccaaca tggcaaaaac ccatctctac taaaagtaca 62880

aaaattagcc aggcatggtg ttacatgtct gtaatcccag ctactcagga ggctgatgca 62940

caagagtcac ttgaacccgg gaggccaagg ctgcagtgag ccaagatcgt gccactgcac 63000

tccagccttg gtgacagagc gcgactctgt cttaagcaaa aaaaagtttt tatttgtata 63060

gttgtgagaa gtgcttatat tgctgactcc attgcttatt tgtgatcata taaatctctt 63120

ttcttctttc tctagtgtga tttaaactta atccttaaag aacatgtatt tcagctggtt 63180

agagtttttt agctagtaga aacctgagta tatgaatcaa agaaaactgc tccttacatg 63240

ccacagactt agttttcttt ctttctttct ttctttcttt ctttctttct ttctttcttt 63300

ctttctttct ttctttcttt ctgtactaag atttatttta ggtatagtaa tttgttaaag 63360

ccaagagctc ctatggaatg aagttagatg ggaggggggc attgagtagt aaggtcatcc 63420

ttataataga gatgccactc ttgcagatat tgacagctat tgggcccaaa aaattgtaac 63480

caaatgttgg aaagacaaga tatgagcaac ttctgattgc tgcagtctca aatatcaaga 63540

aataccgtta tcctcaggac aatgaataca caatcaaaaa agactcacag actagattag 63600

ctgtcatgta tcatcaaaca gggactggat ccttgtcaaa caccacccaa cagagattgt 63660

ccccagaaat tcacttcata acatcaaaac cagaaaccca cattgatgcc actgatggca 63720

gaaatcagtg atgcaaaaat gagagagaga gaaagagaga gagatagagg aaggatttgt 63780

ccaattacaa tgcttagtat ataaaagcaa atgagatcaa tttttgctca tgaacacaga 63840

agaactgagg gaacaagagc caatagctct aaggatccaa gtctgcacca ggggcctgca 63900

ggattctgtt ggctcccatg atgggtctca gatgggctaa tttttatgga catagcctct 63960

gaaaaaaatt agtcaagaaa taatatacag acacttatct gcatgcccat tctccattaa 64020

tatagaaggg aaactggcat tagatggttt tcatcaaata aaagtaaaat caagatttat 64080

aaataaaaga ggaaagagga gaaaaaaatg tctgggacca ttgtgatttt gctccctttg 64140

gataagtgac ttggcagaag gctgtcaaag aggtcactgt gacctgtctt ctctggacca 64200

atgtgacttg gtcaccacag gaaacaatta caggctgggc acagtggctt atgcctataa 64260

taccaacact ttgggaggct gaggtgggcg gaccacttga gctcacgagt tggagagcag 64320

cctgggcaac atgatgaaac tctgtcttgg agagcagcct gggcaacatg atgaaactct 64380

gtctgtacaa aaaatacaaa atttagccgg gcatggtggt ccatgctagt agtcccagct 64440

actcaggagg ctgaggtggg aggatcccct gagcccaggg aggtcgaggc tgcagtgagc 64500

catgattaca ccactgcact ccagcctggg tgacagagta agaccatgtc tcaaaataca 64560

taaattaaat ttgaaaaaga aacaaggcca ggcacagtgg ctcatgcata taaccccagt 64620

actctgggag gccaagatgg gagtatcact tgagctcagg agttcgagac cagcctgggc 64680

aacatagtga gacctcatct ctaaaaacta catacatata tatattaata aatatgtata 64740

ataaaatata atatataatc aaatatatac atattataca taatatataa ttgtatatat 64800

ttactttcag ataattataa taaaatatgt atatatttta ttatatatac aataaaataa 64860

aaaagaaaca attacagatg ttggaggagt ttaaaatatc tcagccaggt gcagtggttc 64920

aggctggtaa tcccagcact ttgggagact gaggcaagag gatcacttga gcccaggagg 64980

ctgaggctta cagtaaacta tgaccatacc actgcactgc aacctgggca acagaaaaag 65040

accctatctc aaaaagtaaa atgtcttaga agtttagctg tccatcctat gcaactgaat 65100

acctgtgtgt gatgtccacc atgtcctcct gttgtacctc ccccttactt aagccaggaa 65160

ggcacttgtc ccgttattag aatatcattg gcacatggcc aggcgtgctg gctcatgact 65220

gtaataccag catgttggga ggctggtcag gagtttgaga ccagcctggc caacatgatg 65280

aaaccttgtc tctactaaaa atacaaaaat tagccaggtg tgatggtaca ggcttgtaag 65340

ctgagatcac tacaatgcac tccagcctgg gcaacagagc aagactgtgt ctcaaaaaaa 65400
aaaaaaaaaa aagaaagaaa ataatattat gggcacatat atagaaagtt tgttaagccg 65460
ggtacggtgg ttcacacctg aaatcccagc actttgggaa gccaaggcag gcgaatctcc 65520
tgagcccaga agttggggtt cctgggccca acttgggcaa cattgcaaga ccctatctaa 65580
aaaaaaaaga aagaaaaaga agtaggattt gatggtgcac atctgtagtt ctagctattc 65640
tggaggatca ctagagccgg ggtggcaggg atgggcggtg agtttgcagt gagctgagat 65700
cacgccactg cactccatcc tgggtgacag agcgacacgt agtctcaaaa aaaaagggag 65760
ggggggggag aaaatctgtt gtgtcggcaa gtaaatgaag aaagggacgt tattagagtt 65820
gagaaataaa ttggaatatg atttggtacc tttcccacag aagataagtt tgctaaatgt 65880
gctgaaatta gaagcattat ataagacccg gtgcagtggc tcatgcttgt aatcctagca 65940
ctttgggaga cctcggaggt gcctgagccc aggagtttgg ggaccagcct gggcaatata 66000
atgaaatcct ttctctacaa aaatacaaaa gttagctagg cctttcggaa gcgacaactg 66060
attgtgccac agagacacaa ccctagtggc ttaggactct gggaagatat aatctccccc 66120
acttatctag tgatcgataa tgcacgaacg ctttaaaagc tggagcaggc ggctgggcac 66180
ggtggctcac acctgtaatc ccagaacttt gggaggccga ggcgggcgga tcacgaggtc 66240
agctgatcga gaccatcctc gccaacaagg tgaaccctcc ctctctacta aaatacaaaa 66300
cattagccag gcagggtggc gtgcgcctgt attcccagct actcgggagg ctgaggcagg 66360
ggaatcgcct gaacctggga ggcagaggtt gcagtgagcc gagatcgccc tactgcactc 66420
cagcctggcg acagagcaag actctgtctc agaaaaaaaa aaaaaaaaaa atctggagca 66480
ggcttggagc agcagccgtc cctcagtggg ttctagccac caacctttag attaacagcc 66540
gaacgctcta accgattgtg ccacagagac acgtagtgta ccttcttctg ggcgctatag 66600
gaagggtgca ctcaccaaac attccccaac cctcccatcc tcagagccca ccagacagta 66660
ggactgtgca aggccttgga aaaccggaga gattacagcg gtgaattgtg ttggtctcgt 66720
tggagacccg caggttggga gattctggaa ccaggacgac cttgccccta gcctgcttta 66780
gaagcccccg gaaacgcccc ggccccgacc cggacctgag ccacctgggg gcctaaggga 66840
agctgaatgc ccagtgggct cccgggatgg ctcttcccgt tctttgcgca gccttcaccc 66900
agtgagggag cctgtgccca ccctgcccag tcactttcga ggccgctgcg gaacttccgc 66960
tgccatcttc cgatcctgtg tcccgcactg ggacacagag cagggattgt ggtgagggtg 67020
gctcgtgggt cctctcgcgg agagcagggt ctggcactta ccagcgcgca cgactaggac 67080
ttgttgaatt aatccattgc ccctttagct tttagtcctt tgaagggccc tgaaaatgga 67140
aatcatgaaa tatttttcca cggggaagtt ttgttttgct tttgagatag ggtctcgctt 67200
ggtcgcccag gctggagtgc agtggtgcaa acactgctca ctgcagcttc gacctcccgg 67260

gggccatcgt cgcctttagc ttttagtctc ttgaagaacc ccaagaatag aaatcatgag 67320

atttttccat ggggaagtta ttttttcaaa gggtctattc acgttgattt ctcagcatcc 67380

cctggggcgg gcaacgggca gggcctccag tgcacctttc tgccgcggtg gagccgcggg 67440

ggctcagctg ggcggtggta gggtcctgag gcagaagggc aggagcgggg gaagggaaaa 67500

gtaaaagcag ggaaagaagc cggggagcgg tggaccagac atccagacct cccgaaggcc 67560

tcttgcagag gcacaggctg gatcttctgg aggtgagaat tgttttttgt tgttgttgtt 67620

gaagcagaat agggaggaac tgaggggaaa attgagagag aacacggaat agctccaaac 67680

gcgaggacct ataactccca aacaataaca tcttccagaa gaactagaca gaaaactagg 67740

catctgggaa ccctgaaatc ccaggagaag tagcatcatc atgaccctct gtgttccttt 67800

tggcaaagga cttgcttcgt ttgtacaatt gtttgtgttt gttaaataaa taaaaccctt 67860

ttcatgtatc tttgaaagta cattggctct attattttat ggttacaaat aatgctgcag 67920

tcatcattct tgtacacttc tcattggcca ctggtgtatt tctatagtgt agaggcctgg 67980

agagcagttg ctccagcata gtgtttacat ggtttttata tcattccatt ttctttcctt 68040

tcttggctta ttatctataa ctgtttcttt catctggtcc accgctccct agcttctttt 68100

cctgcttttg ctttttcagt gatggctttc gggtttccag aatacatctt catcagtgcc 68160

atcaagtgac attatacctc cccttctggc cattatgcta gtgttgtcat gtaatttgat 68220

tttagacatg ttacaaaccc cagaatccat tattattgct tttgtttaat cggtcaaatt 68280

attttaaaag atttatataa taagaacata tatatttaac tatgtacata ccaatttcca 68340

gtactctcca tttctttatg tagatccagt tttctgtctg gtatccttat ccttaggcct 68400

gggggactcc ttttgcattt cttgcagtgt gggttgctga gttctttcat tttttgtatg 68460

tctttaaatg tcctcatttc agtcacattc tggaaagatt tttcatttcg acatagaatt 68520

ctaggaaaac tttatttctt tcagtacttt aggatgttgc cactttgttt ttgtaaaact 68580

ggcataaagt gggcttcctg tacttgttat acaatttttg gaatgtgtat ttaaattaaa 68640

aacattaaaa tgggctggac gaggttgttc atgcctgtag tcccagcact ttgggaggcg 68700

aacacaggaa gatcaattga ggcccagagt tggagaccag tgtgggcaag atagcaagac 68760

cctgtctctc atacacacac acacacacac acacacacac acacacacac acatacgtac 68820

atacattgtg aatgtcctag attcatctta agttccacca acagtgcact taaagtaaaa 68880

tgtgcccaac ctgagggtca aacctaactg ctgatgtgca gtctgtgttt gtgagacatt 68940

ctcaacagca tttgctttcc ctagcatagc ggttttcgtg ttttcctcat acctgaatgt 69000

cttaagtgca aaacctgtca gaaatcattt cctttgccaa aagattctaa aatacttttt 69060

ttttttttga gatcagctca ctgcaacttc cacttcccac gttcacagga ttctcctgcc 69120

tcagcctctg gagtagctgg gactacaggc tgccgccacc atgcccggct aattcttttg 69180

tatttttagt agagatgggg tttcactgtg ttagccagga tggtctcggt ctcctgacct 69240

cctgatccgc ccgcctcagc ctcccaaagt gctgggatta caggcgtgag ccaccatgca 69300

tggccaaggt ttgactttta tacactcttc tgaaaggggg ggttggctag tttgaattgc 69360

gtcgcgggaa tttgctggcg caaaactcgc tgggcaggct agagggctta cagaagcaga 69420

aagaaggcag ttaatcaaac tgacaggtct tgcaacgcaa gcatagctgg tgaccttgca 69480

gctgcactga agggaagtca ggaacttaca aaaccagaaa tggtaagggg gaaagagaag 69540

gtagtaaagg catttgttgt tttttcctct tatccttatc cgtgctaggg gcggactgtg 69600

tagacagagt ctctggaact cattccctgg ggctctgtct tttcagacag tgttatcaag 69660

gatctgctag ggctctatct attgctggtc ttggagtaag tcaagtactg tgaagctcag 69720

ttatgcagtt gagcttcaca cattagggga aacaacaaga gaacagcaca tctggagtgc 69780

aatggatgag cctcaaactt aggacgaatc actttggtaa ccataggatt tcccctgcca 69840

gataagtggc agtccacata cctccgccct gccacagctc catatgcctc accctttaca 69900

cccacggtta cttgccctga gaaccacccc cgcctatccc caaccacccc aagcccttct 69960

agccctaaca cacagctggg actctcacat ccaccagcgg tcctggactt gctcctacag 70020

cacggggaat tctccctggc gaagcagcaa ccctgtgctg cctcatctac acagaaatgc 70080

cctatcggtg atgtcaccga cagtgccttt cccagtcccc ttctgttctt ccgccccacc 70140

ctctgccact cagccgacca acacgctgcc ggagcaggcg ggggaagtga cgtctgcctg 70200

tccctccttt ccttcccgcc ttgtcctttg gggaggtgcg ccaagaccct gcatctagtc 70260

tctcccaaag aggtgactac ttaacctgtg ccctgcggag gaaccttttg gcggccagct 70320

ggaagcctgt gcacccgtct tcaaataaag gcttttaatt cgcagactag aacgtttagg 70380

attacaaaga aaactggttc ctttcaaatc tggttatgtt tgtgaaatag cattccgctt 70440

aaaattggaa gcccttcaat atcagggaga aatcatatct atgaaactag agaggcaaac 70500

cagccatcct tactggtttt accagtagta gtgttataaa gaaagttgtc caatttcatg 70560

aatcttgtag gtttggtttt ttgtttttca aatacagtac tgtacaaaaa ggaagcggaa 70620

tgtggttgca gtcttaatgt aatgcctctc tgggcctgag aatttgaaaa ggctcacact 70680

cctcataaaa gttctcttct tcgccgggac ggacagtggc tcacgcctgt aatcccagca 70740

ttttgggagg ccgaggcggg tggatcacct gaggtcagga attcgagacc tgcctgacta 70800

acatggtgaa accccgtctc tactgcatgg gaaatattta tgtatatgca acaaaaggta 70860

tttgccacca cacatacatc tccctgctca gctaaatatg tcatctaaag tgatgtgatt 70920

atctagtaca accttagcca gtgaataaat agctttttat tgagctgcaa aagaggctgc 70980

agtttcaaca gcagtgtttt tctttctttt tctttcgaag acagtgtctg gctctgtggc 71040

ccaggctgga gtgcagttgt gccagcacag ctcactgcag cctcgaactc ctgggcttaa 71100

gtgatcctcc tgcctcagcc acagagtcac tgggattaca ggccagagcc accatgcctg 71160

attttcatca gcaatgtttc ctgaggttac agaaagattt gtaatcctgg gaggaaacag 71220

tcccctgaag caaagtgttc tcccccaaaa atgcaaagag ctatcttctt gatagcgagg 71280

cagataattc tcaggttttg ccccacagaa tctctatcta aaatagagca gtggtcatga 71340

ctagtgaaaa gtttgaggga actcacccaa tgttgggttt cttcagaaat atatatatat 71400

ttacataaat atatatttat atatatatat aaatatatat ccaaatatcc taaaagacac 71460

tgcccttcat cattccatgc tgttagacat ttataggacc atggatggtg atctcctcca 71520

gacaaaaata aatgctggtg cagaacaggt aagtgtacta taatttgagt acatcagctt 71580

ttgggcattt taaaccatgg gtcagacatg ttagagcaag ggaccaggtt gatagcaaga 71640

gggagtgttt tccttttaat tttccaatag caaagtgatg tttgccactg tcatttcaga 71700

gtgatatgac aatttgttgt tgttgcttgg ttttgtgggg ttttttgttt gtttgttttt 71760

gagacaaggt ctcactgtca cccaggctgg agtgtggtgg catgatcagg gtttacttct 71820

gccttgatct cacgaattca agcaaacctc cttactaatc ctcccaagta gctgggacta 71880

caggtgcgtg acaccacacc ctggggtgtg aactgggatt tgatgttttc agttggctcc 71940

ctaatggaat aggttccttt actattctgg aatacagatt gtcattctcg tgtgcattat 72000

atttctacta ccctgctaat ttttttcttt tttttttttt ctttttaaga tggagtcttg 72060

ctgtgttgcc caggctctat tgcagtggca ggatctcagc tcactgcaac atccacctcc 72120

tgagttcaag tgattctttt gcttcagcct cctgagtagc cacccggcta atttttgtat 72180

ttttagtaga gacagggttt caccatgttg gcaggccagt ctctaactcc ttacctcaag 72240

tgatccacct gcctcggcct cccaaagtgc tgggattaca ggcatgagct accacgcctg 72300

gctctcaaac cattttgagt aagcaaagac agatatgtct ggctttttag tacgttgctg 72360

aattagcctc caatctatat ttttaggaag gacctgggga atggcaacat ggagatattt 72420

ggctaaaatg tgaacctttc atgttctgct tcagtctctt tttgaaaatc cttccaattt 72480

gccttttgca accagttagt ggccttgcct tcttcaccat catgtgaatt aattgataaa 72540

gttcagaata tttgggctca aagatttcaa cagttaagtc aaattttctg ccacaccctg 72600

tgggatcttg gagcctgctt tagaaacaga agagcgaaat gtatttaaat ttagatcagg 72660

gaagtccttt ataatcttaa ttcacgtttt ggtgaagcgg tatacacaat ggtaggctcc 72720

cctcttcctg tgggtttggg ttttaccttg aaaggggctg tcagaacaga agtttcaggg 72780

aaggggccag agctctgggg actttcctca ggtgatggta atggaagaag aggcaaggca 72840

ggacaggaag gctcaggtaa gaaagactat gcaggtgcag gggcaggagg aaaaggagca 72900

gttggaggcg aaagagacaa agcctcctca atatttctca attcagaaaa tgtcagttta 72960

cctccttcag cttatttcct caatggaggc aattttctca gttcttttag aaatttagaa 73020

atttagaaat ttctaggtct cattggaagt aagtctccca tttaatttgt ctggttgtaa 73080

aaccaaattt ctctcccttt tttttttttt tttttttttt tttttgagac atggtctcac 73140

tatgtcaccc agactggagt gcagtagtgt gatctcagct cactgcacac tgcactcact 73200

gcttgctgca tgacagccag taagtccagg gacaaggtgt aggggcaggg aaggtgactt 73260

tattccagag agccaccaaa ctgaagagat ggtgaacaaa tattctaaag aaccatctta 73320

aattaatatg attttcaggc tccttgtacg ttagggaagg gaggaagaag gaggtggttg 73380

aggtgaagag gtctgacaat gacagactta tgggctgcag tgagagccaa aggggatggt 73440

gaaaattctt tgtcttttgt caggtcacaa tgctcttata aatcttcaac ataacactgt 73500

tacttgtgca tacaacctct ttatcttttc aggagtcagt ttggggaagg gattatgatc 73560

atctgtgttt taaagttaaa ctgtaagcta aatccctccc atagatagct cgggaggggc 73620

ctatctgcag aaataaggaa aagcagttgg cctgaaagat atcaccacag ggcaggaagg 73680

gttaggagca aaatgcagtt agtcatgcta agcctccttt tcattgctat gtatttaatt 73740

tacttgaaca cataatttta attttatata tatatatata tttttgagac agagtctcac 73800

tctgtcaccc aggctggagt gcagtggcat gatcttggct cactgcaact tctgcctccc 73860

tggttcaagc aattctcctg actcagcctt ctgagtaact gggattacag gagcccacaa 73920

ccacgcctgg ctaatttttg tatttttagt agagacaggg tttcaccatg ttggccaggc 73980

tggtctcgaa ctcctgacct tgggctccca aagtgctggg attacaggca tgagccactg 74040

cgcccagcct aactaagatc attaaaccat tctaatttgt caaaagagtc acactgattt 74100

ttgaaaaata atgtaatggg ctgggtgcag tggctcacgc ctgtaatccc agcactttgg 74160

gaagccgtag agggcagatc acaaggtcag gagtttgaga ccagccggac caacatggtg 74220

aaaccccgtg tctactaaaa atacaaaaat tagccaggtg tggtagtgtg cgcctgtaat 74280

cccagctact caggaggctg aggcaggaga atcacttgaa cccgggaggc agaggttgca 74340

gtgagctgaa attgcaccac tgcacttcag cctaggcaac agagtgagac tccatctcaa 74400

aaataaaata aaataaaata aattaaaaaa ataataacat aatgaatttt ttcatgtctt 74460

tatatattaa atatttacat tatttaaatt gttcaaaagc atcaaagatt cctctctatc 74520

aacttcattt catttatttt attgtaccaa actatcaaga ccattaattt aatctacagc 74580

taaatctatt attttttcat gttagaaatt ccacaagaaa atttttccct aatgaaacct 74640

cacatttcaa gcatcctaag taggctgggc gaggtggctc acacctgtaa tcccagtact 74700

ttgggatgct gacaggtgca taacttgagg tcaggagttt gagaccagcc tgggaaaaat 74760

ggtgaaaccc tgtctctact aaagctataa aaattagctg ggcatggtgg catgcgcctg 74820

taatcccagc tactctagag gctgaggcag ggaaatagct tcaacctggg aggcggagct 74880
tgcagtgagc taagatcgca ctactgcact ctagcctggg ctacagagca agactctgcc 74940
tcaaaaataa ataaataaat aatcataagt agtaaaaaag taaaatgaaa taaaaaataa 75000
ggcacagtgt cttgctctgt tatccaggct agagtgcagc agggcaatca tagcagacca 75060
acttggaact tctgggctca ggcaatcctc ctggttcagc ttgccttgca tttttttaga 75120
gatggggtct tgccctgttg cccaggctgg tctccaactg ctggcctaaa gcaatccttc 75180
tgcctcagcc tgttgagttg ctgggagtac aggtgcaaga catgcagcct agcattgtag 75240
taaaacaatt ttcaacaaat tcttaatttt ctttcttttt cttttctttt tttttttttt 75300
tttttttttt tttttgagtt gggcgtctta ctctttcacc caggctgcag ggcagtggtg 75360
caataatagt tcactgcagc ctgcctcagt ctccctagta gctgagatta cagtcatgca 75420
ttatcatgcc cagccaactt taaaaaaatt agctaatact taaaaatttg tagagacagg 75480
ggtttcacta tgttgcccag gctggtcttc aactcctaac ctcaattgat tctccctcct 75540
cagcctccta aagtgctggg attgcagcca tgagccacca tacctggttt aattttctta 75600
ttttaatctt atgtaagtga ttattattgt tcctaagata attggggcag tgactccttt 75660
aaaattttag agacctaatt tatctattca cttcactgaa agagtatgcc aatttgtttc 75720
atgagaaaat accctacatt cataaagcag gaaaattcct tccaccaaac tagggggcat 75780
tctaaagaaa caaattttgc taagtaacat cacttaaggt gaaaacagag acaatggtat 75840
ctattaacaa tgtttatcag tgaaggaaat aaactgaaaa tattaacatc attagatcct 75900
tggagggcct tcattgctga aaaatctgag taatattgtg ataccctttt gagtccggca 75960
ggacattctc tttccagggc atataacaat gggtgaataa tttcttttca ttcattttca 76020
ttaagggctg aacttcctta atgttctgga gattattaaa tttgatttgt atagttgtga 76080
aaagtgctca tattgctgat tccattgctt atatgtgatc atataaatct tttctctcct 76140
ttctgtagtg tggtttaaac ttaatcctta aaggacgtgt atttgaattt ttcagctggt 76200
tagaaacctg aatataccaa tcaaagaaaa ctgctcctta catgctacag atttagtttt 76260
cttcttgtac taagatgtct tttagatata gtaaatttgt taaagccaag agctcctatg 76320
gaatgaagtt gaatgggagg ggggatgtgg agtagtaaga tcacccttgt aacagagatg 76380
ccactcttgc agattttgac aactattggg cccataaaac ttttaccaaa tattggaaag 76440
acaagatatg aacagcttat tattgctgca gtctcaaata tcaagaaata ccattattct 76500
caggacaata aataggcaat aaaaaagact cacagaccag attagctgtc caaacaggga 76560
gtggatcctt atcaacacaa cccaacagag attgtcccca gaaattcact tcataacatc 76620
aaaaccaaaa actcacactg atggcaaaaa ataatgatgc aataatgaga gagagacaga 76680

gagagagaga gagagagacc tgtcctatag ccatacttag tgggtaaaag ccaaagagct 76740

caatttctgc tcatgatact taatagaaca gagggaacat gagccaatag ctcagtgggt 76800

tcagatacgc accaagtgcc tgttggatac aggattctac tgtctccaat aatatgtctc 76860

agatggacta gtttttttgt tttttgtctg ttttgttttt gttttttatt ttttgttttt 76920

gagagagagt ctccctctgt cgcccaggct ggggtgcaac ggcacgatct cggctcacgg 76980

caacctccgc ctcccgggtt caagcgattc tcctgcctca gcctcccaag tagttgggac 77040

tacagacgca caccaccacg tctggctatt ttttttgttt tgttttgttt tgttttgtat 77100

ttttaacaga gacagagttt cgccatgttc gtcaggctgg tctcgaactc ctgacctcag 77160

ttgatcagaa gtaggtgagg tcagaaaaca taccctggga gaggctggca caatgcccag 77220

atccgccatc gctaggcctg ggttttcttt ctgtagtatt catgtagtgc agggacaaaa 77280

ccaattagat acttctggga gttaaaaaga gattaatttg ccgggcgcgg tgtctcacgc 77340

ctgtaacccc agcactttgg gagggttcac gaggtcagga gatcgagacc atcctggcta 77400

acgcggtgaa accccgtctc tactaaaaat acaaaaaatt agccgggcgt ggtggccggc 77460

gcctgtagtc ccagctactg gtaggaggct gaggcaggag aatggcgtga acccgggaga 77520

ctgagcttgc agagagtcga gatcgtgcca ctgcactcca gcctgagcga cagagcgaga 77580

ctccgtcaaa aaaaaaaaaa aaaaaaaaaa agatgaattt acagtgtcat ttgagaaggg 77640

gtattaagga atttgccagg gtactgacgc gtgtcagttg caaactgcag gttgagagag 77700

agctaagtat tttctgtcca tgaaggtgat aagcgagggc ctgaagaaag aggaaggggg 77760

gaggacactg gcgccagaaa taggaaaggg ctgcttgggg gtgggaagga tgggtcgggg 77820

tgctatctag aaagctgcct ggcaatggct gtgggatgcg gaagcgagac atcaaacaag 77880

aagctgtcgc ttaaataaag tctgaagaaa gactagatat gtaaacggca ggagatagta 77940

gggaaactgg acccgtctcc tcataaaact tcccgcctta tatttcaggg aggatcgcag 78000

cgcatttcgg ccaagacagg tgagactgcg gttttgacct gcgggcctcg atgaattgcg 78060

ttaggacacc tgggctccgg gagagccgtt ccactccgca aagtaagcgt gttatgtcta 78120

caacccaacg gggacgcgct aagagcccta aaggccctgc tttcatccca aagaacagcc 78180

cccgcctgcg tagtttgtag ccggctctat gaggtgagaa cacattcccc gctagcacag 78240

aaatcctaca gactcctgag ggggctgcag ctagaagcag aggctgtgtg aaatgtgact 78300

gggggctagg gaaaaacacg tagattttca cagaccctga gaacccaaga gactgcagac 78360

catgggcaga caaatctctg caaaaagcag ccccgcctag ataaaggaag agctgtgcgg 78420

ccttcgcgct agctagcttg tgtaaaggtc gacgattctt atttctcccg gggcggggag 78480

aaagcgacat attactacta tttctcagcc ttcgactgtg agagtaacga ttaagactaa 78540

aacacaagcc agtaaaaaaa acaagtaacc cttgctgtgt agactattct ttaaactaca 78600

aaaaatcagg ggaaagcgcg aacgcagtcc cccactacca caaattatgc agtcgagttt 78660

cccacatttg gggaaatcgc aggggtcagc acatccggag tgcaatggat aagcctcgcc 78720

ctgggaaaac caccttcgtg atcatggtat ctcccctgcc aggtaagtat gagctcttgc 78780

acctccgccc cgccacagcc tcacacgctt caccctttac acacacggtc acttgccccg 78840

cgcagccccc cccccccccc cccccccagc cctcctagcc ctgacacaca gctgggactc 78900

tcaggtccga ccagcggtcc tgaacccgct cccacggcac gggaaatcct tcgtggcgca 78960

gcagcaggtg gggaagcagc agcccctgcg ctgcctcatc tacatagaag tcgccctatc 79020

cgtgatgtca ccgacagtgc ctttcccagt ccccgtctgc ctttctgccg ctcagcctac 79080

caacccgctg ccggagccgg cagggggaag tgatgtctgt ctctcccttt tttccctccc 79140

gccccggcat ctgttctcgc ccgaagaagc tggtccttag cctgcgctgc ggagcaacct 79200

ttcagtggcc acctggagcc tgggcaccgt tcttcaaata atggctttta attctcagac 79260

gagaacgttt aggattacaa aagaaaccgg ttctcttcac atccttatcc ttgtcatgta 79320

gcattccgct tgaaattgga agccgttcaa tgtcagagag aaaccatatt tatgaaatga 79380

aagaggcttc tcagatgact gcaaaccagc cttccttact ggttttatca ctggtaatgt 79440

tataaagaca gttgtccagt ttcatgaatc ttgtaggttt ttgtttgttt atttgtttgc 79500

ttttgatgtt gttgttgttg ctgttgtttt ccaaattcag tattgtagaa aaatatgctg 79560

ccccagaaga gatgattgga cactctccag cgtggtgttg gactttgtca tctcttgcac 79620

agccatctcc agaccttagt gtttacctca cgttagtttt ttatattctg caaagacaaa 79680

accaaaataa tccaaatttg acacaaatac ctgggataca tcttatttga gatgtttaac 79740

aaatgtctgg atcatctttt cttacattgg attataacgc aggaaacact gtgaagtaag 79800

taaagttgga attcccaagt caaagaccat ttgaatattt acaagtagat ttgaggcagg 79860

aataatacag ggtggccgca gggtaacaaa ttctaggcag cagatttaca tgacttgagg 79920

ctatgggctg ataagacgct gaaaaaccag ggtgtggacc aagctggcta agactgactg 79980

gacccaatgt ggtgctggat ttgatgtagg ttttaccgag gccctcatta tatgctcatt 80040

aacatactaa atcacacacc caccagtgcc atgacagttc tgagaccaat atgtgatgta 80100

aaaatggatg gcaccacagt tccgagaaat ctccaccttt acccaggaat tttcatgact 80160

attccactcc ttggttagag aaaccaatca agatgaaacc ccagaaccca ttgttctctc 80220

tcgggtatgc ccgaactccc ctttcttgag tgtgtacttt ttgctttgca ataagtctct 80280

tctttcactg tttgctgact catccttgac tttgttcttg agatggtgtc aagagcctgg 80340

acaccacagc tggggtcgag atcccgctag tgtccgggga ctttccccag gccaccagta 80400

tcagattcta ttccattgct caaatcacaa aacatcgagt ggagagttct ccttggagac 80460

cataaagtaa agattctgtg gcatggtggc cagttaggcc actggaaggc atggcaaaat 80520

attgaaaatg agagattagg tgacagtgta gtaataactg ctgaatacta aatatttgag 80580

ccaggcccca ttccctggat actgacaggg agacacattg tccaggtagt agtggagaaa 80640

tactttctgg gtatctgacc agcctttgtg gaaagaactg gcaccatcct gcagatgtaa 80700

ccgcctgatg ggttcttcct gcccactgta catacaaaat caattcatgg agaccatggc 80760

attgaggtaa agagtttaat tgacacaggc cagccacgac atgtggggga cagagttatt 80820

actcaaatag atctcactga agacttggag gtaaagggtt ttcaaagaca gtttggtggg 80880

gagggggcta gggtttgggc ggtgctgatt gttggggatg aaatcacagg ggtgtggaaa 80940

atggccctcc tgcattgagt cagcttctgg gtgggagcta agggactggt tgatttttgg 81000

gccaaatggt gccatccagt agtcagaaat gcaaaagcct gaaaagacat ctcaagaggc 81060

caatcttagg ttctacaata gtgatgttct tcacagcagt aattggggaa gctgcaaata 81120

ttgtgacctc tggaataatg gctggtaatt atttaacgag gcatacatct tagtagaatt 81180

caggcccctt tcatcctcct aacttggtgg cctttcatta gttttatggg gtaatttagt 81240

tttggggaag gttatcattt taaccaacat ttttggctgt ccccaacctt tttggcacct 81300

gggactggtt tcacggaaga caatttttcc atggaagggg gtggtggatg gtttctggat 81360

gaaactgttc cacctcaggt catcaggcat tagttacagt ctcataagga gtgcgcaatc 81420

tggatccctc acatgtgcag ttcccaatag ggtccgagct cctatgagaa tctaatgccg 81480

atgctgatct gacaagaggc ggagctcagg tggtaatgct ccaaagcctg cagctcacct 81540

cctgctgttc agctgggttc ctaacaggcc atggaccagt acctgtcttg tggccctggg 81600

ggttggggac ccctgattta aactataaac tcaatttttc ccaaagatag cttgggagaa 81660

attgcacagg aacgagcaaa gacagctagc ctgtgaggct agaaccaaga tggagtcagc 81720

catgtcagat ttctctgatt gtcataattt tgcaaaggta gtttctgagg gactaccctg 81780

acacttgtta aaagcatgag gtggatttta ttgtatgtac tacagtaggc aagagagacc 81840

agcagagaac cgagctcaac ctcaaataca gcaggaagag ttgaagatta tagccaattg 81900

gcaagataag aaagtcagtg gatggaaaat tactaagagg aacttgatta gctatcaaag 81960

gtggttggga ggactcttgc taaactagac tcaacagtat tcttttctaa aactggactt 82020

agcaggccaa gaactaatag agaaaagggc tcagaggaaa ctactaaggt ttggtcaaag 82080

atggagtcct tgtcaactct aaattgaagc ttctgcaaat aaacgaagac caaccaaatg 82140

aaaaaaagca aaagctattt attctgagct tgctatggca gggagtcagc cactgttact 82200

tgtgttttgg cagagacttg aaggcagtca gaagggtgag aaagcttttt aaaaagaaag 82260

gcttcaggta tgcttggact ggaggctgtc agtatggtga cgctgtagat agatgaagaa 82320

aataaaaata ttttacccca gaatatattt ctttggtata ttttaagatg gctgtcagag 82380

agccagcaaa cagaagtaac tctgcaaaac tgtcttttgt aggggaaatt tacacctgca 82440

gagaatctgc attaatccag ccttcccttg tcaggattga gaaataaaaa taagccctag 82500

gccctacaac caactgaaca gactccctct tggctgacag gaccacagag aaaccttgaa 82560

agctgtttct ggctgtgaca ggataggagg tcggacgtgc ccccttaaaa cccctccctc 82620

actaactgcc attatgaggc aggagaatag cagagggaat tggaagttgg ataaaaggca 82680

gaatgagtaa aggcagaaac agaagcaagg tgatggggcg ggagagcaag aagcaaaata 82740

aaaggcagaa gttgagcaac ctaaacaaaa agtaagatta aaaaaagcaa ggaagaccgg 82800

gcacggtggc tcacgcctgt aatcccagca ttttgggagg ccaaggcggg cagatcgcct 82860

gaggtcagga gtttgagatc agcctggcca acatggcgaa accccatctc tactaaaaat 82920

acaaaacatt agctgggcat ggtggtgcac tcctgtactc caggctactt aggaggctga 82980

gacaggagca ttgcttgaac ccaggaggca gaggttgcag tgagctgaga ttgcaccact 83040

acactccaga ctgggcaaca gagagagact ctgtctcaaa aaaaaaaaat agcaagcaag 83100

gaccccattg ctggcaagat ccaaaccagt aaaggggcag ctcctcagag acagacatgt 83160

gcattcaaga aaaaaagcat ccttaacatg actccatatg ataatcagct cattaaaact 83220

catgcatatg gactacatat cttgcatgta cttaaaatta tggggtggag acaatgtgca 83280

agcacacgag ggccaaagta actaagcaac ccacccatca atctaaaggc aaacactggc 83340

taaagattag gcatccttgg gaagagaaga aaaaaaaaca cacataaaaa gacccaaagt 83400

acaccaaact aatactgatc tcatctgcca caggtcagcc cactctcccc actccaagaa 83460

tgttactgtg cttaataaac ttttgctttg ctttgctgct ttgtgtgtgt catgtacatt 83520

cttcgtttgg gacaccaaga gcctggaact gcatggcacc atctggtaag aattaggatt 83580

tttttttcct aagggttaac aaaccaactc tttggaaaga tttgcttcac tactattatc 83640

aatcaacagc ctgatacttt ccctcccttt tgtggttttg acaaagcaag caagcagcat 83700

tccctcctga taagagacca ccgacctagg aatgattctg gccagactac agaggatgta 83760

cagtgagagt tttcatgtct tctgcttcag cttttgatgt cagaggccca caaactccac 83820

tctcagatga ttgctaatgc caccatttta tgaacatggg ccccatggag aggcacaaag 83880

ctcaactgca cttctgcaca tttttcctct tataaatatt catattggaa tattatttgg 83940

tactgcttcc atgaaagaca catttgcaga atgtactcaa attagaagca ttatataaac 84000

actataatgt agcagtggtg catccagttc tctctttttt tttttttttt ttttttgaga 84060

cagagttttg ctcttgttgg catgatttca gttcaccgca acctctgcct ccgggttcaa 84120

ctgattctcc tgcctcagcc tcccgagtag ctgggattac aggcatgtgc caccacaccc 84180

ggctaatttt gtatttttag taaggatggg gcttctccat gttggtcagg ctggccttga 84240

actcctgacc tcaggtgatc cgcccacctc ggcctcccaa agtgctggaa ttataggcat 84300

gagccaccat gcccagcccc cagttgtcta tactatacaa atatctgcag tgtagacatt 84360

tccacaatga ccaaagatat gtgtacaaga atggtgactg cagcattctt tgtaatccta 84420

aaaccaaaac aacttcatcc caaaaacatt ttttcttttt tttttttttt tgagatggag 84480

tcacactctg ttgctcaggc tggagtgtag tgatctcagc tcactgcagc ctccaccttc 84540

caagttcaaa tgattctcct gcctcagcct cccaagtagc tgggattaca ggtgcatgcc 84600

accacgcctg gctaaaacat tttgaaaagg gttaaataaa tcatgcacaa actgaggaaa 84660

aatactgttt tcaaaaatgg tggagaggat cactatgatg atgaatgatt ccactggtca 84720

cattattaat agaacaatca gtaaatccag gcacatcctg gagatagtac tgaactccta 84780

ttactaaaat atgaaaaaat ggaggcacgt acattactcg tttaagcgca taagggactg 84840

aattagaatt tatcacacca aaagtgggtt cctaggtatc tgtttcagga tcctgagtta 84900

cacaggtgta aacccgccat ttcaggagat acccggttaa gaatcggatg ggggaggggg 84960

gctggccctt gacatggatc agtcataaat taggggtttg gggctggccc ttaacatgga 85020

tcagtcataa attagtggct tggtactccg ggaagataaa atcttcccca tttgtctagt 85080

gattgacaat gcgtgaatgc tttaaaagtt gcaatcagcg tccctggatg ggctcgaacc 85140

accaaccttt cggttaacag ccgaacgcgc taacccattg cgccacagag acaagcacta 85200

ccagttctaa taggcaatat aggaagggca cactcactaa acttcctccg tcccttctat 85260

tctcagcgcc cgcctggcag gacgactgag caaggctttg gaaaaccaga gagattagag 85320

cggtgagttg cgctggtcac attggatacc tgcgcgttag gagattctgg agccagacgg 85380

agagccgaat ggccttcgcc cgccctgccc ctctcttgct tcagaagccc ccagaaactc 85440

cgcggtcggc gacccagccc gagccgcctg aggtcccaag gggaagctga acgcctggtg 85500

ggctcccggg atggttcttc ccgttctttg tgccgccttc acccagtgaa ggagcctgta 85560

tccaccctgc tcagtcgctt ttgggctgct gcggagcttc cgctgccatc ttcggatcct 85620

gtgttcccca ccggggctcc accagggcag ggatggtggt gagggtcgct cctgggtccc 85680

ctcgcgggga gcagggtctg gcactcacta aggcgcacga ctaggacttg tcgaatgaat 85740

ccatcgtctc ctttagcttt taatccttta aagagcactg agagtggaaa tcaagagatt 85800

ttttccatgg ggaagttctt tttacaaagc attgatttct cggcaccccg cggggcgtgc 85860

aactggcagg gcctctggtg ggctttctgc cgggtggagc tgcgggggct cagctgggcg 85920

ggggtcggtt cctggggccg tagggtggga gcgggggaag ggaaaagcaa aagctgggaa 85980

agaagccagg gagacatgaa ccagacatcc agacctcctg aaaggctcgt gcagacgcac 86040

aggcgggatc ttctggaagt gagaattgtt tttgtttatc tattgtagca gaatggggaa 86100

atggagagag aacctgaaag agccccaaac tcgaggacct attgctcccc aagaataaca 86160

tcttccagaa ctagacagaa actaagcgtc tggaaaccct gaaatccttg gaggagtagc 86220

atcatcctga ccctctgtgc tccttttggc aaaggacttg cttccattgt ttgtttgttc 86280

aattgtctgt ttgttaaata aataaaactc ttttcatata tctttaaaag tacgttggtt 86340

ctattatttt atgattacaa acaatgctgc agtcatcatt cttctacact tgtcattggc 86400

cactggtata tttccatagg gtggaggcct ggagagcagt tgttccagca tagtgcttac 86460

atagatttta tttcattccg ttttctttcc tttgttgctt tattagctat aacccttttc 86520

ttatttcagt ggctgcttta gggtttctgg gatatgtctt taatttatca cagtctgctt 86580

tcaggtgtca ttatgtctcc ctttctggcc tttatcctag tgttatgtaa ttttacttta 86640

tacattataa tccttgtgat tcattattat tacttttatt tattacacca aatatttttt 86700

aaaagattta aattctaaga aaatgtgtag tacatgcttt ctaaatgcca tttccagtat 86760

tcgtttcttt gtataggccc acatttttat ctggtatcct tttgcctctg cctggaggac 86820

ttttaataag atttcttgta acgtgggttg gtgaattgtt ttagcttttg tatgtttttc 86880

gatgtcctta tttcactcac agttttgaaa ttttattttt gcatagaatt ccaggttaac 86940

tttttttctc tgggtacttt aaaaatgtta ccatttatga agcattgcca ttataatatc 87000

atctttcttt cctgtgtttg taagaatggc ataaagccgg tttcttgcgg tggttatata 87060

attttcagaa tgtgtactta aatttaaaat attaaatttt actaaaatac taaaaatatt 87120

aatcatgaac atcctagatt catcttaaat tccaccaaca gtgcacttaa aatgtgccca 87180

acctgagggt caaacctacc tgctgacgtg taatttgtgt ttgtgagaca ttctcaacag 87240

catttgcttt ccctagctga gtgattttcc tcacatctga atgtcttcag tacaaaagga 87300

aacctttgtc tttgcaaaaa tactttcaaa tattcttact tcaagtaagt gcattaaaaa 87360

caaacttctc agttgcatcc ctggaatccg tggaaagccc gggagagaca gtcaagtggt 87420

tcaggatcaa gagctaaaca agggaagaca aagcgtggct tcttcactag gagtccagcc 87480

aaagtcaact gatttggtct ccagtggagg tcggaactcg gttcaccagc gacgtgagga 87540

cccggcccag aggaggcgga ctttctcttc ctggtgcctt cagataggaa atctagaatt 87600

tctttctttc cctttgatct acttctaact ctcccgttct atttcttcaa gacctttttc 87660

ggatccctac tgcgaaggac ctaaggggcc gatgcccttc cctaacggtc cctccctggc 87720

tgagtgtctt cggagcccaa gctcacctgt aaggttactg ccctccggag acagcgagag 87780

gaccaaggag ggcggcgggt gcggtgggaa ccacagagtc accgcgcacc tgcgcctcgc 87840

gggctcctag caaattgaat aaatgatccc tgaagcttct ctgcaggtca cagggaaggg 87900

gagggtaggg gtcgacccgc ggaagaagct tcaagaagca tcgggaggac ctggccctgc 87960

ctctgggcct tgaaaacagg cctggccagg ctgattttgt cgcgtaggcc caaagaaaag 88020

tgtcaagggc ggcccaaacc ctgacctcga gattaaggct cttaaatgtc tgacggtttt 88080
gagactcgtc tgtagaatcg atcccgcctg tatccggaga ccggagactc ctttgccaaa 88140
attcagagac caagaaagag aaagattggg cagatcaaag tctgtaatta acccaaacag 88200
gaaacgtaat ttttctgaca aattgtatgt gtgctctgag aaaaagacta gcctcaagga 88260
gaatctgttt ttttggcttg agtgtaggga agaatggaat tgccaccaac aatgttgcgt 88320
tgaaacggca ggtgcatcag gctgtagatg tgaaggaatt aaaaatattt caccccaaaa 88380
tacatttctt tgacacattt tgagatgtct gttcagaaag ccagctgcag aagtagccct 88440
gcaaagttgt attttgttgg ggagatttgc atctgtaggg aatctgcatt gatgcagcca 88500
agtctttctt tgcccagatc taggaaagat taactgagag tctgacagct gtaaaggtct 88560
caaagaaact tttagggttt actgtctctg agagctgcta cctgtaaggt ttcatttaca 88620
tattaagacc acctttgcta gctaagcctc ctcttctctc cctcctataa cctgtcttgc 88680
ccacaataaa tggatataca tctgactctg atggtacctg gttttggcca tgctttgagc 88740
cctcattcat tctgttacct cgagatggtg tataagcttc tgcatcccat tgtgggtagg 88800
gtagggtaat cactctgtgg ccctccccat gtacatgtta gtacatttta tgccttttct 88860
ccaattaatc tgccttttgt gagttgattt ttgagtgaaa cttcagaggg ttaagaggga 88920
gggtttccat tggcccctac agttttggag ctgtaagcag gatagtaaag ctctgctctt 88980
ctggaagctg cagtgaagaa cccaggatct gatcagctgt cataagggta agaatttttt 89040
accagccagg ctcccagcct ccttctctgt gtggaatctg atcaagtgca cagtaaaaat 89100
cactgcttct tttccctctc caaaatcttg attaatggga gaaaaggatt tgtgtgacta 89160
gtcttgggtg tagtgactct ggtgtgcttt tttggtactt tgtggtacca attcatattg 89220
tttaatccct ttcttcccag aaaatgtctg ttcctttgtc tttgtctcta cgtgttattc 89280
tgtcataaaa gggggtactg gttgaggttc cttcttatct tattttatgt ccttgagagc 89340
ttgacttgtg acaaagtggg agcactttct cttggtttcc gctatcgggt aagaggtggt 89400
aattttcagg tcatattagg tggcctgtct gaaaatggct ggaaacccga gtacactttt 89460
tgttctaact atgtcaagct cttggggttt gtcttaagaa gtcccatccc ttcgaggctt 89520
ttgccgtctc aactcttgtt gcctgattag tcctagaaaa gctcagtccc aagagggcct 89580
acctggtatc acagattcac aggtctgtga ctggaagcct ccacaaattt gtgggttact 89640
ggaggcaatc atcatcctta actatctgtg gcctactcct tacattgaac ttttttcttg 89700
gaggggagtc tttgggattg cctcttctat gcccttccca ggaagttaac ctggaaaatg 89760
acatcctggg ctttccacaa agaggctatt tggttgaatt gctattggaa taattatacc 89820
attggatatt ctagttgcca atggcctgaa gatggatccc ttaaattaga cacctgaatt 89880
aaaaaaaaaa aattagagat ctcttatttg aaacagttga taggaagatc aaattaaaag 89940

aaagacacat actagtgtga tagctaccct taaaaattct cttgacaata ttatctgtct 90000

tgtataggga aaaaattctc ttgactaaat taaagagcaa aatttgatcc aaaacaaagt 90060

taaaattctt cataagctca aactgcctgc tctggatccc ttccaggatt cacaataaag 90120

tctgctctac cttgcagttt agtacttaaa gttctgtgct tttgctgcca tagcctgggt 90180

tcaatttcca gtcagggaac cagtttattt tggcttgata tttgtgtgac ttttgacttt 90240

ttggaatacc catttattga tccttttccc gttcatgggc agcttttttt gttctttttg 90300

tttgtttgtt tattaagaca gattctcact ctattaccca ggctggagtg cagtggtgca 90360

atctcggctc actgcaaact ccgcctccca ggttcaagtg attctcccac ctcagtctct 90420

caagtagctg ggattacagc cacccaccac catgcctggc taagttttgt attgtattat 90480

tagtagagac agggttttgc catgttggcc aggctggtct tgagctcctg acctcaagtg 90540

atctgcccgc cttggcctcc caaagtcttg ggattataga tgtgagccac cactcctggc 90600

cattggcagc ttttgatttc ctgtcctccc aacttccaaa gtttatgaag agaaatctgt 90660

tgataatctt attaaagatt tcttacgtgt gatcagttgc ttttctctta ccgatttaaa 90720

gattctctgt ttttgacttt tgaaagtttg attataatgt gttttggtgt cggtctcttt 90780

aagtttatct tacttggagt ttgcctggct actgtgtttg tattcaggtc tttcatcaaa 90840

tttgggaagt tttcagccat tatttcttca aatattctct cttgcccctc tctccttctt 90900

ctttataaat tccacaatgc aaggctgggt atagtggctc atgcctggaa tctcagcatt 90960

ttgggaggtc gaggtgggag gatcacctga ggtcaggagt ttgagaccag cctgaccaac 91020

atggagaaac cccgtctctg ctaaaaatac aaaattaccc aggcatggtg gtgcatgcct 91080

gcaatcccag ctactctgga ggctgaggtg ggagaatcgc ttgaacccaa gaagcagagt 91140

ttgcggtgag ccgagatcac gccattgcat tccagtctgg gcagcaagag cgaaactcca 91200

tccccccacc ccccaccccc aaaacaacaa caacaaaaaa ggaattccac aatgaatatg 91260

ttggtccact tgatggtcac ttaggctctg ttcacttttc ttcaattttt ttttatttct 91320

gttcctcaaa ctggatgatt tccattgtct tatcttcagg ttcactgatt gtttcttctg 91380

cctgctcagc tttcttcaaa tttgactttg aatccctcta gttaattttt tatttcacct 91440

attttacttt tcagatccag cacttttttg tttctttttt gattttctat cctttattta 91500

tattttcatt ttgttcatgc attattttct tgtcttgtgc tacatcttcc tttagttttt 91560

gggtaccttt aagacacctg tttcaaagtc tgtcaagtag cacatccagc aggttgtttt 91620

tttctctctc tctttaatta tttattattt ttcttttcat tttcttcccc catgaccaaa 91680

tcttaacttg actatcaggc atttttcagt gagagtttct gttggttcat ttttttttat 91740

ttatttttat tgttgttgtt gaaaactgga catttgaatc tactagtgtg gttaattcca 91800

gaaattagat tctcctcctt cctcagggtt tgctgttttt ctgttattgt tttgtttttt 91860

gattattgta ggttgtctct atgccaagga tcagcctgag gtataaactt atggtcttca 91920

cagatgtttt ctgagcctga acctttccct gggtgtgtgg tcactcccta attttaccta 91980

tatatgcagt tattcttgaa tattttatcc tttaatgtct ggctcccaaa aggggaaaaa 92040

gagaaaaatt gagggagggt gaaaaaaggg caccatccct ttaaactttc tggaagttgc 92100

ttcagcttag ggtgaggagc ttgctacaat gggggaaggt acagcagtag ctgccagcct 92160

ctttgtctct gtgctgcaat cagacattag agaacagagc cccaatgtgt gtgtgtgtgt 92220

gtgtgtgtgt gtgtgtgtgt gtgtgtgtgt gtgtgttttg agatggagtc tttctctgtc 92280

acccaggcta gagtgcagtg gcatgatctc agctcactgc agcctccgcc tcctgggtca 92340

agtgattctt gtgccccagc ctcctgagtg gctgggatta taggtgcctg ccaccacacc 92400

tgactaattt tagtagagac agagttttac catgtgggcc tggctggtct tgaactcctg 92460

acctcaagta atccacccac ctcggtctcc caaagtgcta ggattacagg catgagctac 92520

tgcacctggc ccagaccccc agtgtttgga gacagcattc tttttgccca ttctggatcc 92580

tgaaagatgt gtgcagctta ctgtaggaac acaagctgac tgccttgggg gtagtgttgg 92640

gtagctgcta ttgtgctaag ggccgaaatt gactgaaatt acctacaatt taccatctgg 92700

gccttcctct ggaagttgca agccttcagt aggctccaga gttctgaaat atttacatct 92760

gacagatcct gcccatgcag ttgttgtgta gattccaggt gcttctcatt ccaccatctt 92820

cccaggatac cctctgacct tctggatttt gaattcagtc aagctgttct ctaacagctg 92880

gggtgaaagc caacaaagat cctgctaaga aagctccctg gaaaaaaaat gttcaaaagt 92940

aacacaaata tcaaactgga aagaaattga tttaggaacc gaacccaggt tgtcatggtg 93000

aaaaaaggag cagaacctta gctaccgaac tgcagcctgg ggtatgaggt gacaaccatc 93060

acttttttag tttggtttgg ctagcaaagg tggccttgtt atacaaataa agccactcag 93120

gtagtcaaaa cctttatttt ctgttttttt tctctctctt ttttttcttt tttgagaagg 93180

agtcttgctc tgtcacccag gctggaatgc actggcgcaa tcacggctca ctgcaagctc 93240

tgcctcccag gttcatgcca ttctcctgcc tcagcctccc aagtagctgg gactacaggc 93300

acccgccacc acgcctggct aattttttgt attttcagta gagacgacgt ttcaccgtgt 93360

tagccaggat ggtctcgatc tcctgacctc atgatccacc tgcctcggcc tcccaaagtg 93420

ttgggattac aggcatgagc caccgtgcct ggccctatgc ctggctaatt tttaaaaatt 93480

ttttgtagaa gcagggtttc gccgtgctcc ccaggttggt ctcaaactcc tgagctcaag 93540

tgctccaacc accttggtct cccaaagtgg tgggattaca ggtgtcaacc actgcaccta 93600

gcctctaaat ccatataaat caattattca cgcactgagc agaatattca ggattttttt 93660

tcttataata taaatgatgt ggaaaactgg agaaccaaag aagaacacca agactatcta 93720

aaagtgttaa atttttcatt agaaagaaaa tgacttcatg acagttctta agacaatgat 93780

agaacttggg gaaatatgta ttccttgatg tttctatgca tttgcaataa taaacaatga 93840

aaatatggtt aaattttaga aagagatagg cctgtggtat tgtcacaagg gtatacaaat 93900

actgagaatg actgctgagg cagtgctctc cattggtggt gaccttcaga agagacctgt 93960

gtttccacat ggcctagaca cctggaaggc agagatccta acattccatg acacccccac 94020

cttctaattc tgttattgtg actccagacc attacctggc accgggtctg atttctggca 94080

ttcctatcag agcctgagtc acagacagta ccttagactc taagcttagg caccttgtgc 94140

cttgtttttg tggctaagga ttctcagagt tgtaggatgc tgacaaagtt tatcccaaag 94200

gtaatttatg aatcagatct gctttctttt gacttgttta aacccttcat ttaatgtaaa 94260

gaaactttat tgaggtataa tttacatact ataaaattta cccattataa ttgtacagtt 94320

caataatttt tagtaattta cccaagtagt gcaaccatta ccacaatcca tgcatttgtt 94380

tttaatttat taattgtctg ggtttattct ccctaggctc tgattgtact ttctccacaa 94440

gcttcagcat ggcagtatct ctcaccactt gttttggtcc aagggcagaa atgagcatcc 94500

tggaaaccaa tcagtacttg ctctctgaac tggaaaagtg caaagagaac ttccgagacc 94560

tcacagagaa attcctgaca tccaaagcta ctgcctactc cctggccaat cacctgcaga 94620

aatatagtag gtcccatagg ggcatggtca ccaaagtgat aaatgattgt ctgtctttcc 94680

tctgtgaaat gaaacactgc agactttatt ctttatcaaa attaatttta tcctaactac 94740

actcccagaa aggtagaagt gggcctttta ctctcaattt gccaaggaca gaaaaactga 94800

ggcacaaagg tgtaaagttt gcagggcgag tgtagtgagg aatagagact aaaaccttgg 94860

tttaggcatg aaactgtctt tcccctctca gcaatgaata tcagattcta caaacatgta 94920

acatcactgt tggttaaaac atctcaggat ttgatttaaa cttttttaag agcgcccatt 94980

tgcaaagcac tgtgccaaca gcaccaggga tgaaagaagt aatagtatac cctatctgcc 95040

ttgaaggaac ttaaaactac ctattctcaa ctgagtcaca gatatgtgga gatgcaaact 95100

tcaatagcag acacggagaa aaatctttat gatacctgga tcaagattct cttcccacat 95160

aagttcacat aagccattcc tcatgttctg gttagattga gggtatttcc ataaaaatca 95220

gtgactttgt tacacccttt ctgtctctct ctgagtatca actttacaga cagcaagcaa 95280

ctgcaggtgg atgtccagga taagagctat gagacatatg gccaaaatga gaacaaggcc 95340

aagtggaaga gaccgccatt ctcagtagac ggccaggcca ggttaggggc tggccctttc 95400

ctctccacag ttagctctca catttttaaa tccaatgtaa aatcccaaac ctgttactaa 95460

tagtcggaga agggggaagc tataatgaca aggaaacatg ggaacaaggg catgagagga 95520

acagggatat tagaagagga ttctggttaa cattgaaatt gagtttgctt tccaatttct 95580

acaattgttt ttaaataaat catcgtgtgg ggggtggtta aatttgctgt ctaacttact 95640

gtcatagaaa agtaaagcca aaagcccact tattaaagaa gtgaaaagat gagctttggt 95700

ttgttgaaaa actagttaaa aaaaaaaaaa acttcattca ttataattgt aggaaaaata 95760

tttctcaagc tagagaagaa gaaagatatt acctaagacc ttagtataaa ggtattattg 95820

tgcagtgatt aaagagcata taccctaggg ccaaatctcc tgggttcaaa tctaacttat 95880

tgtctctgtg cctcagtttt ctcatctgta atggtaacag agtaggaact acctctttgg 95940

atagtggtaa tgatcatatg atcataattc ttaaaaacct actgcaacag taccatgaca 96000

aacactatgt gttggttctc cctttaaaac actattagca tttgggcata tatcatttgg 96060

atctttctcc tccatataca gttttacaca gttgtataca gataaatgtt taaatctttg 96120

aaatattttc gaattattga aactcttgaa acaggagaac catcagttct acagttctta 96180

gtatcttcct aaatacacag gaagtcatta tttcatgccc ttatgttcat gttttactta 96240

agaggctgta aagcttggtg aattggcctt aagatttgaa gtcatggccg ggtgcagtgg 96300

ctcacttact gtaatcccag cactttggga agccaaggtg ggcggatcat ttgagatcag 96360

gagttcgaga gcagcctggc caacatggtg aagccctgtc tctactgaaa atacaaaaac 96420

tagcagggtg tggtggggca cacttgtaat ctcagctact tggaaggctg aggcaggaga 96480

ataacccggg agtcagaggt tgcagtgagc tgagatcgtg ccactgcatt ccagcctggg 96540

gaacaaagtg agattccatc tcaaaaaaaa gaaaagaaaa gaaaagaaaa agatgtggag 96600

tcagacccca tgggacgtga attctgactg cttcattcca gttgaatcat cttgtcaagt 96660

tactttatcg gcctgtgggc ttttctttcc tcatctctaa aacagggagt agttgagaga 96720

ctgaggaata aagatatgga aatcccagtg taaagcctgg taccggggat acttttatcc 96780

ttgagataga ccctgcctcc tgccctgcag gcagtgacca cagcagcatg gccagccttc 96840

catgatgcag gcatgtctgt cttctctcag agtgtgaaga gtgcaaagac cttatagaat 96900

ctgtgctgga ggaggagctg cagtttcagg agagggagct gaccaaattg ccaaggccag 96960

cagcaaggct ccggtaagga ggtacctctg tgggaagtgt ataaatactg tttgaggtgg 97020

ggcagctcag gcaggtagaa caaagagcaa atttgtgcaa ggagaagggc agaaatgtat 97080

gaggcaggta cagaactaga ctaagacaca atgaggctat agtctagtta ggtctctagg 97140

gttttccatt aaagaacata ttagtaaaat ggagacagct gcctgcattg gaatactagc 97200

aacgtggctt ctatttgtgt atccttggtc aaatttactg cacttttctg catgtcatta 97260

acctaggcta cctcatttat aaaacggaaa tactgacagt atctacctta gagggttatg 97320

aagattaaat gcacaattgc aagtagttaa aaccatctct aacacatata aaactctgaa 97380

caactatttt ctgtaattat tttatttgtt cccatttata gatgtggtga ttgtcattaa 97440

catttcctat aagagatatt ttcaaacttt ccaaagtgcc tttcatgata agaaagtttc 97500

tcctccaaac ttttttaaaa agtattatac taggtgaagt attaactggt agggctattg 97560

ggtagataga tgattgagca gctgttgtgt ggaaaatgaa ttattaattc actcaggacc 97620

agcattgact atccttagag ggtttcagac ataaggtaga ttgtagcatt tgaaacttct 97680

tttaatcctt tgttgaaatg aaatcataga agctcaaatg tgtaaaactc accaaagcag 97740

agatatttga ttgaaggagg gataggtctg gagcccctgt ttctcgccag tatgtgtctt 97800

tacttcccct gacactgtct agtaactctg tcgaatagag tttgaaagag gacctgcaga 97860

taacaacttt gaagaatagc agggagaaaa ttcaagcatt agatggggta gatgactgta 97920

aatgttcagt ccagctctgg gattacatgg ttctaaacat taactgaata actacttgta 97980

tgtgtacata ctaccaggtg ctgtgagttg ggtaataggt ggcactgtct ttagactcac 98040

agagctcaca ctctacattg gaacccatgt gtattagtcc attctgacac tgctataaac 98100

atgtacctga gtctgggtga tttataaaga aaagaggttt aatcggctca tggttctgtg 98160

ggctgtacag gcttctgctt ctgggcaggc ctcaggaaac acaatcattg cagaagttga 98220

aggggaagca ggcatggtct tcacatggcc agaacaggag agagagagag aacaaagagg 98280

gaggtgctac actttcaaac aaccagacct catgagacct cactcactac cacgagaaca 98340

ggaaggggga agtccacccc cgtgatccag tcactttcca gcaggtccct cccccaacat 98400

tggagattac aatccaacat gaggtttagg tggggacaca gagccaaacc atatcaccat 98460

gagaaatatt tgaacaataa ttagaaagca atgaaatccc acatagagta atgaagttac 98520

tactcagaac attacttctt ttttttgaga tggagtctta ccctgttgcc gaggctagag 98580

tgcagtggtg tgatctcagc tcactgcaac ctccacctcc taggttcaag cgatcctcct 98640

aggtcagcct cccaaggagc tgaaagtaca ggtgcacacc accacaccag ctaattttag 98700

tatttttagc agagatgggg tttcgccatg ttggccaggc tgctctcaaa actcctgacc 98760

taaggtgatc cgcctgcctt ggcctcccaa agtgctggga ttacaggcat gagccactgc 98820

acatggccgg aactttagtt ttaactgcct tcttactgat tatggtctac cttcatgtca 98880

ttcaaaagaa tactgccctt cttgaccttt tcactgattt ttctttttaa ctttcaatta 98940

ataccagaga tacctgccca acacttttga atctggcttt ttttttttct gccagttact 99000

gcataatgct tgatgtatat tgtatgaaaa tgtacttggg ctaaagtggg gaaccaaagg 99060

ggcatctttt taaagcaaag gtagtaaaat aacagacaaa cccaaggctg gttcttcatt 99120

ccttctgcca caggcaagtc acacaacttg tacaagaatt aatcttgggc cagatgcagt 99180

ggctcatgcc tgtagtccca gcactttggg acaccgaggt gcacgagtca tttgaggtca 99240

ggggttcgag actagcatgg ccaacatggc aaaaccccat ctctacaaaa aatatataaa 99300

ttatcctggc atggtggtgg gcacctgtaa tcccagctac ttggaaggct gaggcaagag 99360

aattgcttga acctgggagg cggaggcaga ggcggaggct gcagtgagcc gagatcttgt 99420
cactgcactc cagcctgggc gacagagtaa gattctgtct caaaaaaaaa aaaaagaaaa 99480
aaaagaattc aacttgggag ttccttacag ggattctaag agatctctcc tctgaaactt 99540
ctataaccta ttattcacta cattccctct tacattttac tcttctttcc caccttagga 99600
tacatgatcc cttaattcag gctcaggctg aagaactgac ccacttacga cagaagatac 99660
aagaagggag aggtgtctgc taccttttca cccagcatgt gaagaacaca gtcaagtctt 99720
ttgagggcct tctcaggaac actggcattg cctactacca gagacagaga ttctgtgagc 99780
aaatggtaca aggaagccag ctaacagaga tccttgtcag aaaacttgcc acaggtaagt 99840
tggctacagg ctctgaagac ccttacctcc tcccaagtgc cccaaagtgg ggagaaaaaa 99900
tgcaaagtta tctccattgt agattttatt ttagttttga ctatggtcct taatctgcat 99960
tctattgttt tctctttaga gcactcaggt aatcactttt catattcttt caagagtcct 100020
gagttttgtt ttttattttg agacagagtc tgctctctaa cccgagctag agtgcagtga 100080
cgcgatcttg gctgactgca gtctgtgcct cctgggctca agcagtcatc ccacctcagc 100140
ctcccaagta gctgggacta caggtgtgtg ccaccacacc tggctaattt tcatattttt 100200
tgtagagaca gggtttctcc atgttgccca ggctggtctt gaactactga gctcaagtga 100260
cccatctgct gtggcctccc aaagttctgg gattacagat atgagccact gtgcctggcc 100320
tgagagttct tagtttcaat cagtaagaga ggtagagtat agtggattac ttcatcttga 100380
tgagcataag aactcacctt catttctgga agatattttc actggatgta ggattctagt 100440
tgacagtttg tgtttttaaa aagtgtttga aaagtgatat tccactgtct tctggcttac 100500
attgtttcca acaagaagtc ttccataatt cttatttttg tttctctgta cataatatgt 100560
ctttttctct ggccgctttt gagattttct gtttatcact ggtttgaagc agtttgatta 100620
aggtaagacc atgtatagtt ttatctttta agtattgttt tactttattt agtttcttgc 100680
atctgcatgt ttatagttct catcaaattt gaatagtaat gacatatata gtcttatcta 100740
tggcatatac agtatatttg gcatatatat atcaaatata tataatgtat attctattct 100800
ttctctctat cctctccttc aaagactcca gttatatttg tattagctgc tttgaaattt 100860
taccatagct gactaatggt ctgttcattt ttgttgtatc ttttttttct ctctttgttt 100920
ctttctattt ccatgtctcg gaattgatta atctttcttt agcagcatct aatctgctgt 100980
taatcctatc cagtgtattt tcatctcaaa ctttattttt cttttttttt ttattttcca 101040
ctttgtcatt gtgtgttggt tttttttttt ttttttttta ttatacttta agttttaggg 101100
tacatgtcca caatgtgcag gttagttaca tatgtatata tgtgccatgc tggtgtgctg 101160
cacccactaa cacgtcatct agcattaggt atatctccca atgttatccc tcccccctcc 101220
ccccactcca caacagtccc tagagtgtga tgttcccctt cctgtgtcca tgtgttctca 101280

ttgttcaatt cccacctatg agtgagaata tgcagtgttt ggttttttgt tcctgcgata 101340

gtttactgag aatgatgatt tccaatttca tccatgtccc tacaaaggac atgaactcat 101400

cattttttat ggctgcatag tattccatgg tgtatatgtg ccacattttc ttaatccagt 101460

ctatcattgt tggacatttg ggttggttcc aagtctttgc tattgtgaat aatgctgcaa 101520

taaacatacg tgtgcatgtg tctttatggc agcatgattt atagtccttt gggtatatac 101580

ccagtaatgg gatggctggg tcaaatggta tttctagttc tagatccctg aggaatcgcc 101640

acactgactt ccacaatggt tgaaatagtt cacagtccca ccaacagtgt aaaagtgttc 101700

ctatttctcc acatcctctc cagcacctgt tgtttcctga ctttttaatg attgccattc 101760

taactggtgt gagatggtat ctcattgtgg ttttgatttg catttctctg atggccagtg 101820

atggtgagca ttttttcatg tgttttttgg ctgcataaat gtcttctttt gagaagtgtc 101880

tgttcatgtc cttcacccac tttttgatgg ggttgtttgt ttttttcttg taaatttgtt 101940

tgagttcatt gtagattctg gatattagcc ctttgtcaga tgagtaggtt gcgaaaattt 102000

tctcccattt tgtaggttgc ctgttcactc tgatggtagt ttcctttgct gtgcagaagc 102060

tcttcagttt aattagatcc catttgtcaa ctttggcttt tgctgccatt gcttttggtg 102120

atttagacat gaagtccttg cccatgccta tgtcctgaat ggtaatgcct aggttttctt 102180

ctagggtttt tatggtttta ggtctaacgt ttaagtcttt aatccatctt gaattgattt 102240

ttgtacaagg tgtaaggaag ggatccagtt tcagctttct acatatggct agccagtttt 102300

cccagcacca tttgttaaat agggaatcct tttcccattg cttgtttttc tcagatttgt 102360

caaagatcag atagttgtag atatgcggca ttatttctga gggctctgtt ctgctccatt 102420

gatctatatc tctgttttgg taccagtacc aggctgtttt ggttactgta gccttgtagt 102480

atagtttgaa gtcaggtagt gtgatgcctc catctttgtt cttttggctg aggattgact 102540

tggtgatgcg ggctcttttt tggttccata tgaactttaa agtagttttt tccaattctg 102600

tgaagaaagt cattggtagc ttgatgggga tggcattgaa tctgtaaatt accttgagca 102660

gtatggccat tttcacgata ttgattcttc ctacccatga gcatggaatg ttcttctatt 102720

tgtttgtatc ctcttttatt tccttgagca gtggtttgta gttctccttg aagaggtcct 102780

tcacttccct tgtaagttgg attcaaactt tatttttcat ctaaatttaa tgtatgtctt 102840

tctttaaggt cttctatgta actcttcatc atttcgtatt ttgatctagc ttctttattt 102900

tattttattc tattttttga gacagagtct cactctgttg cccaggctga agtgcagtgg 102960

tgagattttg gctcactgaa accttcacct cctgggttca agtgattcct atgcctcctg 103020

tgcctcagcc tcctgagtag ctggggttac aggcacatgc ctccacaccc agctaatttt 103080

catatttttc agagagacgg ggtttcacca cattggccag gctgatctca aactcctgac 103140

ctcaggtgac ccacccgcct tggcctcgca aagtgctggg attacaagca tgagccacca 103200

caccagccta gcttctttaa tacaaggaat atagttatgt ttgtttcaat gtgcttgtct 103260

acagattctg tcatctgtgt cctttttatt gttttttaat tctcacctga tgtgtccttt 103320

ctaagtcagt ttaagtagat tgattttttc ccccttctta tcatggatta tagttttctg 103380

tctgtctgca ttcctaacat tatttgattt ggggttagac gttgcaaatt gtaacttact 103440

gagttctgga tttttttgaa tttagtgtta aaattatttt agctttgttt tggtatacaa 103500

taaagttact taaaaacaat tttcatcttt tcaggatggc ttttaaattt ttttaggcag 103560

gataagagca gctattagtc aactgctatc tttttcccac tgtggaggca aaatgacttt 103620

taagaaaata gaccttattt tttagagtag tttcagattc acagcaaatg agcaggaagt 103680

acaaaaagtt cccatatacc ccctgcctcc acacatgcat agcctcctag attatcaaca 103740

tctctctcca gaatggcaca tttgttacag ttgatgaact tacaatgaca tgtcattatc 103800

acacaaagtc catagtttac attgggagca ataatctttt gaagattcta agtcagtgat 103860

ctcccacaga tttggcacca gggactggtt gcatgaaaga caatttttcc acagacaagg 103920

tgggggtggg gtggtaggga ttgggaattg ggtggtttca gtacgaaact gttccacctc 103980

agatcatcag gcactagtta agagtctcat aaggagcgtg caacctagat ccctggcatg 104040

tacagttcac aatagggttt gtgttccaat gctaatctaa tgtcatcgct gatctgacag 104100

aaggctgagc tcaggtggta atgctttctc gctggctgct ccccttctcc tgtgcagccc 104160

cattcctaac aggccatgga ccagtgctgg gggttgggga ctcctgttct aaccaatgct 104220

ccctctatta aaaggatttt tcactctagc tgatggaaac accaagtgtt gcaagctatg 104280

tgtgagctac agagattatt ccttttggga agttctttgc cctgctttgc ataatttcct 104340

caaatacatg tactaatcag tacttagctg aagattgaag acagatatcc aaggcattct 104400

atctgtgcag caacctattc tctagtctgt cctgtgatgt ctagccaccc tgacttcttc 104460

aaacttacta ccaggctctg cctgggtcct ccctccctgc tctacagttg acaatctcag 104520

gcagcaaact ggagcaatta tagggctcac ctcttttatt tattatagta ctgcactata 104580

tccaatgttg aaggtagagt ttaaaatact ttttccagtt tctcagttgt ttaaggtgga 104640

ggacaaatca ggtctccatt actccaaatt ggccagaagc agaagtctcc attccatctt 104700

tgtggtcaga aagaacagta tgacagaagg cagcttgtta gaatgggaag acccatgggc 104760

taagaatcag cattacctag gctctagtct cagcactgta tttactaact ttgtgcaagt 104820

gattgatcct tcttgaattt ctgtttcccc atttgtaaag tgggtcaaat cataattcca 104880

tggctgctgt acagatcagt tgaagtagta tcttaatgtt catgagtgta tttctctttc 104940

agggttattt atttttttat ttttgggtcc caataaaccg tccaaatctt ttggggttag 105000

ttcaggcact gtatatagta gaagctggtg ttagaaaagt tgatttatac tggaatattt 105060

ttcacagaat caaccccaat gctcactaac aaactgagaa gaaaaaaaaa gaattacccc 105120

caacaacatc tgaggaagca gaacagggac agacttgaag gcacctgggc agtcctctgg 105180

ttttggagta gaggtaggac agggctattt gtcctctcag agactggagc aggttctgtg 105240

tgtgagtagc aatgggacaa acagtcgtgg tctggcaacc agcctgtagc aggaaggaag 105300

gttacaactg gagaagtcca aagggcaacc aggcaagtat tctttgagct ggggaacaaa 105360

aggcatttta aactttttgc tctgcttcag gcagctgacg tctggtgatg attcaccata 105420

tcaggaacac cctgtccagt gggctcaggc aagcctgcca ggccacttct ctaaagacac 105480

aacctgaggc ctcctgcaat aattctcaca gagcattctg aacatacatg taaggtgtta 105540

tgtccctgat catatagggc ccatgtgtgc aatgtgtgaa atgggacctg cttaaattct 105600

gactagctct ttctaatttt ggcttgcaga aaatcacaat ggtaagaaaa tgaagacagg 105660

caaaagccac tggctcccag gtaactctac atgatgaagg ggctgataat ggactgacca 105720

aatctagaga gaattccaga agcaaggact caagaggtta aaggtcccag atctagaaaa 105780

agcagaaact gctgattgtt cctttttttg ttgactgatt atacaatgtg tccttttaaa 105840

aatggttctc tgttctcttt gcagttgttg tattggttcc tagttcctag taatttctca 105900

cacttagtta accagtcagt cttaccgaaa agtaaactaa ccagggatcc ctggtttctg 105960

tatctttaaa acctataggg ttacaaacca aagtgagatg cctatctggt ttctgcacag 106020

ttgtcctgaa ttatgttggc agatatttta tagccagaaa tttaccttga aaaataaatt 106080

gaaaccatac caagtattta gagctagggg tctaaaatct gcaaaagccc tgggaggttg 106140

tgttgtctca agagccagta ttcagtggac cactccacat catattaaat aaaatttaaa 106200

cagcattttg aagccgacag tatgggtcac tgtgtgtcct gtgtgccgtg agcgtattgt 106260

agagtagttg ttgagtctgc tgccttatct gtgtgttatg aggtcaatgc actgatcact 106320

tgcttttttc acttctatac taactctagg cacactttac tcccagtcaa aagtggactg 106380

tatacttcct ctttgatgtg atggtccctt tggttttggt gcctcttcca tgagcttcag 106440

atcagaacta gctatgtggt tccaggctgc cctggtttaa tcccttgtct tgcctacttc 106500

acctggctca agagggaagt ccaggaggaa gaaatggatg aagtcctgga ggactcacta 106560

gatgaacagt atttgactca ttccagctgc catgactccc accagcttcc cagcagcaat 106620

gccttcctct ttgatgcaca ggaaggcccc tcggctgtgg aaatagccag taagtgctcc 106680

tttgataaga aaaataaagc tccatcaatc tctcagtagc tcctacattc tacatgctca 106740

ccacctctgt ctaaactgag agatgttatt ctctgcaggc aggccctaaa cattcacttt 106800

aaaaaagtga ttaggctgaa cacagctctg ggatcaactc tcaagtacag gtccctggtg 106860

ggtcaggtaa tttgctctgt tcctgtcctc acctcagtcc ctctggtcca gacgggcatc 106920

tgcaagccgg tatactcaaa gcaggccaga atggagagag tgaagagatt atggcagact 106980
ctctgccact actgccacac atgtacaaat ctgtttaaca gatcttcttc tttaaaatgg 107040
gacatctcat ctttcctgaa atatatatat tttttgagac agggtctctt gttctgtcac 107100
ccaggctaga gtgcagtggc atgatcttgg ctcaccacaa cctaaaaccc agggtcaaat 107160
gaccctccca cctcagcctc ctgagtagct gagaacacag atgcatgcca ctactcctgg 107220
ctattttttt ttgccttttt ggtagaggtg ggttcttgct tttttgccca ggctggcctc 107280
aaactcctga actcaagcaa ttcacccacc cctgcctccc aaagtgctgg gattacagat 107340
gtgagccact gcacccagcc ttcctgaaat attcttggta attatagttc ctgaacagtt 107400
ttcccaagct gttctagcca tcccaagaat gttggcaggc ttgtccatat gtttggaggt 107460
gcaattataa tggttcctgg gtttcaagct ccagtctcca tctcccctca ggtgcttgct 107520
ttcccataag gttccatctg actactggaa tatcagggga tttccataaa gacaagactt 107580
tgatcttggc aaccctttgg taaatagcca gggttctcaa tctttgtgca gcaccaaggt 107640
atttttaaga tgtctgttag tttagtctca gtgttgtaat tccatctaaa taaacaatga 107700
ggctatctgc tggagaaaag gctgaatcta agagcaatgc tgccagcatg ccctcttccc 107760
agaataactc cacgctagcc attggagctt cattaggtat ataagagccc tttggtttgg 107820
tagcctctct ggtgtgtgta tagattaaat ccttgaccta tatctcctct cccttacttg 107880
aatgtttgac tttactttgg gggcaaaagt ttggattgtt aggagactat atgctttata 107940
tactcttcta tgcaataaaa agagattata ttaattcact cattgagctt tcacagtaat 108000
gtaaaccata ttaggcttgt cattctgtca gtgatgggtg taagtttgtg gctttaatta 108060
gtcataccat cttctgacaa aggcatcctt atttctcttt ccaatttcac agtaaggtgt 108120
agaagaaggt ctttaccatc tgttaaagat gttcagggcc gggcacggtg gcgcacacct 108180
ataatcccag ccctttggga ggcctaggtg ggtggatcac ttgaggtcag gagttcaaga 108240
ccagcccggc caacatgctg aaaccccgtc tctactaaaa atgcaaaaat tagctaagca 108300
tagtggtgca cgcctgtagt ctcagccact tgggaggctg aggcatgaga atcctttgaa 108360
cctgggagac ggaggttgca ctgagccaag atggtgcctc tgcactccag cttgggtgac 108420
agaatgagac tctgtcaaaa aacaacaaca aaaacaaaat atgatcagga gagatatttt 108480
gtagagagga agatattccc tagtacctat aagtcatctc aaggaggtgg tcataccttt 108540
tctagtgcta gaaaaggcct actcagagac cttcagtgtt ttcccacctc ctgtcaacct 108600
gccttatact tcccatatct ccatgtggga ggcatccctg tggagtttgc ttagcacttg 108660
gccatgcttt ctttgaaacc ttaaaagctc gtaaagaaag ttgccttatg ttccctgatg 108720
agaagatgaa gttggcaaaa gaatggtgaa aggcagggat cttgatgaga ggcccctgac 108780
tatcccttta gtgatcagca tcttcacttc ccatctcatt ccatgtcctg gtggaatggc 108840

tgcagaagat gagatccaga gccagtggca gcacctgaaa gagatccttt tcatcaacaa 108900
ctgcctgtga gaaaagctag aacatcatca cagcagcttt gacgaagaaa atggtaggag 108960
aggcccaagt cagcctgctt ctggcaatat gtatttttct aaaggcatgt gactggggca 109020
gagttttata gatttcaagc actttgacat actttacttc acttattcat tacaaatgtc 109080
ccagctagag aggtagggca gctactatca ttcctattta taggtaaata aataggctta 109140
aagatttgcc ttcatcttag tggtatctac ctctaaaatc attcttgtga ttcctaggct 109200
gtcttctgtc ttctgtctgc tgctgtacac tgcctgctgt ctctctgctt cttatgtgat 109260
tggcattcag tagaacccag gtttctcagc acctcacgta gcagaatgag agtctcctct 109320
gctgccatcc tcatcagttt cccttatgtc ttggtgactc ttcattctac caccccaagg 109380
tggttctctc caacccccct cctcttagat tgttctcaca cagaggccgt catttcataa 109440
cattgtgctg ggccagaaga atcataagca ggtcccttcc cataccaaag cagtaactat 109500
tttggaaaag cctccttcat attgcagcca ttggacatga taatacatgt gatgtgctgg 109560
catttgttga gaatcatgtg aaaattaagc ttccacccca tcacactaac gttccagccc 109620
tccacggttt tctccctagg ctaagataga gaggaagaac atgaaatgtg cagaaagcat 109680
tctcttaagc aaacgtgtat aacttgcgta aattgtaatt atttcctgga acaggatgca 109740
cctctaacct ctacaggcaa atcatagatt ccattgtcca gctgtaaaat gaaaacagag 109800
ttctcagaga agaataccgg agactccccc gacttgcctg aatcaaattt ccagaggtaa 109860
gtggtgaaag gctacaatat gaagtcactc tcagatgggt cccttctctt gccacactcc 109920
actgtagaaa taaaagagga cggggaggcc agagctgctc aaaaccttat aacccacaat 109980
tctcatagtc attctcaact tagagagggc tgctttctag acacaccccc tccaggcccc 110040
cagactgcca ggctccagga ataagtagct ctaagaaata cagtatttcg atgcgagcca 110100
caccccacta ttgcaaattg gagaagagaa ggacaaactt aaaaggtgaa aagatttaac 110160
agaacaacag ggtgaaaccc catctctaat aaaaatacaa aacttagctg ggcgtggtgg 110220
catgcacctg tagtcccagc tactcaggag gctgaggcag gagaatcatt tcaacccagg 110280
aggcagacgt tgcagcgagc caagtttgca ccactgcatt ctagcctgag tgacagagca 110340
tgactccatc tcaaaaaaaa aagaaaaaaa gaaagacaga aaagatttaa gagaacaatt 110400
attttaattg aatacatttt gtcaagatta aatcaacttt cccctagaat actgaaagaa 110460
taagcaatca taatttagat tctaagttac tggcctttga gaaagtgtga aatccaaaaa 110520
gatgccatga gccaaaaaag agcaaaagtc ctaattttca aactgatgaa aataaagtac 110580
taaataattg aaatcatcgt agttcatctt ggtccttggc aaaataaaaa tttattgatt 110640
gaaattataa tatcctagct acctttttgt gcctagctct taccactgag gagaaaaacc 110700

aaacacaaac aaaagtatgt tcaaattggc aaaaatgttt acaaacctgc aaaatctctt 110760

cagcctatcc cttaaactgc agacttggta tttactaagc tctgctgtgt acaataccca 110820

ccttaatatc cattcttgca caagttctgt taggtataag ccaaaatcac atgggatttt 110880

tttttttacc ctctcatgcc tatgtttgct tttgcttcca aagacccttc tgagtaagga 110940

ctgtcctcag actactggag tcacttagtc acaagcttgc ctgaggctca gagctggcaa 111000

tacctggcag tttatgtcaa tcccacatgc aaccctgcct gcaaagtatt tacccatgat 111060

tgacaggtgc aggtgtatga aagcccagct ccttgacttg aggggtgcgt ttgtggtgaa 111120

ttagctttgg gaaaagggaa tccagaggcc aactttgtaa aaatccagat aggtaaatgg 111180

agagaaatct ttgccttgga agtccaattg agaaagatag tgatttactt cggactgtga 111240

aaggagagac taaaatcttt actttctcat gggaaagaag cctatggtga aagagaaaca 111300

aagaaaatgg tgggaaatag ggtaggtcta ctcagtagct ttaaaccatt ctgtcatatt 111360

ataaagcctg gtaaggtttt aactaacaca agtgcctgga ctctacacat agatattctg 111420

atttaaatgc ttccaggtgg gtggaagaca ccagtagtct taaaaagctc ccaagtaatt 111480

ttaacatgta gctaaagatg aaaaggacaa tctatctact agaatgtcaa gtcgtcagca 111540

taggagtgtc ccaaatgtgc agctacctca tagaaaaggc ttaaccccac ccattgatgg 111600

aattcgcctt cctctctcca tggtcttgtt tgcatagcat ggaaaaaaag agaagagaaa 111660

aagaggtaga tggatagatt gaacctataa tttgccaatg tggagcagtt tctcaaactt 111720

ttctgaccac aagatgcagt gagaaataca caatttttgg tgatcagttt ttaaaaaatc 111780

ttttattatg tgtaatccac ccttcctggt atttaaataa agcctgtagt agtaagcagt 111840

ccactatatt gatttcacaa cccataagtt gttcagaatt gcaattagaa aatttttaat 111900

tttggccagg cgtggtggct cgcacctgta atcccagcac cttgggagga tcacctgagg 111960

tcgggagttt gagaccagcc ggaccaacat ggaggaaccc tgtctctact aaaaataaaa 112020

aaaaaaattt ttttaaatta gctgggcgtg gtggtacctg cctgtaatca cagctacttg 112080

ggaggctgag gcaggagaac tgcttgaacc ctgggggtgg acattgtggt gagccaagat 112140

tgtaccattg cactccagcc tgggcaacaa gagcaaaact gtctcaaaaa aaaaaaaaag 112200

aaagaaagaa agaaaaagac aaagaaagaa agtaagaaag aaagaaaatt ttttttcact 112260

tttgctcggc ctttttatca aggaaaatcc caaaagaaca tttactgtaa aagaacattg 112320

actataaaca tctttctgtg aatccttatt tctgtcccag ccaccatgac ccaataatct 112380

ctaaaacctg ttagtgttct ccagattgaa gccttccctc taactgctcc taaatctgtg 112440

atgtagtgga aaaggtttga ggccttgatt ctcagcccag gtttttcacc tacagatcat 112500

atggccttag cctcttgaca ccattgtctc cttatggcca aagatggaac tgtacttggg 112560

tggtatctga gcaccctgtt ggctttgaca ttctagggct cttcccttgg caccactctc 112620

ttatttaaaa ggaataggat gtggacttag aagggttatt tctctgcaaa ctatcctctc 112680

tttcttatga caagctattt ctgaatgtgc acatttgagg ctaagcaaat cacttcctgg 112740

gaggtgtgaa agacctttat ttctctccag aggagtatta tggaggtggt agagacctag 112800

ctaactcatc tgcagcggct ttgagcaagg cttttagaac cataaggtgg ctggggtcct 112860

gtgtcctaca ccacaggcca gctagttcat ttcaggagac tttcttttca atctattatt 112920

actatcttct tctagactgt agttcactac cagagattat ccacttagct gcactcagtt 112980

tacacctggc ctgtatcacc acttagctct cactctagcc tcagttaggt atcagcaaga 113040

cctcaccaaa gattattgtt tcgttgtatg tgtgtgtgtg tttttgtttg tttcaagtag 113100

caactaatgt tggaactatg gaaagttctt ctctactttt aacaaagctt agtcacaaac 113160

agttccccag ttgatgagaa aaactaaaac aacagaacaa ttgaaagtcc aaatctgaaa 113220

gctccatctc tgagaagcaa attttatagc cactccagat ttgtattcca aatggatagt 113280

ttgattatag aaatcacatc ccttcagtct gcaaatgtga taactgacca agagacagtg 113340

atgcctagat ccataaataa tcccctttcc cccaacctat atataactgg agtcaacagc 113400

agctagagga aaatggcaag gacttggaat caagattaag ttagaataac actgctgtag 113460

actgttcatc agctcttcag catatgtcca ttttccttga gggataagcc tttagaaacc 113520

tctgacaata aagtttattt tacacacatt ctcttgccta tgattttttt gtacaaatca 113580

caattacaaa actttcttgc tccagctaaa agcaggaaac tcagtcacaa atgtgttcac 113640

tacatgtata ccacagaatg atagcagcca gtctgaatgc atcacttgat tccaaaatta 113700

aatgttagca ctcagtagtg aaactacatg tatctccaac tctggaagcc ccaggcaaga 113760

tactcctgtt tcctgcaggg aaacagatct ataagcaggg cagcagtctc acacatgtac 113820

attcctggga aacccaagga aacaatgata gtgatgcagg gcaggcgagc ctcaaaactg 113880

gtttgtagcc cgggaaattt cttccagact gtagtttcct tggctttgcc aaggaaagaa 113940

ttcaagggca agccgctggt gaaagaaagc aacttttatt gaactggtgc tgctacttgc 114000

agagcagggc taacccatag gcaatgtgcc cagagttggt gcctgtgggc tgctagctag 114060

ttgtttttag acccactttt aattacatgc caattaagag gtaggttatt cagaactttc 114120

tggaaaaggg gcagggagtt tccagaaccg tataaagtaa cttccaggcc attgccaagg 114180

tgtgttgcca tgacatttgt aaactggcca tggtcccggt ggtagtgtct ttatgctcat 114240

aagtagtaag agcaactaga ggtcactttc atcatcatct gctggtttca gcaggcttct 114300

tcactgcacc ttgttttgac cagatcctgc tctgttcaat ggggttgtga ctggaaaata 114360

agtccttctg gtctcctacc tcatttcccc atcagagatt atatactcct ccttaatctt 114420

aaggggcttg tagaaggtgg gaggttcatc ttctgtaact gcttcctgct gatgttatgg 114480

gcatagaacc tgcctagcac tggagaagta aaatctctgg atacctgttc taaaagaccc 114540

aaaggcagga tgtctttatt ttctgggtca gaagagggga tgggttgaaa gccttgtgcc 114600

agcatcgtct ttacatggaa ttattgtaat ctagaagaca caaactttac aaggaggtta 114660

aacaagcaag gaccaaagat tagtaataac aagatatctg ttaaaggtcc taggaagggt 114720

aaaaatcaag tgtctgctgg tagagattcc tttattgaat tccatcagca gggcaaagcg 114780

gtaagagaga aaacattata attaggagta agagaaaaac tttcatgctc atccacagca 114840

tcatgttatt aatatttatc tgtgcatttg caaaacaata gctttaagtc ttctgggagt 114900

ttgcaagtat aggtcatggc atcttcctct gacatctgtg aggactcata agaatctttg 114960

aggctgggca cagtggctca tgcctgtaat cccagcactt tgggaggctg aggcaggcat 115020

ttcacaaggt caggagttca agaccagctt ggccaacatg gtgaaacctc atctttacta 115080

aaaatacaca cacacacaca aaaaattagc caagcctggt ggcacgtgcc tgtaatccca 115140

gctactcagg aggctgagac aggaggattg cttgaaccca gaaggtggag gttgcagtga 115200

gccgagatcg agccattgta ctccagcctg ggcgacagag caagacttca tcaaaaaaaa 115260

aaaaaaaatt aactttggag tttaacagca gtgggagtgc ttaataatgc ctgatagggg 115320

ccctttctat tttggttgta attgattctt gggggatcct tctttctaag tttttagtaa 115380

gactaagtct cctggttgaa tgggggagtt aatccttttc cttgtgggaa gttacgtttc 115440

atttccatat gattgaaggg ccttttgaac ctggcctaag ttgataatct atgtgtctct 115500

tcatcaaaca gaaggtctaa agttaaaaag ggccttccga gctgctcagg cgacccagtg 115560

ctcggttggc ccgggccgcg cttaactgtc ccaggtcagt taagcccgtg ggagcccagg 115620

tcatggggca gccagggcct ctgcagtgtg aggcgccggg ctgcgggcgg gcaggcggga 115680

ggcttagggt gggtcgcggt cgcggccccc gccgcaggcc ctggcggcgg catcgtgaac 115740

atagacgtgg agttccacat ccggcacaac tacccctgga acaagttgcc ggccaacgtg 115800

aggcagtcat taagtggaga tagctcatca tggaggtctt catctttgtc ttcacatcaa 115860

gtaggctgag gaggaggaaa tacaggaagt attggtcttg ctgttcagag tggcagagga 115920

gaaagaaaat cccagccgtc ttggaaattc acagagagaa tatgaaaatc aggttgtcct 115980

gtacagtatc tgcaatcagt tacgatacag aaataactta aacatgtcaa gaaagatgaa 116040

cgcagatact atgaggaact gctaaactac agccgagatc atctcatgct gtacccttac 116100

cgtctattgg atattatggt gaagagcttg agcataacac cattttcata ttacactggg 116160

attatggaga atattatgaa cagtgggcac aattttactg ctgctgactg tctaaggctt 116220

cttggcatag gaagaaacca gtatattgat cttatgattc agtgtagatc atcaaaaaaa 116280

ttcttcagaa ggaaaacagc cggtgatctt ctaccaataa agccagtgga aattgccata 116340

gaggcatggt gggtggtgca ggctggatat atcacagaag atgacatcaa gatatgcact 116400

tggcctgaga aatgtgctac tgataagacc gttgattcag gccctcaact ctctggatca 116460

ctagattaca atgtagtaca tagtttgtat aaaaaaggat ttatttatct ggatgtacca 116520

atgtctgatg acagttgtat agcagttgca ccccttgaag gttttgtaat gaatcgagtg 116580

cttattttga ttattttgaa actctactct ataagatatt tgtttcagta gatgagcaca 116640

caaatgtggc agagcttgca aatgtccttg agattgactt atccctggtt aagaatgctg 116700

tttcaatgta ttgctgattg ggctttgccc ataagaaggg acaagtaata aatttggatc 116760

aacttcattc atcatggaag aatgttccat ccataaacag attaaagagt accttagatc 116820

catagaagat gctcttgtca tggggtggag gggaaagtag gaggcctgta caagaagctt 116880

catcggcaac tgacactgat acaaatagtc aagaagatcc agctgacaca gccagtgtaa 116940

gaagcctgag tctgtctgca ggacacacga agcacatcgc attcctgttt gactccactc 117000

ttactgcctt cttaaagatg ggaaatcttt caccagttca gagcactggt gaaagagaag 117060

cacagagata ttttgatcat gcgcttactc tgagaaacac aatactgttt ctgcgtcata 117120

acaaagatct agttgtgcaa actgcacagc cagaccaacc caattatggt tttcctctgg 117180

atctcttacg ctgtgaaagc cttcttggtc tggaccctgc aactggcagc agagttctaa 117240

acaaaaatta cacactgctt gtttccatgg ctcccctcac caatgaaatc cggcctgtca 117300

gcagctgcac ccctcagcat attggaccag ctatcccaga agtcagctct gtctggttta 117360

aacagtacat ttacgtttat catatcactg gacaaggacc accatccctt ttattatcca 117420

aaggtacaag acctcgaaaa ctgccagata tatttcagag ttatgatcga ttgctaataa 117480

catcttggtg tcatgatcct ggagtagttc ctacctcaaa tgtgctcacg atgttgaatg 117540

atgctttaac acattctgca gttttaattc agaggcatgg tctgcatggg ataggagaaa 117600

ctgtccatgt cccatttcca tttgatgaaa cagaactaca aggagagttc actcgtgtca 117660

atatgggtgt tcataaagca ttgcagatac taaggaacag agtggactta caacatctct 117720

gtggatatgt caccatgttg aatgcttcca gccaacttgc aaatagaaaa ctcagtgatg 117780

cttctgatga gagaggaaaa cctgatttgg cttctggctc agatgttaaa tgggagtaca 117840

gagtcatttg aaatggtcat tgaagaagca actatagatt cagcaacaaa gcaaacctct 117900

ggtgccacaa cagaagcaga ttgggttcct ctcgagctgt gctttggaat tccactgttc 117960

agttccgaat taaaccggaa agtttatagg aaaattgcta cacatggcct ttgcagaaaa 118020

gagagccttc aaaacctctt acattccagt agaaaactct ctctgcaagt ccttaacttt 118080

gttcactcat tccaggaagg tgcttcaaca ttggatattc acacagagcc cagtttttca 118140

agtttgcttt cacagtcatc gtatgctgac atgggtgttc cacttcctgc aaaaaattta 118200

atatttaaag atggtgtctt atcagaacgg agtggatggt caccttcctc acttattgct 118260

aatctccatt tgcaataatt tggttacacc atttgctgct cacactttct gccttttttc 118320

tttcttaacg ttagctttat agtgtcagcc actaaaaagc atcctgctgc tgtggagcaa 118380

ttcttgcttt actaatatta aaagtttggg gaacatattc atgttttctg aagttttgct 118440

ccttattgca catcttattg caacaaagtg ctttttagca tccagcactg tattttttac 118500

cttgagacaa tctgcatttc ttttataaaa ctaagtatat actttatagg ctttatgatg 118560

actgttatgt ttaaaagcag tcactgtgaa aattgcaatg ataattttat atgttagttt 118620

atcaaacatc aatcttgttt aactttatat tttgttacct atactttggg ggatcaaggg 118680

aagagatgga actcttcctc tgaaaatgct tcttggtact taaagtagta aaactataaa 118740

acagtaaaca tccagtattg ggagatgata tgatagggca ttatgaattc ctatgggtat 118800

ctaaattatg tatgtcaatt ggacattgta gaaggtatgt aaatcatcat ggttatgtat 118860

aacttaacct tgatttataa ggtcttaatt cagattatga ctattcgttg acatctcatg 118920

agaagcttta gaaaactttc tatttttaaa caccatttat atgtggactt ctgttgtcac 118980

tgactttggg ctttatattt tcacagagtc tttatggaaa aagtagaatt tattttccac 119040

tcttgtagct atagctgctg cacattttca tcctgattta tttttttgtt tcttaccttt 119100

gatgttttca aaccaaggat tgtgatttta ggttagaatt acatattaga aacattaaga 119160

ctatgtcttt ggatcaaaat gctttagtga ttaacctact ttgaagacat actcttaagc 119220

aatctgtatc ttaaatttat gtgaatacat ttttagaaaa tgataaagaa aaatggaatt 119280

acttcaaagt gtttcttgag tcattgattc ttttagcatc tcaaatgtta attagaataa 119340

ttggaatcac tttttaaact tttcaagtta ccttccttgg gaagtttgtg tagtgttaca 119400

gtttagttta gctcctctta tagggcaatg gtttgctagt ttaaaactgt aaccaaatga 119460

actggtcaga caactcatat ctaaaacact taaaatgtta gaacgtttgg gaatgttata 119520

acctaagcgt ttttgctgat aactttttgt tatttataga catttgtgta tttaacatac 119580

ttacttctgg aaatatatgc ctttcctaaa acttaaccac acatccacta ccatggccta 119640

tgtatagaat tgaatatttt ggaccatgtt atctgtggca cagtcagtgc tgtgtttgag 119700

gtaaatgcag taacggttag ttttctactt tgccttataa aaggtagaaa ccatgtgtat 119760

gttatgtttg tctataaaag aaaaatacta atattaaatt atttcttatg actgtgagtc 119820

actcacttat ttttccaata attgatattg tacattctta gtgccattag gtatgtatgt 119880

atgtaacttt tacagttttt cagctgaaag ttgtattttt ttttaatcag ggctctttaa 119940

tctcatttta atttcctttg tttgaatgaa ctgtagttat tttctgtatt cctatattaa 120000

ccatctaaac caactgtaat gacacgtaca ctaatgaaga attgaacatt tgtatttgtt 120060

ggcagtgagc ccagttgttg gtgaatttaa agcttaaaat atgggagtga tttgctgcta 120120

tatttctttt gagagataaa ggaggaagaa atggaaccta atggtaatca tgaattttag 120180

ggaaagtact gaaaaaccat gcggtcccct ctggtttctt gtgttgaatg aggcaagagt 120240

aatcatctga ttcttagctg aagacttctc atactctcaa ggagggagag tgcattttta 120300

gagcttttag caaaaatgtg aaaagttcat gtttgcactt tgctttgtga atttggcttc 120360

attttactta tacattaact catgtaatgt cttcaatctt acaagtattg atccatttca 120420

acaaaaaggt aaatttaaaa tgcagacttt gttacttgtc aaagaagatt catgaaaaat 120480

ttatatccaa ttattttcca aatagttaat ttaatttggc tttttaccac attccttcct 120540

ttctttcccg acttctttaa tgtaatttaa accctggaaa acattcttta gaaaccaaga 120600

ggaaaagaag aacacatatc aaaaaagaca tataacttaa aatggtacat ttcacatcta 120660

aaatgggttt gaagaaacgc aactttatat cattaaaaaa taagatcatc tgatttcctt 120720

tatttctttt ttaaattatg taatcagatg attttatatt ttttgggggg ggcggaatat 120780

tggtttcttt tacttgatgt tttcagtttt ctctggcatt caagtttctt ttttgtgttc 120840

agtgtttcaa atacaatttg tatttaagga ttttaaaata ccaaactgta aatgagtaca 120900

gtggatcttt ttctgttagg atgttaatat tatacaatga aatctataaa gtgttgtcaa 120960

tttgatcatt gacacatatg acacgtttac aattaaactg tggtgttgat caagttaaaa 121020

aaaaggcgga ggccttccat aagtcatttc aaatggacta agctttaaag ttctctttgg 121080

agccatcctc acatgcaaaa gggttatgag taggaggaaa actcaggcct ctgaggtctc 121140

ctgaaagagt tttgctaatg tcacaactgg ctttctatgt tatctgaaga ttgagatctc 121200

catgaagagt gaagataggt agtgcttaag gctgaggcat tacctcactg ggtcacctta 121260

gctgggaagg gaggtcagtt atccccttgc aaacattttg gtaatccaaa tcttgggatg 121320

atttctttaa gtaagaattt agacacttcc aatacttttc ttgtccttgt ggggaaagct 121380

tctatccacc tggtgaaagt gtttataaat actagcaaat attgtagtcc cctgtaaggt 121440

ggcatctggg ttaaatctgt ctgccagtct tcactatggt atgttccgtg gtgttgtaca 121500

agtttaagca gggagcgggg tatggggtgg cttcctgatt ggtaaatctt tttatagttt 121560

agaacagtcc cttccccaag aatatttagg aaactaattt gaatggaaaa tcctgtccca 121620

aatgtgagga atcatgaaaa tgttaaatta tttcccattt gtcagcctca ggaatagagt 121680

ttgttctttt ctagcaagcg tctagagggg tcttactgga agccttttac tcagtccctt 121740

taatttcctt gggtgtatag tacggttcca ctgacatgga tgacctggta gtagcgcagc 121800

agcctgaaat accagggttt ccttagctgt ggccttagct gctctttcca aaagggaatt 121860

tcctctaata agaacagtgt ctcccttctg gtgtcccctg cagtgaatac ttgttatttc 121920

tttgggaatt ggatggcatc taaaagttcc aagatctgag taaagttgta tgggggatcc 121980

cttggctctt gatagtcccc tttccttccc tatggttgca tgagcctgaa gcacccggaa 122040

accatactta gagtcagtaa acacattgac tcttgagggt tttttttttc ttttttgaaa 122100

cggagtcttg ctctgtcgcc caggctggag tgcagtggca tgatcttggc tcactgcaaa 122160

ctctgcctct cagtttcaag tgattctcgt gccccaccct cccgtgtagc tgggattaca 122220

ggtgcccacc accatacccg gataattttt gtatttttag tagagacagg gtttcgccat 122280

gttggccagg ctggtctcga actcccaaca tcagatgatc cacccacctc ggcctgccaa 122340

agtgctggga ttacaggcgt gagccatcca acccaggctt aagtcttttc acagttggaa 122400

ggtcctgatt agagcagcta attctgcttt ttgagcagaa ttctgagaag gtaaaccctt 122460

ggcctcaatg acttcttgtt agctaacctt cctttcttac tccctcatga ataaagctat 122520

ttccatctct aaaccactca acactggggt tagacaaggg cttgtcttca aggttggacc 122580

ttctagagta gatctcttcc atggtttcca cccaggagtc aatgagttgg ggatctgttt 122640

cttgggatgt gaggtccagc aacagagtag cagggtttaa aaatcagcat actttaaggg 122700

taacatctgg ggtgtcaagc agaagagcct gatatttaag taactggccc cctattagcc 122760

atgggtatac ttttgcctct aggaccctct gtactgtact tcatggggtg gtggggggcg 122820

ggggtggtat ggcatctact tgttgtccca aggtaaactt actggcttct tccaccaata 122880

gagtgatggt agctacagat ctcaagcttc ctggtcactc agctgccacc tggtctagct 122940

gtttagagaa ataagccact ggtctgaggc aattcctgac ccttcgagtt agaacatcca 123000

aagctgtccc ttgttattca tccacataga ggatgaaagg tttttctaag ttcgagagtc 123060

ttgaagcagg ggctgtccct ggcttttctt ttaaggctaa gaatgccttt tgacagtctc 123120

cattgcaatt caaaggctta tgaccactca cttttggagg ttcatagagt ggttttgcta 123180

tgagcccaaa cctgggaatc caaacccagc agaatccagc tattctcaaa aaagaatttg 123240

agggacctgg agtgccagga tggtctcttt tcatccctgg gtcaagatcc tggtcccagg 123300

agtgagaatg tgtcccaaat acgtgaccct ttggacagaa atttgggcct ttcgggggga 123360

tacctgatac ccttgttttg tttcaaaatt aaggacttga agttgtatgt ttctcagagt 123420

cttcccctag tagggctgga aattaatagg tgatacacat atggtaagag ggatccatta 123480

attaactgta gttcccatac ctcttttgcc aatgcattgc caaacagatg gggattgtcc 123540

taaaaccttg gggacaggac agtccaggta agctgagatg cagcatgagt gtctggatta 123600

gtccattcaa atgcaaaaat gtattgggtg tccaggtgta aaggtgtgtg aaagaaaaca 123660

tcttttaaat ttaatactat gaaccaataa gcatcttcag ggacttgagt tattattgta 123720

taaagattag gaactattgg gtgactggaa ctaatgcctc attaactgcc ctaagatcct 123780

gaacaaatct atattccccg tttggcttat taacaggaaa aatgggggtg ttacgttggg 123840

actgatagga ttgcaataat ccatacttca ttatcagggg atggatgccc cttggagcca 123900

aaggtctaaa aggatactgg atttttcttt tttttttttt ttgagtcaga gtcttgctct 123960

gttatccagg ctggagtgcg gtggcgcaag gctcactgct acctctgcct tccgggttca 124020

agcaattctc ctgccacagc cttctgagta gttgggatta cagatgtaca ccaccatacc 124080

tagctaattt ttgtgttttt agtagagatg gggttttgcc ttgttggcca ggctggtctc 124140

aaactcctag cttccagtga tatgcccacc ttggcctccg aaagtgcaga gattaaaagc 124200

atgggccacc acacttaaaa ggatattttt ttccccatag ataattaaca ctgggtttca 124260

aaataacctg gactgggaga acatttgcag ttcttccagg aactaccatg tcacaagcag 124320

acgggtctac ttgagaagta atatttgatg gaaggaatga cttttcttta tctgtgttaa 124380

gacatgaact tagagctagg agaagtgtcc cttcccagcc tgtggccttc tcttgaggtg 124440

ctccaaattg aactgcagcc tgttattggg aaagtaagtc tcttcccagc aaaggaataa 124500

ggcactcaag actaagcaaa aatctgtgga aaaacatggt tccccacagt gcaactaaag 124560

gaatgggtga atctcctagc ttttagctgg tcatcaatcc tcattacagt acaggagtgg 124620

gaagacagtg gcaccaagga ctagatcaga actgagaagg cggctcgcat atccaataac 124680

tcaatatttt tacccgccat gtcaagaatt acctgaggct cttctgtaga gatggcaagg 124740

tgtctggtgg aagctgtagg gaatcttggg ccctgtcagt cctctgttct cttagccatt 124800

aggggtccaa gaagctcaga ctccgtttgg agactgaggc agtctttcct ccagtggccc 124860

tcttgcttac agaaggcaca ctgattccag cctaggggat gatgagctgg cttttgtctg 124920

ggcatcccaa agccactctt gcaacacttt ctgaggatgg gcagctcaaa agcagtagga 124980

ggctgaaagc agctgcaaac aattgcattt tttggctatt tcttttggtt ttatcctctt 125040

cctctgcctt gtccctattg ttgtaaactc caaaggccat gtttaagagt tggctcatgg 125100

agtttggggt cccattcctg ccttttgtaa cttcctccta atgtcaagga tagattgagt 125160

aatacaacgc atgcccagaa gaccccaccc ttccgggttg tctgggtcta cattagtata 125220

tttcctgagt gcctcaacca agcagcccta aaacagaatg ggattttcat ccttccactg 125280

agttacttct ctatccttgt cataattaac tggcttaatc acacaccttt tcatactttc 125340

tattaaacaa gttagcatgt gatttctatg ttcaagttct tgggaaccct tctggtaatc 125400

ccactgaggg tctctatgtg gaactgcatc tccccctaca taaatggtat ggccttggat 125460

atgagcagct actctaactg catattcaca ggccagtacc cagaatccct ctcttctcct 125520

ctataataca ggaagaggac aataatattt gcaagttgtg ccaagttaaa tcaaaggaca 125580

tcatcaacct gacaaactcc tctataaact tccctggatc cttcaaaaac cagccatatt 125640

tctccttgaa gaaagccaat ttggacaaag gaaatggcac atatactctg attgttcccc 125700

tatctctgcc agctaactcc agcaatggac acagattcaa tttgaggggc tgatagagag 125760

ctccactcct ggtggttctg gttgggctta cttccttggg cagtggggat acaggctgga 125820

gctagctaaa taaaagggag ggttttctga tgacattggg gtgaaatccc acactggaga 125880

actggcagga cccctcgcag aactggggga ctgagaagac cctggagagg gcatgggccc 125940

tccagggggt gccgccagga agggagcatc gaggatatcc cacgcggttt cttggtgcct 126000

ggaagtaaca tgagccagac acatcctgca gttgactctt aagtcagaat cctggtaggg 126060

ggccgtagaa gcctgtacgt aaggaacttc tccccatttt ccttcctttt tactgaataa 126120

gtccaattgt aaaatagtat gataatgtaa agaacaagtt tcaggccaaa tttcttgttc 126180

tcccaagaaa attgtttctt tttctttgag ccatctcatt tgaatttgct ccaattgcct 126240

taaagacacc ctagtgggga gtcctttggg atgctcgcct ttgtccccat gtctaacaag 126300

gatctctact acacccagaa gtttttctaa gtctagcaaa agctcagtta ctttcccttt 126360

caaattccca cctcctgcag agatgtaagt acagttagca aagtgttatg aaagtagatt 126420

atgaaatatg aatgcgaagt aaaatgatgg gtctgaattc cacagagtga agaaagcatt 126480

ttagtttggg caaagagggg ttaaaagaaa caaggtgatc atggaaggga ggaagggaaa 126540

gcatgaacag cgttgtccag actgaggtgc cagaaaatct acccagcagc cacagagact 126600

gaggaaaatt atttgggcaa aatgtttgga tggctgctgc ttgtctgcca ctatgggtag 126660

ttgtcacttg ggccaggggt ccaggaatcc ctggtccctg tgaccaagag ttcaatatgg 126720

gcttgagcaa agtcacgtag gaggggcccc acaactggct gttatggaaa atcgtaatct 126780

tttaatttaa gggcggagaa gatgacatcc acactcctcc agcgaaggag ctctaatcca 126840

caatcctata gggaatgtca atgctgaaaa ccccagagca tccagggaga ggccaacagt 126900

gacagtggaa ctctcaccac aacgaaatgc caaaaaccca gagtacccaa gtatcagcca 126960

gtgatggtcc ccacactgaa tgctgaaact gcaggggagg caatggtgaa ccccaaaggc 127020

caagttaggg gccactgaac aatgtgactt tggcatctca gggtcagcac agtaagggac 127080

ttttcacgac caagtgtcct gccttaaaca gttcttgact ctgtcctgtc ctgtcctgtg 127140

ttatatcatg tcctgtccca tcttgtcttg tctttgtggt caccagatga tgcaaggaag 127200

gacaacccca aaattggggc ttagccaagg agagttcttg gctttgccca ggtaagaatt 127260

caagggcaac ccagcggtga aagaaagcaa cttttgttaa actggtactg cttctcgtgg 127320

aggaaggcta acccatgggc agtgtgtggg ttgctagcta gctgtattta tttagatgct 127380

tttaattaga tgctaattag gaagtgggct attcagaact ttttggaaaa gggatgagga 127440

gtttccagaa gcatataagg taacttcctg gccattgcca tggcatgttg acatggcatt 127500

tataaactgt catggcactg gaaaggagtg tctttatgct aatgagcagt gagagcaact 127560

agaggtagct tttgtctcca tctgctggtt ttggtcagct tctttactgc accctgtttg 127620

gaccagatcc tgctctgatc aactgggcca caaccgcaaa taagtcctgc cactttccta 127680

cctcagtaga atctctgcca aagaatgatc ttgaaagttt gctgtttact catcatctgg 127740

aaaaatcttc ctgtataggc caaattccca cccttgagat ttctgaagga ccaacaagta 127800

aaaacagtat actccaatgg gactgttaga tttcaactaa cttactctcc tttggatttc 127860

tttttctgaa aataaattct tcacacacac tgacttgtct tttgcaatcc aatgtaaaag 127920

tgggacatct tcttcagaag ctttgaaaca tacaaattag cctcaaactt acctaccaga 127980

cttccaggct agaaggctga ttcatgtaag aaggactaag aagacacttc acccatatcc 128040

tgggtaacag gaggggatcc tttagatagg tttgtacacc aggctggtaa cctacacttt 128100

cttctagcag tgagaggtac tgagatccgt caccccctcc tgcaaggcca agacccagag 128160

agagccaaat tttagcctag aattcagcca gctcttcaca gtctcctatg ttttacattt 128220

tcctccacaa tcatttactt tgaaagactt caaacctata gaaaaattga aaaaaaaaga 128280

actcctctac accattcacc caagttcaac agttaacatt cctttacctt ttttctctgt 128340

ctacacacac ctacacacac attcatattt catagtaaat gcagaccaaa aaaactccac 128400

ttcatcccta aatagttcag catgtatttc atgagattaa ggacattatc ctatataatt 128460

ataatacaat tcaattatca aacctaagga tattaacatt tattcaataa cattatctaa 128520

tatatagtcc agatagaaat tttctgttta gaaaatgtca catctataca catatatgtg 128580

catgtatata gatatttatg catacatagt gtgtgcatgt gtgtatgtat gtgtatatat 128640

aacacacatg cacattacat atgcataaat gttttaattt aataatattt atatgcatca 128700

atatcttaaa gatatatacg tatcattagc tggaagctgc ctctatccat atattaaagg 128760

tttctccata tgtagtgaac tgtaacctaa ctggatgtgt aaacaacctg taacctactc 128820

ttgagccaat gactgagttt tagccaatca aaggtggcca actgttcaaa ctgggttcaa 128880

ataagacaaa ctcagagctg tagccaatcc gattattttt gtacctcact tctgttttct 128940

gtaagtcact ttcccttttc catccataaa tcttcttcta ccacatggta gcactggaac 129000

ctctctgaac ctattctggt tctggaaatt tctgattcag gaattcttct ttgcttaatt 129060

aaactctgtg tgtgtgtgtg tgtgtgtgtg tgtgtgtgtg tattatatat tatatacaca 129120

tgtaatatat atatgtatgt gtatatataa tatttattta agagagagag agaagtgact 129180

tgtggtactg atgttgcttc cctaggttcc aagtaaagtt cctcaattgc actttactta 129240

cctagagctg tccctccatt tctttgtcct tgttgctaag tttttgaagt ggataggcta 129300

gttatcttgt tcaacatcct gcattctgat caaactaaac atttttggaa agaacagaat 129360

ataggaattt gtgcttctta ccactcaacc tgtcagaaag caagtgatgc aagaacaaaa 129420

tattgtgaat atgtttacat cgaaagtgtt aaagaaaact cagcatctgc cagcaagttt 129480

atggcgtgcc acggtggtat agtgattggc actttgtgtt gtgaatgcag aaccttgatt 129540

ccaatccaaa atggcagtgt gtggcatctt tttggggaga aactgagctg tcttttggtt 129600

tgttttcaat ccgtgcactg actgagtcct ttagcagggt ttaccactga ctcctgcaat 129660

gcagtgcagt caggcaaaaa ggagacccca cattgcacat acctagggcc caggacatgc 129720

cctatgtctc accattgagc agaaaatatc ccaaagggta aaatgtcacc tttcaaaccg 129780

ccatgttggt agcttccact aatgcagtgg tgtggtcata actggttttt actgttcctt 129840

acaatggctg caacccttat ttgtgactag agaacaaggc ttaccactga attgccagca 129900

tgatacagcc agggccatca aaaacacacc tgcactagat cattgggaac tttggaggtg 129960

ccttacatct gggcatgcat tgaaccacca cacaaggtga aatgcctctt ctcaaagagg 130020

cttgcctgca gtttccacta atgaaattgt gagtcatagc tgttcttctg tttgagatat 130080

taggagggat tttgtggggg gtgagggggg atgaaagtga gcttttactg agtttaaaat 130140

aaaatccaaa ctccttgcca tggtctacac tggcttgtct ccatcttcca tcccccaccc 130200

cccatgttta tgctaaagag cagactattg aaatgtaatt cacacagtgt aaaattcaaa 130260

tatattaact aggacaggtt attaaatatt gacaaatgaa atcatcaccc agatcaagtt 130320

atagaaaatt tccatcatcc caaaaatgtc tgtggatgcc aggcacggta gctcatgctt 130380

gtaatcccag cacttgggag gctgaggcgg aaagattgac tgagcccagc aatttgagac 130440

cagcctgggc aacatggcaa gacccccatc cctataaaaa ttttttttca aattcaaaaa 130500

ataaaaaata tttaaaaagg tccaagagtc cctttccagg caatcccacc cttctcccct 130560

tggtttatca ctattctgat gtttatcacc atagattatg ttgctctctt cttgaaattc 130620

atataaattc actttttgtg ttagcttttt ttcacatctt tgaaatgtat ccatattatt 130680

atatgtatct agtttatttg ggtgtaactg gtgagtgtta tccaatatgt gaatacacca 130740

ctgttgatgg acaatttggt tatttctagt ttggactatt atgaagaagg ctgctgtgca 130800

tgaatattat tgtacgaatc tatttatcaa caaatattct gtgttttctt agattttgta 130860

tttgtctcta tttggcatct attcaccgca gaacaattat aataactata ttttcttatt 130920

ttctttattt ttttcttaaa acaatttatt gatgtatgcc tggtatacta aaagctgtac 130980

atatgtaatg aacacaattt gctgagattg gaaataagta tggacacatt aaatcatcac 131040

cacaggccgg gtgcggtggc tcacgcctgt aatcccagca cttttggagg ccgaggtggg 131100

tggatcatga ggtcaggaga ttgagaccat cctggctaac acggtgaaac cctgtctcta 131160

ctaaaaacat acaaaaaaaa gattagccag gcgtggtggt gggcgcctgt agtcccagct 131220

acttgggagg ctgaggcagg agaatggcgt gaacccggga ggcagagctt gcagtgagtg 131280

gagatcgcac cactgcactc cagcttgggc aacagagcaa gactccatct caaaaaagaa 131340

aaaaatcatc accacaatgc atacaataaa actattcagc tgggcacagt ggctcacacc 131400

tataatccca gcagtttggg aggcccaggc tggtggatca cttgaggtca ggagtttgat 131460

accagcctgc ctaacatagt gaaactccat ctctaccaaa aatacaaaaa ttggtacaga 131520

ataattatac atgtttatag gagtacatat gatattttga tatatgccta cgatgtgtaa 131580

tgatcaaatc aggatgttta ggatatccat catctcagac attttatcat ttctttgtgt 131640

tgggaacatt tcaaattctc tcttcttagc tcctttgaaa tgtacaatat actgctggta 131700

gctgtagtca ccctactgtg ctgtttcttt tctgagattc cacctaaccc cttgatgttc 131760

caactgctgt ggttgtctaa aactttgtcc tctgttcctt taatctagga agacagtttg 131820

gttttgtatt gtaatttagc taatccaagt aagggggagc aaagtttgtt ctataaacga 131880

aaaataaaat cctaagtccc ccaactgact aaatggaccc cccgttggcc aacgggaccc 131940

taggcaaacc ttataaactg agtcccaggc caagaaggaa cgggaggcca gacaagcctc 132000

attatattcc cacccttttg caatttagac aaaactgacc aacattaatg ttatcatgga 132060

gatcataaga ctgacggaac agactcttta tggcaataag acaccaaatt ataaatagga 132120

cttaggacca ttccgggcaa ggattaactc acacacccca tatatgcaaa gaataaacta 132180

tgttctaact gccacaagga ttttctctag cagctaaaca agcactggcc ttgagaatag 132240

ggagtgttga agcacttgct gctcacccct caccagatac cgaaccccgc tgttccacaa 132300

gccataacta cagccttgac tgacaagaga ctgatttcag taactttctt ctgatgagaa 132360

gaccagcgac catggactgg ttctgtcagt tttacagtat ctgtgcattt gagtgccttt 132420

gtgtcctgaa aagacctttg tgtatagggc ctaattgtaa tgcatttaaa tgctaagtat 132480

ccataacaag gtgaacaagg ttgtatatta cgtgaatgtt tgttcaataa gcatgcatca 132540

ggaccccttc acaaatattc ataaatcctc ctataacttg ttgaatatgt attttaggcc 132600

cacccattca acttaaattc ctgtcttgcc cctccctccc tcaaagtgcc tgcttctggg 132660

cttcccgcga ttcccagcct gtcaggatga ccaccttaca tgctgtaacc ctttagaaga 132720

aataaattct cctctgtaag ctcacaaatt gtatggtttt tcagttaaca gccctctgct 132780

gaaagtttgc aaaatgggat actcactacc tgccattctt ccatatgtcc gcctgctttt 132840

gttcagtctt ccagcacctt caaattatat aatatatata tgaaataaat ttttctagtt 132900

tatagtcgtt atctgtggga gagttactct gaaagcagca agaagccatt accagatgca 132960

gatctcattc tgtcagtaat ggcaacccag caaatagggc aggagcccca cccaggtgtg 133020

gatcaatgca tggctgtata accttgaaca agctgcttag tcgacctctg aagtcagtac 133080

tgctgtatga gaaatggaga tatgaaaaga acaggagtgc tagggtgccc aagggagaaa 133140

agagtaggta acaagatttt cggtcaggag catgagaaac agggagaggg tcgccattaa 133200

ctccccttct tcaccagagt tttctggcac ccaagactgc agaaactctc ctgcagtggg 133260

ctttggggca catcgcaagt tccagtaaca agggctaagg tcgcccgctt ttggagacct 133320

tacaaagcat gtattccctg accaggaatc gaactcgagc tgcaggcggt gaaagcgccg 133380

aatcctagcc actagaccac cacggaaaca acagcaagca cttttcattc ctcttgcctt 133440

ccaaaggttt ctccaaaagg tgaacctgtc tgcaatgctc acgaagcccg ccagatgcac 133500

aagtcaaaac tgatctagag attctgaagt acgctgtgct ccttgagaag tgcggagcca 133560

ctggaaccac aagcagcttc agacaggaac caatgaggcc atcaaaaccc tcaacagagg 133620

catctgtgag ttcattgtgg tggctgcaga caccacgctg caagagagca ttctgcactc 133680

ccactgctgt gtgaagagaa gaatgtgctg ggcctgcctg gaatgtttgc gcactccagg 133740

cgggccctgg ggcgggcctg tggggtctcc agtcctgtca tcacctgttc tgtcaccatc 133800

aaagaaggct cacagctgat ccagtccatt cagcagtccg ttgaaaggct cctagtctaa 133860

acctgtggcg tctaaacccg tggcctctgc tgcacaatct ctgctgactc ctccccctga 133920

ggttattctc agctactttc tattgctata aaatattata gtactaaatc tggtttctgg 133980

ggttttgtat agtttttgtt ctgttttaca gggttgtttt cccccttctc catgcccacc 134040

ctttctctgc catcctgcat cctcttcaac tctctattga aaaatgaaca aatgttcaga 134100

acagaggaag tagagtggtg gcaccatcaa aggcaggaag ggccaggaga acctgatggg 134160

agcggggata cagacctggt tctagcttcc agtcttccag tcactaactt tctgctgtgt 134220

gcagggcaca atggaagtaa acaccaccca ctatatatcc cctgtgcctg gcatacagaa 134280

tcattcacac atgttgaccg aagggtttcc tttgcttcta ggggattatg tatcattttg 134340

ggaggaagca tgtattctgt gaggttgttt agtttatgtc caagcgccat ttactaatgt 134400

atccctgctc tttgctttcg gtatgtatgt tcttcctcca cctgacaatt gtgcccccaa 134460

tggtggccag gcagcagcat accaaagaga tgtgctgcaa gatttcagag gtgggtgagt 134520

gagacatggg aaagtggact caggtcttga aagagtcagg agtggccagg gcaaagaaca 134580

tgaactggtg ctggaatgaa ggattctggg aaggttgtgg agacctggct ggtagctaga 134640

gcagagatga tggaatccaa ggaaacaact tctctccggt gaatcaagat ttcttcagtg 134700

gacacttagt cccagctctg atagccctta cccctgtttc ctgccacagt gtgggtcata 134760

tgtattcttt atcatatgag gagagtgcta attaatgtgt catttatttt gtgagcatgc 134820

taatacatat attcatattc caatttagtg aaaaaaaact gatctagaga tgcccctttt 134880

cgaggtgaca gcatggctct ggagagatgg ccacaggaac catggcagtg gaccaggttg 134940

ctgggagaag gcaaaagggg aggcacccaa gctgagaagg ggctaagcac tcagcccctg 135000

ggaccaccaa cagcaggcct gagacacatg caggaaacca gacagcctgg atgaaatttc 135060

tttcaagcaa ggccagtggt ccctgacaga acaccagagg tttcccccta aatcctttcg 135120

catctgtacc atttccatct tagttggagc tctttgttct catccttgca atacgcagag 135180

aaaaaaaaat cacaggttgt tgattgtttg aagatagggt attgctctgt cactcaggct 135240

ggagtgcagt agcatgatca cggctcactg cagcctcgaa catccgggct caagtgattc 135300

tcccacgtca gcctctggtg tagttgggac caaaggtgtg ccaccacaga ggcagggttt 135360

tgccattttg cccagaatgg tctcaaactc ctgagctcaa gggatcagcc tgcctaggct 135420

tcccaaagtg ctgggactac aggcatgaga caccttgccc agccaaccag agttcttttt 135480

gatgccgata ttttactcgg actccacatc taggtaggaa ttcatgtttt caagtacgga 135540

cattgctcta cacaatcctc tcctttcctg atcactaaat tagagtaaat cactgtcctg 135600

aagaaggtac ccttgggaga gtgggttggc ctgtctttcc accctttctt gactctgccc 135660

tcaccttcaa gcctctttct ggcctgtgtt caaggtccag aggcatgctt aagacctctc 135720

tgtgtggctc ttcttgaaga gtcaggaacc ccatacttcc tgttcagcgg gcagagaagc 135780

ttcaagccgc acagttttgg gtatgctagg atcccacaag gcacagatgt gcagtggccc 135840

acgaagtgag tttgtgggtc tccagttacc tgcagccctt tctgctcctg taggttaaag 135900

ggcaattgcc tttttttttc tgtccaggaa ggaccccaga atgttctgag tcagaaagac 135960

agtgaatcct ctcctctggg tcagtgggga gggagagaga gagatttcag agggaagaaa 136020

gcagaaaaag atgtcacaaa attgcctaat caaaagttac catgatgcct gcctcatctg 136080

gaactgggct cccagtctct agtgagggag acatagtcca gtccagtccc agtctccagt 136140

ctgagccaaa gcagaacaaa gctggacact gcagcctggc atccaacgaa cattttgtca 136200

ctttccccag ggtgggagca tttgaacttt ggatgtgaag gaaacatggt aaccactgca 136260

gtaagaagag caactgctgg agaaaatggc actttcttcc ccaaatttgt ttttacactc 136320

atgtctaagg aacaccttag aaaccacatt aacatctttc agcaaaagga aattctgcct 136380

gttgatcttt tgctgtgaga caaacttgaa aagttcacag acactgcctt tctctgaaaa 136440

ctgcccttac ctctttgtta taattcactt caagatctgt tgagtaggaa catcaaaaat 136500

gcaaataaaa agtttcaagt attatgaaag aatgcaataa aactgccttt aaaaaaaatc 136560

tcaagatgga ggtggagatc ctgtgatcat gaaatctgaa taggctgctc ttgtgtcctt 136620

cagtggtgat ggctctgaaa cccttgtgtt ttatgaactt catgccctgg tcttcaaacg 136680

aattcagtgc aggccaagaa gcacagtttg gcctgggttg gatgctctat attttcaaag 136740

agtgaaatgt cttgagacac agcctttgct atagtacctg ctaaaacttc aacgtgtgga 136800

taaatatagc ttacattgaa tcgaatagac tcagcgagag tggcagtgaa ggatgccctc 136860

tggaagattc gaggtgctga ggccaaaggg ctagcaagga aagagcgtgc gggtcaggcc 136920

cttcaacctg gtggaagagc aggcagggag ggtcctatgg ttcctgggag gacccgaagc 136980

atgatcgcct gtcaagagcc aagagcccta atggccagcg ccggggaaag ggcccccgag 137040

gggcagtcag cagccctgca ggggcggcgt tcacactcct gtgtggcacg ggacactcat 137100

ggctccacca gctcctgaag cttctgggag ccagagagtc agcagggaga atcccacaca 137160

cgggcactca gttcctggaa accctgcatg agatgcggga tcccgaagcc agattggggg 137220
tccctagggt cttggcttcg gggctaggcg cggaaccgta aatgagggtt cagcctgtct 137280
gcctgaggga ttgtgtttcc tgccgtattt atcactcaaa ttttgaaaag cagtcgaatt 137340
tgttatattg gaaaccctag tagttaaaaa cagcagatgg taacatttca ggaatatgtc 137400
ttgtgccagg aactgtgagg ggtttctgta gtgtagtgat tacatgttcg cttcacatgt 137460
gaaaggtctc aggtttggga cctttcatca tcaacatgat gaaaccccgt ctccagtaaa 137520
aatacaaaaa ttagccaggc gtggtggtgc atgcctgtaa tcccagctcc tcgggaggct 137580
gaggcaggag aattgcttaa ttccaagagg tagatgttgc ggtgagctga gattgtgcca 137640
ttgcactcca gcctaggcag caagagtgag actacgtctt aaaaaaaaaa aaaagaaaga 137700
aagaaaagaa aacagaggac tttaaaaccg ttattatgtc aatattctat gggtccaaag 137760
aattaagcag aggtatcaaa catataaaaa tggtcagaat caaactttgg agattaaaaa 137820
ctacaacatc tgagataata aattcactgg atgcaattaa cagcagatta gacactgcag 137880
aaaaaaagat tatgaaacac aaagccgccg ggcaccgtgg ctcaggcctg taatcccagc 137940
actttgggag gcggaggcgg gtggatcacg atgtccagag atagagccca tcctggctaa 138000
aacagtgaga cccccgtctt tacaaaaaaa aaaaaaaaaa aaaaaaaaaa tatatatata 138060
tatatatata tatatatata tatatatata tatatatata tatataaatt agctggacat ggtggcgggc 138120
gcctgtagtc ccagctaatc gggaacctga ggcaggagaa tggcatgaac ccgggaggcg 138180
gagcttgcag tgagccgaga tggtgccact gcaccccagc ctgggcgaca gagcaagact 138240
ccctctaaaa aaaaaaaaaa aaaaaaaaaa aaaaaccgca ctgaggggaa aagtcaatgt 138300
gaaattctac aaaagctgaa aattcactac caccagccat taccacaaaa aaaaaaaaaa 138360
gttgaaggag tcctccaggc agaacgaaac tgacatcgga tgaaaatctg gttgtacaaa 138420
aaaaaaaaaa aaaaaaacta gacatgacat ctaatagacg aatatataat tttaacatct 138480
gactgtttag ctgggcgtgg tggctcacgc ctgtaatccc agcactttgg gaagctgagg 138540
caggcagatc acttgaggtc aggtggttga gaccagcttg gccaatatgg tgaacccctc 138600
tctctactaa aaatacaaaa attagtccgg tgtggtggcc cacgcctgta atctctgcta 138660
ctcaggagtc tgagtcagga caatcgcttg aacccgggaa gcagaggttg cagtgagcca 138720
agattgttcc actgcactcc agcctatctg gaacctagtt tgatctggag ggatcttatt 138780
ttataggagg cttggagggg gaccttttgc tttgtgtact tataggatgc acaagacaca 138840
agggaacaat ctctcacttt ttaaatacag tgataggcct gaggagaaac aactgcaatt 138900
tttttttttt tttttttttt aatgagatgg gattctcact ggtcttgaac tgagctcagg 138960
cgaggcttcc cctacgtcgg cctctcaaag tgttgggatt aataggcgtg agccaccgcg 139020
cctgaccaca actaaccaac atttaaaagc acgtccctgg gtaggctcga accaccaact 139080

ttccggttaa cagccgaacg cgctaaccaa ttgctccacg gagataacct cagttggtcg 139140

ctttcatctc aacatagatt aagcaatcac taaactctag gggttgccat tcgctttctg 139200

caggacaact gtgcagacta caaagcttcg gaaaaccgga gaggctgagt cgactaatcg 139260

tgttgttgca cgttagaaac gcttgcattg cctgactctg aaaccagaag ggcggccgaa 139320

tggccttcac cctccattaa ccctcgcctc cttcagaagc cagtgcctct ggaaatgcct 139380

ggatctgcga ccccagcctg agcctagtag ggcccaaggg aagctgaacg ccccgacggc 139440

tctcatggta ggtagctctt tctgtttttt tgcgccgcct tcaggcaatc atctactccg 139500

cttgctatcc cctcactcaa ctcggcttca gtagatgggg ttggtggggc gggagtggga 139560

aagaggcagg ggagtcaaaa gggaaaacct gaaaagaagg agggagaaga agcaggggag 139620

accaggacga gacaatggga cagcccagga tgccggtgca gagggcagcg gctggatgca 139680

gagaagatgg gacacgtatc agaatggact ggagaagacg tgagggaaaa tcacaagaac 139740

ctgtagctgc ccaagaataa acacgtaaaa atcgcataaa tgtttttaca ttaaaaaaaa 139800

aatcgggaga ccgggcgcag tggctcacgc ctgtaatccc agcactttgg gaggccgagg 139860

tgggtggatc actcacttga agtcaggagt tcgagaccag ccgggccaac atggtgaaag 139920

cccgtctcta ctaaaaatac aaaaattagc tgggtgtggt ggcgacgctt gtagtcccag 139980

ctactcagga ggctgaggca ggagaatcgc ttgaacctga gaggcagagg tggcagtgag 140040

ccgagatcgc gccactgccc tccagcctgg gcgacaaagc gaaattctgt ctctcaaaaa 140100

aaatgcataa ataaataaaa gaggggtggg gaagcaaaac gacgggcagt aggtgtgggg 140160

cgcattggga ttctatagtg gttagtaccc tgccttgtgc ctgcagcaac ctctgttcta 140220

atctgaatcc tggtacagtc agactctatc ttggacccac tggggcgaac ccacgagtct 140280

tttggtttgc ttttaattcc tgcaccagct gcggccttta tctgcagcca gaaagcaggg 140340

tttaccgctg gccccacagc gccatacggt ctggggaaaa gaaggaaacc caatagtaca 140400

caaacaaagg cccaaagaga aaccttccaa gtgctctatg cctcacggtt tagcagaaaa 140460

tatcaagcaa ctctcaacct agctggtctg tagcttccac aaatgaaata ctgtattcat 140520

tgcagccttt ctggttgaga tatttcaaat atttggtgga gcttttaatg agacggagag 140580

acactctcga gtgtggaaga aaaacatgag ggggtgtgag gataaggcga ctttaggaca 140640

gaaaaaacaa agagacaagg aagccacgta aacgttttcg ggtaggcgtg aggcgatgtc 140700

agttttgaac cccgtttatg ttaggtagag agcgcagccc tcttctagca caaacaccgt 140760

ttcccacatt gaagaaatca cagagatcag caactctaga gtgcgatgaa gaagcttcac 140820

tctgggagaa cccccttcgt gaccacggtc tctttcctgc caggtaagtg ggaatgagcg 140880

catgccctgc agggacagca cagcgtcctc gccctggtcg gacgctcagg gtcaccaccc 140940

tacccactgc ccccttcgcc attcttccaa accactctct gccaaagatt ccaccgacag 141000

tcaccccaca cgacaaccca ggccgccttt cagcagtggc tcccgccccg caaccacgcg 141060

ccctctcacc cccgcggttc tgcccgccgc ctctgtccag tctgtgcact tcacctccct 141120

ggctcccgct ctcccctgag cttacagtgg acgcggggtt cttccaaacc cctcttggga 141180

atactgaatg gaaaaggggg agcgtgcgca agtgcttggt agagtgtaga cattgtggga 141240

tttgactgtg gtaccatcgc tttgacgtcc tagtgctgat ttttacacct gcattctgct 141300

tagggcactg gcaacagttt tccgtttgtg cctactccac ctgctgtctt tgttgggtca 141360

gcgaacatcg cctccctcta ccgctcaatc agcaaaaggg accgcccttg aggacctcac 141420

ccgccgctca ctcccctccc aacttcgcgg gcatcgcctc cggtcgcctc ttccgaaggc 141480

ctaacgagca tgttagctgc gaacggaggt gaggaggctc cgctgactga ccggtgccca 141540

cgtccagggc acgcacaaac gccatgactt ggcttggcct ctctcttagt tattcacagc 141600

tcagcccgat aggcacctct ggggcggcga cggcaaagag ggtgcgctta ttaagtgcag 141660

ctccacgggg actggcctct ctgcacggct gtgtacacct gagcgagacg ctcagtcgct 141720

ctctaaagcc gcttctgcgg atgacagaca cggagataaa cgtgagaggt ggcccaccac 141780

gacttgccct cctttgcccg ggtttgcccc tcgctgcgga ggctgttcta catctggccc 141840

ttggagcagg ccggctgaca gcgtggtaaa ggaagatttc tgcgggaggg cggccagtgc 141900

aaaacaattc cctgaccggg aatcgaaccc gggccgtggc tctttcagca ccgaatccta 141960

gccactagac aaccatgcag atgcggaaag ctgctttctc tcccttcttc gacctgaagc 142020

gacactttcc tgtgctctag gaggacttgg gtcttgtgag agtcgccctt tgctcctgga 142080

gtcgtctcac aaggccgttc actccctgct ttcttcaaaa aaagaacctg caggcgacac 142140

accaaggact ccacgaggga gtcctgagta ctggagcgag ttgcggccac gcggccgcag 142200

ctcaccactg gcctagagat gccctttgcg aggcggcagc aactgacaag atggtcgcgg 142260

gtcgccgggt ccggagccgc ccaccaggtt gccaggagga ggcgggagcg gggaggcgcc 142320

cgaggtgaga caggggcacc ctctgcatca taaaggaccc agaccccggc accctcaaca 142380

tcataaggaa tcagacggat gcggaaaccg agacgggctg gataggaaac tctttccagg 142440

aaggctccgg ggcactcaac tggtctccaa ccttcccctg caacctgtga cgcctgccat 142500

tttcccattt taggcgatgg caacgcaacc cctccgtttg ctctgggcaa aacttcgaga 142560

gttccctctg aagctggagc tttttcctca gatccaagat ccaattggtc accaattcgt 142620

gatttccgtc ggccaagtgc gtgggcattg atctacacgc gagtttctcc acctctgccg 142680

aatggctact tcggggtggg ggagggcccc tcccgccgtg gattgcaagg tgtttagcag 142740

catctgtctc ctccgctgac tagacacatg ccagggggat aacattctcc ctcccgcttc 142800

ccccagccgc ggcctagtgt cccagcgggg ttgggagagg catgtgaggg cgaagttgcc 142860

ccctgttgag aaccattgct gcgcgtagtc cttctctctg aacttgtgca gaggactctc 142920

caggtgaagg ctcaagggtg gatccagctc gagacaccct cgctccccct cacagtcgga 143980

ccttaggatt taggctttaa catctccaca tcatgagatt cgaaaccttt aggtcttgtc 143040

ttccgttctg tcctccaaat cggcctcttc cgagcctgtt gaccagggcc agccgggcag 143100

agggctgggc tcgctcaacg aggctcctct cgcacctcct ggagcttcag gcttctttcc 143160

gttgcagaga agctttatgg gccaattcgt tcggcatccc cgggggcagg tgcgcggtgc 143220

gcggggaaga agaggatttg actgcggttc tccacccccg gcgcccaacc tccaccccgg 143280

tgcgcgcgct cttccaggct cctgctggtc ccacttgcca ggagttaggt ctcaggtcag 143340

cctgagctcc tgagacgccc aggcccggaa agacacgtag gggaaaccat ctgctcactt 143400

ctgtcctgtc cggaagggat ccctttctga cgggaaagaa aggcggtgag tcctgtcctg 143460

ttgagtaggc ggaagagaga tcaaagggaa gacaagaaaa atcctgtgag ttttcaggat 143520

ctaaagttac catgaggtcg acctaacctc ctctggaggt cctcccggtc ctcccgtggc 143580

tgtcgaaggt gaatctagct tccgtctcca gttcgccaag gcggacaaag ccgacgacaa 143640

tgggcctgtc cactatcttc tttcatatgc acaaaatgtc agctcttctt gtttctaact 143700

tgcaacatcc cacctgatga ccagctcagc aaattagaga ccctccatgg gattccatct 143760

ctgtcttagt tcgggcttcc ataactatat accataaact gggtggctaa ttcacgacag 143820

aaatttattt ctcacagttc tggaggttgg aagtccgaga tcaaggtgcc aacatggtag 143880

ggttatgatg agggactttt ttctggttgt agactgccac cttctcattg tatcctcagg 143940

gggcagagag agctccctgg ggtccctttt atagtggcat tagtcccact cagactaacg 144000

ggactaaatc cagacccagt tattgcaatg tgtgcaaaag aacaaggact tgtactatct 144060

gacttcaagg cttactataa gctattacag acaaggcatc aggagggaca aatagataaa 144120

cagactgagt taagagacct gaaactgatc cacagccata cagtcaataa atgagctttc 144180

aatgaaagca gttcaataga agaaaataaa tcatttcaat taatggactt tcatatggag 144240

gtgggggaga ccaacaatgt tattctccct cacactacat acaaaagtaa tttgaggtgc 144300

attatacacc aaaacttaaa agttaaagat ataaagcatt tcaaggatac tctgtaggta 144360

aagattagcc taccaacaag taggacactg aaaaaatata tataaaagac atgataaatt 144420

agacttcatc aacattagcc ataccttctc atcaaaagat accactaaga aagtgaaaag 144480

gcaagcaagc cacagacaga gagaaaatag tcacaaaacg tatctgacct ccacatcctg 144540

taattagaat tattgtggtc tggtacactg cacccagttt ctgcaggagt actttctggg 144600

tgtctctaat gagtaagaga gggccccatg ggatattcct acagttccca gatgaacagt 144660

gggaaagact ctacgttgac aaaccccggg gacctgaaaa ctcaggtcct caaggagggt 144720

agaggatacc tggaccctga cccagacccc tagatgggct gtgccaagag acccagcaag 144780

ggaagggatt ccctcctgcc tcaggttctc tgttcttctg tggttagaag acctgaaccc 144840

aactccctct ccaagcagtg gagatagggc ttttccaagg gctggggatc ttgctgtcct 144900

aaggacagct gagcaagggg gtcgaggagg agcttgggtg gtggaggaga ggaaaccggg 144960

taagatgtgt gaagcagtcg gctataccag gcacagagag gacccactgg gacacaagag 145020

cctgcatgtg aagccaggcc ttgggccacc ttgttcctca aaggggtgct tacttccatg 145080

ggatcttcaa agggactgtg gaaagagaag ccttcagccc acacctctga atgcttttcc 145140

accacagcat gccctgtggc ctgtatcctg ctggtgtgga acagtcagac ccctgcaggg 145200

ctgcagagcc tctgtactgg gcggcatccc agcctgagtg ccagagctca gtgggcaggc 145260

ccccgagcaa gcagagagga gggcaccttt tggacagaac ctgtgggaca agagcgacgt 145320

ctcatccttt caggttcctc acaaaatgac agtcaggaag atcagggtgc agacctgatt 145380

tcccacgaag ggctgaaagc agacaaccgg agggagagca gcacctgggc caatgaggta 145440

gaagacagaa gaccacagtg tacttctgcc ctcaacctca ccccctccca cctacatcct 145500

ccacaccccc tgaccacctt cttcagaaac gtaataggaa tcaagatccc ccctggcctg 145560

gttgctatgg gaggcacagt ggcctgatgg agcctgaggc aggtgtggga agatgtggat 145620

tgtctaactg gaggttggga gtccagggtg cagaaggaga agcttggagt gcaggatttg 145680

gtggtatgtg tgtggcagta ggcactatgt tctaattgcc agtttttttt tcttcttcct 145740

tttttctctt agctaaacaa gcactggcct tgagataagc aatgctgaag cacttgcagc 145800

tcacctatta ccataaactg actgagccct ccctacacaa gccgtaacta ctgctttgat 145860

tggacaagag actgatttca gtagttttct cttgataaga gaccactggc cgtgggcggg 145920

ttctggacag tttacagaag ctatgcactt gattgccttt gtgtccctgc ttcacctttt 145980

gaagcatagg gcctaattat aatgtattta aatgttgtct ccaccccaaa gtgaacatgg 146040

gttgcatgta acaggcatgt ttactcagca tgcatgcagc aggatccctt cacaaatatt 146100

cagagctccc cctattccct gttgaatatg tatatgtggc cagccagatc aacgtaaatc 146160

actattcgcc ctcccctccc tggaaaccta cttttcgggt ttcagcagga agctatgcct 146220

ccccgtctgt cagaatggcc acttgcaggc tgtaaccctt tataaaaaaa taaaatctcc 146280

tttctaaatt tataaattgt gtgatttttc agttgacagc tttcagttgt cagtcaagtc 146340

attgactggg aaaagtcatt tgcaatatat ttatttgaaa aatgactcaa ctcctgaata 146400

tataagagaa ctttcataaa tcagtaatat agagctaagc caaataaaat cgggcaaaat 146460

attcgaatag gcctttgcaa aggagaattt cttatatgct ggaagccata agaaaatatg 146520

cttcatagta ttgctcatta ggcaagtaca aattaattcc acactgagat accactaacc 146580

aatcaccagt gtgtggctac tttttttttt ttctctgaga cagggtctct ctcacccaag 146640

ctggagtgca atggtgcgat tggtgcaatc ttgagtcact gcaacctccc cctcctgagt 146700

agctgggact acaggtgcat gccaccatgc ctggctaatt tttatatttt cagtagagat 146760

ggggttttgc catgttggcc agtttggtct gagagcatag ctacatttta aaaagttagt 146820

acaccaaatg ctgacaagaa tttggtgcca cttcaactgt cattgctggt gaaaaaacat 146880

tctagaagac tggcaattta tactaatgtt aaacttgtac tcaggtcgtg acccagcaat 146940

tgaagtactt ccatgaatct caagtgcaca aaaagacctg tagaagaata ttcatcacaa 147000

gaattcaata acaccaaaat tgagaagtga tctatgaaac tacgtggata tatctcatga 147060

gtataatgaa catacctgca gaaaaaaggc cagatacaaa agatatgtcc attcactcat 147120

gtgaacttta agaacaggca attgtaacct atgggaatag acatcagaat agtgattaac 147180

taagaggaca cagggtggga attgcctgga aaggggctct aacaggcctt tctcagatga 147240

tggcaatttt ctataacttg agctgggtgg tgattacatt catcaaaaat aaacgaactg 147300

cactaaagat ttgtgcactt tatgtgaact gtagtttatt tactgttctc attgcttgaa 147360

cccgggaaac gggacgttgc agtgagccga gattgagcca tggcactcca gcctgggtga 147420

cagaacaaga ctacatctca aaaataatag taatagtaat aatttactgt tctcataaaa 147480

attagcggat ggggaatgga ggcaagccgg tgtagaccat gacaactagt ttagatttta 147540

ttgtaaactc attaaaagct cgttctcgtt ttgtgttttt aaaaaatccc actgatacag 147600

ccgttttctc taccagatga gactataacc gtattatttc atcagtggaa gctacagaca 147660

aagggccctt gagaggcggc atcttcacct atgggaattt tttctgctcc attgtgagac 147720

aaagagcatg tccgagtttt cattttggcc aggccgcccc ctagtttacg cactgtgggc 147780

taaactccag aagctggcgc ccttccgggc cagcggttta ctccgctctc tggaggctgc 147840

taggattaaa ggcaaagcaa acgacaggtc ttttagctac aatcgcagga gagaaaacac 147900

tactgtgact cagattagaa cccaggttgc ggcaaccaca gctgcaagta ttgaccacta 147960

cacgaccaaa aagcctgctg acaaccattg tacttcttat atttttttat gtaaaaacac 148020

tcatactatt ttctctgctt tattctcgaa cgtctgcaga ttttcgtgct tttctctctt 148080

tcatgcgctt ctccgttact ctctccccat tccgctacat aatttaaaaa acatctcatc 148140

tctcaggacc tggccactgc ctctacaaca agcctcctgg gaagtctcgt tgtcccatcg 148200

acacctctcc cttctttcgc tccatttttt tttttttttt tttttgacgg agttgctctg 148260

tcgcccaggc tggagtgcag tagcgcgatc ttggctcact gcaacctccg cctcccgggt 148320

tcaagcgatt ctcctgcctc agcctctcaa gtagctggaa tagcaggtgc acgccaccac 148380

attcggctga tttttgtatt tttagtagag acgggatttc accatgttag ccaggctggt 148440

cttgaactct tgacctcaag cgatccatcc gcctcggcct cacaaagtgc tgggattaca 148500

ggcgtgagcc accgtgcccg gccaaatttc aggccaacac ctgttgacac acattgccag 148560

acacacggaa tccctcactg aacaccgatg ggcccacaaa acacgcggag gccacggtgg 148620

ctgaagatgt tagcaaattc ggttcgcggt gtctggggta cagcctcgag ggtccattgg 148680

ctacctctgt gcaaggacca ctctgcgcaa ggaccagtca ccgctgctct cctcatctcc 148740

attgagattc tcccgcacac acctcccctt tctttgggcc gctgaggcct cttggacctc 148800

cgaggtgatt gccccgcccg cagcttctct ccttccggga gcttcatttc ttgttcctct 148860

ccgtagtggc tcagcggtaa gcccaaggtc cagcacacga atcaggaaac tgatggttct 148920

tgggtttgca ggaatccgcc cagagaacag atgaaagtaa caggtaccaa tatcaaaact 148980

gcagtgactc accagaaaca ctacgtgctt gccactttgc ttagcggttt gttgagtcca 149040

acaactgcgt gggtcccggg ttagtctcct gaatgtcttt tgctgctact ttggtcagcg 149100

ttgttgtcag tttaccttgt ggtggccaag cccttaaatg cactattagg ttatgcagtg 149160

taattctgca gggcagaagg gtaaagagcc atagtgaaga gcaaaagaac ctcttgcctt 149220

gaccgggaat tgaacccggg tgtcccgcgt gtgaggcgag aattctacca ctgaaccacc 149280

agtgcctctc cccagcaacc cttggagaat tatcggaaag agtatccaga aagacttaga 149340

aacttccaag cggccttttc aagtgtcgac tcaaagctaa caaagacatc caaaccaaat 149400

gtttttatag gaaactttta ctaaacaaag ttataaatat caaaatagct catttgtcgg 149460

atcaaactct taactctgaa aaaggtcttt ctacctgcat tacatacccc tataataaaa 149520

cgtcacaaat tcattcatgt ttcttttttc taatcctaaa tcttcaattg tcaacgtcaa 149580

actgctgcct tagtggttct gagaaggtaa cctaactggt agcttaggta agtaaagttc 149640

taatccaggg aggaaataag aagcagaagc agaattagaa ttagaggaaa gaggaaataa 149700

aaggacagaa tcaaggtaga gataatgaag aaacaaaggt tggtccactg agttagtctt 149760

ttgtcgctgg tttttttggc aaaagagtaa tgatcggtct tgtaaccata atactgttat 149820

ttgtctgctt gaagatgtat aaagcattta aaggaaatgt gatataaaaa gattaataat 149880

gcctgcagtc aatatttcat tgttagagag aatccaattt cctaagttaa tatgctttga 149940

tgtattagct atgtaaggag tagactagtt taaggaaata ttgatggtca aaatattaac 150000

atattagtct tttgatgaag ttcaaataga gagatttctt ttctcaattt tccctggaga 150060

gattaaactg aagagaaaaa ttcagagagc ttgccccaca tgttgggtct gtgagtcatt 150120

atgacttttt caaagacagg agctgtgaca tggaatcatg cttcttctct agctgagaag 150180

ccaagctagg tccaggctgg gtcataaact tgagcccaac aaggaaatca cccttcacat 150240

tgacctcaca gagctttgac tgttctctgt tctttgccca acacccaaga cacacaccag 150300

ctctggccaa caaaccttaa catattatct atatcaacca gagctacatt tattcccaaa 150360

tctccttcta aaatacaaac ctgtactttc tactctcaac ttctaaattt acaaaggcct 150420

catatgcatc tcagagtcac agatgctaaa actcaacaca ccggtgagtt ccctgtcagc 150480

aatatgtacc accctctacc tcaaaaccca atgatcagac tcaagcacag catctcccaa 150540

agagtagtgc actgccctgg gtgtttcaat gatcactaaa acctgtttct agccctgcct 150600

agcacactta tcctcaccac catctatcct atttgccaag ccagatcttt ctttggataa 150660

actctctcat tttcataaca caacaatttc agttcaattc aaaaaaaata gcgtttaagt 150720

ttattggata ctccacaagc ttactgccac tttattacca cacttttcca tctatcggct 150780

cttgcatcca ttaacataag cagacaaaaa actggactcc tggccaggtg cggtggctca 150840

agcctgtaac cccagcactt tggaaggcca aggcaggcgg atcacgaagt cagaagattg 150900

agaccatcct gactaacatg gtgaaacccc atctctacta aaactacaaa aaaaaaaaaa 150960

ttagccaggc gtggtggcac ccatctgtag tcccagctac tcgagaggct gaagcaggag 151020

aatcgcttga acccaggagg cagaggttgc aatgagccaa gaccacacaa ctgcactcca 151080

gcctgggtga aaaagcaaga ctccacctca aaaaaaaaaa caacaacaac aacaacaaca 151140

aaaaactgga ctccctacag ccagtgtagt atgcaccact cacttgccac actgtatgct 151200

gagtgaccct tccacagctt aaatctgcac ataacttcag cctagaacca tataatgtcc 151260

ctttatttct tcagtacaat gattaatacc atattctctg taccttcttt cactctatgc 151320

tcatcattct aacagctctt gccaatactc aaatccagga gcagcaaact acttcacatg 151380

tcacaaactt ttcacaggtt cacaccagtg gattccccag tgaaccagct gtcttaactc 151440

aggcttccat aacaaaatac cacagactgg gtggcttaaa cacgaggaat ttctttctca 151500

cagtttgaga ggctgagaag tccaagatca aactgccagc ggattcacct ccttggtgag 151560

ggctctctcc ctggcttgca gactgctaca ttctccttct gtcctcgcat ggtggagaga 151620

aagagagcac tttcgtcttt cttctgtttc ttataatgac actgatctca tcatggagat 151680

cccatggtca tgacctcatc taaacctgat tacctcccaa aggccctacc tccaaaatac 151740

cttcacattg ggggttaagg cttcaatata taatatgaag ggaacacaaa catccattcc 151800

attacactag cacctgcatt tgcaatccag cctctgggcc aggcttagca gtgtagagcc 151860

caaggcttca attgacagga tttttatgct ctcatgctct ggctccttgg ctccagaagc 151920

ttctagaacc agtagggtga tacatccttt agcaactata ttgtaaaaca catggtgggt 151980

aaaagagtgc cctattaagt attttggtta tgcccccatt agtgtgcttg gctcaatata 152040

ttaatagtaa gtagtggttc gcatccaccc tcagtaccgc cctctgcagt catcctgtca 152100

gtttccattc cattctccta tccctggctt gctggttcaa tactcttaag tgatttactg 152160

ctggtgctct tctttttccc tcagagatac cttgtgattt ctttgattct ctccatctct 152220

acaggttaat aagtctaata tttaaaaact ctgtagaggg atgttgggaa gctgtgggga 152280

gagcccttgg gcttttcgcc tgggtaaagt gcagattcct gagaattctg ggccttggct 152340
tccagctgca ctagtgccgg ccactcagct gttctcagca gcgtcacctg gcgcaatggt 152400
gctgaagcgc actgccgaga ggccagaagg caaagcgaga gtcggtggct atggcctgga 152460
acccatttag gcaaaacaga gggcgcgcgg ggacaaagca atagagaagg gtgggcaata 152520
caggatctgt acaccataca ttagcaattg tatttattaa attgttgatt acctaagcct 152580
ccggagagta gtttggacgc ttaccggttt tctgaggatt atttggggaa ggggtatgca 152640
tgcaaatgta tgtacataat tcgtgtgatt tcggaatatt gactccatga gttccagatg 152700
cagatttgga acgtttttaa aaaacatttt gtttttgttg tctcgcaaat cagccagatc 152760
tgcaactgta tcagagtaaa gcgaagccaa gctggaccct taggaaatgc gctaacatgt 152820
catccacttt cggtgtctgc ctgtgaaaat tcaggcgata aagagaatga agagaacctt 152880
aaggaattgc tggaaccaaa gttaatatta agcaggcctg ctgtgacgtc ctcttcctgg 152940
cagaccagtg gaaattgtag cctggtcgac aatctgtata gattgatgag gtctaaaatg 153000
tagtcacagg ttcaactact tttctgtttt ccaacctcga gtaaactcac taaatttaag 153060
ggctaacaaa aaaaaaaaaa caaaacaaaa caaaaaaaac ctctgtttcc gcccagtttc 153120
gaaccgggga cctttcgcgt gtgaggcgaa cgtgataacc actacactac ggaaaccaca 153180
cgcggaacct gcgcggcaaa acataaccat gaaaatctta gatcagccgt ttctattata 153240
gtttccaaat ctaagatcgc accactgcac tccagcctag acaacagagc gagagtctca 153300
aaaaaaagtt aggagaagaa ggtgccattt tccctagttg cttttcttac tgcggtggtg 153360
accgcattgc cttcacacct gaagtccaag tgtttgcacc ctggggaata tgcagacagc 153420
tgggtcactg gacgccaggc cgcagcgccg gcctcgttct gcttttggct ctaaatgcag 153480
tcgggggacg cgactggatc tcaccagaga ctggtgggct gttaccctta ccagcgactg 153540
gagggccaac ctcccaaaga ggcctacttc atgatcgcct taaatcagga aatttcatga 153600
aatcccaccc ctctaccgcc ccattcccat attgtatctc ctcccctgcc cactctgaga 153660
ggattcgccc cctttctgtc tcgtctggac cctttggagt cccacataga caagacaaaa 153720
ggggcattgc cttttcctac aggagcggga agagccgtca cagcggagga aacagagact 153780
cacagcgcgc atccccacat acttgcgccc tgccgagttc cagcaaaccc aacaaagcac 153840
tctgaagctt ctctggaaaa ggaaggagtg tgaggtttct gatacggcaa gaggagccaa 153900
agagagggga cccaggcctt cccctagctc gagagaacaa acccagaaga agggcagatc 153960
cgaaggaaaa gcgcaaaggc ggcccaaccc cacgacctga gcgagccaag ggtcgcgggt 154020
taaattgcgt ttatcagaac aattgtttgc tcactgtccc aaagccctcc tagggacaaa 154080
gaaaacactg gcatggcacc agtcgcagaa gggggcagca ttgacaccca ggaacaggcg 154140
tggaaaagca ggaaaagcag ggatcatctc tctcctaccc cgtatatggc tgcagacata 154200

gcagtggttc ttttctccag gacccagtga gtgtgcacca ggagagaaca ttatgcacgt 154260

tattcatggt ggcaccaccc aggctgaaag tgagcctgtg ctgcttgggc tttcaggaag 154320

gacgggccca actctccctc cctatacaga gctgcagtgc cctggcaatg gaggacagac 154380

catggagttg cctgccctgg actcggggaa gaggctttgc cctaaacccc attttagtgg 154440

tagctgtcag agtgccatat ccacagactt cagctgcacc acagccagga accaaaggac 154500

aaagtcttta taaaatgaag gttgtgagct ctgtgacagg gcatgatagg gaagcggatc 154560

acattcttgc ctactcagga ggaggagcta gtgcacctct gccccttccc ctgagacctc 154620

agcacacccc gacatgatct ctttcccacc atcctgtcag ggcaggtttt tccactggac 154680

accagcctac gtaacagccc tgactcttaa gcaccatcta ctggactgca tcctaaactg 154740

gaccactgaa ttcaaaaacc ctgctaccaa agggcttagt gctagtccat gagataagtt 154800

tcctgagaat tccgtaccct cagcccccaa caagggttag tgtgttagct tttacttcca 154860

atacatcacc acaacaagca gcatctgaga aagctactgc tcagaagcta tccacaacca 154920

agaaacccat acaaagctag ggctccctga aagcacccag aaatgaagcc aaacaatcat 154980

acacaacata caccacagtc ataccatcaa gggaaaaaaa gaataaaaac attaaaaatc 155040

ctcatccaaa caataccaaa ttcaaaacta agaagcaaca gcttcctcgg atgagaaaga 155100

atcagtgcaa taactccagt gtacccagag tatttacaca cttccaaagg atcacactag 155160

tttctagcaa tggatcctag ccagactgaa atgtcggaaa tgacagataa gaattccaaa 155220

tacggattgc aagggaactc aatgagatcc aagaaaaagt tgaagtccaa tgcaaagaaa 155280

tcagaaaaac aatacagaat atgaaagaca agaaaactat attaagaaaa atacaaatag 155340

aacttctata attgaaaaat tcactaaagg aatttcaaaa tacattgaaa gctttaataa 155400

tagactagac aagcagaaga atgaatttca gagcttgaag gttggtcttt caaattcacc 155460

tagtcagaca aaagtaaaga aaaatgaatt tttaaaaatg aacaaagctt ttgagaatta 155520

tggggttatg taaagcgatc aaacctatga cttattggca ttcctgagag agaagaagaa 155580

aagtaagcaa cctggaaaac atatttgaag gaataattca ggaaagaatt ttcctaacct 155640

ttcaagagag attgatattc agatacaaga aattcggaca acttctgcaa gatactatac 155700

aagataacca gtgaaaagca tacagtcatc agacttccca agataaatgt gaaagaaaaa 155760

atcttaaagg cagctgaaga aaagggccag attatctata aaggaaatcc catcagacta 155820

acagcaaact tttcaaagga aatcttacaa gtcagaagaa actggggcct atttttagcc 155880

acctaaaaga aaaaaaaaac tgcctgtcaa agtttcatac tctgccaatt taagcttcat 155940

aaatgaagga gaaatagttt ttcccagaca gagaaatgct aagggaattc attaacacca 156000

gatagtctct ataataaatg ttcaaaggag ttctaaatat ggatggtact tgctaccata 156060

aaagcacatg taattacaaa gctcgtacac cttataaagc aatatataat tgggtctaca 156120

aagcaactag cttaacacta tgacagaaat aacacctcac acatgaatat taaccttgaa 156180

tgtaaatggc ctgaaagttc cacttaacgg gcatagagta gtccaggtgt ggtggctcac 156240

acttgtaatc cctgcagttt gggaggccaa ggtgggtgga tcacttgagg tcaggagttc 156300

gaaaccagcc tggccaacat ggtgaaactt catctccact aaaatacaaa aattagctgg 156360

gcgtggtggt gggcgcctgt aatgccagct actcgggaag ctgaggcagg agaatcactt 156420

gaacccagga agcagaggct gcagtgagcc aagatcgcac cactgaactc agcctgggac 156480

tccatcaaaa aaaaaaaaaa aaggcatagg gtggcaaatt gggaaaaaaa aaaatcaaac 156540

tttccatggc ctttaagaga gacctatctt acatgttata gcccccaggg gctcaaagta 156600

aagtgataga gatctgttat gcaaatggaa aacaagaaag gtcaggagtt gctattcttg 156660

tatcaagtaa aacagactaa accaacaaga gttaaaaaaa gaaaaaagac aaagaagggc 156720

attacataat cagaaagcat tcaattcaac aagaaga 156757

## Description

### FIELD OF THE INVENTION

The invention relates to the field of treatment and, more in particular, to a SETDB1 antagonist, preferably mithramycin or a SETDB1-specific RNAi, for use in the treatment of lung cancer in a subject having gene amplification of SETDB1. The invention also relates to a method for predicting the clinical response of a subject suffering from lung cancer to a treatment with a SETDB1 antagonist based on the amplification of the SETDB1 gene in a sample from said subject; and to a method for selecting a lung cancer patient for being treated with a SETDB1 antagonist.

### BACKGROUND OF THE INVENTION

Routine cancer management using chemotherapy, whether as definitive or adjuvant therapy, has improved patient's absolute survival when compared with non-chemotherapy control. However, not all the chemotherapeutic treatments available are suitable for all patients. The efficacy of chemotherapeutic drugs in patients suffering from cancer is influenced by the presence of certain genetic markers. Patients whose tumours have low probability to respond to a chemotherapeutic treatment may omit chemotherapy altogether or may be candidates for alternative treatments, avoiding unnecessary therapeutic side effects.

Therefore, there is a necessity for a personalized approach for the treatment of the disease, particularly in cancers such as lung cancer.

Lung cancer is one of the leading causes of worldwide death, and non-small cell lung cancer (NSCLC) accounts for approximately 85% of all lung cancers, with 1.2 million new cases worldwide each year. NSCLC resulted in more than one million deaths worldwide in 2001 and is the leading cause of cancer-related mortality in both men and women (31% and 25%, respectively). The other main type of lung cancer, namely small-cell lung cancer (SCLC), is most often more rapidly and widely metastatic than non-small cell lung carcinoma and involves early hilar and mediastinal lymph nodes. Without treatment, SCLC has the most aggressive clinical course of any type of pulmonary tumor, with median survival from diagnosis of only 2 to 4 months.

Advanced lung cancers are treated with chemotherapy. There are a number of possible therapies for drug cancer but there are still many patients who receive chemotherapy from which they do not benefit, typically experiencing unnecessary toxicity and a negative impact on their quality of life. Therefore, the advent of the predictive value of any new biomarker is essential in order to improve the patient's outcome by supporting the fitting of lung cancer patient with the most effective personalized anticancer treatment available.

Gene amplification is a common mechanism in deregulated oncogene expression, mainly in solid tumours. The identification of amplified target genes is of great importance for cancer diagnosis and prognosis, and ultimately for designing targeted therapies. An example is the oncogene HER2 in breast cancer where the copy number amplification is associated to poor outcome and is a predictive factor of response to anti-HER2 therapy (Susini T et al. 2010. Gynecol. Oncol., 116(2): 234-239).

Mithramcycin A was described to be capable of inhibiting the growth of human lung cancer cell lines CL1-5 and H647 and inhibiting the invasion phenotypes of CL1-5 cells. However, said effects are due to the ability of mithramyicin of reactivating SLIT2 expression by decreasing methylation of SLIT2 promoter, and to the inhibition of DNMT1 (Ruo-Kai Lin and Yi-Ching Wang. 2007. Bioformosa, 42, 55-62).

Watanabe H. *et al.* (Watanabe H. et al. 2008. Cancer Cell Int, 8:15) discloses that expression of SETDB1 is increased in non-small cell lung cancer cell lines. However, SETDB1 silencing of immortalized and transformed human bronchial epithelial cells by siRNA treatment did not alter the cell growth rate or the DNA synthesis level and did not induce apoptosis, therefore suggesting that the inhibition of SETDB1 is not a suitable treatment for lung cancer.

However, there is still a need for further markers useful for predicting the response of lung cancer patients to a chemotherapeutic treatment.

### BRIEF DESCRIPTION OF THE INVENTION

In a first aspect, the invention relates to a SETDB1 antagonist for use in the treatment of lung cancer in a subject having SETDB1 gene amplification.

In another aspect, the invention relates to an *in vitro* method for predicting the clinical response of a subj ect suffering from lung cancer to a treatment with a SETDB 1 antagonist comprising detecting whether the SETDB1 gene is amplified in a sample from said subject, wherein amplification of the SETDB1 gene is indicative of a good clinical response of the subject to the treatment with said antagonist.

In yet another aspect, the invention relates to an *in vitro* method for selecting a lung cancer patient for being treated with a SETDB antagonist comprising detecting whether the SETDB1 gene is amplified in a sample from said patient, wherein amplification of the SETDB1 gene is indicative that the subject is a candidate for a treatment with a SETDB1 antagonist.

In yet another aspect, the invention relates to the use of a reagent capable of detecting the amplification of SETDB1 gene in a sample from a subject suffering from lung cancer for predicting the clinical response of said subject to a treatment with a SETDB1 antagonist or for selecting a lung cancer patient for being treated with a SETDB1 antagonist.

### BRIEF DESCRIPTION OF THE FIGURES

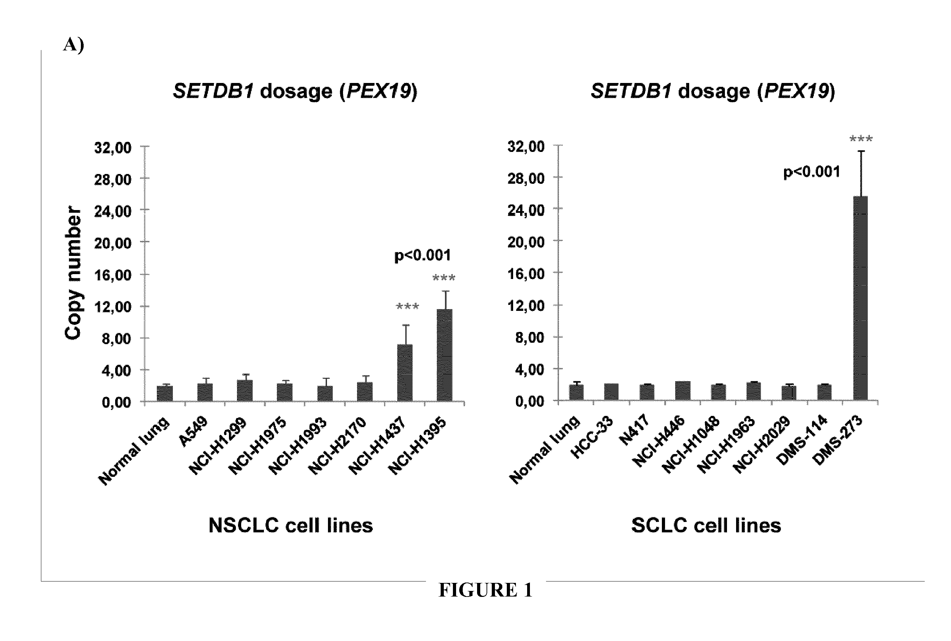

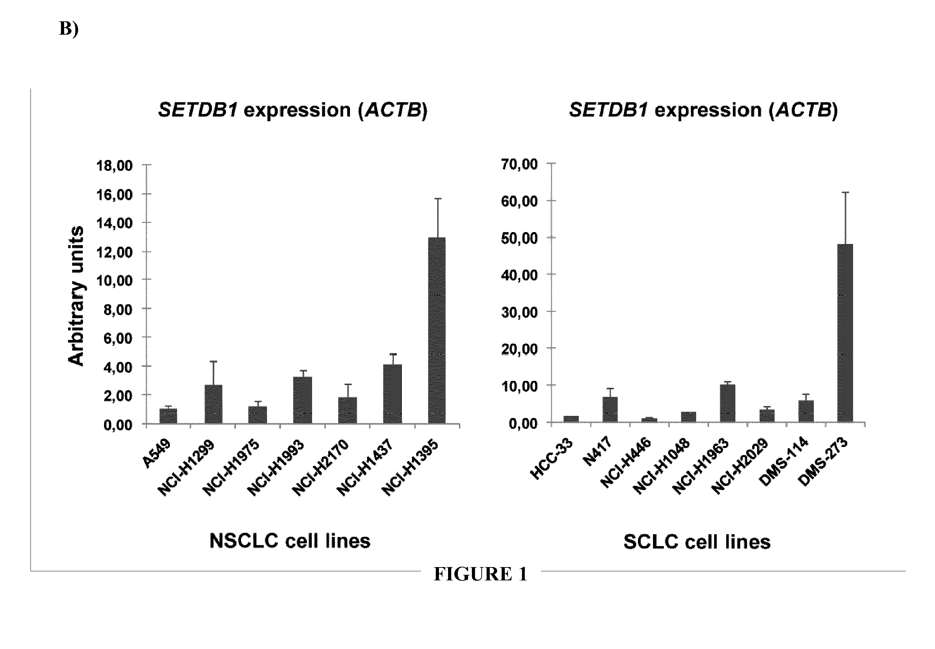

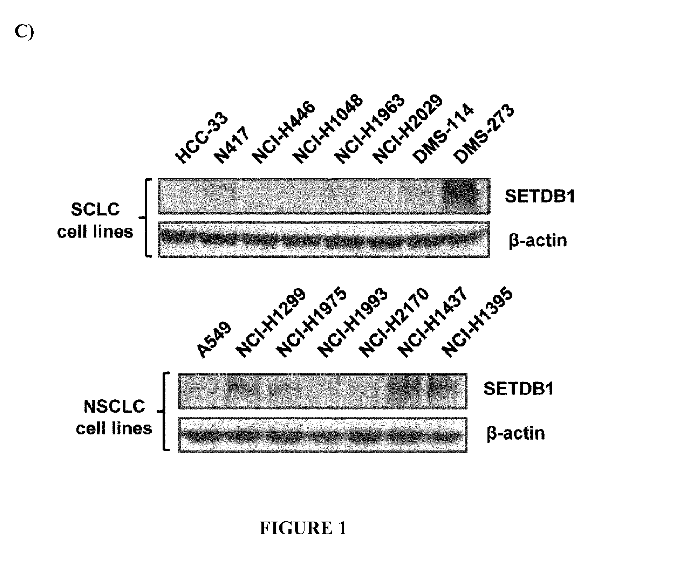

**Figure 1****.** Determination of SETDB1 gene amplification and its association with RNA and protein overexpression in lung cancer cell lines. **(A)** Assessment of SETDB1 copy number by quantitative genomic PCR. Amplification frequency of SETDB1 (evaluated with SYBR Green) was calculated by the standard curve method using the 7900HT SDS program. To define an internal control gene chromosome 1p36.23 was chosen because it is the least aneuploid region among our cell lines (*PEX19* gene). DNA from normal lung was used as the reference standard. Results are reported as n-fold copy number increase relative to the *PEX19* gene. Quantitative RT-PCR **(B)** and western blot **(C)** demonstrate higher levels of SETDB1 mRNA and protein (ab12317, Abcam, Cambridge, UK), respectively, in amplified cancer cell lines (H1437, NCI-H1395 and DMS-273) than in unamplified cells. The internal control gene was ACTB (beta-actin).

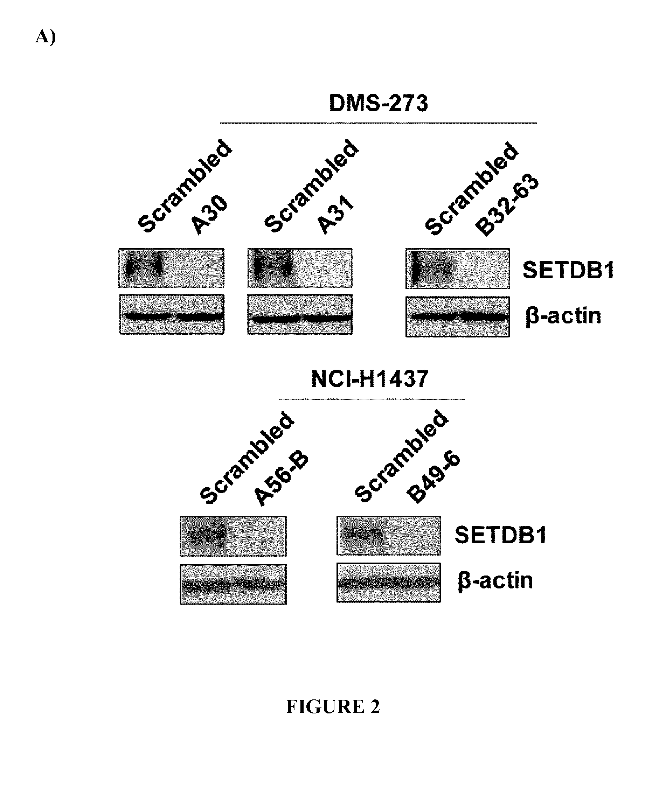

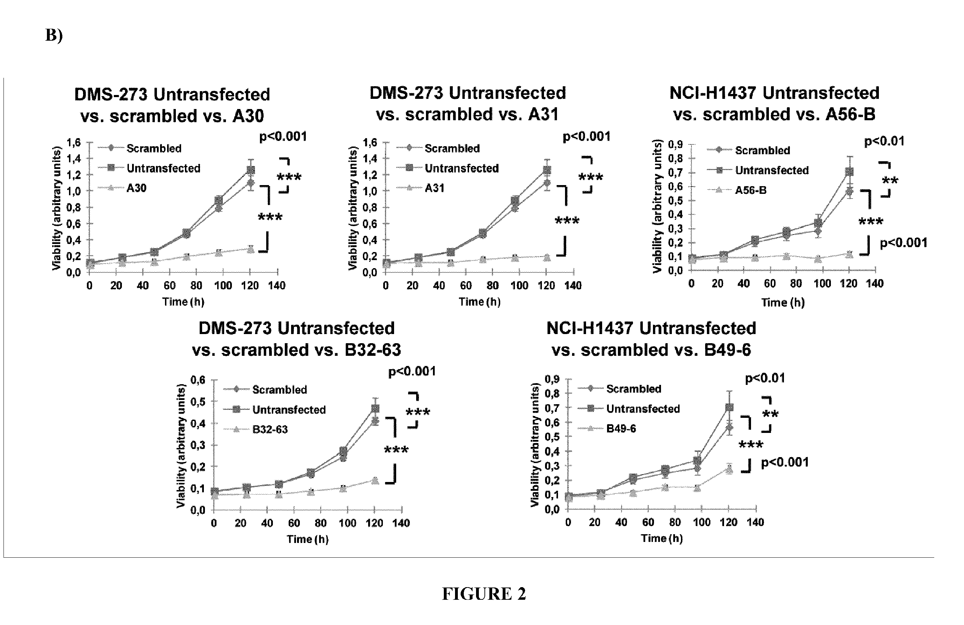

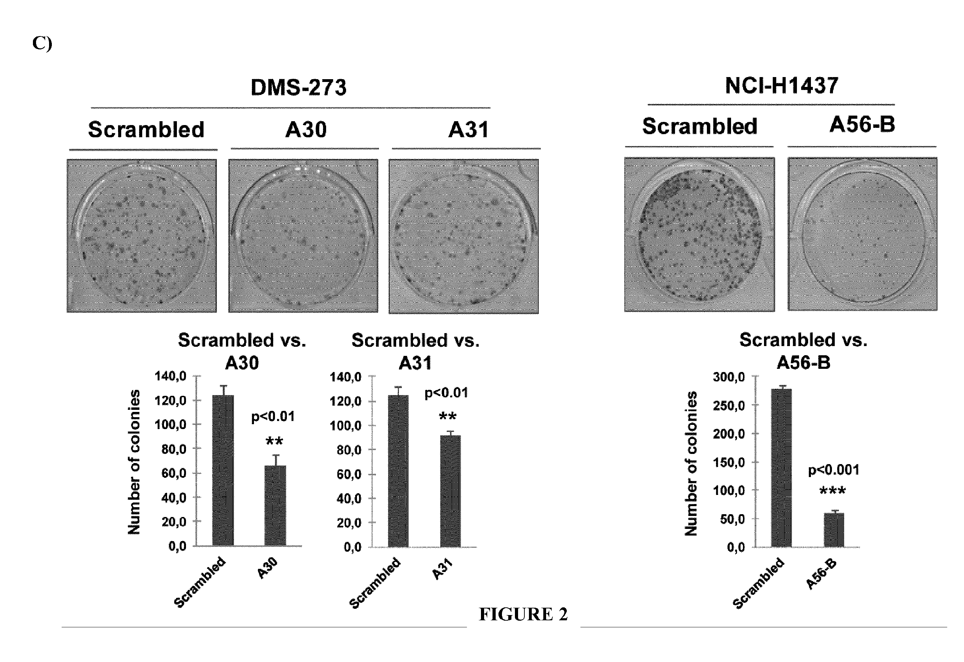

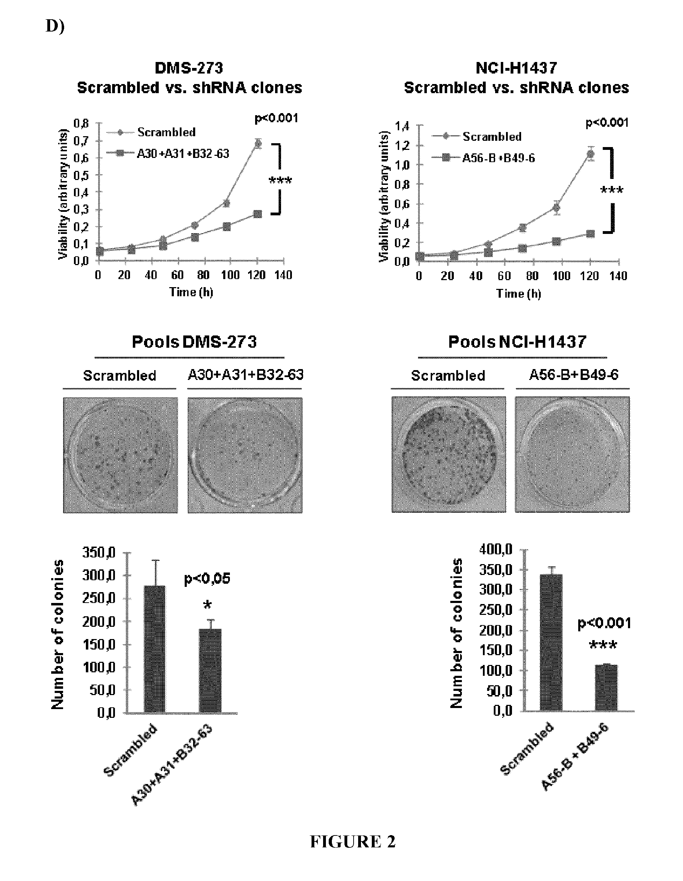

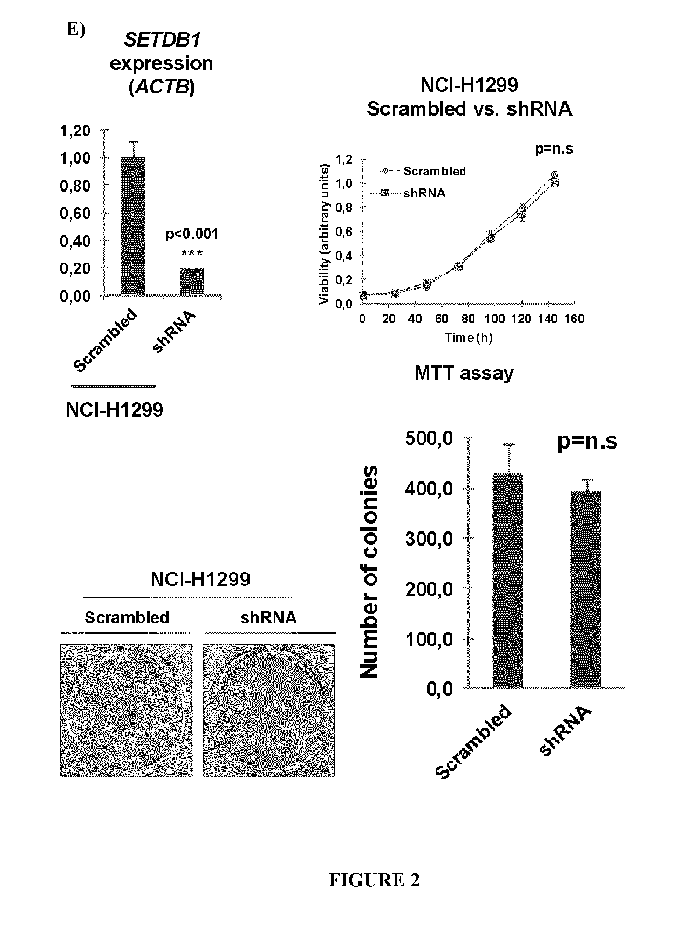

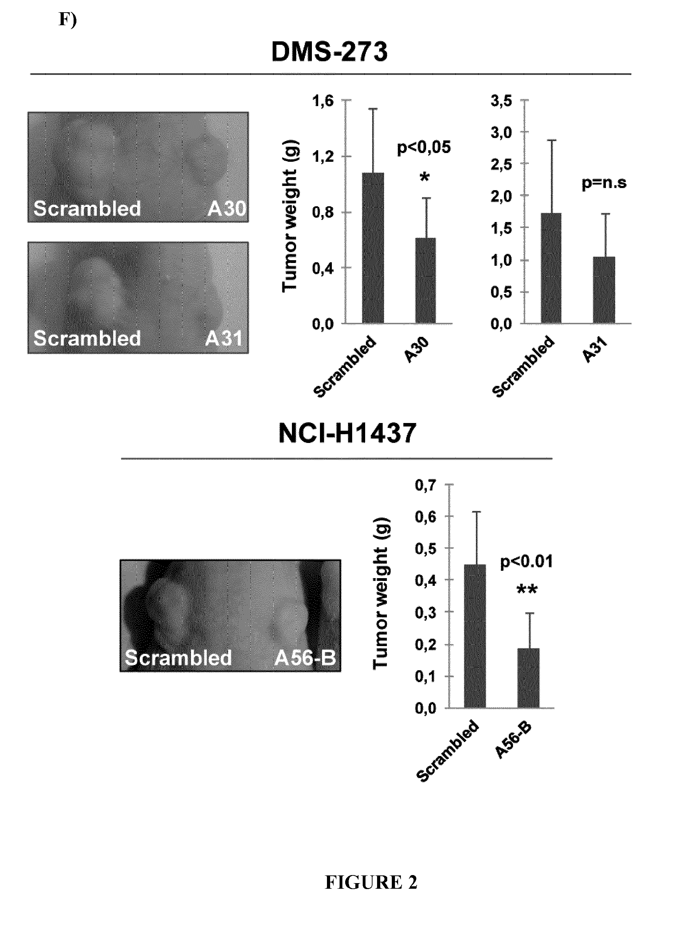

**Figure 2****.** Growth-promoting effects of SETDB1 in lung cancer. **(A)** Stable down-regulation of the SETDB1 gene by short hairpins using two different target sequences for DMS-273 (clones A30/A31 and clone B32-63) and NCI-H1437 (clones A56-B and B46-9). **(B)** The short-hairpin SETDB1-depleted cells were less viable in the 3-(4,5-dimethyl-2-thiazolyl)-2,5-diphenyl-2H-tetrazolium bromide (MTT) assay than in the untransfected or scrambled-shRNA transfected cells (p-values obtained by the ANOVA test). h, hours. **(C)** The colony formation assay showed that DMS-273 and NCI-H1437 cells stably transfected with the shRNA against SETDB1 formed significantly fewer colonies than scrambled shRNA-transfected cells (p-values obtained by the ANOVA test). Data shown are means ± SD, n =3. **(D)** MTT and colony formation assays performed with pools of scrambled-shRNA and SETDB1 shRNA clones of DSM-273 and NCI-H1437 lung cancer cell lines. These experiments further verify the results obtained with individual clones. All p-values were obtained by the ANOVA test. For colony formation assays, data shown are means ± SD, n = 3. h, hours. **(E)** In the non-amplified lung cancer cell line NCI-H1299, stable depletion of SETDB1 by means of shRNA has no effect on cell growth in the MTT assay. Below, colony formation assays show that NCI-H1299 cells stably transfected with the shRNA against SETDB1 formed a similar number of colonies than scrambled shRNA-transfected cells. All p-values were obtained by the ANOVA test. For colony formation assays data shown are means ± SD, n = 3. h, hours; n.s, nonsignificance. **(F)** Effect of SETDB1 shRNA-mediated depletion on the growth of DMS-273 and NCI-H1437 xenografts in nude mice. Tumor volume was monitored over time and the tumor was excised and weighed at 30 days. There was a significant decrease in tumor weight in the SETDB1 shRNA stably transfected cells (p-values obtained by the ANOVA test). Data shown are means ± SD, n =10. n.s, nonsignificance; g, grams.

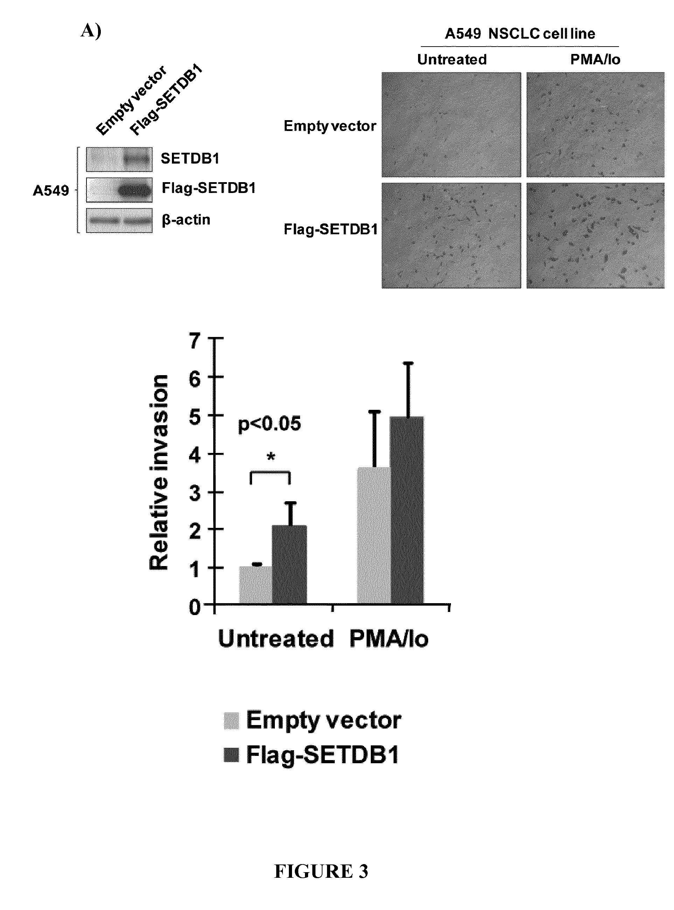

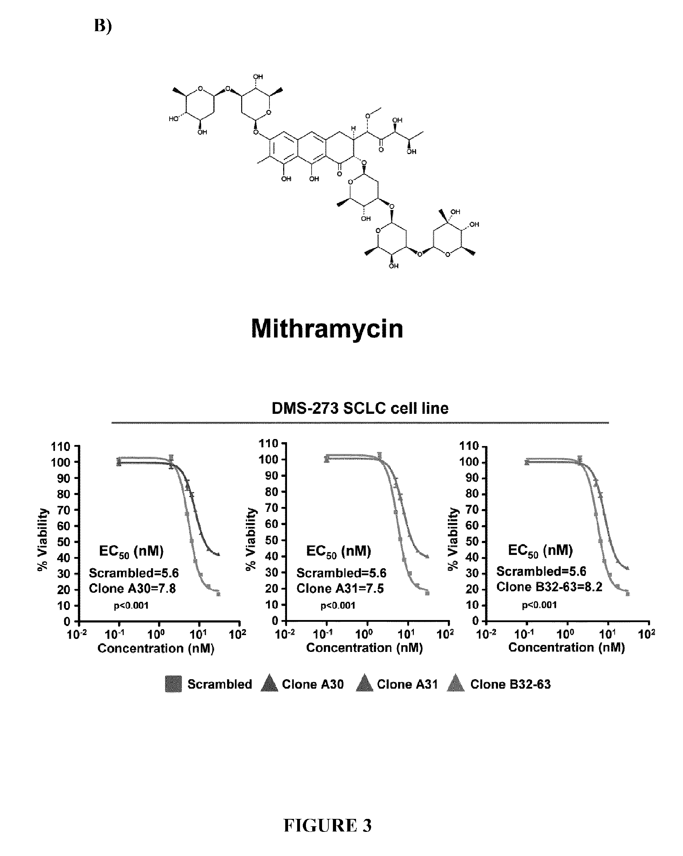

**Figure 3****.** Impact of SETDB1 in invasiveness and chemosensitivity. (A) Effect of SETDB1 on the invasion potential of A549 cells determined by the matrigel invasion assay. Cells were transfected with 3 µg of Flag-SETDB1 or empty vector in 60 mm dishes. After 24 hours cells were stimulated or not with PMA plus Io (PMA/Io) for 30 minutes. Then cells were trypsinized, and 5x10⁴ cells were resuspended in free-serum media and added to the upper compartment of a transwell coated with 1 mg/mL Matrigel. Media with 10% FBS was added in the lower compartment and cells were incubated at 37°C for 42 hours. Invasive cells were fixed with PBS 4% paraformaldehide, stained with 0.5% violet crystal and visualized and photographied under a 10X magnification objective with a microscope. Invasive cells were counted using ImageJ 1.45s (Wayne Rasband, National Institutes of Health, USA) and the percentage of invasive cells was represented. Results are the mean of at least three experiments by duplicate and the significance was determined using ANOVA-test. *p<0.05. PMA, phorbol 12-myristate 13-acetate; Io, ionophore A23187. **(B)** Cancer cells harboring the SETDB1 gene amplification are sensitive to the decrease in cell viability caused by mithramycin. MTT assays in control scrambled shRNA DMS-273-transfected cells in comparison with three shRNA stable downregulated SETDB1 clones (A21, A30 and A31) show enhanced inhibition of viability in cells with SETDB1 gene amplification-mediated overexpression. EC₅₀: half maximal effective concentration.

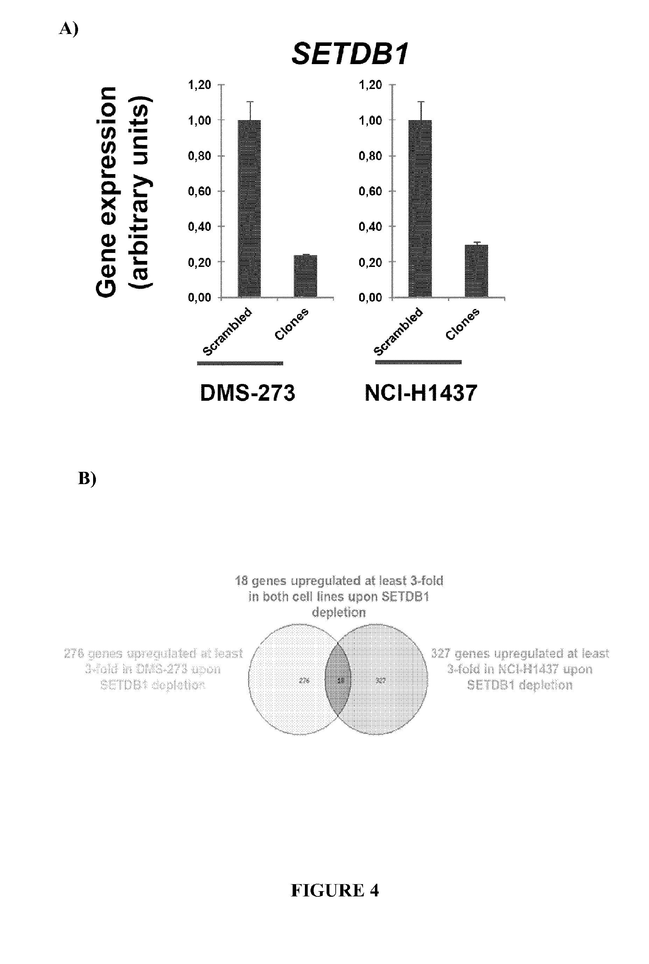

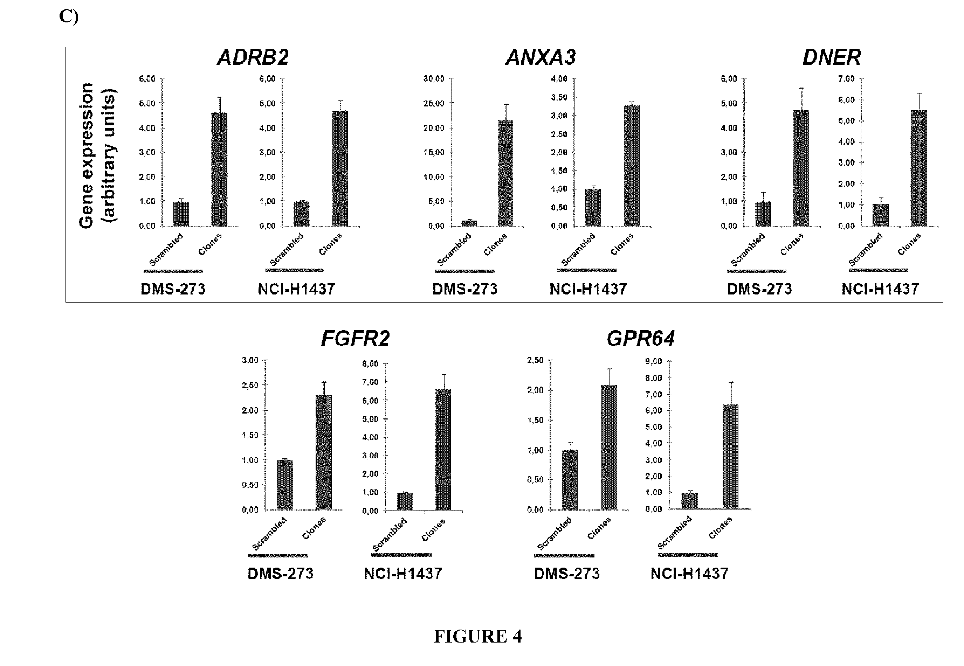

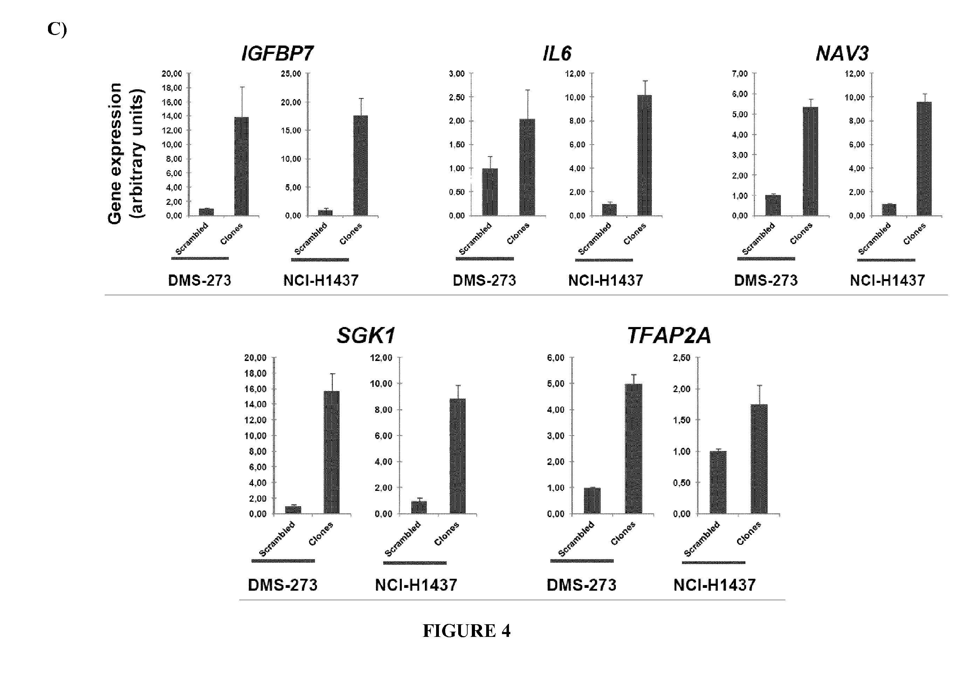

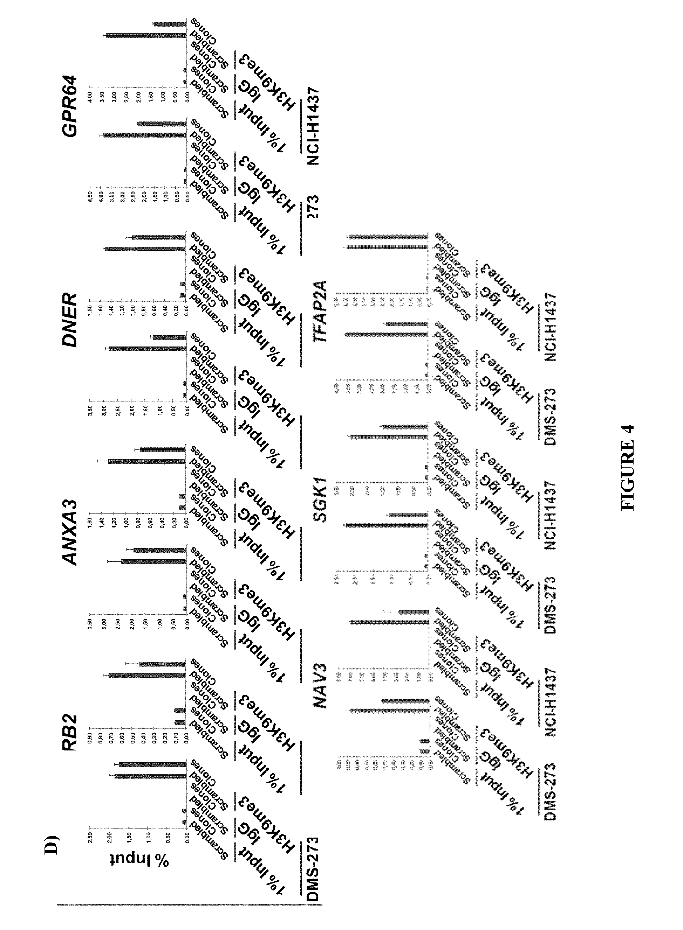

**Figure 4****.** SETDB1 target genes in DMS-273 and NCI-H1437 lung cancer cell lines. **(A)** As a control for the microarray experiments, qRT-PCR experiments were performed to confirm the down-regulation of SETDB1 by means of shRNA in DMS-273 (mix of A30 A31 and B32-63 clones) and NCI-H1437 (mix of A56-B and B49-6 clones) in comparison to their scrambled-shRNA control cells. **(B)** Venn diagram illustrates the most upregulated genes in both cell lines upon SETDB1 depletion (Fold Change ≥ 3). **(C)** Ten transcripts upregulated in both cell lines upon SETDB1 depletion (according to the microarrays) were further selected for validation using qRT-PCR. **(D)** For selected candidate genes quantitative chromatin immunoprecipitation assays (qChIP) using anti-H3K9me3, anti-H3 and anti-rabbit IgG antibodies were performed. ChIP levels are represented as percentage of input chromatin precipitated for each gene promoter region. The experiments depict a reduction of H3K9me3 in DMS-273 and NCI-H1437 cells stably transfected with shRNAs against SETDB1.

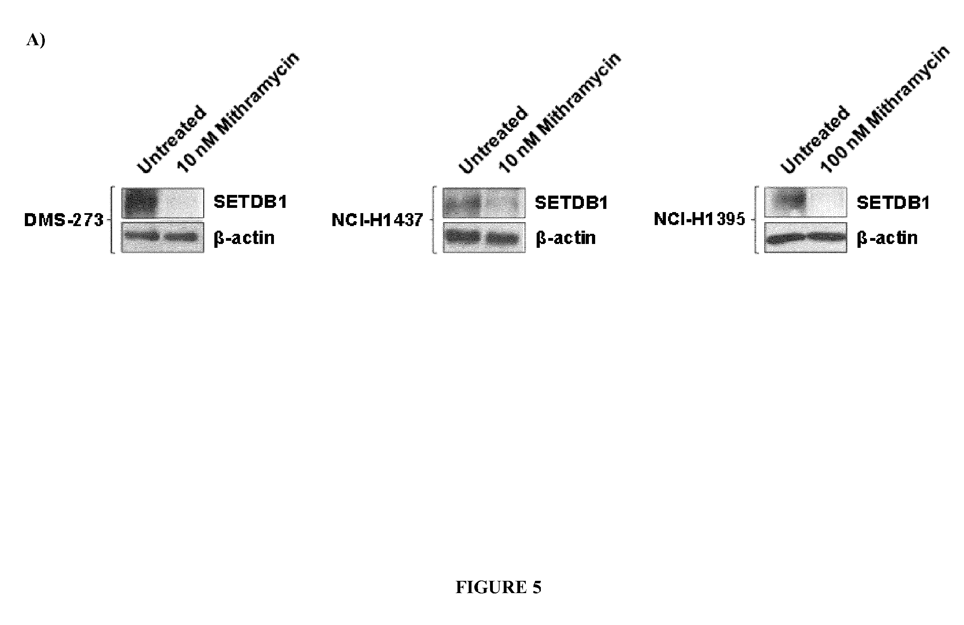

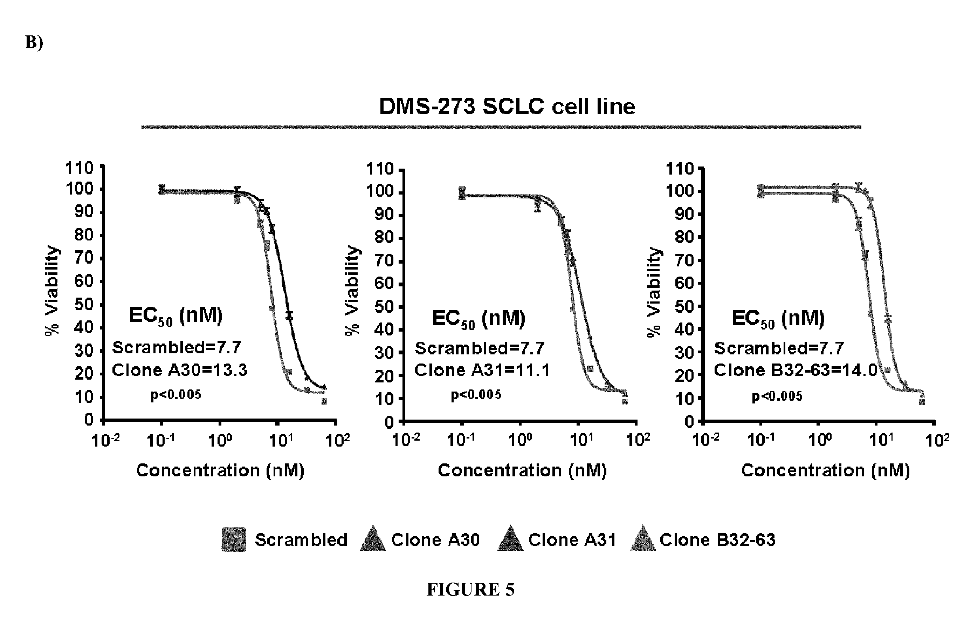

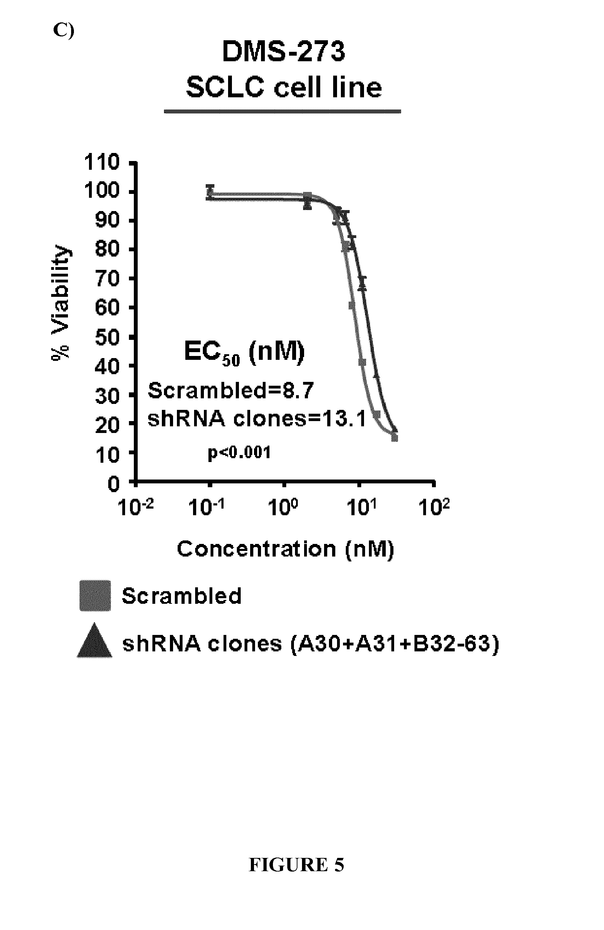

**Figure 5****.** Effect of mithramycin on SETDB1-amplified lung cancer cell lines. **(A)** Western blot experiments show that lung cancer cell lines harboring the SETDB1 amplification (DMS-273, NCI-H1437 and NCI-H1395) undergo a decrease of the protein upon mithramycin treatment. **(B)** Mithramycin diminishes the viability of SETDB1-amplified DMS-273 cells. This experiment, an MTT assay performed with another scrambled-shRNA DMS-273 clone, further demonstrates a higher sensitivity of the scrambled-shRNA DMS-273-transfected cells compared with the 3 different shRNA SETDB1 clones (A21, A30 and A31). (C) Similar results were obtained comparing a pool of control scrambled-shRNA DMS-273-transfected cells with a pool of SETDB1-depleted clones (A21, A30, A31 and B32-63). EC₅₀: half maximal effective concentration.

### DETAILED DESCRIPTION OF THE INVENTION

The inventors of the present invention have discovered that, surprisingly, the histone methyltransferase SETDB1 undergoes gene amplification in non-small and small cell lung cancer cell lines and primary tumors and that the increased gene dosage of SETDB1 is associated with enhanced sensitivity to the growth-inhibitory effect mediated by the drug mithramycin. The inventors have also found that the reduction of SETDB1 expression in gene-amplified cells by using shRNA against SETDB1 has cancer-growth inhibitory features. In this sense, SETDB1 gene amplification has potential value as a genetic marker for predicting a good response to a SETDB1 antagonist, preferably mithramycin or a SETDB1-specific RNAi, in lung cancer patients. Based on these findings, the inventors have developed the methods of the present invention in their different embodiments that will be described now in detail.

The results provided in the example of the present invention clearly show a significant association of SETDB1 gene amplification with a good response to mithramycin or a SETDB1-specific RNAi. Thus, these results suggest that subjects suffering from lung cancer with SETDB1 gene amplification are candidates for a treatment with a SETDB1 antagonist, preferably with mithromycin or a SETDB1-specific RNAi.

### THERAPEUTIC METHODS OF THE INVENTION

The results obtained in the present invention show that SETDB1 gene amplification in lung tumours indicates a high probability of a successful therapeutic treatment with a SETDB 1 antagonist, an indication that this may be the best treatment available for such patients. SETDB1 antagonists would be the choice as first line treatment for patients with SETDB1 gene amplification.

In another aspect, the invention relates to a SETDB1 antagonist for use in the treatment of lung cancer in a subject having SETDB1 gene amplification.

Alternatively, the invention relates to the use of a SETDB1 antagonist for the manufacture of a medicament for the treatment of a subject suffering from lung cancer wherein said subject has SETDB1 gene amplification.

Alternatively, the invention relates to a method for the treatment of lung cancer in a subject comprising administering to said subject of a SETDB1 antagonist wherein said subject has SETDB1 gene amplification.

The term "SETDB1 gene", as used herein, refers to the gene which codes for the enzyme histone H3 lysine 9-specific methyltransferase (EC 2.1.1.43) which is involved in the transcriptional silencing of euchromatic genes and retroelements. The gene that encodes said histone methyltransferase (Gene ID: 9869, human) resides in a recurrently amplified chromosome 1q21 interval. Said gene is also known as ESET, KMT1E, H3-K9-HMTase4, KG1T, TDRD21 or KIAA0067. The term "SETDB1 gene" encompasses the SETDB1 gene of any mammalian species, including but not being limited to domestic and farm animals (cows, horses, pigs, sheep, goats, dogs, cats or rodents), primates and humans. Preferably, the SETDB gene is human. In the present invention "human SETDB1 gene" is understood as the gene defined in the NCBI database, version October 30, 2012 (positions 150898815 to 150937220 in the genomic region shown in the NCBI database under accession number NC_000001.10).

As used herein, the term "SETDB1 antagonist" refers to any agent capable of inhibiting or decreasing the histone methyltransferase activity of SETDB1, including those agents which prevent the expression of the SETDB1 gene, causing reduced levels of mRNA or SETDB1 protein, as well as agents which inhibit SETDB1 causing a decrease in the activity of the enzyme. Said agent can be any organic or inorganic molecule, including modified and unmodified nucleic acids such as antisense nucleic acids, RNA interference (RNAi) agents such as siRNA, shRNA or miRNA, peptides, proteins, peptidomimetics, receptors, ligands, antibodies and small organic molecules that are useful in treating lung cancer.

The SETDB1 antagonist inhibits the histone methyltransferase activity of SETDB1 by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or even 100% in a subject compared to the SETDB1 activity prior to, or in the absence of, administration of the agent.

The person skilled in the art knows how to determine if an agent is a SETDB 1 antagonist using standard assays for determining the mRNA expression levels such as RT-PCR, RNA protection analysis, Northern procedure, in situ hybridization, microarray technology and the like.

The compounds which cause reduced levels of SETDB1 protein can be identified using standard assays for determining the protein expression levels such as immunoblot or Western blot, ELISA (adsorption enzyme immunoanalysis), RIA (radioimmunoassay), competitive EIA (competitive enzyme immunoassay), DAS-ELISA (double antibody sandwich ELISA), immunocytochemical and immunohistochemical techniques, techniques based on the use of protein microarrays or biochip which include specific antibodies or assays based on colloidal precipitation in formats such as reagent strips.

The determination of the inhibiting capacity of the biological activity of SETDB1 is detected using standard assays to measure the activity of SETDB1 histone methyltransferase. An assay to assess if a compound is a SETDB1 antagonist suitable for the present invention consists of comparing the catalytic ability of SETDB1 to methylate H3K9 before and after the addition of the compound. In one assay, wells of a multi-well plate are coated with an appropriate immobilized substrate, such as an assembled recombinant nucleosomes or a histone peptide (preferably including an H3K9-comprising target peptide for SETDB1) immobilised via biotin-streptavidin linkage. To each well is added a reaction solution comprising SETDB1, S-adenosyl-methionine co-factor and the compound to be tested. If the candidate compound acts as an inhibitor of SETDB1 then methylation of amino acid residues on histone H3 (comprised within the nucleosome substrate) or on the H3 peptide can be reduced or prevented. The determination of methylation status of the lysine residues in the histone H3/peptide can be determined by use of an antibody that specifically binds to the methylated target lysine residue in histone H3 (i.e. H3K9me3). The antibody can be linked to a colour generating reaction, so as to form an ELISA-type assay. In this way, wells of the multi-well plate that show a colour reaction correspond to reactions where inhibition of SETDB1 has not occurred, whereas candidate compounds present in the unco loured wells are identified as candidate inhibitors of SETDB activity.

In a preferred embodiment the SETDB1 antagonist is an agent that silences said enzyme, preferably a SETDB1-specific RNAi. The term "SETDB1-specific RNAi" refers to an RNA interference (RNAi) agent such as siRNA, shRNA or miRNA capable of decreasing the expression of SETDB1 protein. An enzyme is silenced when its expression is decreased with respect to the expression basal levels by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

The RNAi has to be specific for the mRNA of SETDB1 ("SETDB1-specific RNAi"). This refers to the capacity of the nucleic acids for binding specifically to their target sequence and not to other sequence. The specificity is validated by performing BLAST analysis, by the use of negative controls, and by determining changes in mRNA levels of unrelated genes.

The expression "basal levels" refers to the level of expression of a gene in a normal cell in conditions in which no manipulation of said cell has taken place.

The expression "expression levels" or its grammatical equivalent, as used herein, means a measurement of the amount of nucleic acid, e.g. RNA or mRNA, or protein of a gene in a cell, or alternatively, the level of activity of a gene or protein in said cell.

As the person skilled in the art understands, the expression levels of SETDB1 gene can be measured by determining the mRNA expression levels of said gene or by determining the protein levels encoded by said gene, i.e. the SETDB1 protein.

In order to measure the mRNA levels of the SETDB1 gene, the cell may be treated to physically, mechanically or chemically disrupt cell structures, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

Determination of the levels of SETDB1 mRNA can be carried out by any method known in the art such as qPCR, northern blot, RNA dot blot, TaqMan®, tag based methods such as serial analysis of gene expression (SAGE) including variants such as LongSAGE and SuperSAGE, microarrays, nucleic acid sequence based amplification (NASBA), Fluorescence In Situ Hybridization, including variants such as Flow-FISH, qFiSH and double fusion fish (D-FISH) as described in WO2010030818, Femino et al. (Science, 1998, 280:585-590), Levsky et al. (Science, 2002, 297:836-840) or Raj et al. (PLoS Biology, 2006, 4:e309).

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts cells. Preferably, the control RNA is mRNA derived from housekeeping genes and which code for proteins which are constitutively expressed and carry out essential cellular functions. Examples of housekeeping genes for use in the present invention include β-2-microglobulin, ubiquitin, 18-S ribosomal protein, cyclophilin, GAPDH, PSMB4, tubulin and β-actin.

The expression levels of the SETDB1 gene may also be measured by determining the protein levels encoded by said gene, i.e. the SETDB1 protein or variants thereof. Practically any conventional method can be used within the context of the present invention to quantify the levels of SETDB1 protein such as Western blot or immunohistochemistry.

The value obtained for the expression of SETDB1 nucleic acid or protein levels in a modified cell is then compared with basal levels of SETDB1 in an unmodified cell. This allows detecting a decrease in the expression of SETDB1 under basal levels. A decrease in the expression under basal levels, as used herein, refers to a change of expression of a given gene with respect to the expression basal levels of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

The SETDB1-specific RNAi can be a siRNA (small interfering RNA), which is a class of double-stranded RNA molecule, 20-25 nucleotides in length with phosphorylated 5' ends and hydroxylated 3' ends with two overhanging nucleotides that interferes with the expression of specific genes with complementary nucleotide sequence. siRNAs can be used in a naked form and incorporated in a vector as shRNA.

The SETDB1-specific RNAi can be a shRNA (short hairpin RNA), which is a sequence of RNA that makes a tight hairpin turn that can be used to silence target gene expression via RNA interference (RNAi). Expression of shRNA in cells is typically accomplished by delivery of plasmids or through viral vectors using polymerase II and polymerase III promoters.

siRNAs have been described in Brummelkamp et al., Science 296; 550-553,2002, Jaque et al., Nature 418; 435-438, 2002, Elbashir S. M. et al. (2001) Nature, 411: 494-498, McCaffrey et al. (2002), Nature, 418: 38-39; Xia H. et al. (2002), Nat. Biotech. 20: 1006-1010, Novina et al. (2002), Nat. Med. 8: 681-686, and U.S. Application No. 20030198627.

In a preferred embodiment the SETDB1-specific RNAi is a nucleic acid selected from the group consisting of a small interfering RNA (siRNA) and a short hairpin RNA (shRNA) specific for the mRNA of said enzyme.

The SETDB1 antagonist can be a small organic molecule. In a preferred embodiment the SETDB antagonist is mithramycin or a pharmaceutically acceptable salt, solvate, polymorph or cocrystal thereof. In a more preferred embodiment the SETDB1 antagonist is mithramycin or a pharmaceutically acceptable salt thereof.

"Mithramycin", as used herein, is also referred to as plicamycin or mithramycin A. It is an antineoplastic antibiotic produced by *Streptomyces plicatus* which acts as RNA synthesis inhibitor.

The chemical name of mithramycin is (1S)-5-deoxy-1-C-((2S,3S)-7-{[2,6-dideoxy-3-O-(2,6-dideoxy-β-D-arabino-hexopyranosyl)-β-D-arabino-hexopyranosyl]oxy}-3-{[2,6-dideoxy-3-C-methyl-β-D-ribo-hexopyranosyl-(1→3)-2,6-dideoxy-β-D-arabino-hexopyranosyl-(1→3)-2,6-dideoxy-β-D-arabino-hexopyranosyl]oxy}-5,10-dihydroxy-6-methyl-4-oxo-1,2,3,4-tetrahydroanthracen-2-yl)-1-O-methyl-D-xylulose and its chemical structure is represented by the formula depicted in Figure 3B.

The term "pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

The term "pharmaceutically acceptable salt" embraces salts with a pharmaceutically acceptable acid or base. Pharmaceutically acceptable acids include both inorganic acids, for example and without limitation hydrochloric, sulfuric, phosphoric, diphosphoric, hydrobromic, hydroiodic and nitric acid and organic acids, for example and without limitation citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example and without limitation alkyl amines, arylalkyl amines and heterocyclic amines.

The term "solvate" according to this invention is to be understood as meaning any solid form of mithramycin which has another molecule attached to it via noncovalent bonding. Examples of solvates include hydrates and alcoholates, preferably C1-C6 alcoholates, e.g. methanolate.

The term "polymorph" according to this invention is to be understood as a specific crystalline form of a compound that can crystallize in different forms.

The term "cocrystal", as used herein, is to be understood as a crystalline structure composed of mithramycin and at least another component.

The terms "lung cancer" or "lung tumour" refer to the physiological condition in mammals characterized by unregulated cell growth in tissues of the lung. The term lung cancer is meant to refer to any cancer of the lung and includes non-small cell lung carcinomas and small cell lung carcinomas. In an embodiment, the lung cancer is non-small cell lung cancer (NSCLC). In another embodiment the lung cancer is small cell lung cancer (SCLC).

The term non-small cell lung cancer (NSCLC), as used herein, refers to a group of heterogeneous diseases grouped together because their prognosis and management is roughly identical and includes, according to the histologic classification of the World Health Organization/International Association for the Study of Lung Cancer (Travis WD et al. Histological typing of lung and pleural tumours. 3rd ed. Berlin: Springer-Verlag, 1999):
(i) squamous cell carcinoma (SCC), accounting for 30% to 40% of NSCLC, starts in the larger breathing tubes but grows slower meaning that the size of these tumours varies on diagnosis.
(ii) adenocarcinoma is the most common subtype of NSCLC, accounting for 50% to 60% of NSCLC, which starts near the gas-exchanging surface of the lung and which includes a subtype, the bronchioalveolar carcinoma, which may have different responses to treatment.
(iii) large cell carcinoma is a fast-growing form that grows near the surface of the lung. It is primarily a diagnosis of exclusion, and when more investigation is done, it is usually reclassified to squamous cell carcinoma or adenocarcinoma.
(iv) adenosquamous carcinoma is a type of cancer that contains two types of cells: squamous cells (thin, flat cells that line certain organs) and gland-like cells.
(v) carcinomas with pleomorphic, sarcomatoid or sarcomatous elements. This is a group of rare tumours reflecting a continuum in histologic heterogeneity as well as epithelial and mesenchymal differentiation.
(vi) carcinoid tumour is a slow-growing neuroendocrine lung tumour and begins in cells that are capable of releasing a hormone in response to a stimulus provided by the nervous system.
(vii) carcinomas of salivary gland type begin in salivary gland cells located inside the large airways of the lung.
(viii)unclassified carcinomas include cancers that do not fit into any of the aforementioned lung cancer categories.

In a particular embodiment the NSCLC is selected from squamous cell carcinoma of the lung, large cell carcinoma of the lung and adenocarcinoma of the lung.

The term small cell lung cancer (SCLC), as used herein, refers to a proliferation of small cells with unique and strict morphological features, containing dense neurosecretory granules which give this tumor an endocrine/paraneoplastic syndrome associated. Most cases arise in the larger airways (primary and secondary bronchi). These cancers grow quickly and spread early in the course of the disease.

The term "treat" or "treatment" refers to a therapeutic treatment, as well as a prophylactic or prevention method, wherein the goal is to prevent or reduce an unwanted physiological change or disease, such as lung cancer. Beneficial or desired clinical results include, but not limiting, release of symptoms, reduction of the length of the disease, stabilized pathological state (specifically not deteriorated), retardation in the disease's progression, improve of the pathological state and remission (both partial and total), both detectable and not detectable. "Treatment" can mean also to prolong survival, compared to the expected survival if the treatment is not applied. Those who need the treatment include those who are suffering from lung cancer and have SETDB1 gene amplification, as well as those with tendency to suffer from lung cancer.

The term "subject", as used herein, refers to all animals classified as mammals and includes, but is not restricted to, domestic and farm animals, primates and humans, e.g., human beings, non-human primates, cows, horses, pigs, sheep, goats, dogs, cats or rodents. Preferably, the subject is a male or female human of any age or race. In the context of the present invention, the subject is a subject suffering from lung cancer or previously diagnosed with lung cancer and having SETDB1 gene amplification. In a preferred embodiment the subject is a mammal, preferably a human being.

The term "gene amplification" (and variants such as "amplification of a gene") refer to a process by which multiple copies of a gene or gene fragment are formed in a particular cell or cell line. The copies of the gene are not necessarily located in the same chromosome. The duplicated region (a stretch of amplified DNA) is often referred to as an "amplicon". The amount of the messenger RNA (mRNA) produced, i.e. the level of gene expression, also increases in proportion to the number of copies made of the particular gene.

The gene amplification can be detected in any kind of sample. The term "sample", as used herein, relates to any sample which can be obtained from the subject and which contains genetic material. The present method can be applied to any kind of biological sample from a subject, such as a biopsy sample, tissue, cell or fluid (serum, saliva, semen, sputum, cerebral spinal fluid (CSF), tears, mucus, sweat, milk), brain extracts, samples obtained by bronchial lavage, bronchoscopy, fine needle aspiration biopsy (FNAB) and the like. In a particular embodiment, the subject has SETDB1 amplification in a tissue sample, preferably a lung tumour tissue sample. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In a preferred embodiment samples are obtained by bronchial lavage. In another preferred embodiment samples are obtained by fine needle aspiration biopsy (FNAB).

Generally, tissue samples are prepared by fixing and embedding the tissue in a medium (such as paraffin). In other examples, samples include a cell suspension which is prepared as a monolayer on a solid support (such as a glass slide) for example by smearing or centrifuging cells onto the solid support. In further examples, fresh frozen (for example, unfixed) tissue sections may be used in the methods disclosed herein.

In some examples an embedding medium is used. An embedding medium is an inert material in which tissues and/or cells are embedded to help preserve them for future analysis. Embedding also enables tissue samples to be sliced into thin sections. Embedding media include paraffin, celloidin, OCT™ compound, agar, plastics, or acrylics.

Many embedding media are hydrophobic; therefore, the inert material may need to be removed prior to histological or cytological analysis, which utilizes primarily hydrophilic reagents. The term deparaffinization or dewaxing is broadly used herein to refer to the partial or complete removal of any type of embedding medium from a biological sample. For example, paraffin-embedded tissue sections are dewaxed by passage through organic solvents, such as toluene, xylene, limonene, or other suitable solvents. The process of fixing a sample can vary. Fixing a tissue sample preserves cells and tissue constituents in as close to a life-like state as possible and allows them to undergo preparative procedures without significant change. Fixation arrests the autolysis and bacterial decomposition processes that begin upon cell death, and stabilizes the cellular and tissue constituents so that they withstand the subsequent stages of tissue processing, such as for *in situ* hybridisation (ISH).

Tissues can be fixed by any suitable process, including perfusion or by submersion in a fixative. Fixatives can be classified as cross-linking agents (such as aldehydes, e.g., formaldehyde, paraformaldehyde, and glutaraldehyde, as well as non-aldehyde cross-linking agents), oxidizing agents (e.g., metallic ions and complexes, such as osmium tetroxide and chromic acid), protein-denaturing agents (e.g., acetic acid, methanol, and ethanol), fixatives of unknown mechanism (e.g., mercuric chloride, acetone, and picric acid), combination reagents (e.g., Carnoy's fixative, methacarn, Bouin's fluid, B5 fixative, Rossman's fluid, and Gendre's fluid), microwaves, and miscellaneous fixatives (e.g., excluded volume fixation and vapor fixation). Additives may also be included in the fixative, such as buffers, detergents, tannic acid, phenol, metal salts (such as zinc chloride, zinc sulfate, and lithium salts), and lanthanum.

The most commonly used fixative in preparing samples for immunohistochemistry (IHC) is formaldehyde, generally in the form of a formalin solution (4 percent formaldehyde in a buffer solution, referred to as 10 percent buffered formalin). In one example, the fixative is 10 percent neutral buffered formalin.

### PREDICTIVE METHODS OF THE INVENTION

The authors of the present invention have observed that amplification of the SETDB1 gene is indicative of a good response to a SETDB1-specific shRNA and to mithramycin in SETDB1-amplified lung cancer cell lines. The example of the present invention describes that mithramycin diminishes the viability of SETDB1-amplified lung cancer cell lines.

Thus, in another aspect, the invention relates to an *in vitro* method for predicting the clinical response of a subject suffering from lung cancer to a treatment with a SETDB antagonist comprising detecting whether the SETDB1 gene is amplified in a sample from said subject, wherein amplification of the SETDB1 gene is indicative of a good clinical response of the subject to the treatment with said antagonist.

The term "predicting", as used herein, refers to the determination of the likelihood that the subject suffering from lung cancer will respond favorably to a therapy with a SETDB1 antagonist. Especially, the term "prediction", as used herein, relates to an individual assessment of the expected response of a subject suffering from lung cancer if the tumour is treated with a SETDB 1 antagonist.

The term "clinical response", as used herein, refers to the response of the subject suffering from lung cancer to a SETDB1 antagonist. Standard criteria (Miller, et al. Cancer, 1981; 47:207-14) that can be used herewith to evaluate the response to chemotherapy include response, stabilization and progression.

The term "response", as used herein, can be a complete response (or complete remission) which is the disappearance of all detectable malignant disease or a partial response which is defined as approximately >50% decrease in the sum of products of the largest perpendicular diameters of one or more lesions (tumour lesions), no new lesions and no progression of any lesion. Subjects achieving complete or partial response were considered "responders", and all other subjects were considered "non-responders". As will be understood by those skilled in the art, such an assessment is usually not intended to be correct for all (i.e. 100 percent) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley and Sons, New York 1983. Preferred confidence intervals are at least 50%, at least 60%, at least 70%, at least 80%, at least 90 percent, at least 95 percent, at least 97 percent, at least 98 percent or at least 99 percent. The p- values are, preferably, 0.1, 0.05, 0.01, 0.005, or 0.0001. More preferably, at least 60 percent, at least 70 percent, at least 80 percent or at least 90 percent of the subjects of a population can be properly identified by the method of the present invention.

The term "stabilization", as used herein, is defined as a <50% decrease or a <25% increase in tumour size.

The term "progression", as used herein, is defined as an increase in the size of tumour lesions by >25% or appearance of new lesions.

Any other parameter which is widely accepted for comparing the efficacy of alternative treatments can be used for determining a response to treatment and include, without limitation:
- disease-free progression which, as used herein, describes the proportion of subjects in complete remission who have had no recurrence of disease during the time period under study.
- disease-free survival (DFS), as used herewith, is understood as the length of time after treatment for a disease during which a subject survives with no sign of the disease.
- objective response which, as used in the present invention, describes the proportion of treated subjects in whom a complete or partial response is observed.
- tumour control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- Time to progression (TTP), as used herein, relates to the time after a disease is treated until the disease starts to get worse. The term "progression" has been previously defined.
- six-month progression free survival or "PFS6" rate which, as used herein, relates to the percentage of subjects wherein free of progression in the first six months after the initiation of the therapy and
- median survival which, as used herein, relates to the time at which half of the subjects enrolled in the study are still alive.

The method of the second aspect of the invention comprises detecting whether the SETDB1 gene is amplified in a sample from said subject.

In a preferred embodiment detecting the amplification of the SETDB1 gene comprises determining the copy number of the SETDB1 gene.

The term "copy number", as used herein, refers to the number of copies of a nucleic acid molecule in a cell. The copy number includes the number of copies of one or more genes or portions thereof in genomic (chromosomal) DNA of a cell. In a normal cell (such as a non- tumour cell), the copy number of a gene (or any genomic DNA) is usually about two (one copy on each member of a chromosome pair). In some examples, the copy number of a gene or nucleic acid molecule includes an average copy number taken from a population of cells.

Methods of determining the copy number of a gene or chromosomal region are well known to those of skill in the art. Said methods include, without limitation, *in situ* hybridisation (such as fluorescent, chromogenic, or silver *in situ* hybridisation), comparative genomic hybridisation, or polymerase chain reaction (such as real-time quantitative PCR). In a particular embodiment, the copy number of the SETDB gene is determined by *in situ* hybridisation (ISH), such as fluorescence *in situ* hybridisation (FISH), chromogenic *in situ* hybridisation (CISH), or silver *in situ* hybridisation (SISH). In a preferred embodiment, the copy number of the SETDB1 gene is determined by FISH.

The term "fluorescent *in situ* hybridisation" or FISH, as used in the present invention, refers to a cytogenetic technique that is used to detect and localize the presence or absence of specific DNA sequences on chromosomes. FISH uses fluorescent probes that bind to only those parts of the chromosome with which they show a high degree of sequence similarity. As the probe for FISH, a DNA fragment, a PCR product, a cDNA, a PAC clone or a BAC clone (each of which has a sequence of interest) may be used. In a typical method using FISH, a DNA probe (a CHKA probe) is labeled with a fluorescent dye or a hapten, typically in the form of fluor-dUTP, digoxigenin-dUTP, biotin-dUTP or hapten-dUTP that is incorporated into the DNA using enzymatic reactions, such as nick translation or PCR. The sample containing the genetic material (chromosome samples) may be smears on slide glass prepared from cultured cells of an isolated cancer. Alternatively, chromosome samples may be slide samples sliced from formalin-fixed, paraffin-embedded cancer-containing tissue blocks. After hardening for prevention of falling off from the slide glass during hybridisation process, chromosome sample slides are denatured by formamide treatment. The labeled probe is then hybridized to the sample containing genetic material on slides under appropriate conditions, which will be determined by the skilled person in the art. After hybridisation, the labeled sample is visualized either directly (in the case of a fluor-labeled probe) or indirectly (using fluorescently labeled antibodies to detect a hapten-labeled probe).

In the case of CISH, the probe is labeled with digoxigenin, biotin, or fluorescein and hybridised to the sample containing genetic material (chromosome or nuclear preparations) under appropriate conditions. The probe is detected with an anti-digoxigenin, -biotin or -fluorescein antibody, which is either conjugated to an enzyme (such as horseradish peroxidase or alkaline phosphatase) that produces a coloured product at the site of the hybridized probe in the presence of an appropriate substrate (such as DAB, NBT/BCIP, etc.), or with a secondary antibody conjugated to the enzyme. SISH is similar to CISH, except that the enzyme (such as horseradish peroxidase) conjugated to the antibody (either anti-hapten antibody or a secondary antibody) catalyzes deposition of metal nanoparticles (such as silver or gold) at the site of the hybridized probe. For any ISH method, SETDB1 gene copy number may be determined by counting the number of fluorescent, coloured, or silver spots on the chromosome or nucleus.

Any label that can be attached to a nucleic acid molecule (such as SETDB1 nucleic acid) can be used to label the SETDB1 probes, thereby permitting detection of the nucleic acid molecule. Examples of labels include, but are not limited to, radioactive isotopes, enzyme substrates, co-factors, ligands, chemiluminescent agents, fluorophores, haptens, enzymes, and combinations thereof. Methods for labelling and guidance in the choice of labels appropriate for various purposes are discussed for example in Sambrook et al. (Molecular Cloning: A Laboratory Manual, Cold Spring Harbor, New York, 1989) and Ausubel et al. (In Current Protocols in Molecular Biology, John Wiley and Sons, New York, 1998).

In a preferred embodiment the determination of the copy number of the SETDB1 gene is carried out by *in situ* hybridisation.

*In situ* hybridisation comprises contacting a sample containing genetic material with a polynucleotide under conditions adequate for the hybridisation of the polynucleotide with the SETDB 1 gene or genes present in the sample and determining the copy number of the SETDB 1 gene based on the hybridisation of the polynucleotide with the sample containing genetic material.

The "conditions adequate for the hybridisation" will be adjusted by a skilled person in the art and will vary depending upon the nature of the hybridisation method and the composition and length of the hybridizing nucleic acid sequences of the composition of the invention. Generally, the temperature of hybridisation and the ionic strength (such as the sodium concentration) of the hybridisation buffer will determine the stringency of hybridisation. Calculations regarding hybridisation conditions for attaining particular degrees of stringency are discussed in Sambrook et al., (1989) Molecular Cloning, second edition, Cold Spring Harbor Laboratory, Plainview, NY (chapters 9 and 11) and in Ausubel et al., Short Protocols in Molecular Biology: A Compendium of Methods from Current Protocols in Molecular Biology, 4th ed., Wiley and Sons, 1999.

The authors have identified a genomic fragment that allows the detection of the amplification of the SETDB1 gene. Thus, in a more preferred embodiment the *in situ* hybridisation is carried out with a polynucleotide comprising the human genomic fragment present in the RP11-42A12 BAC (SEQ ID NO: 1) or a variant thereof which hybridizes under stringent conditions to said polynucleotide.

The BAC is hereinafter referred using the NCBI-recommended clone nomenclature. For instance, the term "RP11-42A12" indicates a clone in BAC library "RP11". More precisely, it is a clone found in microtiter dish "42" at the intersection of row A and column "12".

The term "RP11-42A12" corresponds to a BAC which comprises a region of chromosome 1 having a length of 156757 bp and starting at position 150878829 and ending at position 151035585 according to the sequence of chromosome 1 in the USC Human Genome Browser (GRCh37/hg19 Assembly of Feb. 2009). Its nucleotide sequence is shown in SEQ ID NO: 1.

The term "polynucleotide", as used herein, shall mean any single or double stranded nucleotide polymer of genomic origin or some combination thereof, which by virtue of its origin the isolated polynucleotide (1) is not associated with all or a portion of a polynucleotide in which the isolated polynucleotide is found in nature, (2) is linked to a polynucleotide to which it is not linked in nature, or (3) does not occur in nature as part of a larger sequence.

The term "human genomic fragment", as used in the present invention refers to a specific fragment comprising a gene or a fragment of a gene of human origin or a variant thereof. A person skilled in the art will understand that the present invention relates not only to the genomic fragments mentioned in the present invention, but also to variants thereof. A person skilled in the art will also understand that the variant of the human genomic fragment can be of any mammal, such as simians, mice, rats, pigs, dogs, rabbits, cats, cows, horses and goats.

The term "variant of the human genomic fragment" according to the present invention relates to any polynucleotide in which one or more nucleotides of the polynucleotides according to the invention are deleted, added or substituted. As is known in the state of the art, the identity between two nucleotide sequences is determined by comparing the sequence of a first and a second nucleic acid. Variants according to the present invention include nucleic acid sequences presenting at least 60%, 65%, 70%, 72%, 74%, 76%, 78%, 80%, 90% or 95% similarity or identity with the original nucleotide sequence. The degree of identity between two nucleic acids is determined by using computational algorithms and methods which are widely known by the persons skilled in the art. The identity between two nucleic acid sequences will preferably be determined by using the BLAST algorithm [BLAST Manual, Altschul, S., et al., NCBI NLM NIH Bethesda, Md. 20894, Altschul, S., et al., J. Mol. Biol. 215: 403-410 (1990)].

The variant of the human genomic fragments include fragments which preserve the capacity to hybridize under stringent conditions to the polynucleotides comprising the human genomic fragments present in the BAC as mentioned above.

"Stringent hybridisation" is used herein to refer to conditions under which nucleic acid hybrids are stable. As known to those of skill in the art, the stability of hybrids is reflected in the melting temperature (Tm) of the hybrids. In general, the stability of a hybrid is a function of ionic strength, temperature, G/C content, and the presence of chaotropic agents. The Tm values for polynucleotides can be calculated using known methods for predicting melting temperatures (see, e.g., Baldino et al, Methods Enzymology 168:761-777; Bolton et al, 1962, Proc. Natl. Acad. Sci. USA 48: 1390; Bresslauer et al, 1986, Proc. Natl. Acad. Sci USA 83:8893-8897; Freier et al, 1986, Proc. Natl. Acad. Sci USA 83:9373-9377; Kierzek et al, Biochemistry 25:7840-7846; Rychlik et al, 1990, Nucleic Acids Res 18:6409-6412 (erratum, 1991, Nucleic Acids Res 19:698; Sambmok *et al, ad supra*); Suggs et al, 1981, In Developmental Biology Using Purified Genes (Brown et al, eds.), pp. 683-693, Academic Press; and Wetmur, 1991, Crit Rev Biochem MoI Biol 26:227-259. All publications are incorporated herein by reference). In some embodiments, the polynucleotide encodes the polypeptide disclosed herein and hybridizes under defined conditions, such as moderately stringent or highly stringent conditions, to the complement of a sequence comprising the genomic fragment present in the BAC as defined above.

The term "moderately stringent conditions" as used herein refers to conditions that permit target-DNA to bind a complementary nucleic acid that has about 60 percent identity, preferably about 75 percent identity, about 85 percent identity to the target DNA, with greater than about 90 percent identity to target-polynucleotide. Exemplary moderately stringent conditions are conditions equivalent to hybridisation in 50% formamide, 5X Denhardt's solution, 5X SSPE, 0.2 % SDS at 42°C, followed by washing in 0.2X SSPE, 0.2% SDS, at 42°C.

The term "highly stringent conditions", as used herein refers generally to conditions that are about 10 degrees centigrade or less from the thermal melting temperature Tm as determined under the solution condition for a defined polynucleotide sequence. In some embodiments, a high stringency condition refers to conditions that permit hybridisation of only those nucleic acid sequences that form stable hybrids in 0.018M NaCl at 65°C (i.e., if a hybrid is not stable in 0.018M NaCl at 65°C, it will not be stable under high stringency conditions, as contemplated herein). Exemplary high stringency conditions can be provided, for example, by hybridisation in conditions equivalent to 50% formamide, 5X Denhardt's solution, 5X SSPE, 0.2% SDS at 42°C, followed by washing in 0.1X SSPE, and 0.1% SDS at 65 °C. Another high stringency condition is hybridizing in conditions equivalent to hybridizing in 5X SSC containing 0.1% (w:v) SDS at 65°C degrees centigrade and washing in 0.1X SSC containing 0.1% SDS at 65°C.

Other high stringency hybridisation conditions, as well as moderately stringent conditions, are described in the references cited above.

In a preferred embodiment, the human genomic fragments are found within cloning vehicles in order to allow their propagation. The skilled person will understand that the cloning vehicles are those which can accommodate human genomic fragments having the size of the genomic fragments according to the invention. Thus, preferably, the cloning vehicle is an artificial chromosome. The cloning vehicle can comprise an artificial chromosome comprising a bacterial artificial chromosome (BAC), a bacteriophage P1-derived vector (PAC), a yeast artificial chromosome (YAC), or a mammalian artificial chromosome (MAC). In a preferred embodiment, the human genomic fragments are provided in BACs.

The term "bacterial artificial chromosome" (BAC), as used herein, refers to a DNA construct, based on a functional fertility plasmid (or F-plasmid), which has a low copy number because of the strict control of replication, used for transforming and cloning in bacteria, usually E. *coli.* The BAC's usual insert size is 150-350 kbp in which medium-sized segments of DNA (100,000 to 300,000 bases in length) that come from another species are cloned into bacteria. Once the foreign DNA has been cloned into the bacteria's chromosome, many copies of it can be made (amplified) and sequenced. In addition, BAC libraries of human genomic DNA have more complete and accurate representation of the human genome than libraries in cosmids or yeast artificial chromosomes. BACs are described in further detail in U.S. Application Nos. 10/659,034 and 61/012,701, which are hereby incorporated by reference in their entireties.

The BACs are commercially available, for example, the RP11 BAC library is available from the BAC Resources PAC (Children's Hospital Oakland Research Institute), from Empire Genomics, imaGenes or Invitrogen.

In another example, SETDB 1 gene copy number is determined by comparative genomic hybridisation (CGH). See, e.g., Kallioniemi et al, Science 258:818-821, 1992; U.S. Pat. Nos. 5,665,549 and 5,721,098. In one example, CGH includes the following steps. DNA from tumour tissue (such as a lung cancer sample) and from normal control tissue (reference, such as a non-tumour sample) is labeled with different detectable labels, such as two different fluorophores. After mixing tumour and reference DNA along with unlabeled human Cot-1 DNA (placental DNA that is enriched for repetitive DNA sequences such as the Alu and Kpn family) to suppress repetitive DNA sequences, the mix is hybridized to normal metaphase chromosomes. The fluorescence intensity ratio along the chromosomes is used to evaluate regions of DNA gain or loss in the tumour sample. In a further example, SETDB1 gene copy number is determined by array CGH (aCGH). See, e.g., Pinkel and Albertson, Nat. Genet. 37:S11-S17, 2005; Pinkel et al, Nat. Genet. 20:207-211, 1998; Pollack et al, Nat. Genet. 23:41-46, 1999. Similar to standard CGH, tumour and reference DNA are differentially labeled and mixed. However, for aCGH, the DNA mixture is hybridized to a slide containing hundreds or thousands of defined DNA probes (such as probes that are homologous to portions of the SETDB1 gene). The fluorescence intensity ratio at each probe in the array is used to evaluate regions of DNA gain or loss in the tumour sample, which can be mapped in finer detail than CGH, based on the particular probes which exhibit altered fluorescence intensity. In one example, the array is an Agilent Human Genome CGH 44B Oligo Microarray (Agilent Technologies, Santa Clara, CA). In another example, the CGH array is a Whole Genome Tiling, Custom, or Chromosome specific Tiling Array (for example, a Chromosome 15 Tiling Array) as provided by Roche NimbleGen, Inc. (Madison, WI).

In general, CGH (and aCGH) does not provide information as to the exact number of copies of a particular genomic DNA or chromosomal region. Instead, CGH provides information on the relative copy number of one sample (such as a tumour sample, for example a lung cancer sample) compared to another (such as a control sample, for example a non-tumour cell or tissue sample). Thus, CGH is most useful to determine whether SETDB1 gene copy number of a sample is increased or decreased as compared to a control sample (such as a non-tumour cell or tissue sample or a reference value).

In another preferred embodiment the determination of the copy number of the SETDB1 gene is carried out by quantitative genomic PCR.

Additional methods that may be used to determine copy number of the SETDB1 gene are known to those of skill in the art. Such methods include, but are not limited to Southern blotting, multiplex ligation-dependent probe amplification (MLPA; see, e.g., Schouten et al., Nucl. Acids Res. 30:e57, 2002), and high-density SNP genotyping arrays (see, e.g. WO 98/030883).

Also disclosed herein is a method of scoring (for example, enumerating) copy number of a gene in a sample from a subject (such as a subject with neoplastic disease), wherein the sample is stained by ISH (such as FISH, SISH, CISH, or a combination of two or more thereof) for the gene of interest and wherein individual copies of the gene are distinguishable in cells in the sample. Typically, the method includes identifying individual cells in a sample with the highest number of signals per nucleus for the gene (such as the strongest signal in the sample), counting the number of signals for the gene in the identified cells, and determining an average number of signals per cell, thereby scoring the gene copy number in the sample. In additional embodiments, the method further includes counting the number of signals for a reference (such as a chromosomal locus known not to be abnormal, for example, centromeric DNA) and determining an average ratio of the number of signals for the gene to the number of signals for the reference per cell.

The scoring method includes identifying individual cells in the sample (such as a tissue section or tumour core) having the highest number of signals (such as the highest number of spots per cell or the brightest intensity of staining) for the gene of interest in the cells in the sample. Thus, the disclosed method does not determine gene copy number in a random sampling of cells in the sample. Rather, the method includes specifically counting gene copy number in those cells that have the highest gene copy number in the sample. In some examples, identifying the individual cells having the highest number of signals for the gene includes examining a sample stained by ISH for the gene under low power microscopy (such as about 20X magnification). Cells with the strongest signal (for example, highest amplification signal under higher power) are identified for counting by eye or by an automated imaging system. In some examples, such as when the sample is a tissue section, the sample is examined (for example, visually scanned) to identify a region that has a concentration of tumour cells that has amplification of the gene. Gene copy number in the cells with highest amplification in the selected region is then counted. In other examples, such as when the sample is a tumour core (such as a tumour microarray), most of the sample is visible in the field of view under low power magnification and the individual cells (such as tumour cells) with the strongest signal (for example, highest amplification signal under high power) are separately identified for counting. In particular examples, the cells chosen for counting the gene copy number may be non-consecutive cells, such as cells that are not adjacent to or in contact with one another. In other examples, at least some of the cells chosen for counting the gene copy number may be consecutive cells, such as cells that are adjacent to or in contact with one another.

The disclosed methods include counting the number of ISH signals (such as fluorescent, coloured, or silver spots) for the gene in the identified cells. The methods may also include counting the number of ISH signals (such as fluorescent, coloured or silver spots) for a reference (such as a chromosome-specific probe) in the identified cells. In some examples, the number of spots per cells is distinguishable in the identified cells and the number of spots are counted (or enumerated) and recorded. In other examples, one or more of the identified cells may include a cluster, which is the presence of multiple overlapping signals in a nucleus that cannot be counted (or enumerated). In particular examples, the number of copies of the gene (or chromosome) may be estimated by the person (or computer, in the case of an automated method) scoring the slide. For example, one of skill in the art of pathology may estimate that a cluster contains a particular number of copies of a gene (such as 10, 20, or more copies) based on experience in enumerating gene copy number in a sample. In other examples, the presence of a cluster may be noted as a cluster, without estimating the number of copies present in the cluster.

The number of cells identified for counting is a sufficient number of cells that provides for detecting a change (such as an increase or decrease) in gene copy number. In some examples, the number of cells identified for counting is at least about 20, for example, at least 25, 30, 40, 50, 75, 100, 200, 500, 1000 cells, or more. In a particular example, about 50 cells are counted. In other examples, every cell in the sample or every cell in a microscope field of vision, or in a number of microscope fields (such as at least 2 microscope fields, at least 3, at least 4, at least 5, at least 6 microscope fields, and the like) which contains 3 or more copies of the gene of interest (such as 3, 4, 5, 6, 7, 8, 9, 10, 15, 20, or more) is counted. In some examples, the biological sample is a tumour sample, such as a tumour which potentially includes a gene amplification. Exemplary biological samples include neoplastic cells or tissues, which may be isolated from solid tumours, including lung cancer (e.g., non-small cell lung cancer, such as lung squamous cell carcinoma).

The inventors have found that lung cancer cells having SETDB1 gene amplification have eighteen genes repressed. The inventors have validated the genes that appear in Figures 4C and 4D. Therefore, in another preferred embodiment, detecting the amplification of the SETDB1 gene comprises detecting decreased expression levels of one or more genes selected from the group consisting of ADRB2, ANXA3, DNER, FGFR2, GPR64, IGFBP7, IL6, NAV3, SGK1 and TFAP2A with respect to a reference value.

The expression "expression levels" or its grammatical equivalent, as used herein, means a measurement of the amount of nucleic acid, e.g. RNA or mRNA, or protein of a gene in a cell, or alternatively, the level of activity of a gene or protein in said cell.

As the person skilled in the art understands, the expression levels of said genes can be measured by determining the mRNA expression levels of said genes or by determining the protein levels encoded by said genes.

In order to measure the mRNA levels of said genes, the cell may be treated to physically, mechanically or chemically disrupt cell structures, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

Determination of the levels of mRNA of said genes can be carried out by any method known in the art such as qPCR, northern blot, RNA dot blot, TaqMan®, tag based methods such as serial analysis of gene expression (SAGE) including variants such as LongSAGE and SuperSAGE, microarrays, nucleic acid sequence based amplification (NASBA), Fluorescence In Situ Hybridization, including variants such as Flow-FISH, qFiSH and double fusion fish (D-FISH) as described in WO2010030818, Femino et al. (Science, 1998, 280:585-590), Levsky et al. (Science, 2002, 297:836-840) or Raj et al. (PLoS Biology, 2006, 4:e309).

In a particular embodiment de expression levels of said genes are determined by a microarray. In another particular embodiment the expression levels of said genes are determined by quantitative PCR, preferably Real-Time PCR.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts cells. Preferably, the control RNA is mRNA derived from housekeeping genes and which code for proteins which are constitutively expressed and carry out essential cellular functions. Examples of housekeeping genes for use in the present invention include β-2-microglobulin, ubiquitin, 18-S ribosomal protein, cyclophilin, GAPDH, PSMB4, tubulin and β-actin.

In one embodiment relative gene expression quantification is calculated according to the comparative Ct method using GAPDH, β-actin or PSMB4 as an endogenous control and commercial RNA controls as calibrators. Final results are determined according to the formula 2-(ΔCt sample-ΔCt calibrator), where ΔCT values of the calibrator and sample are determined by substracting the CT value of the target gene from the value of the control gene.

The expression levels of said genes may also be measured by determining the protein levels or variants thereof encoded by said genes. Practically any conventional method can be used within the context of the present invention to quantify the levels of said proteins such as Western blot or immunohistochemistry.

As previously mentioned, variants of said proteins can be used for measuring the expression levels of ADRB2, ANXA3, DNER, FGFR2, GPR64, IGFBP7, IL6, NAV3, SGK1 or TFAP2A genes in order to put into practice the method of the invention.

Thus, variants of the ADRB2, ANXA3, DNER, FGFR2, GPR64, IGFBP7, IL6, NAV3, SGK1 or TFAP2A proteins may be: (i) one in which one or more of the amino acid residues are substituted with a conserved or non-conserved amino acid residue (preferably a conserved amino acid residue) and such substituted amino acid residue may or may not be one encoded by the genetic code; (ii) one in which there are one or more modified amino acid residues, e.g., residues that are modified by the attachment of substituent groups; (iii) one in which the protein is an alternative splice variant of the proteins of the present invention and/or; (iv) fragments of the proteins. The fragments include proteins generated via proteolytic cleavage (including multi-site proteolysis) of an original sequence. Variants are deemed to be within the scope of those skilled in the art from the teaching herein.

Variants according to the present invention include amino acid sequences that are at least 60%, 70%, 80%, 90%, 95% or 96% similar or identical to the original amino acid sequence. As known in the art the "similarity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Additionally, the proteins may include unnatural amino acids formed by post-translational modification or by introducing unnatural amino acids during translation.

In a particular embodiment said variant is a mammal variant, preferably a human variant, more preferably with at least 60%, 70%, 80%, 90%, 95% or 96% similarity or identity to the original amino acid sequence.

The ADRB2 gene, as used herein, refers to the gene which codes for the protein adrenergic, beta-2-, receptor surface; also named adrenoceptor beta 2, surface. The human gene (UniGene Hs. 2551) is defined in the NCBI database under accession number NC_000005.9 (positions 148206156 to 148208197 in the genomic region), version October 30, 2012.

The ANXA3 gene, as used herein, refers to the gene which codes for the protein annexin A3. The human gene (UniGene Hs.480042) is defined in the NCBI database under accession number NC_000004.11 (positions 79472673 to 79531605 in the genomic region), version October 30, 2012.

The DNER gene, as used herein, refers to the gene which codes for the protein delta/notch-like EGF repeat containing. The human gene (UniGene Hs.234074) is defined in the NCBI database under accession number NC-000002.11 (positions 230222345 to 230579286 in the genomic region), version October 30, 2012.

The FGFR2 gene, as used herein, refers to the gene which codes for the protein fibroblast growth factor receptor 2. The human gene (UniGene Hs.533683) is defined in the NCBI database under accession number NC_000010.10 (positions 123237844 to 123357972 in the genomic region), version October 30, 2012.

The GPR64 gene, as used herein, refers to the gene which codes for the protein G protein-coupled receptor 64. The human gene (UniGene Hs.146978) is defined in the NCBI database under accession number NC-000023.10 (positions 19007425 to 19140755 in the genomic region), version October 30, 2012.

The IGFBP7 gene, as used herein, refers to the gene which codes for the protein insulin-like growth factor binding protein 7. The human gene (UniGene Hs.479808) is defined in the NCBI database under accession number NC-000004.11 (positions 57896939 to 57976551 in the genomic region), version October 30, 2012.

The IL6 gene, as used herein, refers to the gene which codes for the protein interleukin 6. The human gene (UniGene Hs.654458) is defined in the NCBI database under accession number NC_000007.13 (positions 22765503 to 22771621 in the genomic region), version October 30, 2012.

The NAV3 gene, as used herein, refers to the gene which codes for the protein neuron navigator 3. The human gene (UniGene Hs.655301) is defined in the NCBI database under accession number NC_000012.11 (positions 78224685 to 78606790 in the genomic region), version October 30, 2012.

The SGK1 gene, as used herein, refers to the gene which codes for the protein serum/glucocorticoid regulated kinase 1. The human gene (UniGene Hs.510078) is defined in the NCBI database under accession number NC_000006.11 (positions 134490384 to 134639250 in the genomic region), version October 30, 2012.

The TFAP2A gene, as used herein, refers to the gene which codes for the protein transcription factor AP-2 alpha (activating enhancer binding protein 2 alpha). The human gene (UniGene Hs.519880) is defined in the NCBI database under accession number NC_000006.11 (positions 10393419 to 10419892 in the genomic region), version October 30, 2012.

The value obtained for the expression of ADRB2, ANXA3, DNER, FGFR2, GPR64, IGFBP7, IL6, NAV3, SGK1 and/or TFAP2A nucleic acid or protein levels in a sample from the subject suffering from lung cancer is then compared with a reference value.

This comparison allows detecting a decrease in the expression of one or more of said genes with respect to the reference value. The expression "decreased expression levels" of one or more genes selected from the group consisting of ADRB2, ANXA3, DNER, FGFR2, GPR64, IGFBP7, IL6, NAV3, SGK1 and TFAP2A means a decrease in the expression of a given gene with respect to the reference value of at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%.

The reference value can be determined by techniques well known in the state of the art, for example, determining the median value of expression levels of each of ADRB2, ANXA3, DNER, FGFR2, GPR64, IGFBP7, IL6, NAV3, SGK1 or TFAP2A genes measured in a collection of tumour tissue in biopsy samples from subjects suffering from lung cancer who do not have amplification of SETDB1 gene or from normal tissue. In a preferred embodiment the expression levels of ADRB2, ANXA3, DNER, FGFR2, GPR64, IGFBP7, IL6, NAV3, SGK1 or TFAP2A genes are measured in a collection of lung tumour tissue in biopsy samples from subjects suffering from lung cancer who do not have amplification of the SETDB gene or from normal lung tissue. Once this median value is established, the level of this marker expressed in tumour tissues from the subject can be compared with this median value, and thus be assigned a level of "decreased" (low) or "increased" (high) expression level. The collection of samples from which the reference level is derived will preferably be constituted from subjects suffering from the same type of lung cancer or a mixture of lung tissues from normal individuals not affected of lung cancer. Alternatively, the use of a reference value used for determining whether the expression level of a gene is "increased" or "decreased" could correspond to the median value of expression levels of each gene measured in a RNA sample obtained by pooling equal amounts of RNA from each of the tumour samples obtained by biopsy from subjects suffering from lung cancer who do not have amplification of the SETDB1 gene, preferably from subjects suffering from the same type of lung cancer. Once this median value is established, the level of this marker expressed in tumour tissues from subjects can be compared with this median value, and thus be assigned a level of "increased" or "decreased". For example, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

In the present invention, the "reference value" may be an arbitrary cut-off point, established according to ROC methodology. Once this cut-off point is established, the level of this marker expressed in tumour tissues from the subject can be compared with this cut-off point, and thus be assigned a level of "low" expression if it is under this cut-off.

Once the level of the amplification of the SETDB1 gene has been determined (for example, when the SETDB1 gene copy number has been determined or when a comparison between the expression levels of one or more of ADRB2, ANXA3, DNER, FGFR2, GPR64, IGFBP7, IL6, NAV3, SGK1 or TFAP2A genes and the reference value has been made), the method of the invention allows making a prediction as to whether the subject will show a good clinical response to a treatment with a SETDB1 antagonist, preferably to a SETDB1-specific RNAi or to mithramycin. Thus, patients having amplification of the SETDB1 gene will have a good clinical response to the treatment with said antagonist.

The expression "good clinical response", as used herein, means that the subject will show a favourable response to the treatment with a SETDB 1 antagonist. As will be understood by those skilled in the art, such the assessment of the probability, although preferred to be, may usually not be correct for 100% of the subjects to be analyzed. The term, however, requires that a statistically significant portion of subjects can be identified as having a predisposition of responding to the chemotherapeutic treatment. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%.. The p- values are, preferably, 0.1, 0.05, 0.01, 0.005, or 0.0001. More preferably, at least 60 percent, at least 70 percent, at least 80 percent or at least 90 percent of the subjects of a population can be properly identified by the method of the present invention.

In some examples, an increased SETDB gene copy number includes SETDB1 gene copy number per nucleus (such as average SETDB1 gene copy per nucleus) in the sample of greater than about two copies of the gene per nucleus (such as greater than 2, 3, 4, 5, 10 or 20 copies). In other examples, an increased SETDB1 gene copy number includes a ratio of SETDB1 gene copy number to Chromosome 1 copy number (such as an average SETDB1 gene: Chromosome 1 ratio) in the sample of greater than about 2 (such as a ratio of greater than 2, 3, 4, 5, 10, or 20). In further examples, an increased SETDB 1 copy number includes an increase in SETDB 1 gene copy number relative to a control (such as an increase of about 1.5-fold, about 2-fold, about 3-fold, about 5-fold, about 10-fold, about 20-fold, or more).

In other examples, both the SETDB1 gene and Chromosome 1 DNA (such as Chromosome 1 centromeric DNA) are detected in a sample from the subject, for example by ISH. Chromosome 1-specific probes are well known in the art and include commercially available probes. The SETDB1 gene and Chromosome 1 DNA may be detected on the same sample (for example, on a single slide or tissue section, such as a dual colour assay) or in different samples from the same subject (for example, SETDB1 gene is detected on one slide and Chromosome 1 DNA is detected on a matched slide from the same subject, such as a single colour assay). The SETDB1 gene and Chromosome 1 DNA are detected with two different detectable labels for dual colour assay (such as two different fluorophores, two different chromogens, or a chromogen and metal nanoparticles). The SETDB1 gene and Chromosome 1 DNA may be detected with the same label for single colour assay. The SETDB1 gene and Chromosome 1 DNA copy number may be determined by counting the number of fluorescent, coloured, or silver spots on the chromosome or nucleus. A ratio of SETDB1 gene copy number and Chromosome 1 DNA number is then determined.

Thus, in a particular embodiment, if the sample of the subject which has been amplified has more than 2 copies of the SETDB1 gene, it is indicative of a good clinical response of the subject to the treatment with a SETDB1 antagonist. In a particular embodiment, if the sample of the subject which has been amplified has more than 6 copies of the SETDB1 gene, it is indicative of a good clinical response of the subject to the treatment with a SETDB1 antagonist.

The skilled person will appreciate that the particular embodiments developed in the first aspect of the invention are also applicable to the second aspect of the invention. Therefore, in a preferred embodiment the method predicts the clinical response of a subject suffering from lung cancer to a treatment with a SETDB1 antagonist, wherein the SETDB1 antagonist is a SETDB1-specific RNAi. In another preferred embodiment the SETDB1 antagonist is mithramycin or a pharmaceutically acceptable salt, solvate, polymorph or cocrystal thereof. In another preferred embodiment the subject is a mammal, preferably a human being. In another preferred embodiment the sample is a lung tumour tissue sample.

The terms "subject", "lung cancer", "treatment", "SETDB1 antagonist", "mithramycin", "pharmaceutically acceptable salt", "solvate", "polymorph", "cocrystal", "SETDB1-specific RNAi", "SETDB1 gene" and "sample" have been described in detail above in the context of the first aspect of the invention and are used with the same meaning in the context of the second aspect of the invention.

### SELECTIVE METHODS OF THE INVENTION

The authors of the present invention have observed that amplification of the SETDB1 gene is indicative of a good response to a SETDB1-specific shRNA and to mithramycin in SETDB1-amplified lung cancer cell lines. Therefore, the detection of the SETDB1 gene amplification can be a marker useful for selecting a patient for being treated with a SETDB1 antagonist. In this way, subjects can proceed directly to adequate therapies while avoiding ineffective therapies and secondary effects associated to them.

Thus, in a third aspect, the invention relates to an *in vitro* method for selecting a lung cancer patient for being treated with a SETDB1 antagonist comprising detecting whether the SETDB1 gene is amplified in a sample from said patient, wherein amplification of the SETDB1 gene is indicative that the subject is a candidate for a treatment with a SETDB1 antagonist.

All the embodiments of the second aspect of the invention are also applicable to the third aspect of the invention. Therefore, in a preferred embodiment the method allows selecting a lung cancer patient for being treated with a SETDB1 antagonist, wherein the SETDB1 antagonist is a SETDB1-specific RNAi. In another preferred embodiment the SETDB1 antagonist is mithramycin or a pharmaceutically acceptable salt, solvate, polymorph or cocrystal thereof. In another preferred embodiment detecting the amplification of the SETDB1 gene comprises determining the copy number of the SETDB1 gene, preferably more than 2 copies, or detecting decreased expression levels of one or more genes selected from the group consisting of ADRB2, ANXA3, DNER, FGFR2, GPR64, IGFBP7, IL6, NAV3, SGK1 and TFAP2A with respect to a reference value. In another preferred embodiment the patient is a mammal, preferably a human being. In another preferred embodiment the determination of the copy number of the SETDB1 gene is carried out by *in situ* hybridisation, preferably with a polynucleotide comprising the human genomic fragment present in the RP11-42A12 BAC (SEQ ID NO: 1) or a variant thereof which hybridizes under stringent conditions to said polynucleotide. In another preferred embodiment the determination of the copy number of the SETDB gene is carried out by quantitative genomic PCR. In another preferred embodiment the sample is a lung tumour tissue sample.

All the terms have been defined previously in the context of the first and second aspects of the invention and are used with the same meaning in the context of the third aspect of the invention.

### KITS OF THE INVENTION AND USES THEREOF

The inventors of the present invention have found that the copy number of the SETDB1 gene can be determined by FISH (fluorescent *in situ* hybridization) using a specific polynucleotide, namely, RP11-42A12.

In another aspect, the invention relates to the use of a reagent capable of detecting the amplification of SETDB1 gene in a sample from a subject suffering from lung cancer for predicting the clinical response of said subject to a treatment with a SETDB1 antagonist.

In another aspect, the invention relates to the use of a reagent capable of detecting the amplification of SETDB1 gene in a sample from a subject suffering from lung cancer for selecting a lung cancer patient for being treated with a SETDB1 antagonist.

The term "reagent", as used herein, refers to any compound or composition which can be used for detecting the amplification of SETDB1 gene (for example any reagent capable of detecting the copy number of the SETDB1 gene or any reagent capable of detecting decreased expression levels of one or more genes selected from the group consisting of ADRB2, ANXA3, DNER, FGFR2, GPR64, IGFBP7, IL6, NAV3, SGK1 and TFAP2A). In a preferred embodiment the reagent is a reagent for detecting the copy number of the SETDB1 gene. In another embodiment the reagent is a reagent for detecting the expression levels of one or more genes selected from the group consisting of ADRB2, ANXA3, DNER, FGFR2, GPR64, IGFBP7, IL6, NAV3, SGK1 and TFAP2A, i.e. for detecting their genes, proteins or variants thereof. The reagent can include, optionally, reagents for detecting one or more housekeeping genes or the protein encoded by said housekeeping gene(s). This set of reagents can include, without limitation, nucleic acids capable of specifically hybridising with the SETDB1 gene and/or antibodies or fragments thereof capable of specifically binding to SETDB1 protein or to variants thereof (including fragments thereof containing antigenic determinants).

The reagents for use in the method of the invention may be formulated as a "kit" and thus, may be combined with one or more other types of elements or components (e.g., other types of biochemical reagents, containers, packages such as packaging intended for commercial sale, substrates to which the reagents are attached, electronic hardware components, etc.).

In a preferred embodiment, the reagents for determining the copy number of SETDB1 gene are probes for the identification of this gene, particularly a polynucleotide comprising the human genomic fragment present in the RP11-42A12 (SEQ ID NO: 1) or a variant thereof which hybridizes under stringent conditions to said polynucleotide.

In another embodiment the reagents for determining the amplification of SETDB1 gene are primers and/or antibodies.

Nucleic acids capable of specifically hybridizing with the SETDB1 gene are, for example, one or more pairs of primer oligonucleotides for the specific amplification of fragments of the mRNA (or of their corresponding cDNA) of said gene.

Nucleic acids capable of specifically hybridizing with one or more genes selected from the group consisting of ADRB2, ANXA3, DNER, FGFR2, GPR64, IGFBP7, IL6, NAV3, SGK1 and TFAP2A can also be included.

As the skilled person understands, the oligonucleotide primers and probes of the kit of the invention can be used in all techniques of gene expression profiling (RT-PCR, SAGE, TaqMan, Real Time-PCR, FISH, NASBA, etc).

Antibodies, or a fragment thereof, capable of detecting an antigen, capable of specifically binding to a protein or to variants thereof are, for example, monoclonal and polyclonal antibodies, antibody fragments, Fv, Fab, Fab' and F(ab')2, ScFv, diabodies, triabodies, tetrabodies and humanised antibodies. The antibodies of the kit of the invention can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc.

Said reagents, specifically the probes and the antibodies, may be fixed onto a solid support, such as a membrane, a plastic or a glass, optionally treated in order to facilitate fixation of said probes or antibodies onto the support.

The kits of the invention optionally comprise additional reagents for detecting a housekeeping gene, a polypeptide encoded by a housekeeping gene or the mRNA encoded by said housekeeping gene. The availability of said additional reagent allows the normalization of measurements taken in different samples (e.g. the test sample and the control sample) to exclude that the differences in expression of the biomarker are due to a different amount of total protein in the sample rather than to real differences in relative expression levels. Housekeeping genes, as used herein, relates to genes which code for proteins which are constitutively expressed and carry out essential cellular functions. Preferred housekeeping genes for use in the present invention include β-2-microglobulin, ubiquitin, 18-S ribosomal protein, cyclophilin, PSMB4, GAPDH, tubulin and β-actin.

All the particular embodiments disclosed for the methods of the present invention are applicable to the kit of the invention and uses thereof.

Particularly, in a preferred embodiment the SETDB1 antagonist is selected from the group consisting of: (i) a SETDB1-specific RNAi, and (ii) mithramycin or a pharmaceutically acceptable salt, solvate, polymorph or cocrystal thereof. In another preferred embodiment the subject is a mammal, preferably a human being. In another preferred embodiment the sample is a lung tumour tissue sample.

The present invention is also directed to:
[1]. A SETDB1 antagonist for use in the treatment of lung cancer in a subject having SETDB1 gene amplification.
[2]. A SETDB1 antagonist for use according to [1] wherein the antagonist is selected from the group consisting of:
   (i) a SETDB1-specific RNAi, and
   (ii) mithramycin or a pharmaceutically acceptable salt, solvate, polymorph or cocrystal thereof.
[3]. A SETDB1 antagonist for use according to [1] or [2] wherein the subject is a mammal.
[4]. A SETDB1 antagonist for use according to [3] wherein the mammal is a human being.
[5]. A SETDB1 antagonist for use according to [1], [2], [3] or [4] wherein the subject has SETDB1 gene amplification in a lung tumour tissue sample.
[6]. An *in vitro* method for predicting the clinical response of a subject suffering from lung cancer to a treatment with a SETDB1 antagonist comprising detecting whether the SETDB1 gene is amplified in a sample from said subject, wherein amplification of the SETDB1 gene is indicative of a good clinical response of the subject to the treatment with said antagonist.
[7]. The method according to [6] wherein the SETDB1 antagonist is selected from the group consisting of:
   (i) a SETDB1-specific RNAi, and
   (ii) mithramycin or a pharmaceutically acceptable salt, solvate, polymorph or cocrystal thereof.
[8]. The method according to [6] or [7] wherein detecting the amplification of the SETDB1 gene comprises determining the copy number of the SETDB1 gene or detecting decreased expression levels of one or more genes selected from the group consisting of ADRB2, ANXA3, DNER, FGFR2, GPR64, IGFBP7, IL6, NAV3, SGK1 and TFAP2A with respect to a reference value.
[9]. The method according to [8] wherein more than 2 copies of the SETDB1 gene are detected.
[10]. The method according to [6], [7], [8] or [9] wherein the subject is a mammal.
[11]. The method according to [10] wherein the mammal is a human being.
[12]. The method according to [8], [9], [10] or [11] wherein the determination of the copy number of the SETDB1 gene is carried out by *in situ* hybridisation.
[13]. The method according to [12] wherein the *in situ* hybridisation is carried out with a polynucleotide comprising the human genomic fragment present in the RP11-42A12 BAC (SEQ ID NO: 1) or a variant thereof which hybridizes under stringent conditions to said polynucleotide.
[14]. The method according to [8], [9], [10] or [11] wherein the determination of the copy number of the SETDB gene is carried out by quantitative genomic PCR.
[15]. The method according to [6], [7], [8], [9], [10], [11], [12], [13] or [14] wherein the sample is a lung tumour tissue sample.
[16]. An *in vitro* method for selecting a lung cancer patient for being treated with a SETDB1 antagonist comprising detecting whether the SETDB1 gene is amplified in a sample from said patient, wherein amplification of the SETDB1 gene is indicative that the subject is a candidate for a treatment with a SETDB1 1 antagonist.
[17]. The method according to [16] wherein the SETDB1 antagonist is selected from the group consisting of:
   (i) a SETDB1-specific RNAi, and
   (ii) mithramycin or a pharmaceutically acceptable salt, solvate, polymorph or cocrystal thereof.
[18]. The method according to [16] or [17] wherein detecting the amplification of the SETDB1 gene comprises determining the copy number of the SETDB1 gene or detecting decreased expression levels of one or more genes selected from the group consisting of ADRB2, ANXA3, DNER, FGFR2, GPR64, IGFBP7, IL6, NAV3, SGK1 and TFAP2A with respect to a reference value.
[19]. The method according to [18] wherein more than 2 copies of the SETDB1 gene are detected.
[20]. The method according to [16], [17], [18] or [19] wherein the patient is a mammal.
[21]. The method according to [20] wherein the mammal is a human being.
[22]. The method according to [18], [19], [20] or [21] wherein the determination of the copy number of the SETDB gene is carried out by *in situ* hybridisation.
[23]. The method according to [22] wherein the *in situ* hybridisation is carried out with a polynucleotide comprising the human genomic fragment present in the RP11-42A12 BAC (SEQ ID NO: 1) or a variant thereof which hybridizes under stringent conditions to said polynucleotide.
[24]. The method according to [18], [19], [20] or [21] wherein the determination of the copy number of the SETDB gene is carried out by quantitative genomic PCR.
[25]. The method according to [16], [17], [18], [19], [20], [21], [22], [23] or [24] wherein the sample is a lung tumour tissue sample.
[26]. Use of a reagent capable of detecting the amplification of SETDB1 gene in a sample from a subject suffering from lung cancer for predicting the clinical response of said subject to a treatment with a SETDB1 antagonist or for selecting a lung cancer patient for being treated with a SETDB 1 antagonist.
[27]. The use according to [26] wherein the SETDB1 antagonist is selected from the group consisting of:
   (i) a SETDB1-specific RNAi, and
   (ii) mithramycin or a pharmaceutically acceptable salt, solvate, polymorph or cocrystal thereof.
[28]. The use according to [26] or [27] wherein the subject is a mammal.
[29]. The use according to [28] wherein the mammal is a human being.
[30]. The use according to [26], [27], [28] or [29] wherein the reagent capable of detecting the amplification of SETDB1 gene is a polynucleotide comprising the human genomic fragment present in the RP11-42A12 BAC (SEQ ID NO: 1) or a variant thereof which hybridizes under stringent conditions to said polynucleotide.
[31]. The use according to [26], [27], [28], [29] or [30] wherein the sample is a lung tumour tissue sample.

The following example is provided as merely illustrative and is not to be construed as limiting the scope of the invention.

### EXAMPLE

The inventors first screened a collection of 15 human lung cancer cell lines for SETDB1 gene copy number alterations. These included seven non-small (A549, NCI-H1299, NCI-H1975, NCI-H1993, NCI-H2170, NCI-H1437 and NCIH1395) and eight small (HCC-33, N417, NCI-H446, NCI-H1048, NCI-H1963, NCI-H2029, DMS-114 and DMS-273) cell lung cancer types. The lung cancer cell lines were purchased from the American Type Culture Collection (ATCC) and were grown and maintained in 10% FBS in RPMI medium. Primary normal tissues such as lung epithelium and leukocytes were used as normal SETDB1 copy number control samples. Using a quantitative genomic PCR approach, the inventors observed that two non-small (NCI-H1437 and NCI-H1395) and one small (DMS-273) cell lung cancer lines had a greater than fourfold change in SETDB1 gene copy number **(****Figure 1A****).** This increase was particularly important in the small lung cancer cell line DMS-273 **(****Figure 1A****).** The remaining eleven lung cancer cell lines did not present any evident change in SETDB 1 gene copy number (i.e., amplification, half gene dosage or homozygous deletion). Fluorescence *in situ* hybridization (FISH) analyses were performed in order to confirm the presence of SETDB1 gene amplification suggested to occur in the three lung cancer cell lines by the competitive genomic PCR approach. The UCSC genome browser (http://genome.ucsc.edu) was used to select the BAC clone spanning the 1q21 region for the SETDB1 gene: RP11-42A12. A telomeric BAC clone located in the telomeric 1p36.23 region was used as a control. The BACs were obtained from the BACPAC Resource Center at the Children's Hospital Oakland Research Institute (Oakland, CA). SETDB1 and telomeric probes were labeled with Spectrum Green and Red dUTP (Abbott), respectively, using a CGH Nick Translation Reagent Kit (Abbott Molecular Inc., Des Plaines, IL). The samples were counterstained with DAPI in Vectashield antifade solution. The FISH technique confirmed the presence of SETDB1 gene amplification in NCI-H1437, NCI-H1395 and DMS-273 (data not shown). The applied FISH technique was validated by the observation of the normal copy number of the SETDB1 gene in lymphocytes and probes were verified to give a single signal on normal commercial lymphocyte metaphase slides (CGH Reagents, Abbott, Wiesbaden, Germany). Using reported Copy Number Variation (CNV) data, the inventors have determined the size of the amplicon that contains SETDB1 in the three studied lung cancer cell lines: DMS-273 (1,038,210 bp), NCI-H1437 (4,539,342 bp) and NCI-H1395 (4,623,419 bp). SETDB1 is genomically located in the middle of all three amplicons, even in the case of the smallest one (DMS-273). It was calculated that NCI-H1437 and NCI-H1395 have 7 copies of SETDB1 gene and DMS-273 has 17 copies of SETDB1 gene.

The inventors next considered the possible existence of an association between extra copies of the SETDB1 gene and overexpression of the corresponding RNA transcript and protein using quantitative reverse transcription-PCR (qRTPCR) and western blot approaches, respectively **(****Figure 1B** **and** **1C****).** They found that the expression of SETDB1 for both mRNA and protein was enhanced in cancer cell lines harboring the SETDB1 gene amplification event relative to non-amplified cancer cells **(****Figure 1B** **and** **1C****).** In the smallest SETDB1 amplicon (present in DMS-273 cells), thirty-four genes are also co-amplified (data not shown). Using reported microarray expression data, the inventors have observed that, in addition to SETDB1, only 7 of these other 34 genes (21%) are overexpressed in DMS-273, NCI-H1437 and NCI-H1395 in comparison to unamplified lung cancer cells (NCI-H1299 and A549) (data not shown). Said seven genes are TARS2, GOLPH3L, ARNT, C1Orf56, GABPB2, LYSMD1 and SELENBP1. In this regard, the inventors have confirmed by qRT-PCR that six of these seven genes are overexpressed in the SETDB 1 amplified lung cell lines (data not shown).

Once the presence of SETDB1 gene amplification and its associated overexpression in the lung cancer cell lines were demonstrated, the inventors examined its contribution to the tumorigenic phenotype *in vitro* and *in vivo.* They first analyzed the effect of SETDB1 depletion in lung cancer cells harboring its gene amplification and its associated overexpression. **Table 1** includes all the used short hairpin RNA (shRNA) sequences.

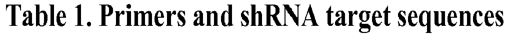

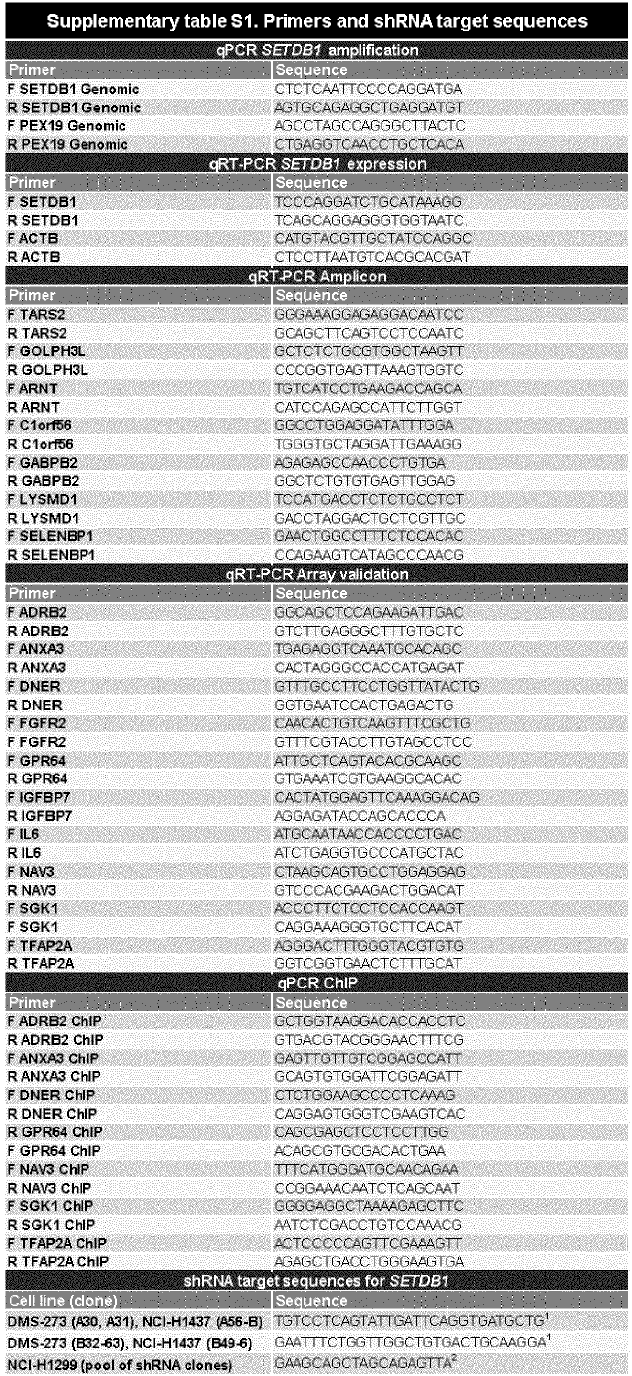

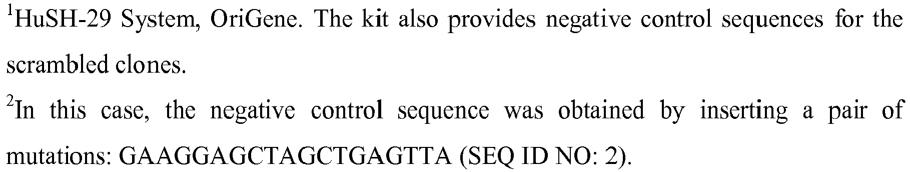

Three SETDB1 shRNA depleted clones were established for DMS-273 cells (A30, A31 and B32-63) and two clones for NCI-H1437 (A56-B and B49-6). Experiments for each clone were performed in triplicate. The inventors observed that the reduction of SETDB1 expression in the gene-amplified cells had cancer growth-inhibitory features **(****Figure 2****).** Upon stable transfection of shRNAs against SETDB1 in the gene-amplified DMS-273 and NCI-H1437 lung cancer cell lines and efficient depletion of the SETDB1 protein **(****Figure 2A****),** the cells proved less viable in the MTT assay **(****Figure 2B** and **2D****)** and had a markedly reduced percentage colony formation density in the assay developed on plastic plate **(****Figures 2C** and **2D****).** Transfection of the scramble short hairpin RNA did not reduce cell viability **(****Figure 2B** and **2D****)** and had no impact on the colony formation assay **(****Figures 2C** and **2D****).** Confocal microscopy experiments in the NCI-H1437 lung cancer cell line confirmed the expected nuclear staining of the SETDB1 protein (DAPI colocalization) and the disappearance of the nuclear signal upon SETDB1 shRNA-mediated depletion (data not shown). SETDB1 knock-down by the shRNA approach in a lung cancer cell line without gene amplification (NCI-H1299) did not cause a significant effect in cell growth determined by the MTT experiments or the colony formation assay **(****Figure 2E****),** suggesting the SETDB1 dependence for cell growth only in the amplified cancer cells. The inventors next tested the ability of SETDB1 shRNA-transfected DMS-273 and NCI-H1437 cells to form tumors in nude mice compared with scramble shRNA-transfected cells **(****Figure 2F****).** DMS-273 and NCI-H1437 scramble shRNA-transfected cells formed tumors rapidly, but cells with shRNA-mediated depletion of SETDB1 had much lower tumorigenicity **(****Figure 2F****).**

The inventors also studied the oncogenic potential of SETDB 1 by evaluating its ability to enhance cell invasion in a lung carcinoma cell line without SETDB1 gene amplification (A549) **(****Figure 3A****).** To this end, the inventors transfected the plasmids driving the expression of SETDB1 and performed a matrigel invasion assay. A significant increase in the number of invasive cells upon SETDB1 transfection in comparison with empty vector-transfected cells was observed **(****Figure 3A****).**

The inventors also wondered about gene targets in the amplified lung cancer cells whose expression could be regulated by SETDB1 mediated H3-K9 promoter methylation and that could further explain the above observed impact in cell growth and invasiveness. To find downstream targets of SETDB1 in the amplified lung cancer cell lines DMS-273 and NCI-H1437, the inventors developed expression microarray analyses (Agilent G4851B 60K) of both SETDB1 shRNA depleted cell lines in comparison to their corresponding shRNA-scrambled control cell lines. The microarray expression data obtained are freely available at the Gene Expression Omnibus database: http://www.ncbi.nlm.nih.gov/geo/query/acc.cgi?token=tdexdacygiayejy&acc=GSE4517 5. Using this approach, the inventors identified eighteen common genes repressed in both SETDB1 amplified lung cancer cell lines that become upregulated at least 3-fold upon SETDB1 shRNA-mediated downregulation (Table 2; Figure 4B).

**Table 2. Common upregulated genes in both SETDB1-depleted lung cancer cell lines (DSM-273 and NCI-H1437)**

| **Gene** | **Description** |
|---|---|
| ***ADARB2*** | Homo sapiens adenosine deaminase, RNA-specific, B2 |
| ***ADRB2*** | Homo sapiens adrenergic, beta-2-, receptor, surface |
| ***ANXA3*** | Homo sapiens annexin A3 |
| ***C8orf31*** | Homo sapiens chromosome 8 open reading frame 31 |
| ***DNER*** | Homo sapiens delta/notch-like EGF repeat containing |
| ***FGFR2*** | fibroblast growth factor receptor 2 |
| ***GPR64*** | Homo sapiens G protein-coupled receptor 64 |
| ***IGFBP7*** | Homo sapiens insulin-like growth factor binding protein 7 |
| ***IL11*** | Homo sapiens interleukin 11 |
| ***IL32*** | Homo sapiens interleukin 32 |
| ***IL6*** | Homo sapiens interleukin 6 (interferon, beta 2) |
| ***LOC100506718*** | PREDICTED: Homo sapiens hypothetical |
| | LOC100506718 |
| ***NAV3*** | Homo sapiens neuron navigator 3 |
| ***PLAG1*** | Homo sapiens pleiomorphic adenoma gene 1 |
| ***SGK1*** | Homo sapiens serum/glucocorticoid regulated kinase 1 |
| ***SPANXA1*** | Homo sapiens sperm protein associated with the nucleus, X-linked, family member A1 |
| ***TFAP2A*** | Homo sapiens transcription factor AP-2 alpha (activating enhancer binding protein 2 alpha) |
| ***TFPI2*** | Homo sapiens tissue factor pathway inhibitor 2 |

The inventors also confirmed the expression changes of the candidate genes by quantitative RT-PCR **(****Figure 4C****)** and the shift in H3-K9 methylation status in their respective promoters by quantitative chromatin immunoprecitation (qChIP) using anti-H3K9me3, anti-H3 and anti-rabbit IgG antibodies **(****Figure 4D****).** The experiments depicted a reduction of H3K9me3 in DMS-273 and NCI-H1437 cells stably transfected with shRNAs against SETDB1. Related to function, gene ontology (GO) analysis of these genes determined "Regulation of cell proliferation" as the most significantly enriched biological process (FDR = 0.0006). Representative examples included the tumor suppressor roles of Delta/Notch-like epidermal growth factor-related receptor (DNER) and insulin-like growth factor binding protein 7 (IGFBP7).

The observation that the presence of the SETDB1 gene amplification with associated overexpression was critical for the tumorigenesis of these lung cancer cells prompted the inventors to examine whether drugs targeting this pathway might find a therapeutic "niche" for their use in the described subset of cases with extra copies of this gene. No highly specific inhibitor for SETDB1 has been described in the publicly available literature, but one agent-mithramycin- is a clinically approved anti-tumor antibiotic that binds to DNA by interacting with the minor groove and displacing transcriptional activators that bind to GC-rich binding sites. Thus, the inventors tested whether a putative growth-inhibitory effect of this drug in lung cancer cells was dependent on SETDB1 expression. First, western blot analyses for the SETDB1 protein in the SETDB1 amplified lung cancer cell lines DMS-273, NCI-H1437 and NCI-H1395 upon the use of the drug were developed. The inventors found that mithramycin was able to inhibit SETDB1 expression in the three cell lines **(****Figure 5****).** Using the small lung cancer cell line DMS-273, harboring the previously identified SETDB1 gene amplification, in comparison with three stable short hairpin SETDB1-depleted clones, we observed that the scramble shRNA DMS-273 cells were significantly more sensitive to the growth-inhibitory effect mediated by mithramycin than any of the depleted clones (A30, A31 and B32-63) **(****Figure 3B** **and** **5****).** Experiments for each clone were performed in triplicate. Furthermore, we extended the cell viability experiments, using the MTT assay, to the other two SETDB1 amplified lung cancer cell lines (NCI-H1437 and NCI-H1395) and to three SETDB1 non-amplified lung cancer cell lines (DMS-114, A549 and NCI-H1299). The determination of the corresponding EC50 values further confirmed that SETDB1 amplified cell lines (EC50=13.6 nM for NCI-H1437 and EC50=14.7 nM for NCI-H1395) are more sensitive to the drug than the non-amplified cell lines (EC50=347.5 nM for DMS-114, EC50=122 nM for A549 and EC50=32 nM for NCI-H1299) (ANOVA, p<0.001). Thus, the presence of SETDB1 gene amplification could "mark" lung cancer cells that are more sensitive to the inhibition of cell viability associated with the use of mithramycin.

Finally, the inventors demonstrated that the presence of SETDB1 gene amplification was not a specific feature of *in vitro* grown lung cancer cell lines and that it also occurred in primary tumors of lung cancer patients. FISH analysis for the SETDB1 locus were performed using a collection of 59 primary lung tumors, corresponding to 40 non-small cell lung tumors (20 squamous cell carcinomas and 20 adenocarcinomas) and 19 small cell carcinomas (data not shown). The UCSC genome browser (http://genome.ucsc.edu) was used to select the BAC clone RP11-42A12 spanning the 1q21 region of the SETDB1 gene. The telomeric BAC clone RP11-60J11 located in the telomeric 1p36.23 region was used as a control. The inventors identified SETDB1 gene amplification in nine tumors corresponding to 20% (4 of 20), 20% (4 of 20) and 5% (1 of 19) of adenocarcinoma, squamous and small cell lung cancer cases, respectively. Most importantly, the inventors also demonstrated that the presence of extra copies of SETDB1 in these nine primary tumors was associated with overexpression of the SETDB1 protein as determined by immunohistochemistry in all cases (HPA018142, Sigma-Aldrich, St. Louis, MO, USA) (data not shown). Among the remaining fifty unamplified cases, eight (14%) also showed enhanced SETDB1 expression that could be associated with other upstream regulatory events. Fisher's test, two-tailed P value < 0.0001.

## Claims

1. A SETDB1 antagonist for use in the treatment of lung cancer in a subject having SETDB1 gene amplification.

2. A SETDB1 antagonist for use according to claim 1 wherein the antagonist is selected from the group consisting of:
(i) a SETDB1-specific RNAi, and
(ii) mithramycin or a pharmaceutically acceptable salt, solvate, polymorph or cocrystal thereof.

3. An *in vitro* method for predicting the clinical response of a subject suffering from lung cancer to a treatment with a SETDB 1 antagonist comprising detecting whether the SETDB1 gene is amplified in a sample from said subject, wherein amplification of the SETDB1 gene is indicative of a good clinical response of the subject to the treatment with said antagonist.

4. The method according to claim 3 wherein the SETDB1 antagonist is selected from the group consisting of:
(i) a SETDB1-specific RNAi, and
(ii) mithramycin or a pharmaceutically acceptable salt, solvate, polymorph or cocrystal thereof.

5. The method according to any of claims 3 or 4 wherein detecting the amplification of the SETDB1 gene comprises determining the copy number of the SETDB1 gene or detecting decreased expression levels of one or more genes selected from the group consisting of ADRB2, ANXA3, DNER, FGFR2, GPR64, IGFBP7, IL6, NAV3, SGK1 and TFAP2A with respect to a reference value.

6. The method according to claim 5 wherein the determination of the copy number of the SETDB1 gene is carried out by *in situ* hybridisation.

7. The method according to claims 5 wherein the determination of the copy number of the SETDB1 gene is carried out by quantitative genomic PCR.

8. An *in vitro* method for selecting a lung cancer patient for being treated with a SETDB antagonist comprising detecting whether the SETDB gene is amplified in a sample from said patient, wherein amplification of the SETDB1 gene is indicative that the subject is a candidate for a treatment with a SETDB1 1 antagonist.

9. The method according to claim 8 wherein the SETDB1 antagonist is selected from the group consisting of:
(i) a SETDB1-specific RNAi, and
(ii) mithramycin or a pharmaceutically acceptable salt, solvate, polymorph or cocrystal thereof.

10. The method according to any of claims 8 or 9 wherein detecting the amplification of the SETDB1 gene comprises determining the copy number of the SETDB1 gene or detecting decreased expression levels of one or more genes selected from the group consisting of ADRB2, ANXA3, DNER, FGFR2, GPR64, IGFBP7, IL6, NAV3, SGK1 and TFAP2A with respect to a reference value.

11. The method according to claim 10 wherein the determination of the copy number of the SETDB1 gene is carried out by *in situ* hybridisation.

12. The method according to claim 10 wherein the determination of the copy number of the SETDB1 gene is carried out by quantitative genomic PCR.

13. Use of a reagent capable of detecting the amplification of SETDB1 gene in a sample from a subject suffering from lung cancer for predicting the clinical response of said subject to a treatment with a SETDB1 antagonist or for selecting a lung cancer patient for being treated with a SETDB1 antagonist.

14. The use according to claim 13 wherein the SETDB1 antagonist is selected from the group consisting of:
(i) a SETDB1-specific RNAi, and
(ii) mithramycin or a pharmaceutically acceptable salt, solvate, polymorph or cocrystal thereof.

15. The use according to any of claims 13 or 14 wherein the reagent capable of detecting the amplification of SETDB1 gene is a polynucleotide comprising the human genomic fragment present in the RP11-42A12 BAC (SEQ ID NO: 1) or a variant thereof which hybridizes under stringent conditions to said polynucleotide.
